# EUROPEAN PATENT SPECIFICATION

(11) **EP 2 456 435 B1**
(45) Date of publication and mention of the grant of the patent: **24.06.2015**
(21) Application number: 10802849.9
(22) Date of filing: 21.07.2010
(51) Int. Cl.: A61K 31/33, C07D 213/75, C07D 213/89, C07D 215/38, C07D 231/40, C07D 239/42, C07D 241/20, C07D 277/46, C07D 401/10, C07D 413/10, C07D 417/10

(54) **HETEROARYL BENZAMIDES, COMPOSITIONS AND METHODS OF USE**
HETEROARYLBENZAMIDE, ZUSAMMENSETZUNGEN DARAUS UND VERFAHREN ZU IHRER VERWENDUNG
HÉTÉROARYL BENZAMIDES, COMPOSITIONS ET PROCÉDÉS D'UTILISATION

(30) Priority: 13.04.2010 US 323681 P; 21.07.2009 US 227213 P
(43) Date of publication of application: 30.05.2012
(73) Proprietor: The Board of Trustees of the Leland Stanford Junior University, Stanford, CA 94305-2038 (US); Auckland UniServices Limited, Auckland (NZ)
(72) Inventor: SUTPHIN, Patrick, Boston MA 02114 (US); CHAN, Denise, San Francisco CA 94117 (US); TURCOTTE, Sandra, Quebec J6A 3T6 (CA); DENNY, William, Alexander, Pakuranga Auckland (NZ); HAY, Michael, Patrick, Mt. Eden Auckland 1024 (NZ); GIDDENS, Anna, Claire, St Pameli Auckland (NZ); BONNET, Muriel, Mt. Eden Auckland 1024 (NZ); GIACCIA, Amato, Stanford CA 94305 (US)
(74) Representative: Sexton, Jane Helen
(86) International application number: PCT/US2010/042742
(87) International publication number: WO 2011/011514

(56) References cited:
- WO-A1-03/000267
- WO-A1-03/099773
- WO-A1-2005/042489
- WO-A2-2005/004810
- WO-A2-2008/008374
- US-A1- 2007 072 862
- US-A1- 2008 058 302
- US-A1- 2008 076 762
- US-A1- 2008 161 345
- US-A1- 2008 293 711
- US-B1- 6 316 450
- US-B1- 6 458 845
- US-B2- 7 312 234
- DATABASE PUBCHEM COMPOUND [Online] 29 February 2008 XP008151815 Retrieved from NCBI Database accession no. 24538156

<110> The Board of Trustees of the Leland Stanford University Auckland UniServices Limited
<120> HETEROARYL BENZAMIDES, COMPOSITIONS AND METHODS OF USE
<130> N115924
<140> EP10802849.9
   <141> 2010-07-21
<150> US 61/227,213
   <151> 2009-07-21
<150> US 61/323,681
   <151> 2010-04-13
<160> 16
<170> PatentIn version 3.5
<210> 1
   <211> 21
   <212> DNA
   <213> artificial
<220>
   <223> synthetic primer
<400> 1
   ggccaagagt gtgctaaaga a 21
<210> 2
   <211> 20
   <212> DNA
   <213> artificial
<220>
   <223> synthetic primer
<400> 2
   acagcgttga tgccagacag 20
<210> 3
   <211> 21
   <212> DNA
   <213> artificial
<220>
   <223> synthetic primer
<400> 3
   gtcactggga ccctggtttt c 21
<210> 4
   <211> 23
   <212> DNA
   <213> artificial
<220>
   <223> synthetic primer
<400> 4
   agttgttgat agcttttcgg tca 23
<210> 5
   <211> 21
   <212> DNA
   <213> artificial
<220>
   <223> synthetic primer
<400> 5
   tggcctatta cttcacggag c 21
<210> 6
   <211> 23
   <212> DNA
   <213> artificial
<220>
   <223> synthetic primer
<400> 6
   ggaatggacc ttacgaatgt tgg 23
<210> 7
   <211> 21
   <212> DNA
   <213> artificial
<220>
   <223> synthetic primer
<400> 7
   tttgaccaca ttgccgaatg c 21
<210> 8
   <211> 21
   <212> DNA
   <213> artificial
<220>
   <223> synthetic primer
<400> 8
   ggtccatgag accaggaaac t 21
<210> 9
   <211> 19
   <212> DNA
   <213> artificial
<220>
   <223> synthetic primer
<400> 9
   catcccccat cctacgtgg 19
<210> 10
   <211> 21
   <212> DNA
   <213> artificial
<220>
   <223> synthetic primer
<400> 10
   ccccataggg tgagaaaacc a 21
<210> 11
   <211> 22
   <212> DNA
   <213> artificial
<220>
   <223> synthetic primer
<400> 11
   ctgtgatacg gatcagaaac cg 22
<210> 12
   <211> 23
   <212> DNA
   <213> artificial
<220>
   <223> synthetic primer
<400> 12
   tccaccaaac aataaagagt gct 23
<210> 13
   <211> 20
   <212> DNA
   <213> artificial
<220>
   <223> synthetic primer
<400> 13
   cctgggcgga gctaaagttg 20
<210> 14
   <211> 23
   <212> DNA
   <213> artificial
<220>
   <223> synthetic primer
<400> 14
   tctcagcttt ggacattagg tct 23
<210> 15
   <211> 23
   <212> DNA
   <213> artificial
<220>
   <223> synthetic primer
<400> 15
   caacatcacc atgcagatta tgc 23
<210> 16
   <211> 22
   <212> DNA
   <213> artificial
<220>
   <223> synthetic primer
<400> 16
   cccacaggga ttttcttgtc tt 22

## Description

This invention was made in part with U.S. Government support under Contract number CA-082566, awarded by the National Cancer Institute. The government may have certain rights in the invention.

Provided herein are certain heteroaryl benzamides, compositions, and methods of their manufacture and use.

Tumor hypoxia has a well defined role in driving tumor progression and metastasis, as well as resistance to therapy. A key mediator of hypoxic stress is HIFα. HIF is a bHLH heterodimeric transcription factor, made up of an oxygen-labile subunit (HIF-α) and a constitutive subunit (HIF-β).

In the presence of oxygen, hydroxylation on proline residues 564 and 402 by prolyl hydroxylases (PHDs) marks HIF-α for recognition and binding with Von Hippel-Lindau protein (pVHL), leading to degradation of HIF-α. Under hypoxic conditions, activity of the PHDs decrease, which prevents the recognition of HIF-α by pVHL. In cells that lack VHL, stabilized HIF-α binds HIF-β to activate the transcription of genes involved in several processes. HIF transcribes genes that mediate glycolysis, angiogenesis, tissue remodelling, epithelial permeability and vascular tone. These genes, and processes driven by these genes, act to promote tumor growth and survival in hypoxic conditions.

Functional studies indicate that pVHL, the protein product of VHL, is an E3 ubiquitin ligase that targets the α-subunit of the hypoxia-inducible factor (HIF) for proteasomal degradation under normoxia. In addition to its role in HIF regulation, pVHL has been implicated in a variety of processes including extracellular matrix assembly, regulation of microtubule stability, polyubiquitination of atypical PKC family members, regulation of fibronectin, and RNA polymerase II subunits.

There is considerable interest in the identification of HIF inhibitors and a variety of pharmacological HIF inhibitors have been identified, although the interaction of these agents is not directly with HIF, but via modulation of cellular processes in which HIF is integral.

An extension of this therapy would be in the treatment of cells defective in the von Hippel-Lindau gene and diseases associated with such defects.

While many solid tumors respond to different combinations of cytotoxic chemotherapies, kidney cancer is a particularly intractable disease. Renal cell carcinoma (RCC), the most common type of kidney cancer, has proven to be particularly challenging, resistant to both radiation therapy and standard systemic chemotherapies. To date, immunotherapy using interferon or interleukin-2 has had mild success with responses in less than 10% of patients with metastatic RCC. The recent development of anti-angiogenic therapies sunitinib (Sutent) and sorafenib (Nexavar) is encouraging although few patients have durable responses and exhibit increased survival. The targeting of receptor tyrosine kinases, which is not specific to the development of RCC, has become the standard of care for advanced RCC.

One key distinguishing feature in RCC is the loss of function of the *VHL* tumor suppressor gene, an essential and frequent mutation. In order to specifically target RCC cells without toxicity to normal cells, a synthetic lethal approach, seeking to identify compounds that exhibit selective cytotoxicity to cells that have lost functional VHL, can be used. The concept of synthetic lethality, or conditional genetics, describes the genetic interaction of two genes, both involved in an essential process. When either gene is mutated alone, the cell remains viable. However, the combination of mutations in these two genes results in cell death. In the case of chemical synthetic lethality, the first mutation is essential to the development of cancer, while a second gene is inhibited by a small molecule, resulting in cytotoxic cell death. This approach is particularly attractive because it should not affect normal, non-cancerous tissue.

WO 03/000267 describes amino nicotinate derivatives as glucokinase (GLK) modulators. US2008/0076762 A1 describes 4-aminopiperidine derivatives that exhibit parasite aspartic protease inhibiting properties. WO 2005/042489 describes N-substituted benzenesulfonamides for use in treating or preventing cognitive disorders. US 7312234 B2 describes chemokine receptor binding heterocyclic compounds. WO 2008/008374 describes CCR2 inhibitors useful for treating inflammation.

Compounds that function in a synthetic lethal manner to the loss of *VHL* and/or selectively target RCC are described herein. Provided is at least one compound of Formula I: 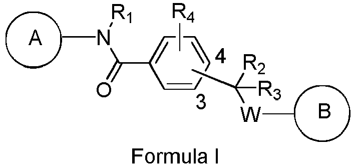 or a pharmaceutically acceptable salt thereof,
wherein:
A is 3-pyridinyl which is optionally substituted; 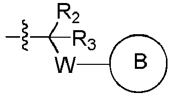 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen or C₁-C₈ alkyl;
W is -NRSO₂-, wherein_R is hydrogen or C₁-C₈ alkyl; and
B is an optionally substituted aryl ring,
provided that if A is 3-pyridinyl, R₁, R₂, and R₃ are each hydrogen, and W is -NHSO₂-, then B is not 3-methoxyphenyl, 3,4-dimethylphenyl, 2,3,4-trifluorophenyl, 2,3,5,6-tetramethylphenyl, 2,5-dimethylphenyl, 3-chlorophenyl, 3-trifluoromethylphenyl, 4-methoxyphenyl, 4-tertbutylphenyl, 4-fluorophenyl, or 4-acetylphenyl;
   wherein "aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic.

Provided is a pharmaceutical composition comprising at least one compound of Formula IA: 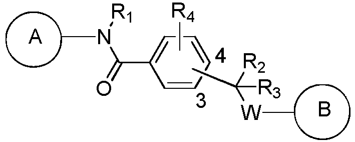 or a pharmaceutically acceptable salt thereof,
wherein:
A is 3-pyridinyl which is optionally substituted; 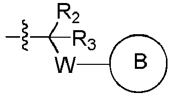 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen or C₁-C₈ alkyl;
W is -NRSO₂-, wherein R is hydrogen or C₁-C₈ alkyl; and
B is an optionally substituted aryl ring,
   wherein "aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic;
and at least one pharmaceutically acceptable carrier.

Methods are described for treating diseases mediated by HIF-1α and/or HIF-2α.

Methods are also described of targeting cells which express HIF-1α and/or HIF-2α.

Also provided are compounds for use in a method for treating a disease mediated by defective pVHL protein.

Methods are also described of targeting cells which have defective pVHL protein.

### Brief Description of the Figures

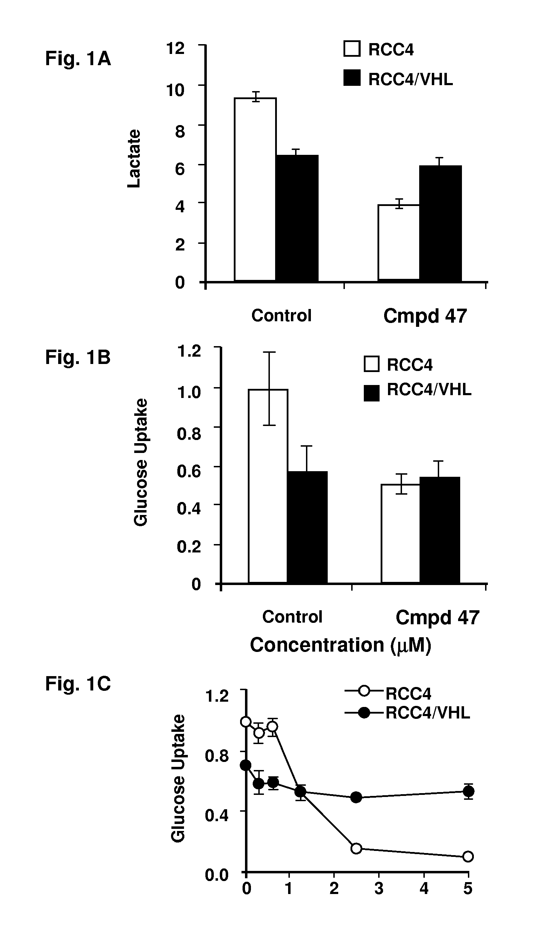

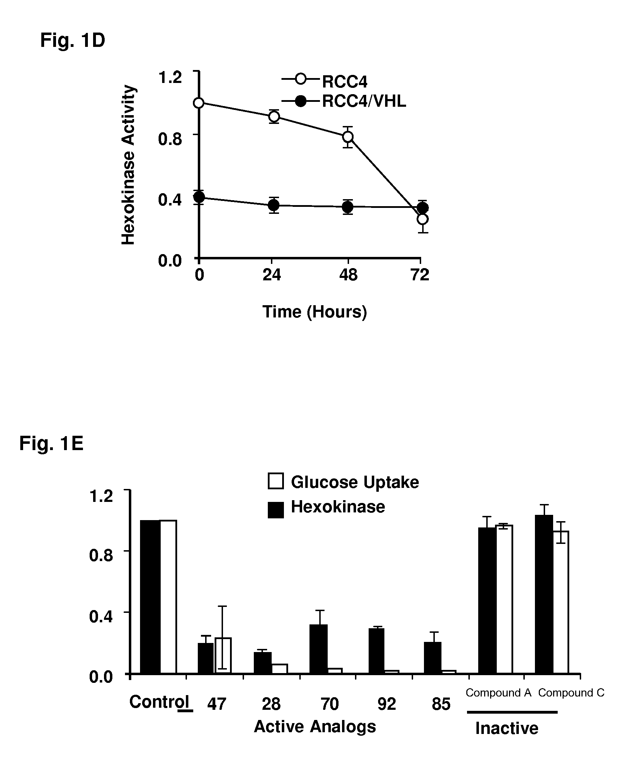

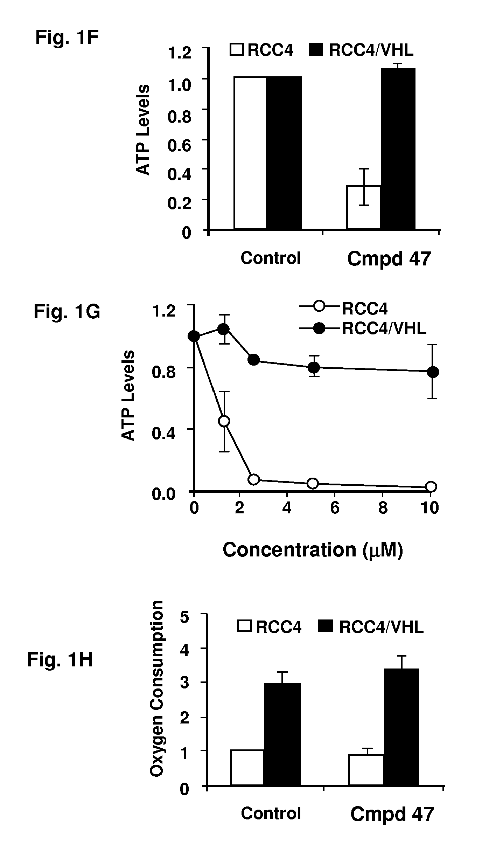

**Fig. 1****. (A)** Compound **47** inhibits glycolysis in VHL-deficient cells. Lactate µM/cell), which is converted from pyruvate, the end product of glycolysis, was measured in RCC4 and RCC4/VHL cells treated with either vehicle or compound **47** (5 µM). **(B)** Relative glucose uptake is inhibited by compound **47** in VHL-deficient cells. Following treatment with compound **47**, cells were starved of glucose for 1 hour and then pulsed for 1 hour with ³H-2-deoxyglucose. **(C)** Inhibition of glucose in cells that have lost VHL is dependent on compound **47** concentration. Cells were treated with the indicated concentration and glucose uptake was measured. **(D)** Relative hexokinase activity is impaired by compound **47** specifically in cells without VHL. Whole cell lysates were examined for hexokinase activity following compound **47** treatment. **(E)** Glucose uptake and hexokinase activity were measured for active and inactive compound **47** analogs. Only active analogs affected both glucose uptake and hexokinase activity in VHL-defective cells. **(F)** Relative ATP levels are decreased in response to compound **47** in cells that have lost VHL. **(G)** Decrease in ATP levels in VHL-deficient cells is dependent on compound **47** concentration. **(H)** Oxygen consumption (nmol/min/10⁶ cells) does not change in response to compound **47**.

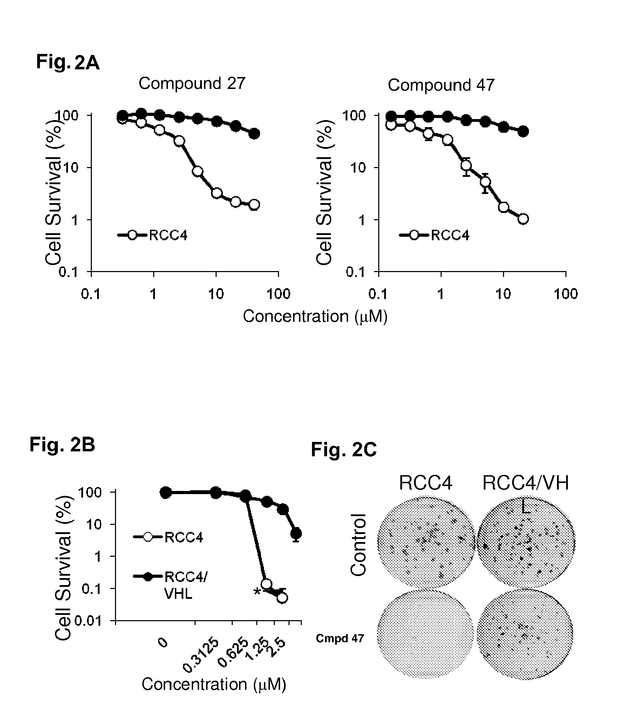

**Figure 2A-G** Chemical synthetic lethal screen identifies compounds that specifically target loss of *VHL* in renal carcinoma. (A) XTT validation of 4-phenylsulfonamido-*N*-(pyridin-3-yl)benzamides (PPBs): Compound **27** and compound **47** were identified from chemical synthetic lethal screen of renal carcinoma cells that have lost *VHL.* (B) Clonogenic survival of RCC4 with and without *VHL* in response to compound **47** (*p<0.00005). (C) Representative plates of clonogenic survival in RCC4 and RCC4/VHL cells. Three hundred cells were treated with 5 µM of compound **47** for 10 days. (D) Compound **47**-induced cell death is irreversible after three days. Cells were treated with compound **47** (5 µM). The media was replaced after the indicated time and cells were allowed to grow for a total of 10 days (*p<0.0005). (E) Clonogenic survival of ACHN with and without shRNA to VHL in response to compound **47** (*p<0.0001). (F) Compound **47** induces a necrotic cell death. RCC4 and RCC4/VHL cells were treated for 3 days with 5 µM of compound **47** and amount of cell death was examined by trypan blue staining (*p<0.01). (G) Compound **47** toxicity is mediated through HIF. RCC4, RCC4/VHL or RCC4/VHL cell clones overexpressing HIF-2α were treated with compound **47** (*p<0.005). All error bars represent the standard error of the mean.

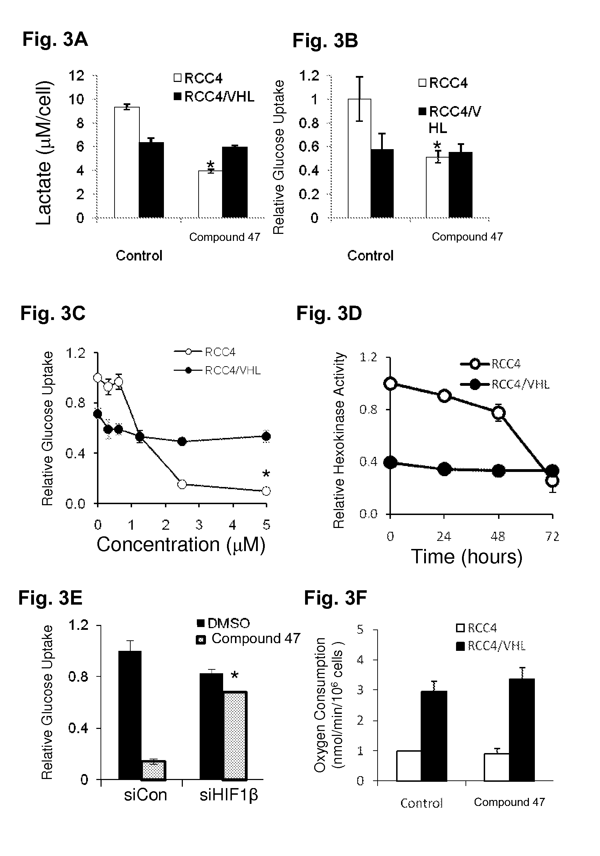

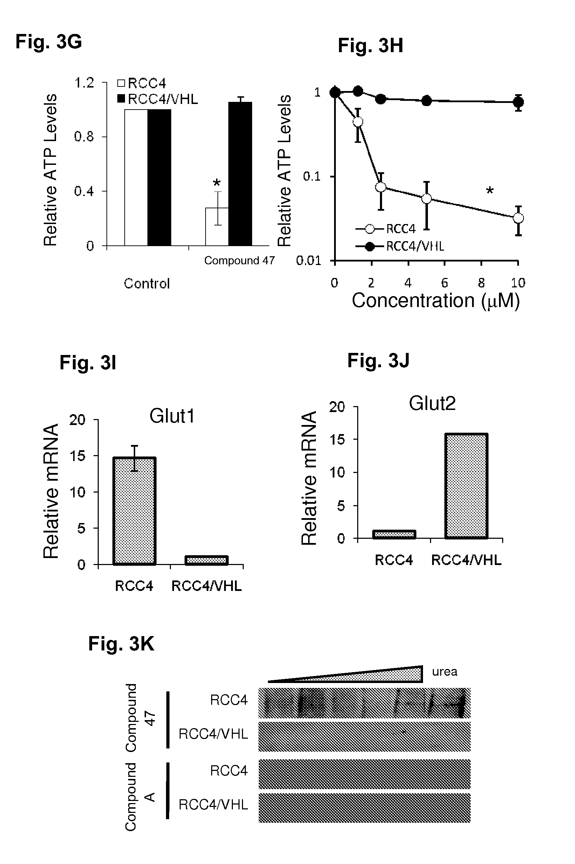

**Figure 3A-K** Compound **47** inhibits glucose metabolism in *VHL*-deficient cells. (A) Compound **47** inhibits glucose uptake and glycolysis in *VHL*-deficient cells. Lactate (mM/cell), which is converted from pyruvate, the end product of glycolysis, was measured in RCC4 and RCC4/VHL cells treated with either vehicle or compound **47** (5 µM)(*p<0.01). (B) Relative glucose uptake is inhibited by compound **47** in *VHL*-deficient cells. Following treatment with compound **47** (5 µM), cells were starved of glucose for 1 hour and then pulsed for 1 hour with ³H-2-deoxyglucose. Counts are normalized to cell number. (C) Inhibition of glucose uptake in cells that have lost *VHL* is dependent on compound **47** concentration. Cells were treated with the indicated concentration and glucose uptake was measured (*p<0.00005). (D) Relative hexokinase activity is impaired by compound **47** specifically in cells without *VHL.* Whole cell lysates were examined for hexokinase activity following compound **47** treatment (5 µM). (E) Compound **47** inhibition of glucose uptake is dependent on HIF. RCC4 cells were transfected with siRNA to HIF-1β, treated with compound **47** (5 µM), and glucose uptake was measured (*p<0.05). (F) Oxygen consumption (nmol/min/10⁶ cells) does not change in response to compound **47** (5 µM). (G) Relative ATP levels are decreased in response to compound **47** (5 µM) in cells that have lost *VHL* (*p<0.005). (H) Decrease in ATP levels in VHL-deficient cells is dependent on compound **47** concentration (*p<0.01). (I) Relative mRNA expression of Glut1 in RCC4 and RCC4/VHL as determined by quantitative real-time PCR and normalized to TBP. (J) Relative mRNA expression of GLUT1 in RCC4 and RCC4/VHL as determined by quantitiative real-time PCR and normalized to TBP. All error bars represent the standard error of the mean. (K) Compound **47** analog, Compound **116**, binds to GLUT1. 4-{2-[1-(6-Aminohexyl)-1H-1,2,3-triazol-4-yl]-4-pyridinyl}-N-(3-methylphenyl)-1,3-thiazol-2-amine does not bind to GLUT1. Cell lysates of RCC4 and RCC4/VHL were incubated with Affi-gel immobilized compound **116** or 4-{2-[1-(6-aminohexyl)-1H-1,2,3-triazol-4-yl]-4-pyridinyl}-N-(3-methylphenyl)-1,3-thiazol-2-amine and eluted with increasing salt concentration. Elutions were probed for GLUT1.

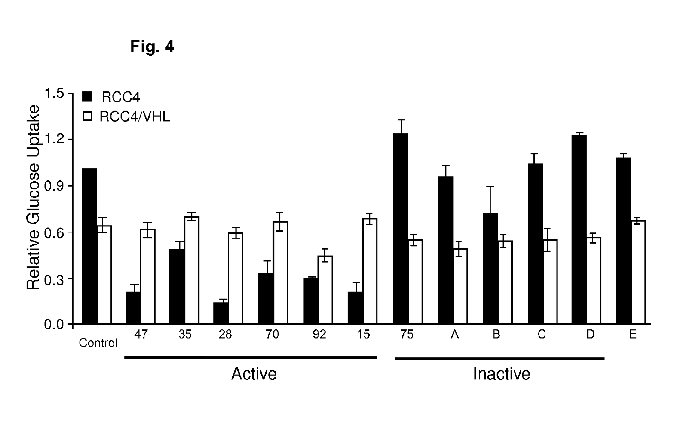

**Figure 4** Glucose uptake and hexokinase activity were measured for active and inactive compound **47** analogs. Only active analogs affected both glucose uptake and hexokinase activity in VHL-defective cells. All error bars represent the standard error of the mean.

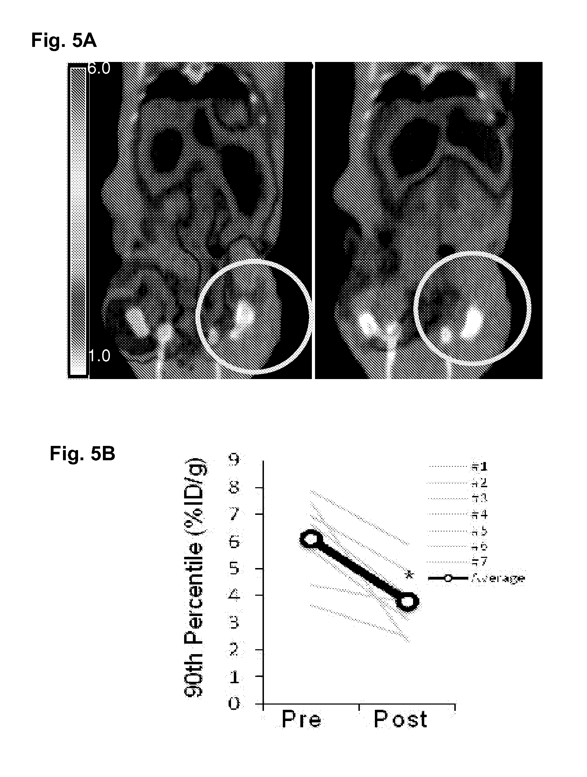

**Figure 5A-E** In vivo monitoring and efficacy of compound **47**. (A) FDG-PET imaging demonstrates an in vivo decrease in glucose uptake in a renal clear cell carcinoma xenograft in response to compound **85**, a more soluble, active analog of compound **47**. 786-O, a renal clear cell carcinoma with a naturally occurring VHL mutation, were implanted subcutaneously into the flanks of CD-1 nude mice. Representative axial cross section of a mouse prior to treatment (left) and following three daily i.p. injections with compound **85** (11.6 mg/kg)(right), overlaid over CT scan. (B) Quantitatively, compound **85** inhibits FDG-PET in mouse xenografts. Quantification of FDG-PET inhibition by compound **85** as determined by the 90^{th} percentile ROI for percent injected dose per gram (%ID/g) (*p<0.01). (C) Compound **85** is not toxic to normal tissues. (a, b) Kidney of vehicle- and compound **85**-treated animals. (c, d) Spleen of vehicle- and compound **85**-treated animals. (e, f) Liver of vehicle- and compound **85**-treated animals. (g, h) Heart of vehicle-and compound **85**-treated animals. (i, j) Salivary gland of vehicle- and compound **85**-treated animals. (k, I) Brain of vehicle- and compound **85**-treated animals. Scale bar represents 100 microns. (D) Compound **85** delays tumor growth. 786-O tumor-bearing mice were treated daily with vehicle or compound **85** (11.6 mg/kg for the first 3 days, followed by 7.8 mg/kg for the next week)(*p<0.005). (E) Compound **85** delays tumor growth in cells that have lost *VHL.* ACHN cells expressing a short hairpin RNA to VHL were implanted subcutaneously into the flanks of immunocompromised mice. Once tumors reached an average of >20 mm³, mice were treated daily with compound **85** or vehicle (*p<0.05). All error bars represent the standard error of the mean.

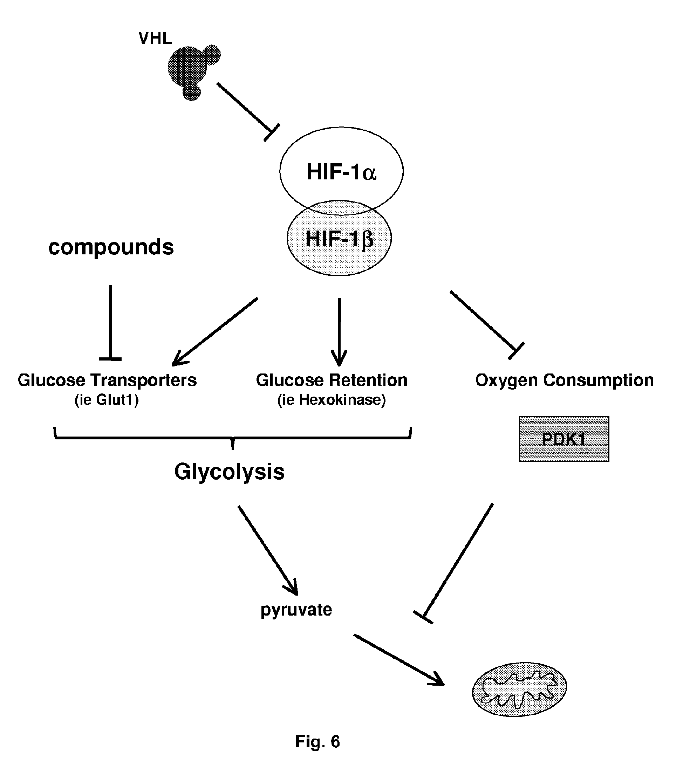

**Figure 6** Model of compound **47** mechanism of synthetic lethality.

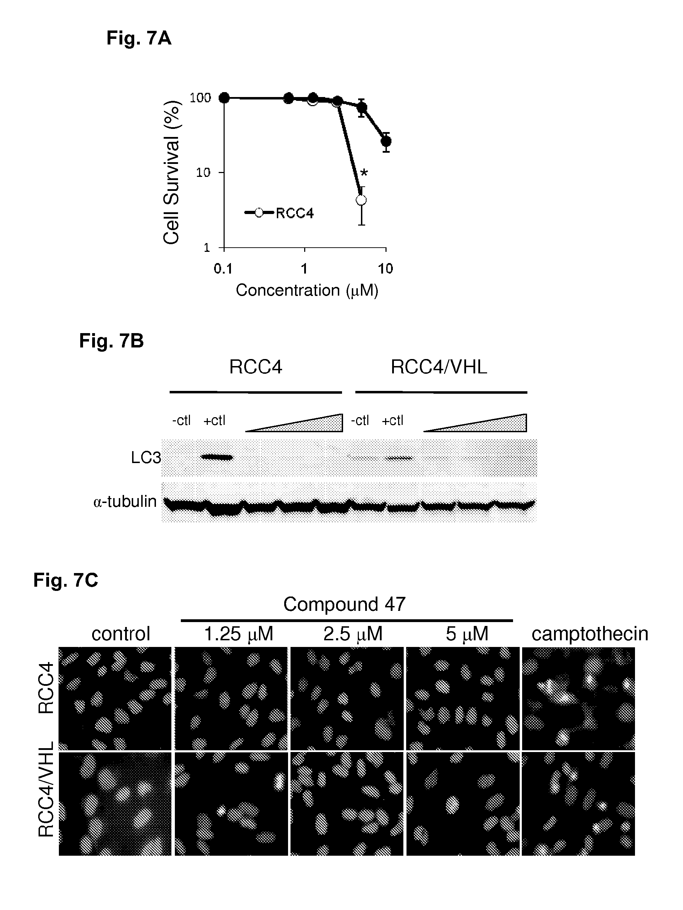

**Figure 7A-E** Compound **47** does not induce autophagy, apoptosis, or DNA damage. (A) Clonogenic survival of RCC4 and RCC4/VHL treated with compound **27** (5 µM)(*p<0.05). All error bars represent the standard error of the mean. (B) Compound **47** does not induce autophagy. RCC4 and RCC4/VHL cells were treated with increasing concentrations of compound **47** (1.25, 2.5 and 5 µM), a negative control (DMSO) and a positive control (4-(pyridin-4-yl)-N-(m-tolyl)thiazol-2-amine). Cells were lysed and probed for LC3, a marker of autophagy, or α-tubulin (loading control). (C) Compound **47** does not induce apoptosis. RCC4 and RCC4/VHL cells were treated with vehicle, increasing concentrations of compound **47**, and camptothecin. Cells were stained with DAPI and nuclear condensation was examined by fluorescence microscopy. (D) RCC4 cells were treated with compound **47** (5 µM) for the indicated time and stained with Annexin V and propidium iodide and subjected to FACS analysis. (E) Compound **47** does not induce DNA damage. RCC4 and RCC4/VHL cells were subjected to increasing concentrations of compound **47** (1.25, 2.5, and 5 µM), a negative control (DMSO), and a positive control (doxorubicin). Cells were lysed and subjected to Western blot with the indicated antibodies.

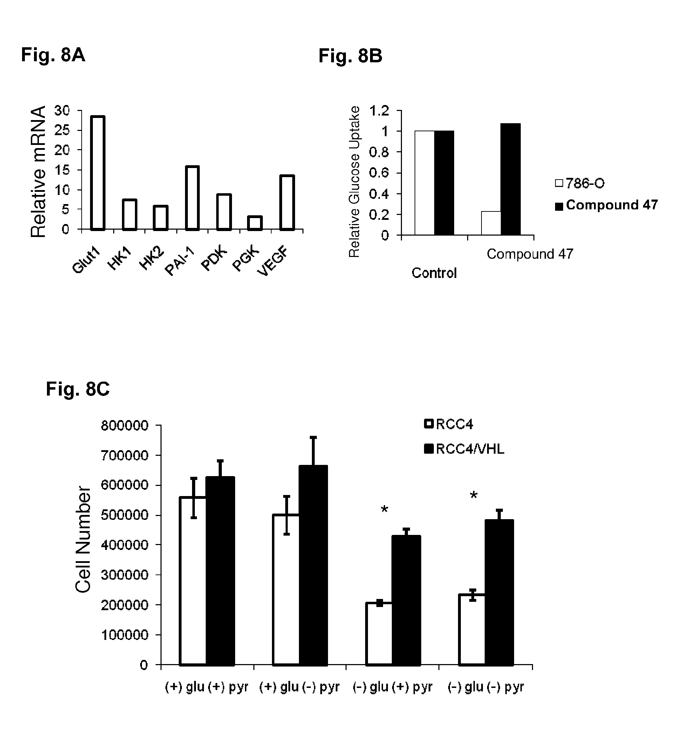

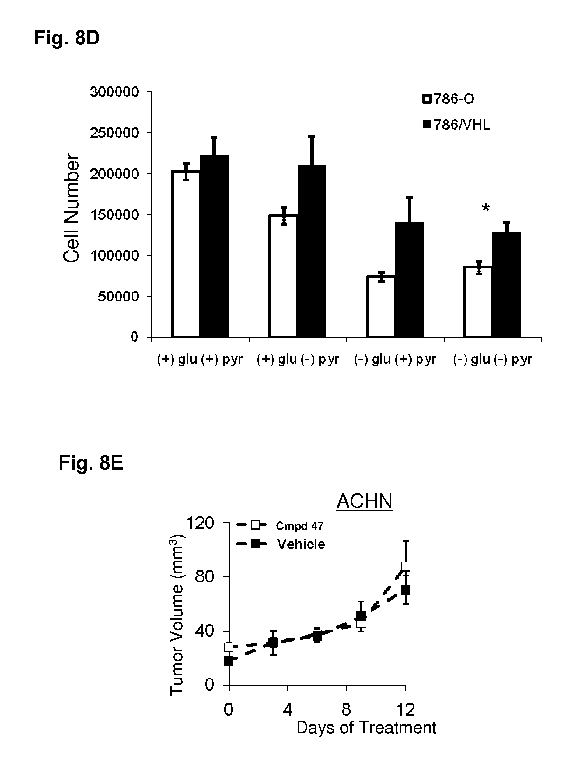

**Figure 8A-E** *VHL*-deficient renal carcinomas are more sensitive to glucose deprivation compared to RCCs with wild-type *VHL*. (A) Relative mRNA expression levels for different genes involved in glucose metabolism in RCC4 cells relative to RCC4/VHL cells. (B) Glucose uptake is impaired by compound **47** (5 µM) in 786-O cells, which are deficient in VHL, but not 786/VHL, which have wild-type VHL restored. (C) VHL mutant RCC4 cells are more sensitive to glucose deprivation than RCC4/VHL. Cells were grown in media lacking glucose and/or pyruvate for 6 days (*p<0.005). (D) *VHL* mutant 786-O cells are more sensitive to glucose deprivation than 786/VHL cells (**p*<0.05). (E) ACHN tumors with wild-type VHL are insensitive to compound **47** treatment (5 µM). ACHN cells were implanted subcutaneously into the flanks of immunocompromised mice. Once tumors reached an average of >20 mm³, mice were treated daily with compound **85** or vehicle. All error bars represent the standard error of the mean.

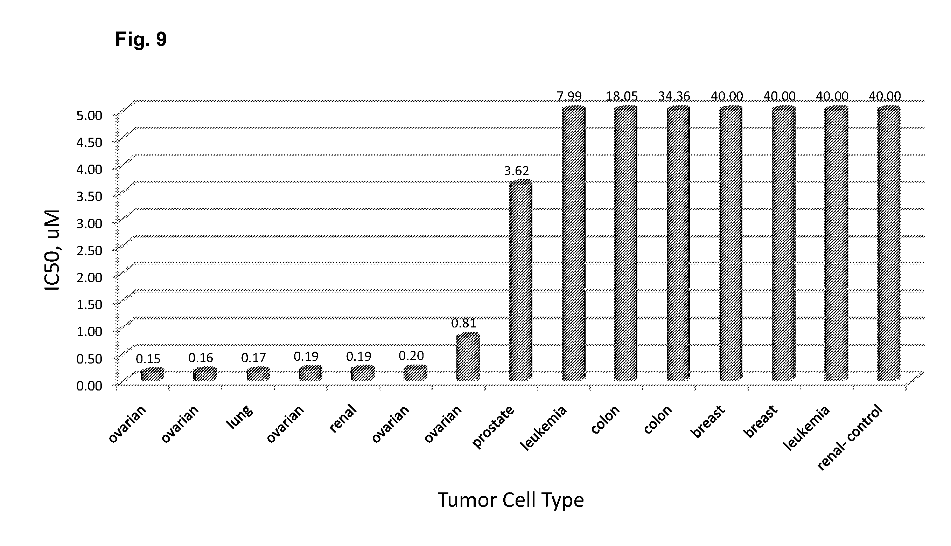

**Figure 9** Sensitivity of cancer cell lines to GIUT1 inhibition.

As used in the present specification, the following words, phrases, and symbols are generally intended to have the meanings set forth below, except to the extent that the context in which they are used indicated otherwise. The following abbreviations and terms have the indicated meanings throughout:

"Subject" refers to an animal, such as a mammal, that has been or will be the object of treatment, observation, or experiment. The compounds and methods described herein may be useful for both human therapy and veterinary applications. In some embodiments, the subject is a human.

As used herein, "treatment" or "treating" refers to an amelioration of a disease or disorder, or at least one discernible symptom thereof. In another embodiment, "treatment" or "treating" refers to an amelioration of at least one measurable physical parameter, not necessarily discernible by the patient. In yet another embodiment, "treatment" or "treating" refers to reducing the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

As used herein, "prevention" or "preventing" refers to a reduction of the risk of acquiring a given disease or disorder.

As used herein, "pharmaceutically acceptable" refers to those compounds, materials, compositions, and/or dosage forms that are, within the scope of sound medical judgment, suitable for use in contact with the tissues of human beings and animals without excessive toxicity, irritation, allergic response, or other problem or complication, commensurate with a reasonable benefit/risk ratio.

As used herein, "parenteral administration" and "administered parenterally" refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

A dash ("-") that is not between two letters or symbols is used to indicate a point of attachment for a substituent. For example, -CONH₂ is attached through the carbon atom.

The term "alkyl" refers to refers to a saturated straight or branched hydrocarbon, such as a straight or branched group of 1-20, 1-8, or 1-6 carbon atoms, referred to herein as C₁-C₂₀ alkyl, C₁-C₈ alkyl, and C₁-C₆ alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, and the like.

The term "alkenyl" refers to an unsaturated straight or branched hydrocarbon having at least one carbon-carbon double bond, such as a straight or branched group of 2-20, 2-8, or 2-6 carbon atoms, referred to herein as (C₂-C₂₀) alkenyl, (C₂-C₈) alkenyl, and (C₂-C₆) alkenyl, respectively. Exemplary alkenyl groups include, but are not limited to, vinyl, allyl, butenyl, pentenyl, hexenyl, butadienyl, pentadienyl, hexadienyl, 2-ethylhexenyl, 2-propyl-2-butenyl, and 4-(2-methyl-3-butene)-pentenyl.

The term "alkynyl" refers to an unsaturated straight or branched hydrocarbon having at least one carbon-carbon triple bond, such as a straight or branched group of 2-20, 2-8, or 2-6 carbon atoms, referred to herein as C₂-C₂₀ alkynyl, C₂-Cₛ alkynyl, and C₂-C₆ alkynyl, respectively. Exemplary alkynyl groups include, but are not limited to, ethynyl, propynyl, butynyl, pentynyl, hexynyl, methylpropynyl, 4-methyl-1-butynyl, 4-propyl-2-pentynyl, and 4-butyl-2-hexynyl.

"Cycloalkyl" refers to a saturated hydrocarbon ring group, having the specified number of carbon atoms, such as, for example from 3 to 7 ring carbon atoms. Examples of cycloalkyl groups include cyclopropyl, cyclobutyl, cyclopentyl, and cyclohexyl as well as bridged and caged saturated ring groups such as, for example, adamantane.

The term "alkoxy" as used herein refers to an alkyl group attached to an oxygen (-O-alkyl-). "Alkoxy" groups also include an alkenyl group attached to an oxygen ("alkenyloxy") or an alkynyl group attached to an oxygen ("alkynyloxy") groups. Exemplary alkoxy groups include, but are not limited to, groups with an alkyl, alkenyl or alkynyl group of 1-20, 1-8, or 1-6 carbon atoms, referred to herein as (C₁-C₂₀) alkoxy, (C₁-C₈) alkoxy, and (C₁-C₆) alkoxy, respectively. Exemplary alkoxy groups include, but are not limited to methoxy, ethoxy, propoxy, isopropoxy, n-butoxy, sec-butoxy, tert-butoxy, pentoxy, 2-pentyloxy, isopentoxy, neopentoxy, hexoxy, 2-hexoxy, 3-hexoxy, 3-methylpentoxy, and the like.

"Acyl" refers to the groups (alkyl)-C(O)-, (cycloalkyl)-C(O)-, (aryl)-C(O)-, (heteroaryl)-C(O)-, and (heterocycloalkyl)-C(O)-, wherein the group is attached to the parent structure through the carbonyl functionality and wherein alkyl, cycloalkyl, aryl, heteroaryl, and heterocycloalkyl are as described herein. Acyl groups have the indicated number of carbon atoms, with the carbon of the keto group being included in the numbered carbon atoms. For example a C₂ acyl group is an acetyl group having the formula CH₃(C=O)-.

"Alkoxycarbonyl" refers to an ester group of the formula (alkoxy)(C=O)-attached through the carbonyl carbon wherein the alkoxy group has the indicated number of carbon atoms. Thus, a C₁-C₆ alkoxycarbonyl group is an alkoxy group having from 1 to 6 carbon atoms attached through its oxygen to a carbonyl linker.

By "amino" is meant the group -NH₂.

"Aryl" encompasses: 5- and 6-membered carbocyclic aromatic rings, for example, benzene; bicyclic ring systems wherein at least one ring is carbocyclic and aromatic, for example, naphthalene, indane, and tetralin; and tricyclic ring systems wherein at least one ring is carbocyclic and aromatic, for example, fluorene.
For example, aryl includes 5- and 6-membered carbocyclic aromatic rings fused to a 5- to 7-membered heterocycloalkyl ring containing 1 or more heteroatoms chosen from N, O, and S. For such fused, bicyclic ring systems wherein only one of the rings is a carbocyclic aromatic ring, the point of attachment may be at the carbocyclic aromatic ring or the heterocycloalkyl ring. Bivalent radicals formed from substituted benzene derivatives and having the free valences at ring atoms are named as substituted phenylene radicals. Bivalent radicals derived from univalent polycyclic hydrocarbon radicals whose names end in "-yl" by removal of one hydrogen atom from the carbon atom with the free valence are named by adding "-idene" to the name of the corresponding univalent radical, e.g., a naphthyl group with two points of attachment is termed naphthylidene. Aryl, however, does not encompass or overlap in any way with heteroaryl, separately defined below. Hence, if one or more carbocyclic aromatic rings is fused with a heterocycloalkyl aromatic ring, the resulting ring system is heteroaryl, not aryl, as defined herein.

The term "aryloxy" refers to the group -O-aryl.

The term "halo" includes fluoro, chloro, bromo, and iodo, and the term "halogen" includes fluorine, chlorine, bromine, and iodine.

"Heteroaryl" encompasses: 5- to 7-membered aromatic, monocyclic rings containing one or more, for example, from 1 to 4, or In some embodiments, from 1 to 3, heteroatoms chosen from N, O, and S, with the remaining ring atoms being carbon; and bicyclic heterocycloalkyl rings containing one or more, for example, from 1 to 4, or In some embodiments, from 1 to 3, heteroatoms chosen from N, O, and S, with the remaining ring atoms being carbon and wherein at least one heteroatom is present in an aromatic ring. For example, heteroaryl includes a 5- to 7-membered heterocycloalkyl, aromatic ring fused to a 5- to 7-membered cycloalkyl ring. For such fused, bicyclic heteroaryl ring systems wherein only one of the rings contains one or more heteroatoms, the point of attachment may be at the heteroaromatic ring or the cycloalkyl ring. When the total number of S and O atoms in the heteroaryl group exceeds 1, those heteroatoms are not adjacent to one another. In some embodiments, the total number of S and O atoms in the heteroaryl group is not more than 2. In some embodiments, the total number of S and O atoms in the aromatic heterocycle is not more than 1. Examples of heteroaryl groups include, but are not limited to, (as numbered from the linkage position assigned priority 1), 2-pyridyl, 3-pyridyl, 4-pyridyl, 2-pyrazinyl, 2-pyrimidinyl, 3-pyrazolinyl, 2-thiazolyl, imidazolinyl, isoxazolinyl, oxazolinyl, thiazolinyl, thiadiazolinyl, tetrazolyl, thienyl, benzothiophenyl, furanyl, benzofuranyl, benzoimidazolinyl, indolinyl, pyridizinyl, triazolyl, quinolinyl, and pyrazolyl. Bivalent radicals derived from univalent heteroaryl radicals whose names end in "-yl" by removal of one hydrogen atom from the atom with the free valence are named by adding "-idene" to the name of the corresponding univalent radical, e.g., a pyridyl group with two points of attachment is a pyridylidene. Heteroaryl does not encompass or overlap with aryl as defined herein. Substituted heteroaryl also includes ring systems substituted with one or more oxide (-O⁻) substituents, such as pyridinyl N-oxides.

"Heterocycloalkyl" refers to a single aliphatic ring, containing at least 2 carbon atoms in addition to 1-3 heteroatoms independently selected from oxygen, sulfur, and nitrogen, as well as combinations comprising at least one of the foregoing heteroatoms. Suitable heterocycloalkyl groups include, for example (as numbered from the linkage position assigned priority 1), 2-pyrrolinyl, 2,4-imidazolidinyl, 2,3-pyrazolidinyl, 2-piperidyl, 3-piperidyl, 4-piperdyl, and 2,5-piperzinyl. Morpholinyl groups are also contemplated, including 2-morpholinyl and 3-morpholinyl (numbered wherein the oxygen is assigned priority 1). Substituted heterocycloalkyl also includes ring systems substituted with one or more oxo moieties, such as piperidinyl N-oxide, morpholinyl-N-oxide, 1-oxo-1-thiomorpholinyl and 1,1-dioxo-1-thiomorpholinyl.

The term "cyano" as used herein refers to -CN.

The term "carboxy" as used herein refers to -COOH or its corresponding carboxylate salts (e.g., -COONa). The term carboxy also includes "carboxycarbonyl," for example, a carboxy group attached to a carbonyl group, for example, -C(O)-COOH or salts, such as -C(O)-COONa.

The term "nitro" refers to -NO₂.

The term "hydroxy" and "hydroxyl" refer to -OH.

The term "sulfinyl" includes the groups: -S(O)-H, -S(O)-(optionally substituted (C₁-C₆)alkyl), -S(O)-optionally substituted aryl), -S(O)-optionally substituted heteroaryl), -S(O)-(optionally substituted heterocycloalkyl); and -S(O)-(optionally substituted amino).

The term "sulfonyl" includes the groups: -S(O₂)-H, -S(O₂)-(optionally substituted (C₁-C₆)alkyl), -S(O₂)-optionally substituted aryl), -S(O₂)-optionally substituted heteroaryl), -S(O₂)-(optionally substituted heterocycloalkyl), -S(O₂)-(optionally substituted alkoxy), -S(O₂)-optionally substituted aryloxy), -S(O₂)-optionally substituted heteroaryloxy), -S(O₂)-(optionally substituted heterocyclyloxy); and -S(O₂)-(optionally substituted amino).

By "optional" or "optionally" is meant that the subsequently described event or circumstance may or may not occur, and that the description includes instances where the event or circumstance occurs and instances in which it does not. For example, "optionally substituted alkyl" encompasses both "alkyl" and "substituted alkyl" as defined below. It will be understood by those skilled in the art, with respect to any group containing one or more substituents, that such groups are not intended to introduce any substitution or substitution patterns that are sterically impractical, synthetically non-feasible and/or inherently unstable.

The term "substituted", as used herein, means that any one or more hydrogens on the designated atom or group is replaced with a selection from the indicated group, provided that the designated atom's normal valence is not exceeded. When a substituent is oxo (i.e., =O) then 2 hydrogens on the atom are replaced. Combinations of substituents and/or variables are permissible only if such combinations result in stable compounds or useful synthetic intermediates. A stable compound or stable structure is meant to imply a compound that is sufficiently robust to survive isolation from a reaction mixture, and subsequent formulation as an agent having at least practical utility. Unless otherwise specified, substituents are named into the core structure. For example, it is to be understood that when (cycloalkyl)alkyl is listed as a possible substituent, the point of attachment of this substituent to the core structure is in the alkyl portion.

The terms "substituted" alkyl, alkenyl, cycloalkyl, aryl, heterocycloalkyl, and heteroaryl (including "substituted" pyridinyl, pyrimidinyl, pyrazinyl, quinolinyl, pyrazolyl, and thiazolyl"), unless otherwise expressly defined, refer respectively to alkyl, alkenyl, cycloalkyl, aryl, heterocycloalkyl, and heteroaryl wherein one or more (such as up to 5, for example, up to 3) hydrogen atoms are replaced by a substituent independently chosen from:
-R^{a}, -OR^{b}, -O(C₁-C₂ alkyl)O- (e.g., methylenedioxy-), -SR^{b}, guanidine, guanidine wherein one or more of the guanidine hydrogens are replaced with a lower-alkyl group, -NR^{b}R^{c}, halo, cyano, oxo (as a substituent for heterocycloalkyl), nitro, -COR^{b}, -CO₂R^{b}, -CONR^{b}R^{c}, -OCOR^{b}, -OCO₂R^{a}, -OCONR^{b}R^{c}, -NR^{c}COR^{b}, -NR^{c}CO₂R^{a}, -NR^{c}CONR^{b}R^{c}, -SOR^{a}, -SO₂R^{a}, -SO₂NR^{b}R^{c}, and -NR^{c}SO₂R^{a},
where R^{a} is chosen from optionally substituted C₁-C₆ alkyl, optionally substituted C₂-C₆ alkenyl, optionally substituted cycloalkyl, optionally substituted aryl, optionally substituted heterocycloalkyl, and optionally substituted heteroaryl;
R^{b} is chosen from hydrogen, optionally substituted C₁-C₆ alkyl, optionally substituted cycloalkyl, optionally substituted aryl, optionally substituted heterocycloalkyl, and optionally substituted heteroaryl; and
R^{c} is chosen from hydrogen and optionally substituted C₁-C₄ alkyl; or
R^{b} and R^{c}, and the nitrogen to which they are attached, form an optionally substituted heterocycloalkyl group; and
where each optionally substituted group is unsubstituted or independently substituted with one or more, such as one, two, or three, substituents independently selected from C₁-C₄ alkyl, aryl, heteroaryl, aryl-C₁-C₄ alkyl-, heteroaryl-C₁-C₄ alkyl-, C₁-C₄ haloalkyl-, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo (as a substitutent for heteroaryl), -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ phenyl, -C(O)C₁-C₄ haloalkyl, -OC(O)C₁-C₄ alkyl, - SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl).

In some embodiments, the terms "substituted" alkyl, alkenyl, cycloalkyl, aryl, heterocycloalkyl, and heteroaryl (including "substituted" pyridinyl, pyrimidinyl, pyrazinyl, quinolinyl, pyrazolyl, and thiazolyl"), unless otherwise expressly defined, refer respectively to alkyl, alkenyl, cycloalkyl, aryl, heterocycloalkyl, and heteroaryl wherein one or more (such as up to 5, for example, up to 3) hydrogen atoms are replaced by a substituent independently chosen from: -R^{a}, -OR^{b}, -COR^{b}, -CO₂R^{b}, NO₂, -NR^{b}R^{c}, -NR^{c}COR^{b}, -NR^{c}CO₂R^{a}, -NR^{c}CONR^{b}R^{c}, -NR^{c}SO₂R^{a} and CN, where R^{a}, R^{b}, and R^{c} are as described herein.

The term "substituted acyl" refers to the groups (substituted alkyl)-C(O)-, (substituted cycloalkyl)-C(O)-, (substituted aryl)-C(O)-, (substituted heteroaryl)-C(O)-, and (substituted heterocycloalkyl)-C(O)-, wherein substituted alkyl, substituted cycloalkyl, substituted aryl, substituted heteroaryl, and substituted heterocycloalkyl are as described herein. In some embodiments, the term "substituted acyl" refers to the groups (substituted alkyl)-C(O)-, (substituted aryl)-C(O)-, and (substituted heteroaryl)-C(O)-, wherein substituted alkyl, substituted aryl, and substituted heteroaryl are as described herein.

The term "substituted alkoxycarbonyl" refers to the group (substituted alkyl)-O-C(O)- wherein the group is attached to the parent structure through the carbonyl functionality and wherein "substituted alkyl" is as described herein.

The term "substituted cycloalkyloxy" refers to cycloalkyloxy wherein the cycloalkyl constituent is substituted (i.e., -O-(substituted cycloalkyl)) wherein "substituted cycloalkyl" is as described herein.

The term "substituted amino" refers to the group -NR^{b}R^{c}, -NR^{c}COR^{b}, -NR^{c}CO₂R^{a}, -NR^{c}CONR^{b}R^{c}, and -NR^{c}SO₂R^{a}, wherein R^{b} and R^{c} are as described herein. The term "substituted amino" also refers to N-oxides of the groups -NHR^{d}, and NR^{d}R^{d} each as described herein. N-oxides can be prepared by treatment of the corresponding amino group with, for example, hydrogen peroxide or m-chloroperoxybenzoic acid. The person skilled in the art is familiar with reaction conditions for carrying out the N-oxidation.

The term "substituted aryloxy" refers to aryloxy wherein the aryl constituent is substituted (i.e., -O-(substituted aryl)) wherein "substituted aryl" is as described herein.

Compounds described herein include, but are not limited to, any stereoisomer, tautomer, rotomer, deuterated analogues, and/or pharmaceutically acceptable salt as defined herein.

The compounds described herein can be asymmetric (e.g., having one or more stereocenters). All stereoisomers, such as enantiomers and diastereomers, are intended unless otherwise indicated.

Compounds that contain asymmetrically substituted carbon atoms can be isolated in optically active or racemic forms. Methods on how to prepare optically active forms from optically active starting materials are known in the art, such as by resolution of racemic mixtures or by stereoselective synthesis. The processes described herein can be stereoselective such that any given reaction starting with one or more chiral reagents enriched in one stereoisomer forms a product that is also enriched in one stereoisomer. The reaction can be conducted such that the product of the reaction substantially retains one or more chiral centers present in the starting materials. The reaction can also be conducted such that the product of the reaction contains a chiral center that is substantially inverted relative to a corresponding chiral center present in the starting materials.

Resolution of racemic mixtures of compounds can be carried out by any of numerous methods known in the art. An example method includes fractional crystallization using a "chiral resolving acid" which is an optically active, salt-forming organic acid. Suitable resolving agents for fractional recrystallization methods are, for example, optically active acids, such as the D and L forms of tartaric acid, diacetyltartaric -acid, dibenzoyltartaric acid, mandelic acid, malic acid, lactic acid or the various optically active camphorsulfonic acids such as ß-camphorsulfonic acid. Resolution of racemic mixtures can also be carried out by elution on a column packed with an optically active resolving agent (e.g., dinitrobenzoylphenylglycine). Suitable elution solvent composition can be determined by one skilled in the art.

Compounds as described herein can also include all isotopes of atoms occurring in the intermediates or final compounds. Isotopes include those atoms having the same atomic number but different mass numbers. For example, isotopes of hydrogen include tritium and deuterium.

The compounds disclosed herein can be used in different enriched isotopic forms, e.g., enriched in the content of ²H, ³H, ¹¹C, ¹³C ¹⁴C and or ¹⁸F. In one particular embodiment, the compounds are deuterated. Such deuterated forms can be made by the procedure described in U.S. Patent Nos. 5,846,514 and 6,334,997. As described in U.S. Patent Nos. 5,846,514 and 6,334,997, deuteration can improve the efficacy and increase the duration of action of drugs.

Deuterium substituted compounds can be synthesized using various methods such as described in: Dean, Dennis C.; Editor. Recent Advances in the Synthesis and Applications of Radiolabeled Compounds for Drug Discovery and Development. [In: Curr., Pharm. Des., 2000; 6(10)] 2000, 110 pp.; Kabalka, George W. and Varma, Rajender S. The Synthesis of Radiolabeled Compounds via Organometallic Intermediates, Tetrahedron, 1989, 45(21), 6601-21, Evans, E. Anthony. Synthesis of radiolabeled compounds, J. Radioanal. Chem., 1981, 64(1-2), 9-32.

Compounds as described herein can also include tautomeric forms, such as keto-enol tautomers. Tautomeric forms can be in equilibrium or sterically locked into one form by appropriate substitution.

Compounds as described herein also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form," "polymorph," and "novel form" may be used interchangeably herein, and are meant to include all crystalline and amorphous forms of the compound, including, for example, polymorphs, pseudopolymorphs, solvates, hydrates, unsolvated polymorphs (including anhydrates), conformational polymorphs, and amorphous forms, as well as mixtures thereof, unless a particular crystalline or amorphous form is referred to. Compounds as described herein also include pharmaceutically acceptable forms of the recited compounds, including chelates, non-covalent complexes, and mixtures thereof.

A "solvate" is formed by the interaction of a solvent and a compound. The term "compound" is intended to include solvates of compounds. Similarly, "salts" includes solvates of salts. Similarly, "salts" includes solvates of salts. Suitable solvates are pharmaceutically acceptable solvates, such as hydrates, including monohydrates and hemi-hydrates.

A "chelate" is formed by the coordination of a compound to a metal ion at two (or more) points. The term "compound" is intended to include chelates of compounds. Similarly, "salts" includes chelates of salts.

A "non-covalent complex" is formed by the interaction of a compound and another molecule wherein a covalent bond is not formed between the compound and the molecule. For example, complexation can occur through van der Waals interactions, hydrogen bonding, and electrostatic interactions (also called ionic bonding). Such non-covalent complexes are included in the term "compound'.

Compound **47** refers to 4-((4-*tert*-butylphenylsulfonamido)methyl)-*N*-(pyridin-3-yl)benzamide, a preparation of which is shown in Example 38. Compound **85** refers to 4-((4-(4-methylpiperazin-1-yl)phenylsulfonamido)methyl)-*N*-(pyridin-3-yl)benzamide, a preparation of which is shown in Example 76. Compound **116** refers to 4-[({[4-(21-amino-4,7,10,13,16,19-hexaoxahenicos-1-yn-1-yl)phenyl]sulfonyl}amino)methyl]-*N*-(3-pyridinyl)benzamide, a preparation of which is shown in Example 100.

Provided is at least one compound of Formula **I**: 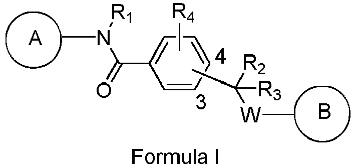 or a pharmaceutically acceptable salt thereof,
wherein:
A is 3-pyridinyl which is optionally substituted; 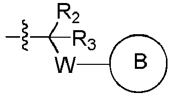 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen or C₁-C₈ alkyl;
W is -NRSO₂-, wherein_R is hydrogen or C₁-C₈ alkyl; and
B is an optionally substituted aryl ring,
provided that if A is R₁, R₂, and R₃ are each hydrogen, and W is -NHSO₂-, then B is not 3-methoxyphenyl, 3,4-dimethylphenyl, 2,3,4-trifluorophenyl, 2,3,5,6-tetramethylphenyl, 2,5-dimethylphenyl, 3-chlorophenyl, 3-trifluoromethylphenyl, 4-methoxyphenyl, 4-tertbutylphenyl, 4-fluorophenyl, or 4-acetylphenyl;
   wherein "aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic.

In some embodiments, R₁ is chosen from hydrogen and optionally substituted alkyl. In some embodiments, R₁ is chosen from hydrogen and lower alkyl. In some embodiments, R₁ is hydrogen or methyl. In some embodiments, R₁ is hydrogen.

In some embodiments, R₂ and R₃ are each independently chosen from hydrogen and optionally substituted alkyl. In some embodiments, R₂ is hydrogen.

In some embodiments, R₃ is chosen from hydrogen and lower alkyl. In some embodiments, R₃ is hydrogen.

In some embodiments, R₄ is chosen from hydrogen, hydroxy, lower alkyl, lower alkoxy, halo, carboxy, and nitro. In some embodiments, R₄ is chosen from hydrogen, methyl, halo, and nitro. In some embodiments, R₄ is chosen from hydrogen and lower alkyl. In some embodiments, R₄ is hydrogen.

In some embodiments, R is chosen from hydrogen and lower alkyl. In some embodiments, R is hydrogen.

In some embodiments, B is an optionally substituted phenyl ring.

In some embodiments, B is phenyl optionally substituted with one or more groups chosen from halo, alkyl, alkenyl, alkynyl, cycloalkyl, aryl, heteroaryl, heterocycloalkyl, hydroxyl, alkoxy, aryloxy, acyl, carboxy, alkoxycarbonyl, NO₂, optionally substituted amino, and CN, wherein each of said alkyl, alkenyl, alkynyl, alkoxy, cycloalkyl, aryl, heteroaryl, heterocycloalkyl, alkoxy, and aryloxy groups may be optionally independently substituted with one or more groups chosen from halo, alkyl, hydroxyl, alkoxy, carboxy, alkoxycarbonyl, heterocycloalkyl, and optionally substituted amino.

In some embodiments, B is phenyl optionally substituted with one or more groups chosen from optionally substituted amino, halo, and lower alkyl optionally substituted with optionally substituted amino, heterocycloalkyl, alkoxy, or hydroxyl.

In some embodiments, B is phenyl optionally substituted with one or more groups chosen from halo, optionally substituted amino and lower alkyl optionally substituted with optionally substituted amino or heterocycloalkyl.

In some embodiments, B is chosen from phenyl, 2-methylphenyl, 2-fluorophenyl, 2-chlorophenyl, 2-bromophenyl, 2-methoxycarbonylphenyl, 2-trifluoromethylphenyl, 2-cyanophenyl, 3-aminophenyl, 3-methoxyphenyl, 3-methylphenyl, 3-fluorophenyl, 3-chlorophenyl, 3-bromophenyl, 3-trifluoromethylphenyl, *tert*-butylphenyl, 4-ethynylphenyl, 3-cyanophenyl, 3-nitrophenyl, 3-phenylphenyl, 3-(2-pyrimidinyl)phenyl, 3-(1-methyl-1*H*-pyrazol-3-yl)phenyl, 3-(5-methyl-1,3,4-oxadiazol-2-yl)phenyl, 3-(5-methyl-1,2,4-oxadiazol-2-yl)phenyl, 3-(2-methyl-1,3-thiazol-4-yl)phenyl, 4-aminophenyl, 4-methoxyphenyl, 4-butoxyphenyl, 4-phenoxyphenyl, 4-methylphenyl, 4-propylphenyl, 4-tert-butylphenyl, 4-(1-adamantyl)phenyl, 4-(3-chloro-1-adamantyl)phenyl, 4-methoxycarbonylethylphenyl, 4-acetamidophenyl, 4-fluorophenyl, 4-chlorophenyl, 4-bromophenyl, 4-iodophenyl, 4-trifluoromethoxyphenyl, 4-methoxycarbonylphenyl, 4-acetylphenyl, 4-trifluoromethylphenyl, 4-cyanophenyl, 4-nitrophenyl, 4'-methoxy[1,1'-biphenyl]-4-yl, 4'-methyl[1,1'-biphenyl]-4-yl, 4-phenylphenyl, 4'-fluoro[1,1'-biphenyl]-4-yl, 4'-chloro[1,1'-biphenyl]-4-yl, 4-(2-pyrimidinyl)phenyl, 4-(1H-pyrazol-1-yl)phenyl, 4-(2-methyl-1,3-thiazol-4-yl)phenyl, 4-(1,3-oxazol-5-yl)phenyl, 3,4-dimethoxyphenyl, 3-tert-butyl-4-methoxyphenyl, 2,3,4,5,6-pentamethylphenyl, 2,4-dimethylphenyl, 3,4-dimethylphenyl, 3,5-dimethylphenyl, 3-fluoro-4-methylphenyl, 3-chloro-2-methylphenyl, 3-chloro-4-methylphenyl, 3,4-dichlorophenyl, 3-cyano-4-fluorophenyl, 2-naphthalenyl, 5-(dimethylamino)-2-naphthalenyl, 2,3-dihydro-5-indeneyl, 2-(dimethylamino)-2,3-dihydro-5-indeneyl, 4-(4-methylpiperazin-1-yl)phenyl, 4-(dimethylamino)methylphenyl, 4-(diethylamino)methylphenyl, 4-(dipropylamino)methylphenyl, 4-(1-pyrrolidinylmethyl)phenyl, 4-(1-piperidinylmethyl)phenyl, 4-(1-azepanylmethyl)phenyl, 4-(4-morpholinylmethyl)phenyl, 4-(4-methoxy-1-piperidinyl)methylphenyl, 4-(4-methyl-1-piperazinyl)methylphenyl, 4-(3-hydroxypropyl)phenyl, 3-morpholinophenyl, 4-morpholinophenyl, 4-(1-piperidinyl)phenyl, (4-methoxy-1-piperidinyl)phenyl, (21-amino-4,7,10,13,16,19-hexaoxahenicos-1-yl)phenyl, {[3-(4-morpholinyl)propyl]amino}phenyl, 3-(4-methyl-1-piperazinyl)phenyl, 4-{[2-(dimethylamino)ethyl]amino}phenyl, 3'-(trifluoromethyl)[1,1'-biphenyl], 4-benzylphenyl, 4-[3-(4-morpholinyl)-1-propynyl]phenyl, 4-[3-(dimethylamino)-1-propynyl]phenyl, 4-[3-(4-morpholinyl)propyl]phenyl, 4-[3-(dimethylamino)propyl]phenyl, 3-(propionylamino)phenyl, and 3-(acryloylamino)phenyl.

In some embodiments, B is chosen from phenyl, 2-methylphenyl, 2-fluorophenyl, 2-chlorophenyl, 2-bromophenyl, 2-methoxycarbonylphenyl, 2-trifluoromethylphenyl, 2-cyanophenyl, 3-aminophenyl, 3-methoxyphenyl, 3-methylphenyl, 3-fluorophenyl, 3-chlorophenyl, 3-bromophenyl, 3-trifluoromethylphenyl, *tert*-butylphenyl, 4-ethynylphenyl, 3-cyanophenyl, 3-nitrophenyl, 3-phenylphenyl, 3-(2-pyrimidinyl)phenyl, 3-(1-methyl-1*H*-pyrazol-3-yl)phenyl, 3-(5-methyl-1,3,4-oxadiazol-2-yl)phenyl, 3-(5-methyl-1,2,4-oxadiazol-2-yl)phenyl, 3-(2-methyl-1,3-thiazol-4-yl)phenyl, 4-aminophenyl, 4-methoxyphenyl, 4-butoxyphenyl, 4-phenoxyphenyl, 4-methylphenyl, 4-propylphenyl, 4-tert-butylphenyl, 4-(1-adamantyl)phenyl, 4-(3-chloro-1-adamantyl)phenyl, 4-methoxycarbonylethylphenyl, 4-acetamidophenyl, 4-fluorophenyl, 4-chlorophenyl, 4-bromophenyl, 4-iodophenyl, 4-trifluoromethoxyphenyl, 4-methoxycarbonylphenyl, 4-acetylphenyl, 4-trifluoromethylphenyl, 4-cyanophenyl, 4-nitrophenyl, 4'-methoxy[1,1'-biphenyl]-4-yl, 4'-methyl[1,1'-biphenyl]-4-yl, 4-phenylphenyl, 4'-fluoro[1,1'biphenyl]-4-yl, 4'-chloro[1,1'biphenyl]-4-yl, 4-(2-pyrimidinyl)phenyl, 4-(1*H*-pyrazol-1-yl)phenyl, 4-(2-methyl-1,3-thiazol-4-yl)phenyl, 4-(1,3-oxazol-5-yl)phenyl, 3,4-dimethoxyphenyl, 3-tert-butyl-4-methoxyphenyl, 2,3,4,5,6-pentamethylphenyl, 2,4-dimethylphenyl, 3,4-dimethylphenyl, 3,5-dimethylphenyl, 3-fluoro-4-methylphenyl, 3-chloro-2-methylphenyl, 3-chloro-4-methylphenyl, 3,4-dichlorophenyl, 3-cyano-4-fluorophenyl, 2-naphthalenyl, 5-(dimethylamino)-2-naphthalenyl, 2,3-dihydro-5-indeneyl, 2-(dimethylamino)-2,3-dihydro-5-indeneyl, 4-(4-methylpiperazin-1 -yl)phenyl, 4-(dimethylamino)methylphenyl, 4-(diethylamino)methylphenyl, 4-(dipropylamino)methylphenyl, 4-(1-pyrrolidinylmethyl)phenyl, 4-(1-piperidinylmethyl)phenyl, 4-(1-azepanylmethyl)phenyl, 4-(4-morpholinylmethyl)phenyl, 4-(4-methoxy-1-piperidinyl)methylphenyl, and 4-(4-methyl-1-piperazinyl)methylphenyl.

In some embodiments, B is chosen from 3-fluorophenyl, 3-chlorophenyl, 3-bromophenyl, 3-(2-pyrimidinyl)phenyl, 3-(1-methyl-1*H*-pyrazol-3-yl)phenyl, 3-(5-methyl-1,3,4-oxadiazol-2-yl)phenyl, 3-(5-methyl-1,2,4-oxadiazol-2-yl)phenyl, 4-butoxyphenyl4-tert-butylphenyl, 4-(2-pyrimidinyl)phenyl, 3,4-dimethoxyphenyl, 3-tert-butyl-4-methoxyphenyl, 3,4-dimethylphenyl, 3,5-dimethylphenyl, 3-fluoro-4-methylphenyl, 3-chloro-4-methylphenyl, 2-(dimethylamino)-2,3-dihydro-5-indeneyl, 4-(4-methylpiperazin-1-yl)phenyl, 4-(dimethylamino)methylphenyl, 4-(diethylamino)methylphenyl, 4-(dipropylamino)methylphenyl, 4-(1-pyrrolidinylmethyl)phenyl, 4-(1-piperidinylmethyl)phenyl, 4-(1-azepanylmethyl)phenyl, 4-(4-morpholinylmethyl)phenyl, 4-(4-methoxy-1-piperidinyl)methylphenyl, 4-(4-methyl-1-piperazinyl)methylphenyl, and 4-(3-hydroxypropyl)phenyl.

In some embodiments, the radical 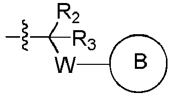 is attached to the phenyl ring at the 3 position. In some embodiments, the radical 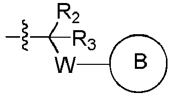 is attached to the phenyl ring at the 4 position.

Also provided is at least one compound chosen from:
4-(Phenylsulfonamidomethyl)-*N*-(pyridin-3-yl)benzamide;
4-((2-Methylphenylsulfonamido)methyl)-*N*-(pyridin-3-yl)benzamide;
4-((2-Fluorophenylsulfonamido)methyl)-*N*-(pyridin-3-yl)benzamide;
4-((2-Chlorophenylsulfbnamido)methyl)-*N*-(pyridin-3-yl)benzamide;
4-((2-Bromophenylsulfonamido)methyl)-*N*-(pyridin-3-yl)benzamide;
Methyl 2-(*N*-(4-(Pyridin-3-ylcarbamoyl)benzyl)sulfamoyl)benzoate;
*N*-(Pyridin-3-yl)-4-((2-(trifluoromethyl)phenylsulfonamido)methyl)benzamide;
4-((2-Cyanophenylsulfonamido)methyl)-*N*-(pyridin-3-yl)benzamide;
4-((3-Aminophenylsulfonamido)methyl)-*N*-(pyridin-3-yl)benzamide;
4-((3-Methylphenylsulfonamido)methyl)-*N*-(pyridin-3-yl)benzamide;
4-((3-Fluorophenylsulfonamido)methyl)-*N*-(pyridin-3-yl)benzamide;
4-((3-Bromophenylsulfonamido)methyl)-*N*-(pyridin-3-yl)benzamide;
4-((3-Cyanophenylsulfonamido)methyl)-*N*-(pyridin-3-yl)benzamide;
4-((3-Nitrophenylsulfonamido)methyl)-*N*-(pyridin-3-yl)benzamide;
4-{[([1,1'-Biphenyl]-3-ylsulfonyl)amino]methyl}-*N*-(3-pyridinyl)benzamide;
4-[({[3-(2-Pyrimidinyl)phenyl]sulfonyl}amino)methyl]-*N*-(3-pyridinyl)benzamide;
4-[({[3-(1-Methyl-1*H*-pyrazol-3-yl)phenyl]sulfonyl}amino)methyl]-*N*-(3-pyridinyl)benzamide;
4-[({[3-(5-Methyl-1,3,4-oxadiazol-2-yl)phenyl]sulfonyl}amino)methyl]-*N*-(3-pyridinyl)benzamide;
4-[({[3-(5-Methyl-1,2,4-oxadiazol-3-yl)phenyl]sulfonyl}amino)methyl]-*N*-(3-pyridinyl)benzamide;
4-[({[3-(2-Methyl-1,3-thiazol-4-yl)phenyl]sulfonyl}amino)methyl]-*N*-(3-pyridinyl)benzamide;
4-((4-Aminophenylsulfonamido)methyl)-*N*-(pyridin-3-yl)benzamide;
4-((4-Butoxyphenylsulfonamido)methyl)-*N*-(pyridin-3-yl)benzamide;
4-((4-Phenoxyphenylsulfonamido)methyl)-*N*-(pyridin-3-yl)benzamide;
4-((4-Methylphenylsulfonamido)methyl)-*N*-(pyridin-3-yl)benzamide;
4-((4-Propylphenylsulfonamido)methyl)-*N*-(pyridin-3-yl)benzamide;
4-[({[4-(1-Adamantyl)phenyl]sulfonyl}amino)methyl]-*N*-(3-pyridinyl)benzamide;
4-[({[4-(3-Chloro-1-adamantyl)phenyl]sulfonyl}amino)methyl]-*N*-(3-pyridinyl)benzamide;
Methyl 3-{4-[({4-[(3 Pyridinylamino)carbonyl]benzyl}amino)sulfonyl]phenyl} propanoate;
4-((4-Acetamidophenylsulfonamido)methyl)-*N*-(pyridin-3-yl)benzamide;
4-((4-Chlorophenylsulfonamido)methyl)-*N*-(pyridin-3-yl)benzamide;
4-((4-Bromophenylsulfonamido)methyl)-*N*-(pyridin-3-yl)benzamide;
*N*-(Pyridin-3-yl)-4-((4-(trifluoromethoxy)phenylsulfonamido)methyl)benzamide;
Methyl 4-(*N*-(4-(Pyridin-3-ylcarbamoyl)benzyl)sulfamoyl)benzoate;
*N*-(Pyridin-3-yl)-4-((4-(trifluoromethyl)phenylsulfonamido)methyl)benzamide;
4-((4-Cyanophenylsulfonamido)methyl)-*N*-(pyridin-3-yl)benzamide;
4-((4-Nitrophenylsulfonamido)methyl)-*N*-(pyridin-3-yl)benzamide;
4-((Biphenyl-4-ylsulfonamido)methyl)-*N*-(pyridin-3-yl)benzamide;
4-({[(4'-Methoxy[1,1'-biphenyl]-4-yl)sulfonyl]amino}methyl)-*N*-(3-pyridinyl)benzamide;
4-({[(4'-Methyl[1,1'-biphenyl]-4-yl)sulfonyl]amino}methyl)-*N*-(3-pyridinyl)benzamide;
4-({[(4'-Fluoro[1,1'-biphenyl]-4-yl)sulfonyl]amino}methyl)-*N*-(3-pyridinyl)benzamide;
4-({[(4'-Chloro[1,1'-biphenyl]-4-yl)sulfonyl]amino}methyl)-*N*-(3-pyridinyl)benzamide;
4-[({[4-(2-Pyrimidinyl)phenyl]sulfonyl}amino)methyl]-*N*-(3-pyridinyl)-benzamide;
4-[({[4-(1H-Pyrazol-1-yl)phenyl]sulfonyl}amino)methyl]-*N*-(3-pyridinyl)benzamide;
4-[({[4-(2-Methyl-1,3-thiazol-4-yl)phenyl]sulfonyl}amino)methyl]-*N*-(3-pyridinyl)benzamide;
[({[4-(1,3-Oxazol-5-yl)phenyl]sulfonyl}amino)methyl]-*N*-(3-pyridinyl)benzamide;
4-((3,4-Dimethoxyphenylsulfonamido)methyl)-*N*-(pyridin-3-yl)benzamide;
4-((3-*tert*-Butyl-4-methoxyphenylsulfonamido)methyl)-*N*-(pyridin-3-yl)benzamide;
4-((2,3,4,5,6-Pentamethylphenylsulfonamido)methyl)-*N*-(pyridin-3-yl)benzamide;
4-((2,4-Dimethylphenylsulfonamido)methyl)-*N*-(pyridin-3-yl)benzamide;
4-((3,4-Dimethylphenylsulfonamido)methyl)-*N*-(pyridin-3-yl)benzamide;
4-((3,5-Dimethylphenylsulfonamido)methyl)-*N*-(pyridin-3-yl)benzamide;
4-((3-Fluoro-4-methylphenylsulfonamido)methyl)-*N* (pyridin-3-yl)benzamide;
4-((3-Chloro-2-methylphenylsulfonamido)methyl)-*N*-(pyridin-3-yl)benzamide;
4-((3-Chloro-4-methylphenylsulfonamido)methyl)-*N*-(pyridin-3-yl)benzamide;
4-((3,4-Dichlorophenylsulfonamido)methyl)-*N*-(pyridin-3-yl)benzamide;
4-((3-Cyano-4-fluorophenylsulfonamido)methyl)-*N*-(pyridin-3-yl)benzamide;
4-((Naphthalene-2-sulfonamido)methyl)-*N*-(pyridin-3-yl)benzamide;
4-((5-(Dimethylamino)naphthalene-1-sulfonamido)methyl)-N-(pyridin-3-yl)benzamide;
4-((2,3-Dihydro-1*H*-indene-5-sulfonamido)methyl)-*N*-(pyridin-3-yl)benzamide;
4-((2-(Dimethylamino)-2,3-dihydro-1*H*-indene-5-sulfonamido)methyl)-*N*-(pyridin-3-yl)benzamide;
4-((4-(4-Methylpiperazin-1-yl)phenylsulfonamido)methyl)-*N*-(pyridin-3-yl)benzamide;
4-[({4-[(Dimethylamino)methyl]phenyl}sulfonyl)amino]methyl-*N*-(3-pyridinyl)benzamide;
4-{[({4-[(Diethylamino)methyl]phenyl}sulfonyl)amino]methyl}-*N*-(3-pyridinyl)benzamide,
4-{[({4-[(Dipropylamino)methyl]phenyl}sulfonyl)amino]methyl}-*N*-(3-pyridinyl)benzamide;
4-[({[4-(1-Pyrrolidinylmethyl)phenyl]sulfonyl}amino)methyl]-*N*-(3-pyridinyl)-benzamide;
4-[({[4-(1-Piperidinylmethyl)phenyl]sulfonyl}amino)methyl]-*N*-(3-pyridinyl)benzamide;
4-[({[4-(1-Azepanylmethyl)phenyl]sulfonyl}amino)methyl]-*N*-(3-pyridinyl)benzamide;
4-[({[4-(4-Morpholinylmethyl)phenyl]sulfonyl}amino)methyl]-*N*-(3-pyridinyl)benzamide;
4-{[({4-[(4-Methoxy-1-piperidinyl)methyl]phenyl}sulfonyl)amino]methyl}-*N*-(3-pyridinyl)benzamide;
4-{[({4-[(4-Methyl-1-piperazinyl)methyl]phenyl}sulfonyl)amino]methyl}-*N*-(3-pyridinyl)benzamide;
4-*tert*-Butyl-*N*-(4-(pyridin-3-ylcarbamoyl)benzyl)benzamide;
4-((4-*tert*-Butylphenylsulfonamido)methyl)-N-methyl-N-(pyridin-3-yl)benzamide;
N-Methyl-4-(phenylsulfonamidomethyl)-N-(pyridin-3-yl)benzamide;
3-((4-*tert*-Butylphenylsulfonamido)methyl)-N-(pyridin-3-yl)benzamide;
3-(Phenylsulfonamidomethyl)-N-(pyridin-3-yl)benzamide;
3-(4-(Phenylsulfonamidomethyl)benzamido)pyridine 1-oxide;
4-((4-lodophenylsulfonamido)methyl)-N-(pyridin-3-yl)benzamide;
4-((4-Ethynylphenylsulfonamido)methyl)-N-(pyridin-3-yl)benzamide;
4-((4-Bromophenylsulfonamido)methyl)-N-(pyridin-3-yl)benzamide;
4-((4-Fluorophenylsulfonamido)methyl)-N-(pyridin-3-yl)benzamide;
3,5-Dimethyl-*N*-(4-(pyridin-3-ylcarbamoyl)benzyl)benzamide;
3,4-Dimethoxy-*N*-(4-(pyridin-3-ylcarbamoyl)benzyl)benzamide;
4-{[({4-[3-(Methyloxy)-1-propynyl]phenyl}sulfonyl)amino]methyl}-*N*-(3-pyridinyl)benzamide;
4-[({[4-(21-Amino-4,7,10,13,16,19-hexaoxahenicos-1-yn-1-yl)phenyl]sulfonyl}amino)methyl]-*N*-(3-pyridinyl)benzamide;
4-[({[4-(3-Mcthoxypropyl)phenyl]sulfonyl}amino)methyl]-N-(3-pyridinyl)benzamide;
4-[({[4-(1-benzyl-1*H*-1,2,3-triazol-4-yl)phenyl]sulfonyl}amino)methyl]-*N*-(3-pyridinyl)benzamide;
4-[({[4-(3-Hydroxy-1-propynyl)phenyl]sulfonyl}amino)methyl]-*N*-(3-pyridinyl)benzamide;
4-[({[4-(3-Hydroxypropyl)phenyl]sulfonyl}amino)methyl]-*N*-(3-pyridinyl)benzamide;
4-({[(4-*tert*-Butylphenyl)sulfonyl]amino}methyl)-*N*-(5-methyl-3-pyridinyl)benzamide;
4-({[(4-*tert*-Butylphenyl)sulfonyl]amino}methyl)-*N*-(2-methyl-3-pyridinyl)benzamide;
4-({[(4-*tert*-Butylphenyl)sulfonyl]amino}methyl)-*N*-(6-methyl-3-pyridinyl)benzamide;
4-({[(4-*tert*-Butylphenyl)sulfonyl]amino}methyl)-*N*-(6-methoxy-3-pyridinyl)benzamide;
4-({[(4-*tert*-Butylphenyl)sulfonyl]amino}methyl)-*N*-(6-chloro-3-pyridinyl)benzamide;
4-({[(4-*tert*-Butylphenyl)sulfonyl]amino}methyl)-*N*-(4-chloro-3-pyridinyl)benzamide;
4-({[(4-*tert*-Butylphenyl)sulfonyl]amino}methyl)-*N*-(2-chloro-3-pyridinyl)benzamide;
4-({[(4-*tert*-Butylphenyl)sulfonyl]amino}methyl)-*N*-(4-methyl-3-pyridinyl)benzamide;
4-({[(4-*tert*-Butylphenyl)sulfonyl]amino}methyl)-*N*-(5-chloro-3-pyridinyl)benzamide;
4-({[(4-*tert*-Butylphenyl)sulfonyl]amino}methyl)-*N*-(2-nitro-3-pyridinyl)benzamide;
4-({[(4-*tert*-Butylphenyl)sulfonyl]amino}methyl)-*N*-[6-(4-morpholinyl)-3-pyridinyl]benzamide;
4-({[(4-*tert*-Butylphenyl)sulfonyl]amino}methyl)-*N*-[6-(trifluoromethyl)-3-pyridinyl]benzamide;
*N*-[6-(Acetylamino)-3-pyridinyl]-4-({[(4-*tert*-butylphenyl)sulfonyl]amino}methyl)benzamide;
4-({[(4-*tert*-Butylphenyl)sulfonyl]amino}methyl)-*N*-(6-fluoro-3-pyridinyl)benzamide;
4-({[(4-*tert*-Butylphenyl)sulfonyl]amino}methyl)-*N*-(5-fluoro-3-pyridinyl)benzamide;
4-({[(4-*tert*-Butylphenyl)sulfonyl]amino}methyl)-*N*-[4-(trifluoromethyl)-3-pyridinyl]benzamide;
4-({[(4-*tert*-Butylphenyl)sulfonyl]amino}methyl)-*N*-(2-fluoro-3-pyridinyl)benzamide;
4-({[(4-*tert*-Butylphenyl)sulfonyl]amino}methyl)-*N*-(4-methoxy-3-pyridinyl)benzamide;
*N*-(6-Bromo-3-pyridinyl)-4-({[(4-*tert*-butylphenyl)sulfonyl]amino}methyl)benzamide;
4-[({[3-(4-Morpholinyl)phenyl]sulfonyl}amino)methyl]-*N*-(3-pyridinyl)benzamide;
4-[({[4-(4-Morpholinyl)phenyl]sulfonyl}amino)methyl]-*N*-(3-pyridinyl)benzamide;
4-[({[4-(1-Piperidinyl)phenyl]sulfonyl}amino)methyl]-*N*-(3-pyridinyl)benzamide;
4-[({[4-(1-Piperidinyl)phenyl]sulfonyl}amino)methyl]-*N*-(3-pyridinyl)benzamide;
4-[({[4-(21-Amino-4,7,10,13,16,19-hexaoxahenicos-1-yl)phenyl]sulfonyl}amino) methyl]-*N*-(3-pyridinyl)benzamide;
4-({[(4-{[3-(4-Morpholinyl)propyl]amino}phenyl)sulfonyl]amino}methyl)-*N*-(3-pyridinyl)benzamide;
4-[({[3-(4-Methyl-1-piperazinyl)phenyl]sulfonyl}amino)methyl]-*N*-(3-pyridinyl)benzamide;
4-({[(4-{[2-(Dimethylamino)ethyl]amino}phenyl)sulfonyl]amino}methyl)-*N*-(3-pyridinyl)benzamide;
*N*-(3-Pyridinyl)-4-[({[3'-(trifluoromethyl)[1,1'-biphenyl]-4-yl]sulfonyl}amino)methyl]benzamide;
4-({[(4-Benzylphenyl)sulfonyl]amino}methyl)-*N*-(3-pyridinyl)benzamide;
4-{[({4-[3-(4-Morpholinyl)-1-propynyl]phenyl}sulfonyl)amino]methyl}-*N*-(3-pyridinyl)benzamide;
4-{[({4-[3-(Dimethylamino)-1-propynyl]phenyl}sulfonyl)amino]methyl}-*N*-(3-pyridinyl)benzamide;
4-{[[(4-*tert*-Butylphenyl)sulfonyl](methyl)amino]methyl}-*N*-(3-pyridinyl) benzamide;
4-{[[(4-*tert*-Butylphenyl)sulfonyl](ethyl)amino]methyl}-*N*-(3-pyridinyl)benzamide;
4-{[[(4-*tert*-Butylphenyl)sulfonyl](propyl)amino]methyl}-*N*-(3-pyridinyl)benzamide;
4-{[({4-[3-(4-Morpholinyl)propyl]phenyl}sulfonyl)amino]methyl}-*N*-(3-pyridinyl)benzamide;
4-{[({4-[3-(Dimethylamino)propyl]phenyl}sulfonyl)amino]methyl}-*N*-(3-pyridinyl)benzamide;
4-[({[3-(Propionylamino)phenyl]sulfonyl}amino)methyl]-*N*-(3-pyridinyl)benzamide;
4-[({[3-(Acryloylamino)phenyl]sulfonyl}amino)methyl]-*N*-(3-pyridinyl)benzamide;
4-({[(4-*tert*-Butylphenyl)sulfonyl]amino}methyl)-2-methyl-*N*-(3-pyridinyl)benzamide;
4-({[(4-*tert*-Butylphenyl)sulfonyl]amino}methyl)-2-fluoro-*N*-(3-pyridinyl)benzamide;
4-({[(4-*tert*-Butylphenyl)sulfonyl]amino}methyl)-3-methyl-*N*-(3-pyridinyl)benzamide;
4-({[(4-*tert*-Butylphenyl)sulfonyl]amino}methyl)-3-fluoro-*N*-(3-pyridinyl)benzamide;
4-(1-{[(4-*tert*-Butylphenyl)sulfonyl]amino}ethyl)-*N*-(3-pyridinyl)benzamide;
4-[(anilinosulfonyl)methyl]-*N*-(3-pyridinyl)benzamide;
4-{[(4-*tert*-butylanilino)sulfonyl]methyl}-*N*-(3-pyridinyl)benzamide;
4-{[(4-fluoroanilino)sulfonyl]methyl}-*N*-(3-pyridinyl)benzamide; and
4-({[4-(4-methyl-1-piperazinyl)anilino]sulfonyl}methyl)-*N*-(3-pyridinyl)benzamide;
4-((4-(tert-butyl)phenylsulfonamido)methyl)-2-methyl-N-(pyridin-3-yl)benzamide;
4-((4-(tert-butyl)phenylsulfonamido)methyl)-3-methyl-N-(pyridin-3-yl)benzamide;
4-((4-(tert-butyl)phenylsulfonamido)methyl)-2-fluoro-N-(pyridin-3-yl)benzamide;
4-((4-(tert-butyl)phenylsulfonamido)methyl)-3-fluoro-N-(pyridin-3-yl)benzamide;
4-((4-(tert-butyl)phenylsulfonamido)methyl)-3-nitro-N-(pyridin-3-yl)benzamide;
4-(1-(4-(tert-butyl)phenylsulfonamido)ethyl)-N-(pyridin-3-yl)benzamide,
4-(N-phenylsulfamoylmethyl)-N-(pyridin-3-yl)benzamide;
4-((N-(4-fluorophenyl)sulfamoyl)methyl)-N-(pyridin-3-yl)benzamide;
4-((N-(4-tert-butylphenyl)sulfamoyl)methyl)-N-(pyridin-3-yl)benzamide;
4-((N-(4-(4-methylpiperazin-1-yl)phenyl)sulfamoyl)methyl)-N-(pyridin-3-yl)benzamide,
or a pharmaceutically acceptable salt thereof.

The methods described herein comprise administering to a subject in need thereof, a therapeutically acceptable amount at least one compound or pharmaceutically acceptable salt thereof described above.

In addition to those compounds and pharmaceutically acceptable salts described above, the methods described herein also may comprise the administration of at least one compound chosen from:
4-((3-Methoxyphenylsulfonamido)methyl)-*N*-(pyridin-3-yl)benzamide;
4-((3,4-dimethylphenylsulfonamido)methyl)-N-(pyridin-3-yl)benzamide;
3-((3,4-dimethylphenylsulfonamido)methyl)-N-(pyridin-3-yl)benzamide;
N-(pyridin-3-yl)-3-((2,3,4-trifluorophenylsulfonamido)methyl)benzamide;
N-(pyridin-3-yl)-4-((2,3,4-trifluorophenylsulfonamido)methyl)benzamide;
N-(pyridin-3-yl)-3-((2,3,5,6-tetramethylphenylsulfonamido)methyl)benzamide;
N-(pyridin-3-yl)-4-((2,3,5,6-tetramethylphenylsulfonamido)methyl)benzamide;
3-((2,5-dimethylphenylsulfonamido)methyl)-N-(pyridin-3-yl)benzamide;
4-((2,5-dimethylphenylsulfonamido)methyl)-N-(pyridin-3-yl)benzamide;
4-((3-Chlorophenylsulfonamido)methyl)-*N*-(pyridin-3-yl)benzamide,
*N*-(Pyridin-3-yl)-4-((3-(trifluoromethyl)phenylsulfonamido)methyl)benzamide,
4-((4-Methoxyphenylsulfonamido)methyl)-*N*-(pyridin-3-yl)benzamide,
3-((4-*tert*-Butylphenylsulfonamido)methyl)-*N*-(pyridin-3-yl)benzamide,
4-((4-Fluorophenylsulfonamido)methyl)-*N*-(pyridin-3-yl)benzamide, and
4-((4-Acetylphenylsulfonamido)methyl)-*N*-(pyridin-3-yl)benzamide, or a pharmaceutically acceptable salt thereof.

In some embodiments, the subject is a mammal. In some embodiments, the subject is a human.

The compounds and pharmaceutically acceptable salts described herein can be administered alone, as mixtures, or in combination with other active agents.

Methods for obtaining the compounds and pharmaceutically acceptable salts described herein will be apparent to those of ordinary skill in the art, suitable procedures being described, for example, in the reaction schemes and examples below, and the references cited herein.

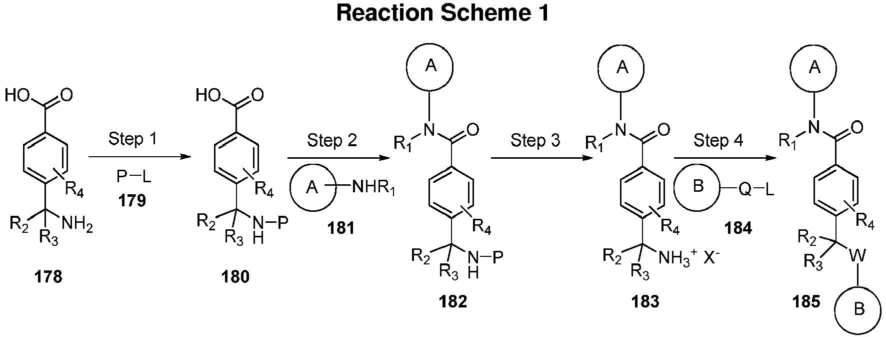

Referring to Reaction Scheme 1, Step 1, a compound of Formula **178**, is combined with an aqueous solution of base (such as NaOH in water), and treated with a compound of Formula **179**, where P is a nitrogen protecting group (such as benzenesulfonyl), and L is a leaving group (such as bromide), to give a compound of Formula **180**, which is isolated and optionally purified.

Referring to Reaction Scheme 1, Step 2, a mixture of a compound of Formula 180 is combined with a halogenating agent (such as oxalyl chloride), an organic base (such as pyridine), in a polar organic solvent (such as DMF and/or THF). A compound of Formula **181** is then added to give the product, a compound of Formula 182, which is isolated and optionally purified.

Referring to Reaction Scheme 1, Step 3, a compound of Formula **182** is treated with an acidic mixture (such as hydrobromic acid and acetic acid), to provide a compound of Formula **183**, where X is a halogen (such as bromide), where the product, a compound of Formula **184**, is isolated and optionally purified.

Referring to Reaction Scheme 1, Step 4, a mixture of a compound of Formula 183 is combined with a compound of Formula **184**, where L is a leaving group (such as chloride) and Q is a substitutent group (such as carbonyl or SO₂), and an organic base (such a pyridine) to give the product, a compound of Formula **185**, which is isolated and optionally purified.

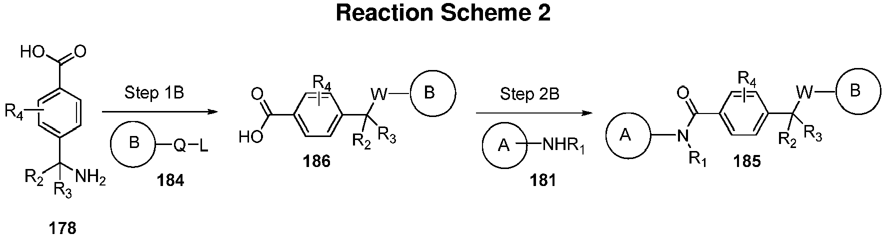

Referring to Reaction Scheme 2, Step 1 B, a mixture of a compound of Formula **178** is combined with a compound of Formula **184**, where L is a leaving group (such as chloride) and Q is a substitutent group (such as carbonyl or SO₂), and an organic base (such a pyridine) to give the product, a compound of Formula **186**, which is isolated and optionally purified.

Referring to Reaction Scheme 1, Step 2B, a mixture of a compound of Formula **186** is combined with a halogenating agent (such as oxalyl chloride), an organic base (such as pyridine), in a polar organic solvent (such as DMF and/or THF). A compound of Formula **181** is then added to give the product, a compound of Formula **185**, which is isolated and optionally purified.

Also provided is a pharmaceutical composition comprising at least one compound and/or pharmaceutically acceptable salt described herein and at least one pharmaceutically acceptable carrier.

The term "pharmaceutically acceptable carrier" refers to any and all solvents, dispersion media, coatings, isotonic and absorption delaying agents, and the like, that are compatible with pharmaceutical administration. The use of such media and agents for pharmaceutically active substances is well known in the art, such as, for example, aqueous solutions such as water or physiologically buffered saline or other solvents or vehicles such as glycols, glycerol, oils such as olive oil or injectable organic esters. The compositions may also contain other active compounds providing supplemental, additional, or enhanced therapeutic functions.

A pharmaceutically acceptable carrier may contain physiologically acceptable agents that act, for example, to stabilize or to increase the absorption of a compound or pharmaceutically acceptable salt thereof. Such physiologically acceptable agents include, for example, carbohydrates, such as glucose, sucrose or dextrans, antioxidants, such as ascorbic acid or glutathione, chelating agents, low molecular weight proteins or other stabilizers or excipients. The choice of a pharmaceutically acceptable carrier, including a physiologically acceptable agent, may depend, for example, on the route of administration of the composition. The pharmaceutical composition also may comprise a liposome or other polymer matrix, which may have incorporated therein, for example, a compound as described herein. Liposomes, for example, which consist of phospholipids or other lipids, are nontoxic, physiologically acceptable and metabolizable carriers that are relatively simple to make and administer.

In some embodiments, a "pharmaceutically acceptable carrier" as used herein means a pharmaceutically acceptable material, composition, or vehicle, such as a liquid or solid filler, diluent, excipient, solvent, or encapsulating material, involved in carrying or transporting the subject compounds from one organ, or portion of the body, to another organ, or portion of the body. Each carrier is typically "acceptable" in the sense of being compatible with the other ingredients of the formulation and not injurious to the patient. Some examples of materials that may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. See Remington: The Science and Practice of Pharmacy, 20th ed. (Alfonso R. Gennaro ed.), 2000.

In some embodiments, a pharmaceutical composition comprising at least one compound and/or salt as described herein may be administered to a subject by any of a number of routes of administration including, for example, orally (for example, drenches as in aqueous or non-aqueous solutions or suspensions, tablets, boluses, powders, granules, pastes for application to the tongue); sublingually; anally, rectally, or vaginally (for example, as a pessary, cream, or foam); parenterally (including intramusclularly, intravenously, subcutaneously, or intrathecally as, for example, a sterile solution or suspension); nasally; intraperitoneally; subcutaneously; transdermally (for example as a patch applied to the skin); or topically (for example, as a cream, ointment or spray applied to the skin). At least one compound and/or salt as described herein may also be formulated for inhalation.

In some embodiments, at least one compound of Formula I or IA or a pharmaceutically acceptable salt thereof, may be simply dissolved or suspended in sterile water. Details of appropriate routes of administration and compositions suitable for same can be found in, for example, U.S. Patent Nos. 6,110,973; 5,763,493; 5,731,000; 5,541,231; 5,427,798; 5,358,970; and 4,172,896, as well as in patents cited therein.

The pharmaceutical compositions described herein may conveniently be presented in unit dosage form and may be prepared by any methods well known in the art of pharmacy. The amount of active ingredient that can be combined with a carrier material to produce a single dosage form will vary depending upon the subject being treated and the particular mode of administration. The amount of active ingredient that can be combined with a carrier material to produce a single dosage form will generally be that amount of the compound that produces a therapeutic effect.

In some embodiments, this amount ranges from about 1 percent to about 99 percent of active ingredient.

In another embodiment, this amount ranges from about 5 percent to about 70 percent, and in a further embodiment from about 10 percent to about 30 percent.

Methods of preparing these compositions include the step of bringing into association at least one compound and/or pharmaceutically acceptable salt as described herein with at least one carrier and, optionally, one or more accessory ingredients.

In some embodiments, the pharmaceutical compositions are prepared by uniformly and intimately bringing into association at least one compound and/or pharmaceutically acceptable salt as described herein with liquid carriers, or finely divided solid carriers, or both, and then, if necessary, shaping the product.

Pharmaceutical compositions suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of at least one compound and/or salt as described herein as an active ingredient. The pharmaceutical compositions described herein may also be administered as a bolus, electuary, or paste.

In some embodiments, compounds and/or pharmaceutically acceptable salts described herein are mixed with one or more pharmaceutically acceptable excipients, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agaragar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

In some embodiments, the tablets, and other solid dosage forms pharmaceutical compositions, such as dragees, capsules, pills and granules, may optionally be scored or prepared with coatings and shells, such as enteric coatings and other coatings well known in the pharmaceutical formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition in that they release the active ingredient(s) only, preferentially, in a certain portion of the gastrointestinal tract, optionally, or in a delayed manner. Examples of embedding compositions that may be used include polymeric substances and waxes. The active ingredient may also be in micro-encapsulated form, if appropriate, with one or more of the above-described excipients.

In some embodiments, liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils, cottonseed, groundnut, corn, germ, olive, castor oils, sesame oils, glycerol, tetrahydrofuryl alcohol, polyethylene glycols and fatty acid esters of sorbitan, and mixtures thereof.

In some embodiments, the emulsifiers are chosen from cottonseed, groundnut, corn, germ, olive, castor, and sesame oils.

Besides inert diluents, the oral compositions may also include adjuvants such as wetting agents, emulsifying and suspending agents, sweetening, flavoring, coloring, perfuming, and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agaragar, and tragacanth, and mixtures thereof.

Pharmaceutical compositions as described herein for rectal, vaginal, or urethral administration may be presented as a suppository, which may be prepared by mixing one or more compounds or salts as described herein with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Alternatively or additionally, pharmaceutical compositions described herein may be formulated for delivery via a catheter, stent, wire, or other intraluminal device. Delivery via such devices may be especially useful for delivery to the bladder, urethra, ureter, rectum, or intestine.

Formulations suitable for vaginal administration also include pessaries, tampons, creams, gels, pastes, foams, or spray formulations containing such carriers as are known in the art to be appropriate.

Dosage forms for the topical or transdermal administration include powders, sprays, ointments, pastes, creams, lotions, gels, solutions, patches, and inhalants. The active compound may be mixed under sterile conditions with a pharmaceutically acceptable carrier, and with any preservatives, buffers, or propellants that may be required.

The ointments, pastes, creams, and gels may comprise excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to a compound as described herein, excipients such as lactose, talc, silicic acid, aluminum hydroxide, calcium silicates, and polyamide powder, or mixtures of these substances. Sprays may additionally contain customary propellants, such as chlorofluorohydrocarbons and volatile unsubstituted hydrocarbons, such as butane and propane.

Transdermal patches have the added advantage of providing controlled delivery to the body. Such dosage forms may be made by dissolving or dispersing the compound in the proper medium. Absorption enhancers may also be used to increase the flux across the skin. The rate of such flux may be controlled by either providing a rate controlling membrane or dispersing the compound in a polymer matrix or gel.

Ophthalmic formulations, eye ointments, powders, solutions, and the like, may also comprise at least one of the compounds or salts as described herein.

In some embodiments, pharmaceutical compositions as described herein suitable for parenteral administration comprise at least one compound of Formula I, IA, or II, or a pharmaceutically acceptable salt thereof, in combination with one or more pharmaceutically acceptable sterile isotonic aqueous or nonaqueous solutions, dispersions, suspensions, or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

These compositions may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, chelators and the like.

In some embodiments, isotonic agents, such as sugars, sodium chloride, and the like may be included into the compositions. In addition, prolonged absorption of the injectable pharmaceutical form may be brought about by the inclusion of agents that delay absorption such as aluminum monostearate and gelatin.

In some cases, in order to prolong the effect of a drug, it may be advantageous to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

Methods of introduction may also be provided by rechargeable or biodegradable devices. Various slow release polymeric devices have been developed and tested in vivo in recent years for the controlled delivery of drugs. A variety of biocompatible polymers (including hydrogels), including both biodegradable and non-degradable polymers, may be used to form an implant for the sustained release of a compound at a particular target site.

As further detailed below, the pharmaceutical compositions described herein may also comprise, or may be used in combination with, one or more known cytotoxic, vascular targeting agents or chemotherapeutic agents including, but not limited to, XelodaTM (capecitabine), PaclitaxelTM, FUDR (fluorouridine) FludaraTM (fludarabine phosphate), GemzarTM (gemcitabine), methotrexate, cisplatin, carboplatin, adriamycin, avastin, tarceva, taxol, tamoxifen, Femora, temezolamide, cyclophosphamide, Erbitux, and Sutent.

In some embodiments, when pharmaceutically acceptable compositions are for human administration, the aqueous solution is pyrogen free, or substantially pyrogen free. The excipients may be chosen, for example, to effect delayed release of an agent or to selectively target one or more cells, tissues or organs. The pharmaceutical composition may be in dosage unit form such as tablet, capsule, sprinkle capsule, granule, powder, syrup, suppository, injection or the like. The composition may also be present in a transdermal delivery system, e.g., a skin patch.

The term "pharmaceutically acceptable prodrugs" as used herein represents those prodrugs of a compound of Formula I or IA or a pharmaceutically acceptable salt thereof, that are, within the scope of sound medical judgment, suitable for use in contact with the tissues of humans and lower animals without undue toxicity, irritation, allergic response, commensurate with a reasonable benefit / risk ratio, and effective for their intended use, as well as the zwitterionic forms, where possible, of the compounds and pharmaceutically acceptable salts described herein. A discussion is provided in Higuchi et al., "Prodrugs as Novel Delivery Systems," ACS Symposium Series, Vol. 14, and in Roche, E.B., ed. Bioreversible Carriers in Drug Design, American Pharmaceutical Association and Pergamon Press, 1987.

The term "pharmaceutically acceptable salt(s)" refers to salts of acidic or basic groups that may be present in compounds used in the present compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including but not limited to hydrochloric, hydrobromic, hydriodic, sulfuric and phosphoric acid, as well as organic acids such as para-toluenesulfonic, methanesulfonic, oxalic, para-bromophenylsulfonic, carbonic, succinic, citric, benzoic and acetic acid, and related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephathalate, sulfonate, xylenesulfonate, phenylacetate, phenylpropionate, phenylbutyrate, citrate, lactate, β-hydroxybutyrate, glycollate, maleate, tartrate, methanesulfonate, propanesulfonates, naphthalene-1-sulfonate, naphthalene-2-sulfonate, mandelate, hippurate, gluconate, lactobionate, and the like salts.

In some embodiments, pharmaceutically acceptable acid addition salts include those formed with mineral acids such as hydrochloric acid and hydrobromic acid, and those formed with organic acids such as fumaric acid and maleic acid.

Compounds included in the present compositions, that are acidic in nature may react with any number of inorganic and organic bases to form pharmaceutically acceptable base salts. Bases may include, for example, the mineral bases, such as NaOH and KOH, but one of skill in the art would appreciate that other bases may also be used. See Ando et al., Remington: The Science and Practice of Pharmacy, 20th ed. 700-720 (Alfonso R. Gennaro ed.), 2000.

In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare non-toxic pharmaceutically acceptable addition salts.

In some embodiments, the pharmaceutically acceptable addition salts of the compounds described herein may also exist as various solvates, such as, for example, with water, methanol, ethanol, dimethylformamide, and the like. Mixtures of such solvates may also be prepared. The source of such solvate may be from the solvent of crystallization, inherent in the solvent of preparation or crystallization, or adventitious to such solvent.

The compounds and pharmaceutically acceptable salts thereof described herein may target cells which express HIF-1α and/or HIF-2α. The compounds and pharmaceutically acceptable salts thereof described herein may target cells which express HIF-1α. The compounds and pharmaceutically acceptable salts thereof described herein may target cells which express HIF-2α. The compounds and pharmaceutically acceptable salts thereof described herein may target cells which express HIF-1α and/or HIF-2α.

The compounds and pharmaceutically acceptable salts thereof described herein may target cells which do not have functional VHL.

In some embodiments, the compounds and pharmaceutically acceptable salts described herein are for use in treating cells, and more particularly, cancerous cells, expressing HIF-1α and/or HIF-2α. In some embodiments, the compounds and pharmaceutically acceptable salts described herein are for use in treating cells, a more particularly, cancerous cells, expressing HIF-1α. In some embodiments, the compounds and pharmaceutically acceptable salts described herein are for use in treating cells, and more particularly, cancerous cells, expressing HIF-2α. In some embodiments, the compounds and pharmaceutically acceptable salts described herein are for use in treating cells, and more particularly, cancerous cells, expressing HIF-1α and/or HIF-2α.

In some embodiments, the compounds and pharmaceutically acceptable salts thereof interfere with glycolysis.

In certain embodiments, the disease treated or prevented is cancer.

In some embodiments, the compounds and pharmaceutically acceptable salts described herein may be for use in treating a disease mediated by defective pVHL protein, such as Von Hippel-Lindau disease (which may also be referred to as angiomatosis retinae, angiophakomatosis retinae et cerebelli, familial cerebello- retinal angiomatosis, cerebelloretinal hemangioblastomatosis, Hippel Disease, Hippel-Lindau syndrome, HLS, VHL, Lindau disease or retinocerebellar angiomatosis). In some embodiments, the compounds and pharmaceutically acceptable salts described herein may be for use in treating a variety of malignant and/or benign tumors of the eye, brain, spinal cord, kidney, pancreas, and/or adrenal glands wherein individuals suffering from VHL may be disposed to such tumors. In some embodiments, the compounds and pharmaceutically acceptable salts described herein may be for use in treating a disease mediated by defective pVHL protein, such as ngiomatosis, hemangioblastomas, pheochromocytoma, renal cell carcinoma, pancreatic cysts and cafe au lait spots.

Also provided is a compound of Formula I or IA, or a pharmaceutically acceptable salt thereof, for use in a method for treating a disease mediated by defective pVHL protein, comprising administering to a subject at least one said compound that is specifically cytotoxic to cells that have elevated HIF levels due to their increased rate and dependence on glucose uptake and glycolysis. In some embodiments, at least one compound of Formula I or IA, selectively disrupts glucose uptake and utilization in the subject. In some embodiments, at least one compound of Formula I or IA, or a pharmaceutically acceptable salt thereof, inhibits HIF-mediated induction of *PDK1.*

Also described is a method of targeting cells which have defective pVHL protein. The cells may be contacted with at least one compound of Formula I or IA, or a pharmaceutically acceptable salt thereof, that selectively disrupts glucose uptake and utilization in the cells. The compound of Formula I or IA, or a pharmaceutically acceptable salt thereof, may inhibit HIF-mediated induction of *PDK1.*

Also described is a method for selectively killing cells which have defective pVHL protein. The cells may be contacted with at least one compound of Formula I or IA, or a pharmaceutically acceptable salt thereof, that selectively disrupts glucose uptake and utilization in the cells. At least one compound of Formula I or IA, or a pharmaceutically acceptable salt thereof, may inhibit HIF-mediated induction of *PDK1*.

Also described is a method for treating a disease mediated by HIF-1α and/or HIF-2α comprising administering to a subject at least one compound of Formula I or IA, or a pharmaceutically acceptable salt thereof, that is specifically cytotoxic to cells that have elevated HIF levels due to their increased rate and dependence on glucose uptake and glycolysis. At least one compound of Formula I or IA, or a pharmaceutically acceptable salt thereof, may selectively disrupt glucose uptake and utilization in the subject. At least one compound of Formula I or IA, or a pharmaceutically acceptable salt thereof, may inhibit HIF-mediated induction of *PDK1*.

Also described is a method for treating a disease mediated by cells comprising genetic or epigenetic alterations that make them highly dependent on aerobic glycolysis for energy production, comprising administering to a subject at least one compound of Formula I or IA, or a pharmaceutically acceptable salt thereof, that is specifically cytotoxic to cells comprising genetic or epigenetic alterations that make them highly dependent on aerobic glycolysis for energy production.

Also described is a method for selectively killing cells comprising genetic or epigenetic alterations that make them highly dependent on aerobic glycolysis for energy production, comprising administering to the cells at least one compound of Formula I or IA, or a pharmaceutically acceptable salt thereof, that is specifically cytotoxic to cells comprising genetic or epigenetic alterations that make them highly dependent on aerobic glycolysis for energy production. At least one compound of Formula I or IA, or a pharmaceutically acceptable salt thereof, may selectively disrupt glucose uptake and utilization in cells comprising genetic or epigenetic alterations that make them highly dependent on aerobic glycolysis for energy production.

Also described is a method for treating a disease mediated by GLUT1 administering to a subject at least one compound of Formula I or IA, or a pharmaceutically acceptable salt thereof. Also described is a method for treating a disease mediated by GLUT 1, comprising administering to a subject at least one compound of Formula I or IA, or a pharmaceutically acceptable salt thereof, that is specifically cytotoxic to cells that have elevated GLUT 1 levels due to their increased rate and dependence on glucose uptake and glycolysis. At least one compound of Formula I or IA, or a pharmaceutically acceptable salt thereof, may selectively disrupt glucose uptake and utilization in the subject. At least one compound of Formula I or IA, or a pharmaceutically acceptable salt thereof, may inhibit glucose transport by GLUT1.

Also provided is a method of identifying a compound as a candidate cancer therapy, comprising exposing a first population of cells that have elevated expression of GLUT1 but not GLUT2 to a test compound and assaying cytotoxicity of the test compound, exposing a second population of cells that have elevated expression of GLUT2 but not GLUT1 to the test compound and assaying cytotoxicity of the test compound, and identifying the test compound as a candidate cancer therapy if the test compound induces significantly higher cytotoxicity in the first population of cells than in the second population of cells. Also provided is at least one compound, or a pharmaceutically acceptable salt thereof, identified by such method.

The subject receiving treatment may be any mammal in need of such treatment. Such mammals include, *e.g*., humans, ovines, bovines, equines, porcines, canines, felines, non-human primate, mice, and rats. In some embodiments, the subject is a human. In some embodiments, the subject is a non- human mammal.

"Therapeutically-effective amount" refers to the concentration of a compound that is sufficient to elicit the desired therapeutic effect (*e.g.*, treatment or prevention of a disease). It is generally understood that the effective amount of the compound will vary according to the weight, gender, age, and medical history of the subject. Other factors that influence the effective amount may include, but are not limited to, the severity of the patient's condition, the disorder being treated, the stability of the compound, and, if desired, another type of therapeutic agent being administered with the compounds and pharmaceutically acceptable salts described herein. A larger total dose may be delivered by multiple administrations of the agent. Methods to determine efficacy and dosage are known to those skilled in the art. See, *e.g.*, Roden, Harrison's Principles of Internal Medicine, Ch. 3, McGraw-Hill, 2004.

Actual dosage levels of the active ingredients in the pharmaceutical compositions comprising at least one compound or pharmaceutically active salt as described herein may be varied so as to obtain an amount of the active ingredient that is effective to achieve the desired therapeutic response for a particular patient, composition, and mode of administration, without being toxic to the patient.

A physician or veterinarian having ordinary skill in the art can readily determine and prescribe the effective amount of the pharmaceutical composition required. For example, the physician or veterinarian could start doses of the compounds described herein employed in the pharmaceutical composition at levels lower than that required in order to achieve the desired therapeutic effect and gradually increase the dosage until the desired effect is achieved.

In general, a suitable daily dose of at least one compound of Formula I or IA, or a pharmaceutically acceptable salt thereof, will be that amount of the compound that is the lowest dose effective to produce a therapeutic effect. Such an effective dose will generally depend upon the factors described herein.

If desired, the effective daily dose of the active compound may be administered as one, two, three, four, five, six, or more sub-doses administered separately at appropriate intervals throughout the day, optionally, in unit dosage forms.

The active compound may be administered two or three times daily. The active compound may be administered once daily.

The optimal frequency of administration and effective dosage will vary from one individual to another and will depend upon the particular disease being treated and may be determined by one skilled in the art.

In some embodiments, effective dosages of at least one compound of Formula I or IA, or a pharmaceutically acceptable salt thereof, may range from as low as about 1 mg per day to as high as about 1000 mg per day, including all intermediate dosages there between.

In another embodiment, effective dosages may range from about 10 mg per day to about 100 mg per day, including all intermediate dosages there between. The compositions may be administered in a single dosage, or in multiple, divided dosages.

As described herein, at least one compound of Formula I or IA, may be used for treating or preventing cancer. In some embodiments, such methods may, further comprise administration of a chemotherapeutic agent.

Chemotherapeutic agents that may be coadministered with compounds and pharmaceutical compositions of Formula I or IA, may include: alemtuzumab, aminoglutethimide, amsacrine, anastrozole, asparaginase, Bacillus Calmette-Guérin, bevacizumab, bicalutamide, bleomycin, bortezomib, buserelin, busulfan, campothecin, capecitabine, carboplatin, carmustine, CeaVac, cetuximab, chlorambucil, cisplatin, cladribine, clodronate, colchicine, cyclophosphamide, cyproterone, cytarabine, dacarbazine, daclizumab, dactinomycin, daunorubicin, dienestrol, diethylstilbestrol, docetaxel, doxorubicin, edrecolomab, epirubicin, epratuzumab, erlotinib, estradiol, estramustine, etoposide, exemestane, filgrastim, fludarabine, fludrocortisone, fluorouracil, fluoxymesterone, flutamide, gemcitabine, gemtuzumab, genistein, goserelin, huJ591, hydroxyurea, ibritumomab, idarubicin, ifosfamide, IGN-101, imatinib, interferon, interleukin-2, irinotecan, ironotecan, letrozole, leucovorin, leuprolide, levamisole, lintuzumab, lomustine, MDX-210, mechlorethamine, medroxyprogesterone, megestrol, melphalan, mercaptopurine, mesna, methotrexate, mitomycin, mitotane, mitoxantrone, mitumomab, nilutamide, nocodazole, octreotide, oxaliplatin, paclitaxel, pamidronate, pentostatin, pertuzumab, plicamycin, porfimer, procarbazine, raltitrexed, rituximab, sorafinib, streptozocin, sunitinib, suramin, tamoxifen, temozolomide, temsirolimus, teniposide, testosterone, thalidomide, thioguanine, thiotepa, titanocene dichloride, topotecan, tositumomab, trastuzumab, tretinoin, vatalanib, vinblastine, vincristine, vindesine, and vinorelbine.

Other useful chemotherapeutic agents for combination with the compounds as described herein include MDX-010; MAb, AME; ABX-EGF; EMD 72 000; apolizumab; labetuzumab; ior-tl; MDX-220; MRA; H-11 scFv; Oregovomab; huJ591 MAb, BZL; visilizumab; TriGem; TriAb; R3; MT-201; G-250, unconjugated; ACA-125; Onyvax-105; CDP-860; BrevaRex MAb; AR54; IMC-1C11; GlioMAb-H; ING-1; Anti-LCG MAbs; MT-103; KSB-303; Therex; KW-2871; Anti-HMI.24; Anti-PTHrP; 2C4 antibody; SGN-30; TRAIL-RI MAb, CAT; Prostate cancer antibody; H22xKi-4; ABX-MA1; Imuteran; and Monopharm-C.

These chemotherapeutic agents may be categorized by their mechanism of action into, for example, the following groups: anti-metabolites/anti-cancer agents, such as pyrimidine analogs (*e.g.*, 5-fluorouracil, floxuridine, capecitabine, gemcitabine and cytarabine) and purine analogs, folate antagonists and related inhibitors (*e.g.*, mercaptopurine, thioguanine, pentostatin and 2-chlorodeoxyadenosine (cladribine)); antiproliferative/antimitotic agents including natural products such as vinca alkaloids (*e.g.*, vinblastine, vincristine, and vinorelbine), microtubule disruptors such as taxane (paclitaxel, docetaxel), vincristin, vinblastin, nocodazole, epothilones and navelbine, epidipodophyllotoxins (teniposide), DNA damaging agents (*e.g.*, actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorethamine, mitomycin, mitoxantrone, nitrosourea, paclitaxel, plicamycin, procarbazine, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes *(e.g.,* L-asparaginase, which systemically metabolizes L-asparagine and deprives cells which do not have the capacity to synthesize their own asparagine); antiplatelet agents; antiproliferative/antimitotic alkylating agents such as nitrogen mustards *(e.g.,* mechlorethamine, cyclophosphamide and analogs, melphalan, chlorambucil), ethylenimines and methylmelamines (*e.g.*, hexamethylmelamine and thiotepa), alkyl sulfonatesbusulfan, nitrosoureas (*e.g.*, carmustine (BCNU) and analogs, streptozocin), trazenes - dacarbazinine (DTIC); antiproliferative/antimitotic antimetabolites such as folic acid analogs (*e.g.*, methotrexate); platinum coordination complexes (*e.g.*, cisplatin, carboplatin), procarbazine, hydroxyurea, mitotane, aminoglutethimide; hormones, hormone analogs (*e.g.*, estrogen, tamoxifen, goserelin, bicalutamide, nilutamide) and aromatase inhibitors (*e.g.*, letrozole, anastrozole); anticoagulants (*e.g.*, heparin, synthetic heparin salts and other inhibitors of thrombin); fibrinolytic agents (such as tissue plasminogen activator, streptokinase and urokinase), aspirin, COX-2 inhibitors, dipyridamole, ticlopidine, clopidogrel, abciximab; antimigratory agents; antisecretory agents (*e.g.*, breveldin); immunosuppressives (*e.g.*, cyclosporine, tacrolimus (FK-506), sirolimus (rapamycin), azathioprine, mycophenolate mofetil); anti-angiogenic compounds (*e.g*., TNP-470, genistein) and growth factor inhibitors (*e.g*., vascular endothelial growth factor (VEGF) inhibitors, fibroblast growth factor (FGF) inhibitors, epidermal growth factor (EGF) inhibitors); angiotensin receptor blocker; nitric oxide donors; anti-sense oligonucleotides; antibodies (*e.g*., trastuzumab and others listed above); cell cycle inhibitors and differentiation inducers (*e.g*., tretinoin); mTOR inhibitors, topoisomerase inhibitors (*e.g*., doxorubicin (adriamycin), amsacrine, camptothecin, daunorubicin, dactinomycin, eniposide, epirubicin, etoposide, idarubicin, irinotecan (CPT-11) and mitoxantrone, topotecan, irinotecan), corticosteroids (*e.g*., cortisone, dexamethasone, hydrocortisone, methylpednisolone, prednisone, and prenisolone); growth factor signal transduction kinase inhibitors; mitochondrial dysfunction inducers and caspase activators; chromatin disruptors.

Pharmaceutical compositions comprising at least one compound of Formula I or IA, may be coadministered with chemotherapeutic agents either singly or in combination.

Combination therapies comprising at least one compound of Formula I or IA, or a pharmaceutically acceptable salt thereof, and a conventional chemotherapeutic agent may be advantageous over combination therapies known in the art because the combination allows the conventional chemotherapeutic agent to exert greater effect at lower dosage. In some embodiments, the effective dose (ED₅₀) for a chemotherapeutic agent, or combination of conventional chemotherapeutic agents, when used in combination with a compound of Formula I or IA, or a pharmaceutically acceptable salt thereof as described herein is at least 2 fold less than the ED₅₀ for the chemotherapeutic agent alone. In another embodiment, the ED₅₀ is about 5-fold less, about 10-fold less, and further about 25-fold less. Conversely, the therapeutic index (TI) for such chemotherapeutic agent or combination of such chemotherapeutic agent when used in combination with a compound or pharmaceutically acceptable salt described herein may be at least 2-fold greater than the TI for conventional chemotherapeutic regimen alone. In another embodiment, the TI is about 5-fold greater, about 10-fold greater, and further about 25-fold greater.

In some embodiments, the compounds and pharmaceutically acceptable salts thereof described herein may be for administration in combination with radiation therapy.

The invention is further illustrated by the following non-limiting examples. If an abbreviation is not defined, it has generally accepted meaning.

Analyses were carried out in the Campbell Microanalytical Laboratory, University of Otago, Dunedin, NZ. Melting points were determined on an Electrothermal 2300 Melting Point Apparatus. NMR spectra were obtained on a Bruker Avance 400 spectrometer at 400 MHz for ¹Hand 100 MHz for ¹³C spectra. Spectra were obtained in [(CD₃)₂SO] unless otherwise specified, and were referenced to Me₄Si. Chemical shifts and coupling constants were recorded in units of ppm and Hz, respectively. Assignments were determined using COSY, HSQC, and HMBC two-dimensional experiments. Low resolution mass spectra were gathered by direct injection of methanolic solutions into a Surveyor MSQ mass spectrometer using an atmospheric pressure chemical ionization (APCI) mode with a corona voltage of 50 V and a source temperature of 400 °C. Solutions in organic solvents were dried with anhydrous MgSO₄. Solvents were evaporated under reduced pressure on a rotary evaporator. Thin-layer chromatography was carried out on aluminium-backed silica gel plates (Merck 60 F₂₅₄) with visualization of components by UV light (254 nm) or exposure to I₂. Column chromatography was carried out on silica gel (Merck 230-400 mesh). DCM refers to dichloromethane; DME refers to dimethoxyethane, DMF refers to dry *N*,*N*-dimethylformamide; ether refers to diethyl ether; EtOAc refers to ethyl acetate; EtOH refers to ethanol; MeOH refers to methanol; pet. ether refers to petroleum ether, boiling range 40-60 °C; THF refers to tetrahydrofuran dried over sodium benzophenone ketyl.

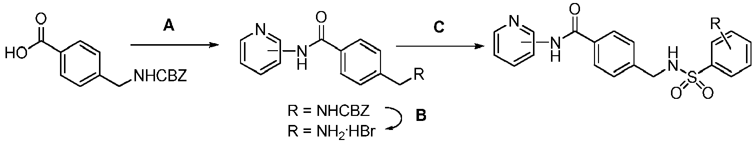

**General Method A.** Oxalyl chloride (13 mmol) was added dropwise to a solution of acid (9 mmol) and DMF (4 drops) in dry THF (40 mL), and the mixture stirred at 50 °C for 3 h. The solvent was evaporated and the residue dissolved in pyridine (25 mL). Aminopyridine (9.6 mmol) was added and the solution stirred at 20 °C for 16 h. Water (150 mL) was added, the mixture stirred for another 2 h, the precipitate filtered off, washed with water and dried to give the amide.

**General Method B.** A suspension of benzylcarbamate (7 mmol) in HBr/AcOH (30%, 30 mL) was stirred at 20 °C for 5 h. Et₂O (150 mL) was added, the mixture was stirred for another 30 min, the precipitate filtered off, washed with Et₂O and dried to give the amine dihydrobromide.

**General Method C.** A mixture of amine dihydrobromide (0.9 mmol) and benzenesulfonyl chloride (1.0 mmol) in dry pyridine (10 mL) was stirred at 20 °C for 16 h. The solvent was evaporated and the residue stirred in water (20 mL) for 1 h. The precipitate was filtered, washed with water (5 mL) and dried. The crude solid was purified by column chromatography, to give the sulfonamide.

### Example 1 (Reference Example)

### Preparation of 4-(Phenylsulfonamidomethyl)-N-(pyridin-2-yl)benzamide (9).

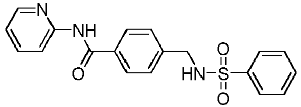

**4-(Benzyloxycarbonylamino)methyl)benzoic acid (2).** Benzyl chloroformate (10.3 mL, 72.7 mmol) and 2 M NaOH solution (33 mL, 66 mmol) were simultaneously added dropwise to a stirred solution of 4-aminomethylbenzoic acid (**1**) (10.0 g, 66.2 mmol) in 2 M NaOH solution (33 mL) and THF (30 mL) at 0 °C. The mixture was stirred at 20 °C for 16 h, then the organic solvent was evaporated and the residue acidified with 2 M HCl until the pH of the mixture was 2-3. The precipitate was filtered, washed with water (250 mL), washed with EtOH (50 mL), and finally washed with Et₂O (100 mL). The solid was dried under vacuum to give acid **2** (16.43 g, 87%) as a white powder: mp 190-192 °C [lit. (Loge et. al., J. Enzyme Inhibit. Med. Chem. 2002, 17, 381-390) mp (toluene) 194-195 °C; ¹H NMR δ 7.85 (br d, 2 H, H-2, H-6), 7.82 (br t, *J* = 6.1 Hz, 1 H, NHCO₂), 7.30-7.40 (m, 5 H, H-2', H-3', H-4', H-5', H-6'), 7.27 (br d, *J* = 8.2 Hz, 2 H, H-3, H-5), 5.05 (s, 2 H, OCH₂), 4.24 (d, *J* = 6.1 Hz, 2 H, CH₂N).

**Benzyl 4-(pyridine-2-ylcarbamoyl)benzylcarbamate (3). Method A.** Reaction of benzoic acid 2 (2.5 g, 8.8 mmol) and oxalyl chloride (1.15 mL, 13.1 mmol), with subsequent reaction with 2-aminopyridine (0.91 g, 9.6 mmol), gave carbamate **3** (2.43 g, 77%) as a pale pink solid: mp (EtOAc) 127-129 °C; ¹H NMR δ 10.69 (s, 1 H, NHCO), 8.37-8.39 (m, 1 H, H-3'), 8.19 (d, *J* = 8.4 Hz, 1 H, H-6'), 7.99 (d, *J* = 8.3 Hz, 2 H, H-2, H-6), 7.84 (br t, *J* = 6.0 Hz, 1 H, NHCO₂), 7.80-7.85 (m, 1 H, H-5'), 7.29-7.39 (m, 7 H, H-3, H-5, phenyl), 7.14-7.18 (m, 1 H, H-4'), 5.07 (s, 2 H, OCH₂), 4.09 (d, *J* = 6.2 Hz, 2 H, CH₂N); ¹³C NMR δ 165.6, 156.3, 152.1, 147.8, 143.8, 137.9, 137.0, 132.5, 128.2 (2), 127.9 (2), 127.7, 127.6 (2), 126.6 (2), 119.6, 114.5, 65.3, 43.5. Anal. Calcd for C₂₁H₁₉N₃O₃: C, 69.79; H, 5.30; N, 11.63. Found: C, 69.54; H, 5.46; N, 11.40%.

**4-(Aminomethyl)-*N*-(2-pyridinyl)benzamide dihydrobromide (4). Method B.** Reaction of benzylcarbamate **3** (2.4 g, 6.6 mmol) gave benzamide **4** (1.65 g, 64%) as a pale pink solid: mp (EtOAc) 281-284 °C; ¹H NMR δ 10.61 (s, 1 H, NHCO), 8.49 (dd, *J* = 5.4, 1.0 Hz, 1 H, H-3'), 8.38 (br s, 3 H, NH₂·HBr), 8.22 (dd, *J* = 7.9, 1.6 Hz, 1 H, H-5'), 8.09-8.14 (m, 3 H, H-2, H-6, H-6'), 7.68 (d, *J =* 8.3 Hz, 2 H, H-3, H-5), 7.46 (t, *J* = 6.2 Hz, 1 H, H-4'), 4.16 (q, *J* = 5.8 Hz, 2 H, CH₂N), 7.06 (br s, 1 H, pyrN·HBr); ¹³C NMR δ 166.4, 149.6, 142.9, 142.7, 138.6, 132.6, 128.8 (2), 128.4 (2), 120.4, 116.1, 41.7. Anal. Calcd for C₁₃H₁₅Br₂N₃O: C, 40.13; H, 3.89; N, 10.80. Found: C, 40.26; H, 4.07; N, 10.53%.

**4-(Phenylsulfonamidomethyl)-*N*-(pyridin-2-yl)benzamide (9). Method C.** Reaction of amine salt **4** (357 mg, 0.9 mmol) and benzenesulfonyl chloride (0.13 mL, 1.0 mmol), followed by column chromatography, eluting with EtOAc, gave benzamide **9** (272 mg, 80%) as a white powder: mp (EtOAc) 169-170 °C; ¹H NMR δ 10.68 (br s, 1 H, NHCO), 8.38 (ddd, *J* = 4.9, 1.8, 0.8 Hz, 1 H, H-6'), 8.24 (br s, 1 H, NHSO₂), 8.18 (br d, *J =* 8.4 Hz, 1 H, H-3'), 7.97 (br d, *J* = 8.3 Hz, 2 H, H-2, H-6), 7.80-7.86 (m, 3 H, H-4', H-2", H-6"), 7.62-7.67 (m, 1 H, H-4"), 7.56-7.61 (m, 2 H, H-3", H-5"), 7.37 (br d, *J =* 8.3 Hz, 2 H, H-3, H-5), 7.16 (ddd, *J* = 7.3, 4.9, 0.9 Hz, 1 H, H-5'), 4.08 (s, 2 H, CH₂N); ¹³C NMR δ 165.5, 152.0, 147.8, 141.8, 140.6, 137.9, 132.7, 132,3, 129.1 (2), 127.8 (2), 127.2 (2), 126.3 (2), 119.6, 114.6, 45.6; MS *m*/*z* 368.4 (MH⁺, 100%). Anal. Calcd for C₁₉H₁₇N₃O₃S: C, 62.11; H, 4.66; N, 11.44. Found: C, 62.17; H, 4.86; N, 11.44%.

### Example 2

### Preparation of 4-(Phenylsulfonamidomethyl)-N-(pyridin-3-yl)benzamide (10).

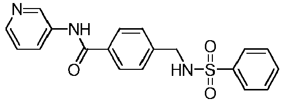

**Benzyl 4-(pyridine-3-ylcarbamoyl)benzylcarbamate (5). Method A.** Reaction of benzoic acid **2** (10.0 g, 35.0 mmol) and oxalyl chloride (4.58 mL, 52.5 mmol), with subsequent reaction with 3-aminopyridine (3.62 g, 38.5 mmol) gave carbamate **5** (7.82 g, 62%) as a white solid: mp (EtOH) 207-210 °C; ¹H NMR δ 10.37 (s, 1 H, NHCO), 8.92 (d, *J* = 2.3 Hz, 1 H, H-2'), 8.31 (dd, *J* = 4.7, 1.5 Hz, 1 H, H-6'), 8.18 (ddd, *J* = 8.34, 2.5, 1.5 Hz, 1 H, H-4'), 7.93 (br d, *J* = 8.3 Hz, 2 H, H-2, H-6), 7.89 (br t, *J* = 6.0 Hz, 1 H, NHCO₂), 7.41 (br d, *J* = 8.3 Hz, 2 H, H-3, H-5), 7.31-7.39 (m, 6 H, H-5', H-2", H-3", H-4", H-5", H-6"), 5.06 (s, 2 H, CH₂O), 4.30 (d, *J* = 6.2 Hz, 2 H, CH₂N). Anal. Calcd for C₂₁H₁₉N₃O₃: C, 69.79; H, 5.30; N, 11.63. Found: C, 69.60; H, 5.40; N, 11.63%.

**4-(Aminomethyl)-*N*-(3-pyridinyl)benzamide dihydrobromide (6). Method B.** Reaction of carbamate **5** (2.2 g, 6.1 mmol) gave benzamide **6** (2.35 g, 99%) as a white solid: mp (EtOAc) 292-296 °C; ¹H NMR δ 11.06 (s, 1 H, NHCO), 9.35 (d, *J* = 2.2 Hz, 1 H, H-2'), 8.70 (ddd, *J* = 8.5, 2.2, 1.1 Hz, 1 H, H-4'), 8.64 (br d, *J* = 5.4 Hz, 1 H, H-6'), 8.31 (br s, 3 H, NH₂·HBr), 8.09 (br d, *J* = 8.2 Hz, 2 H, H-2, H-6), 7.96 (dd, *J* = 8.6, 5.4 Hz, 1 H, H-5'), 7.67 (d, *J* = 8.4 Hz, 2 H, H-3, H-5), 5.95 (br s, 1 H, pyrN·HBr), 4.16 (q, *J* = 5.8 Hz, 2 H, CH₂N); Anal. Calcd for C₁₃H₁₅Br₂N₃O: C, 40.13; H, 3.89; N, 10.80. Found: C, 39.99; H, 3.94; N, 10.36%.

**4-(Phenylsulfonamidomethyl)-*N*-(pyridin-3-yl)benzamide (10). Method** C. Reaction of amine salt **6** (411 mg, 1.1 mmol) and benzenesulfonyl chloride (0.15 mL, 1.2 mmol), followed by column chromatography eluting with EtOAc, gave benzamide **10** (237 mg, 61%) as a white powder: mp (EtOAc) 168-170 °C; ¹H NMR δ 10.37 (s, 1 H, NHCO), 8.93 (d, *J* = 2.3 Hz, 1 H, H-2'), 8.31 (dd, *J* = 4.7, 1.5 Hz, 1 H, H-6'), 8.25 (br s, 1 H, NHSO₂), 8.18 (ddd, *J* = 8.4, 2.5, 1.5 Hz, 1 H, H-4'), 7.90 (br d, *J* = 8.3 Hz, 2 H, H-2, H-6), 7.83 (br d, *J* = 8.0 Hz, 2 H, H-2", H-6"), 7.57-7.69 (m, 3 H, H-3", H-4", H-5"), 7.37-7.42 (m, 3 H, H-3, H-5, H-5'), 4.09 (s, 2 H, CH₂N); ¹³C NMR δ 165.4, 144.4, 141.9, 141.7, 140.5, 135.7, 132.9, 129.1 (2), 127.6 (2), 127.3 (2), 127.2, 126.3 (2), 123.3, 45.6; MS *m*/*z* 368.4 (MH⁺, 100%). Anal. Calcd for C₁₉H₁₇N₃O₃S: C, 62.11; H, 4.66; N, 11.44. Found: C, 62.37; H, 4.82; N, 11.39%.

### Example 3 (Reference Example)

### Preparation of 4-(Phenylsulfonamidomethyl)-N-(pyridin-4-yl)benzamide (11).

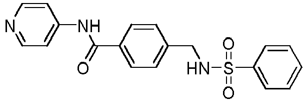

**Benzyl 4-(pyridine-4-ylcarbamoyl)benzylcarbamate (7). Method A.** Reaction of benzoic acid **2** (5.8 g, 20.3 mmol) with oxalyl chloride (2.65 mL, 30.4 mmol), with subsequent reaction with 4-aminopyridine (2.10 g, 22.3 mmol) gave carbamate **7** (2.30 g, 31%) as a pale pink solid: mp (EtOAc) 146-149 °C; ¹H NMR δ 10.51 (s, 1 H, NHCO), 8.47 (dd, *J* = 4.7, 1.6 Hz, 2 H, H-2', H-6'), 7.87-7.93 (m, 3 H, H-2, H-6, NHCO₂), 7.76 (dd, *J* = 4.7, 1.6 Hz, 2 H, H-3', H-5'), 7.42 (br d, *J* = 8.3 Hz, 2 H, H-3, H-5), 7.27-7.38 (m, 5 H, H-2", H-3", H-4", H-5", H-6"), 5.06 (s, 2 H, CH₂O), 4.29 (d, *J* = 6.0 Hz, 2 H, CH₂N). Anal. Calcd for C₂₁H₁₉N₃O₃: C, 69.79; H, 5.30; N, 11.63. Found: C, 69.92; H, 5.39; N, 11.71%.

**4-(Aminomethyl)-*N*-(4-pyridinyl)benzamide dihydrobromide (8). Method B.** Reaction of carbamate **7** (2.24 g, 6.6 mmol) gave benzamide **8** (1.98 g, 82%) as a white solid: mp (Et₂O) 280-282 °C; ¹H NMR δ 14.60 (br s, 1 H, pyrN·HBr), 11.56 (s, 1 H, NHCO), 8.80 (br d, *J* = 7.3 Hz, 2 H, H-2, H-6), 8.30-8.40 (m, 5 H, H-3', H-5', NH₂·HBr), 8.10 (br d, *J* = 8.4 Hz, 2 H, H-2, H-6), 7.69 (d, *J* = 8.4 Hz, 2 H, H-3, H-5), 4.18 (q, *J* = 5.6 Hz, 2 H, CH₂N). Anal. Calcd for C₁₃H₁₅Br₂N₃O·½H₂O: C, 39.22; H, 4.05; N, 10.55. Found: C, 39.40; H, 4.06; N, 10.48%.

**4-(Phenylsulfonamidomethyl)-*N*-(pyridin-4-yl)benzamide (11). Method** C. Reaction of amine salt **8** (394 mg, 1.0 mmol) and benzenesulfonyl chloride (0.14 mL, 1.1 mmol), followed by column chromatography eluting with a gradient (0-5%) of MeOH/EtOAc, gave benzamide **11** (232 mg, 63%) as a white powder: mp (MeOH/EtOAc) 231-234 °C; ¹H NMR δ 10.51 (s, 1 H, NHCO), 8.47 (dd, *J* = 4.9, 1.5 Hz, 2 H, H-4', H-6'), 8.26 (br s, 1 H, NHSO₂), 7.89 (br d, *J* = 8.3 Hz, 2 H, H-2, H-6), 7.83 (br d, *J* = 8.1 Hz, 2 H, H-2", H-6"), 7.78 (dd, *J* = 4.8, 1.6 Hz, 2 H, H-3', H-5'), 7.64-7.68 (m, 1 H, H-4"), 7.57-7.61 (m, 2 H, H-3", H-5"), 7.41 (br d, *J* = 8.4 Hz, 2 H, H-3, H-5), 4.10 (s, 2 H, CH₂N); ¹³C NMR δ 165.9, 150.2 (2), 145.8, 142.0, 140.5, 132.8, 132.3, 129.1 (2), 127.7 (2), 127.3 (2), 126.3 (2), 113.9 (2), 45.6; MS *m*/*z* 368.3 (MH⁺, 100%). Anal. Calcd for C₁₉H₁₇N₃O₃S: C, 62.11; H, 4.66; N, 11.44. Found: C, 62.36; H, 4.84; N, 11.47%.

### Example 4 (Reference Example)

### Preparation of 4-(Phenylsulfonamidomethyl)-N-(thiazol-2-yl)benzamide (13).

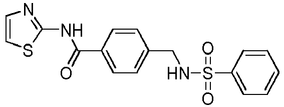

**4-(Phenylsulfonamidomethyl)benzoic Acid (12).** Benzenesulfonyl chloride (1.27 mL, 10.0 mmol) was added dropwise to a stirred solution of 4-aminomethylbenzoic acid (**1**) (1.51 g, 10 mmol) in 2 M NaOH (10 mL) at 20 °C. The mixture was stirred at 20 ° for 3 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The residue was purified by chromatography, eluting with 5% MeOH/EtOAc, to give benzoic acid **12** (2.33 g, 80%) as a white powder: mp 223-227 °C; ¹H NMR δ 12.20 (br s, 1 H, CO₂H), 8.23 (br s, 1 H, NHSO₂), 7.84 (br *d, J=* 8.3 Hz, 2 H, H-2, H-6), 7.78-7.82 (m, 2 H, H-2', H-6'), 7.60-7.64 (m, 1 H, H-4'), 7.55-7.59 (m, 2 H, H-3', H-5'), 7.34 (br d, *J=* 8.3 Hz, 2 H, H-3, H-5), 4.05 (br d, *J*= 5.8 Hz, 2 H, CH₂N). Anal. Calcd for C₁₄H₁₃NO₄S: C, 57.72; H, 4.50; N, 4.81. Found: C, 57.46; H, 4.52; N, 4.73%.

**Method A.** Reaction of benzoic acid **12** (485 mg, 1.7 mmol) and oxalyl chloride (0.22 mL, 2.5 mmol) and subsequent reaction with 2-aminothiazole (185 mg, 1.8 mmol), followed by column chromatography eluting with a gradient (50-70%) of EtOAc/pet. ether, gave benzamide **13** (221 mg, 36%) as a white powder: mp (EtOAc/pet. ether) 189-191 °C; ¹H NMR δ 12.56 (s, 1 H, NHCO), 8.25 (br s, 1 H, NHSO₂), 8.01 (br d, *J* = 8.4 Hz, 2 H, H-2, H-6), 7.82 (ddd, *J* = 8.0, 2.1, 1.6 Hz, 2 H, H-2", H-6"), 7.62-7.66 (m, 1 H, H-4"), 7.56-7.61 (m, 2 H, H-3", H-5"), 7.55 (d, *J* = 3.6 Hz, 1 H, H-4'), 7.39 (br d, *J =* 8.4 Hz, 2 H, H-3, H-5), 7.27 (t, *J* = 3.6 Hz, 1 H, H-5'), 4.09 (s, 2 H, CH₂N); ¹³C NMR δ 164.6, 158.6, 142.4, 140.6, 137.4, 132.3, 130.8, 129.1 (2), 128.0 (2), 127.4 (2), 126.3 (2), 113.7, 45.6; MS *m*/*z* 374.3 (MH⁺, 100%). Anal. Calcd for C₁₇H₁₅N₃O₃S₂: C, 54.67; H, 4.05; N, 11.25. Found: C, 54.94; H, 4.09; N, 11.30%.

### Example 5 (Reference Example)

### Preparation of 4-(Phenylsulfonamidomethyl)-N-(1H-pyrazol-3-yl)benzamide (14).

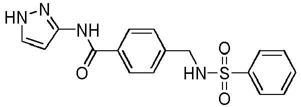

**Method A.** Reaction of benzoic acid **12** (402 mg, 1.38 mmol) and oxalyl chloride (0.18 mL, 2.1 mmol) with subsequent reaction with *1H*-pyrazol-3-amine hydrochloride (126 mg, 1.5 mmol), followed by column chromatography eluting with EtOAc, gave benzamide **14** (396 mg, 80%) as a white powder: mp (EtOAc/pet. ether) 180-182 °C; ¹H NMR δ 12.40 (br s, 1 H, NH), 10.71 (s, 1 H, NHCO), 8.22 (br s, 1 H, NHSO₂), 7.91 (br d, *J* = 8.3 Hz, 2 H, H-2, H-6), 7.82 (ddd, *J* = 8.0, 2.1, 1.5 Hz, 2 H, H-2", H-6"), 7.61-7.66 (m, 2 H, H-5', H-4"), 7.55-7.60 (m, 2 H, H-3", H-5"), 7.34 (br d, *J* = 8.3 Hz, 2 H, H-3, H-5), 6.62 (br s, 1 H, H-4'), 4.07(s, 2 H, CH₂N); ¹³C NMR δ 164.0 147.2, 141.2, 140.6, 132.9, 132.3, 129.1 (2), 128.4, 127.5 (2), 127.1 (2), 126.3 (2), 96.9, 45.6; MS *m*/*z* 357.3 (MH⁺, 100%). Anal. Calcd for C₁₇H₁₆N₄O₃S: C, 57.29; H, 4.52; N, 15.72. Found: C, 57.53; H, 4.60; N, 15.73%.

### Example 6 (Reference Example)

### Preparation of 4-(Phenylsulfonamidomethyl)-N-(quinolin-3-yl)benzamide (15).

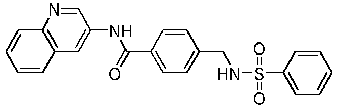

**Method A.** Reaction of benzoic acid **12** (424 mg, 1.46 mmol) and oxalyl chloride (0.19 mL, 2.2 mmol) with subsequent reaction with 3-aminoquinoline (232 mg, 1.6 mmol), followed by column chromatography eluting with EtOAc, gave benzamide **15** (528 mg, 87%) as a white powder: mp (EtOAc) 186-188 °C; ¹H NMR δ 10.63 (br s, 1 H, NHCO), 9.15 (d, *J=* 2.5 Hz, 1 H, H-2'), 8.83 (d, *J* = 2.5 Hz, 1 H, H-4'), 8.27 (br s, 1 H, NHSO₂), 7.95-8.00 (m, 3 H, H-2, H-6, H-8'), 7.84 (ddd, *J* = 8.0, 2.1, 1.6 Hz, 2 H, H-2", H-6"), 7.63-7.70 (m, 2 H, H-5', H-4"), 7.56-7.62 (m, 4 H, H-6', H-7', H-3", H-5"), 7.44 (br d, *J* = 8.3 Hz, 2 H, H-3, H-5), 4.11 (s, 2 H, CH₂N); ¹³C NMR δ 165.6, 145.4, 144.3, 141.8, 140.6, 132.9, 132.7, 132.3 (2), 129.1 (2), 128.4, 127.9, 127.6 (2), 127.3 (3), 126.9, 126.3 (2), 123.3, 45.6; MS *m*/*z* 418.5 (MH⁺, 100%). Anal. Calcd for C₂₃H₁₉N₃O₃S: C, 66.17; H, 4.59; N, 10.07. Found: C, 66.40; H, 4.58; N, 10.27%.

### Example 7 (Reference Example)

### Preparation of 4-(Phenylsulfonamidomethyl)-N-(quinolin-5-yl)benzamide (16).

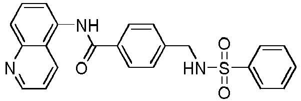

**Method A.** Reaction of benzoic acid **12** (440 mg, 1.5 mmol) and oxalyl chloride (0.20 mL, 2.3 mmol) with subsequent reaction with 5-aminoquinoline (240 mg, 1.7 mmol), followed by column chromatography eluting with EtOAc, gave benzamide **16** (426 mg, 68%) as a white powder: mp (EtOAc/pet. ether) 198-201 °C; ¹H NMR δ 10.48 (br s, 1 H, NHCO), 8.92 (dd, *J* = 4.2, 1.6 Hz, 1 H, H-2'), 8.32 (br d, *J* = 8.6 Hz, 1 H, H-4'), 8.27 (br s, 1 H, NHSO₂), 8.01 (br d, *J* = 8.3 Hz, 2 H, H-2, H-6), 7.97 (d, *J* = 8.3 Hz, 1 H, H-6'), 7.85 (ddd, *J* = 8.0, 2.1, 1.6 Hz, 2 H, H-2", H-6"), 7.80 (dd, *J* = 8.4, 7.5 Hz, 1 H, H-7'), 7.70 (dd, *J* = 7.4, 1.0 Hz, 1 H, H-8'), 7.65-7.68 (m, 1 H, H-4"), 7.58-7.63 (m, 2 H, H-3", H-5"), 7.56 (dd, *J* = 8.6, 4.2 Hz, 1 H, H-3'), 7.44 (br d, *J =* 8.3 Hz, 2 H, H-3, H-5), 4.12 (s, 2 H, CH₂N); ¹³C NMR δ 165.9, 150.4, 148.1, 141.6, 140.6, 134.0, 132.9, 132.3, 132.0, 129.1 (2), 128.8, 127.7 (2), 127.3 (2), 127.0, 126.3 (2), 124.1, 123.6, 120.9, 45.6; MS *m*/*z* 418.5 (MH⁺, 100%). Anal. Calcd for C₂₃H₁₉N₃O₃S: C, 66.17; H, 4.59; N, 10.07. Found: C, 66.40; H, 4.62; N, 10.29%.

### Example 8 (Reference Example)

### Preparation of 4-(Phenylsulfonamidomethyl)-N-(pyrazin-2-yl)benzamide (17).

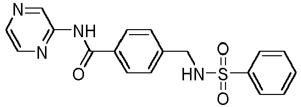

**Method A.** Reaction of benzoic acid **12** (413 mg, 1.4 mmol) and oxalyl chloride (0.19 mL, 2.1 mmol) with subsequent reaction with 2-aminopyrazine (149 mg, 1.6 mmol), followed by column chromatography eluting with EtOAc, gave benzamide **24** (398 mg, 76%) as a white powder: mp (EtOAc/pet. ether) 200-202 °C; ¹H NMR δ 11.00 (br s, 1 H, NHCO), 9.41 (d, *J* = 1.5 Hz, 1 H, H-2'), 8.47 (dd, *J* = 2.5, 1.5 Hz, 1 H, H-6'), 8.41 (d, *J* = 2.3 Hz, 1 H, H-5'), 8.20 (br s, 1 H, NHSO₂), 7.98 (br d, *J* = 8.4 Hz, 2 H, H-2, H-6), 7.84 (ddd, *J* = 7.0, 2.1, 1.6 Hz, 2 H, H-2", H-6"), 7.62-7.67 (m, 1 H, H-4"), 7.56-7.61 (m, 2 H, H-3", H-5"), 7.39 (br d, *J* = 8.4 Hz, 2 H, H-3, H-5), 4.09 (s, 2 H, CH₂N); ¹³C NMR δ 165.6, 148.9, 142.4, 142.4, 140.6, 139.8, 137.4, 132.3, 132.0, 129.1 (2), 128.1 (2), 127.2 (2), 126.3 (2), 45.6; MS *m*/*z* 369.2 (MH⁺, 100%). Anal. Calcd for C₁₈H₁₆N₄O₃S: C, 58.68; H, 4.38; N, 15.21. Found: C, 58.91; H, 4.45; N, 15.26%.

### Example 9 (Reference Example)

### Preparation of 4-(Phenylsulfonamidomethyl)-N-(pyrimidin-2-yl)benzamide (18).

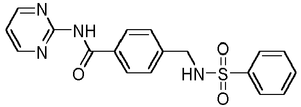

**Method A.** Reaction of benzoic acid **12** (509 mg, 1.8 mmol) and oxalyl chloride (0.23 mL, 2.6 mmol) with subsequent reaction with pyrimidin-2-amine (183 mg, 1.9 mmol), followed by column chromatography eluting with a gradient (0-5%) of MeOH/EtOAc, gave benzamide **18** (262 mg, 41%) as a white powder: mp (MeOH/EtOAc) 180-182 °C; ¹H NMR δ 10.91 (s, 1 H, NHCO), 8.72 (d, *J* = 4.8 Hz, 2 H, H-4', H-6'), 8.24 (br s, 1 H, NHSO₂), 7.89 (br d, *J* = 8.3 Hz, 2 H, H-2, H-6), 7.82 (ddd, *J* = 8.0, 2.1, 1.5 Hz, 2 H, H-2", H-6"), 7.62-7.67 (m, 1 H, H-4"), 7.56-7.61 (m, 2 H, H-3", H-5"), 7.37 (br d, *J* = 8.3 Hz, 2 H, H-3, H-5), 7.26 (t, *J* = 4.8 Hz, 1 H, H-5'), 4.08(s, 2 H, CH₂N); ¹³C NMR δ 165.0, 158.2 (2), 158.1, 141.9, 140.6, 132.9, 132.3, 129.1 (2), 128.1 (2), 127.2 (2), 126.3 (2), 117.2, 45.6; MS *m*/*z* 369.3 (MH⁺, 100%). Anal. Calcd for C₁₈H₁₆N₄O₃S: C, 58.68; H, 4.38; N, 15.21. Found: C, 58.70; H, 4.48; N, 14.91%.

### Example 10

### Preparation of 4-((2-Methylphenylsulfonamido)methyl)-N-(pyridin-3-yl)benzamide (19).

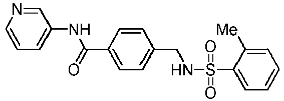

**Method C.** Reaction of amine salt **6** (400 mg, 1.0 mmol) and 2-toluenesulfonyl chloride (216 mg, 1.1 mmol) followed by column chromatography, eluting with EtOAc, gave benzamide **19** (264 mg, 67%) as a white powder: mp (EtOAc) 159-161 °C; ¹H NMR δ 10.36 (s, 1 H, NHCO), 8.93 (d, *J* = 2.3 Hz, 1 H, H-2'), 8.30-8.32 (br s, 1 H, NHSO₂), 8.31 (dd, *J* = 4.6, 1.5 Hz, 1 H, H-6'), 8.19 (d, *J* = 8.3 Hz, 1 H, H-4'), 7.89 (d, *J* = 6.7 Hz, 2 H, H-2, H-6), 7.83 (dd, *J* = 7.7, 1.1 Hz, 1 H, H-6"), 7.50 (dt, *J* = 7.5, 1.3 Hz, 1 H, H-5"), 7.34-7.41 (m, 5 H, H-3, H-5, H-5', H-3", H-4"), 4.12 (s, 2 H, CH₂N), 2.59 (s, 3 H, CH₃); ¹³C NMR δ 165.4, 144.4, 142.0, 141.9, 138.7, 136.3, 135.7, 132.9, 132.4, 132.3, 128.2, 127.5 (2), 127.2 (2), 127.2, 126.1, 123.3, 45.3, 19.7. Anal. Calcd for C₂₀H₁₉N₃O₃S: C, 62.97; H, 5.02; N, 11.02. Found: C, 63.28; H, 5.06; N, 11.13%.

### Example 11

### Preparation of 4-((2-Fluorophenylsulfonamido)methyl)-N-(pyridin-3-yl)benzamide (20).

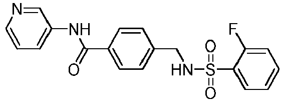

**Method C.** Reaction of amine salt **6** (442 mg, 1.1 mmol) and 2-fluorobenzenesulfonyl chloride (0.17 mL, 1.3 mmol) followed by column chromatography, eluting with EtOAc, gave benzamide **20** (219 mg, 50%) as a white powder: mp (EtOAc) 189-191 °C; ¹H NMR δ 10.35 (s, 1 H, NHCO), 8.92 (d, *J* = 2.2 Hz, 1 H, H-2'), 8.58 (br s, 1 H, NHSO₂), 8.31 (dd, *J*= 4.7, 1.5 Hz, 1 H, H-6'), 8.18 (ddd, *J* = 8.3, 2.5, 1.5 Hz, 1 H, H-4'), 7.88 (br d, *J* = 8.3 Hz, 2 H, H-2, H-6), 7.79 (dt, *J* = 7.6, 1.7 Hz, 1 H, H-6"), 7.65-7.71 (m, 1 H, H-4") 7.36-7.42, (m, 4 H, H-3, H-5, H-5', H-3"), 7.34 (dd, *J* = 7.6, 1.0 Hz, 1 H, H-5"), 4.22 (s, 2 H, CH₂N); ¹³C NMR δ 165.4, 158.0 (d, *J =* 253 Hz), 144.4, 141.9, 141.7, 135.7, 135.0 (d, *J =* 9 Hz), 132.9, 129.4, 128.6 (d, *J* = 14 Hz), 127.5 (2), 127.2 (3), 124.6 (d, *J* = 4 Hz), 123.4, 117.0 (d, *J* = 21 Hz), 45.4; MS *m*/*z* 386.4 (MH⁺, 100%). Anal. Calcd for C₁₉H₁₆FN₃O₃S: C, 59.21; H, 4.18; N, 10.90. Found: C, 59.38; H, 4.29; N, 10.85%.

### Example 12

### Preparation of 4-((2-Chlorophenylsulfonamido)methyl)-N-(pyridin-3-yl)benzamide (21).

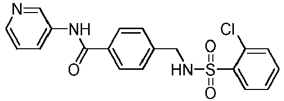

**Method C.** Reaction of amine salt **6** (424 mg, 1.1 mmol) and 2-chlorobenzenesulfonyl chloride (0.16 mL, 1.2 mmol) followed by column chromatography, eluting with EtOAc, gave benzamide **21** (165 mg, 38%) as a white powder: mp (EtOAc) 190-192 °C; ¹H NMR δ 10.35 (s, 1 H, NHCO), 8.92 (d, *J* = 2.2 Hz, 1 H, H-2'), 8.54 (br s, 1 H, NHSO₂), 8.31 (dd, *J* = 4.7, 1.5 Hz, 1 H, H-6'), 8.18 (ddd, *J* = 8.4, 2.5, 1.5 Hz, 1 H, H-4'), 7.95 (dd, *J* = 7.4, 1.2 Hz, 1 H, H-6"), 7.87 (br d, *J* = 8.3 Hz, 2 H, H-2, H-6), 7.57-7.64 (m, 2 H, H-3", H-5"), 7.47-7.52 (m, 1 H, H-4"), 7.36-7.42 (m, 3 H, H-3, H-5, H-5'), 4.21 (s, 2 H, CH₂N); ¹³C NMR δ 165.3, 144.4, 141.9, 141.7, 138.1, 135.7, 133.8, 132.9, 131.6, 130.5, 130.2, 127.5 (2), 127.4, 127.2 (2), 127.1, 123.4, 45.5; MS *m*/*z* 402.4 (MH⁺, 100%). Anal. Calcd for C₁₉H₁₆ClN₃O₃S: C, 56.79; H, 4.01; N, 10.46. Found: C, 56.90; H, 4.07; N, 10.57%.

### Example 13

### Preparation of 4-((2-Bromophenylsulfonamido)methyl)-N-(pyridin-3-yl)benzamide (22).

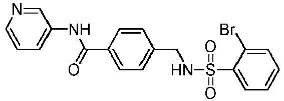

**Method C.** Reaction of amine salt **6** (400 mg, 1.0 mmol) and 2-bromobenzenesulfonyl chloride (289 mg, 1.1 mmol) followed by column chromatography, eluting with EtOAc, gave benzamide **22** (219 mg, 48%) as a cream coloured powder: mp (EtOAc) 200-202 °C; ¹H NMR δ 10.35 (s, 1 H, NHCO), 8.93 (d, *J* = 2.3 Hz, 1 H, H-2'), 8.51 (br s, 1 H, NHSO₂), 8.31 (dd, *J* = 4.7, 1.4 Hz, 1 H, H-6'), 8.18 (d, *J* = 8.3 Hz, 1 H, H-4'), 7.98 (dd, *J* = 7.6, 1.9 Hz, 1 H, H-5"), 7.88 (d, *J* = 8.3 Hz, 2 H, H-2, H-6), 7.81 (dd, *J* = 7.6, 1.4 Hz, 1 H, H-2"), 7.49-7.54 (m, 2 H, H-3", H-4"), 7.37-7.41 (m, 3 H, H-3, H-5, H-5'), 4.22 (s, 2 H, CH₂N); ¹³C NMR δ 165.4, 144.4, 141.9, 141.7, 139.8, 135.7, 135.1, 133.7, 132.9, 130.4, 128.0, 127.5 (2), 127.2 (2), 127.2, 123.4, 119.1, 45.6. Anal. Calcd for C₁₉H₁₆BrN₃O₃S: C, 51.13; H, 3.61; N, 9.41. Found: C, 51.69; H, 3.78; N, 9.52%.

### Example 14

### Preparation of Methyl 2-(N-(4-(Pyridin-3-ylcarbamoyl)benzyl)sulfamoyl)benzoate (23)

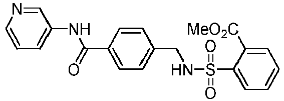

**Method C.** Reaction of amine salt **6** (400 mg, 1.0 mmol) and methyl 2-(chlorosulfonyl)benzoate (365 mg, 1.6 mmol) followed by column chromatography, eluting with EtOAc, gave the benzoate **23** (225 mg, 49%) as a white powder: mp (EtOAc) 155-157 °C; ¹H NMR δ 10.36 (s, 1 H, NHCO), 8.92 (d, *J* = 2.2 Hz, 1 H, H-2"), 8.31 (dd, *J* = 4.7, 1.5 Hz, 1 H, H-6"), 8.18 (d, *J* = 8.4 Hz, 1 H, H-4"), 8.11 (br s, 1 H, NHSO₂), 7.90-7.87 (m, 3 H, H-6, H-2', H-6'), 7.65-7.71 (m, 3 H, H-3, H-4, H-5), 7.67-7.42 (m, 3 H, H-3', H-5', H-5"), 4.20 (s, 2 H, CH₂N), 3.86 (s, 3 H, OCH₃); ¹³C NMR δ 167.7, 165.4, 144.4, 141.9, 141.8, 138.1, 135.7, 132.9, 132.4, 131.7, 130.9, 128.6, 128.1, 127.5 (2), 127.2 (2), 127.2, 123.4, 52.8, 45.5. Anal. Calcd for C₂₁H₁₉N₃O₅S: C, 59.28; H, 4.50; N, 9.88. Found: C, 59.36; H, 4.46; N, 9.77%.

### Example 15

### Preparation of N-(pyridin-3-yl)-4-((2-(trifluoromethyl)phenylsulfonamido)methyl)benzamide (24).

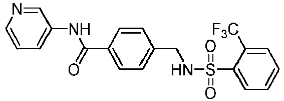

**Method C.** Reaction of amine salt **6** (398 mg, 1.0 mmol) and 2-trifluoromethylbenzenesulfonyl chloride (0.17 mL, 1.1 mmol) followed by column chromatography, eluting with EtOAc, gave benzamide **24** (149 mg, 34%) as a white powder: mp (EtOAc) 234-237 °C; ¹H NMR δ 10.36 (s, 1 H, NHCO), 8.92 (d, *J* = 2.2 Hz, 1 H, H-2'), 8.61 (br s, 1 H, NHSO₂), 8.31 (dd, *J* = 4.7, 1.5 Hz, 1 H, H-6'), 8.18 (ddd, *J* = 8.3, 2.5, 1.5 Hz, 1 H, H-4'), 8.10 (br d, *J* = 8.7 Hz, 1 H, H-6")*, 7.97 (br dd, *J* = 7.2, 1.9 Hz, 1 H, H-3")*, 7.91 (br d, *J =* 8.3 Hz, 2 H, H-2, H-6), 7.80-7.88 (m, 2 H, H-4", H-5"), 7.43 (br d, 2 H, H-3, H-5), 7.39 (ddd, *J* = 8.3, 4.7, 0.5 Hz, 1 H, H-5'), 4.25 (s, 2 H, CH₂N); ¹³C NMR δ 165.4, 144.4, 141.9, 141.7, 139.6, 135.6, 133.1, 133.0, 132.7, 129.9, 128.2 (q, *J* = 6 Hz), 127.6 (2), 127.3 (2), 127.1, 125.9 (q, *J* = 33 Hz), 123.3, 122.8 (q, *J =* 274 Hz), 45.7; MS *m*/*z* 436.5 (MH⁺, 100%). Anal. Calcd for C₂₀H₁₆F₃N₃O₃S: C, 55.17; H, 3.70; N, 9.65. Found: C, 55.40; H, 3.87; N, 9.66%. *Assignments interchangeable

### Example 16

### Preparation of 4-((2-Cyanophenylsulfonamido)methyl)-N-(pyridin-3-yl)benzamide (25).

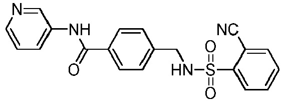

**Method C.** Reaction of amine salt **6** (414 mg, 1.1 mmol) and 2-cyanobenzenesulfonyl chloride (240 mg, 1.2 mmol) followed by column chromatography, eluting with EtOAc, gave benzamide **25** (140 mg, 34%) as a white powder: mp (EtOAc) 217-220 °C; ¹H NMR δ 10.36 (s, 1 H, NHCO), 8.92 (d, *J* = 2.2 Hz, 1 H, H-2'), 8.58 (br s, 1 H, NHSO₂), 8.31 (dd, *J*= 4.7, 1.5 Hz, 1 H, H-6'), 8.15-8.19 (m, 2 H, H-4', H-6"), 8.05 (ddd, *J* = 8.6, 7.6, 1.2 Hz, 1 H, H-5"), 7.91-7.96 (m, 1 H, H-3"), 7.83-7.88 (m, 3 H, H-2, H-6, H-4"), 7.35-7.42, (m, 3 H, H-3, H-5, H-5'), 4.28 (s, 2 H, CH₂N); MS *m*/*z* 393.3 (MH⁺, 100%). Anal. Calcd for C₂₀H₁₆N₄O₃S: C, 61.21; H, 4.11; N, 14.28. Found: C, 61.28; H, 4.08; N, 14.19%.

### Example 17

### Preparation of 4-((3-Aminophenylsulfonamido)methyl)-N-(pyridin-3-yl)benzamide (26).

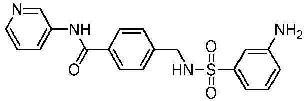

A mixture of nitrophenylbenzamide **34** (252 mg, 0.6 mmol) and Pd/C (50 mg, catalytic) in EtOH/EtOAc (1:1, 80 mL) was stirred under H₂ (50 psi) at 20 °C for 2 h. The mixture was filtered through Celite, the Celite was washed with EtOH (20 mL), and the solvent was evaporated. The residue was purified by column chromatography, eluting with a gradient (0-5%) of MeOH/EtOAc, to give benzamide **26** (166 mg, 71%) as a white powder: mp (EtOH/EtOAc) 211-213 °C; ¹H NMR δ 10.39 (s, 1 H, NHCO), 8.93 (d, *J*= 2.3 Hz, 1 H, H-2'), 8.31 (dd, *J*= 4.7, 1.5 Hz, 1 H, H-6'), 8.19 (ddd, *J* = 8.3, 2.5, 1.5 Hz, 1 H, H-4'), 8.03 (br s, 1 H, NHSO₂), 7.92 (br d, *J* = 8.3 Hz, 2 H, H-2, H-6), 7.44 (br d, *J* = 8.3 Hz, 2 H, H-3, H-5), 7.39 (dd, *J* = 8.3, 4.7 Hz, 1 H, H-5'), 7.20 (br t, *J* = 7.9 Hz, 1 H, H-5"), 7.04 (br t, *J* = 2.0 Hz, 1 H, H-2"), 6.93 (br ddd, *J* = 7.6, 1.6, 0.8 Hz, 1 H, H-6"), 6.77 br ddd, *J* = 8.0, 2.2, 0.8 Hz, 1 H, H-4"), 5.56 (s, 2 H, NH₂), 4.06 (s, 2 H, CH₂N); ¹³C NMR δ 165.5, 149.3, 144.4, 142.0, 141.9, 140.9, 135.7, 132.9, 129.4, 127.6 (2), 127.3 (2), 127.2, 123.4, 117.1, 113.0, 111.0, 45.7; MS *m*/*z* 383.4 (MH⁺, 100%). Anal. Calcd for C₁₉H₁₈N₄O₃S: C, 59.67; H, 4.74; N, 14.65. Found: C, 59.48; H, 4.92; N, 14.45%.

### Example 18

### Preparation of 4-((3-Methoxyphenylsulfonamido)methyl)-N-(pyridin-3-yl)benzamide (27).

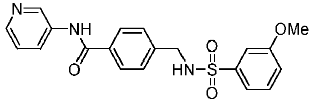

**Method C.** Reaction of amine salt **6** (477 mg, 1.2 mmol) and 3-methoxybenzenesulfonyl chloride (0.19 mL, 1.4 mmol) followed by column chromatography, eluting with a gradient (50-100%) of EtOAc/pet. ether, gave benzamide **27** (247 mg, 51%) as a white powder: mp (MeOH/EtOAc) 148-151 °C; ¹H NMR δ 10.37 (s, 1 H, NHCO), 8.92 (d, *J* = 2.2 Hz, 1 H, H-2'), 8.31 (dd, *J* = 4.7, 1.5 Hz, 1 H, H-6'), 8.25 (br s, 1 H, NHSO₂), 8.17 (ddd, *J*= 8.3, 2.5, 1.5 Hz, 1 H, H-4'), 7.90 (br d, *J* = 8.3 Hz, 2 H, H-2, H-6), 7.51 (dd, *J* = 8.1, 7.9 Hz, 1 H, H-5"), 7.37-7.43 (m, 4 H, H-3, H-5, H-5', H-6"), 7.30 (br dd, *J* = 2.5, 1.7 Hz, 1 H, H-2"), 7.20 (ddd, *J* = 8.3, 2.6, 0.9 Hz, 1 H, H-4"), 4.09 (s, 2 H, CH₂N), 3.82 (s, 3 H, OCH₃); ¹³C NMR δ 165.4, 159.3, 144.4, 141.9, 141.7, 141.6, 135.7, 132.9, 130.3, 128.0 (2), 127.3 (2), 127.2, 123.4, 118.4, 118.2, 111.4, 55.4, 45.6; MS *m*/*z* 398.4 (MH⁺, 100%). Anal. Calcd for C₂₀H₁₉N₃O₄S: C, 60.44; H, 4.82; N, 10.57. Found: C, 60.70; H, 4.90; N, 10.53%.

### Example 19

### Preparation of 4-((3-Methylphenylsulfonamido)methyl)-N-(pyridin-3-yl)benzamide (28)

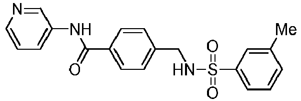

**Method C.** Reaction of amine salt **6** (400 mg, 1.0 mmol) and 3-toluenesulfonyl chloride (216 mg, 1.1 mmol) followed by column chromatography, eluting with EtOAc, to give benzamide **28** (250 mg, 64%) as a white powder, mp (EtOAc) 180-182 °C; ¹H NMR δ 10.37 (s, 1 H, NHCO), 8.93 (d, *J =* 2.3 Hz, 1 H, H-2'), 8.31 (dd, *J* = 4.7, 1.5 Hz, 1 H, H-6'), 8.17-8.20 (m, 2 H, NHSO₂, H-4'), 7.91 (d, *J* = 8.3 Hz, 2 H, H-2, H-6), 7.60-7.64 (m, 2 H, H-2", H-6"), 7.37-7.48 (m, 5 H, H-3, H-5, H-5', H-4", H-5"), 4.09 (d, *J* = 6.3 Hz, 2 H, CH₂N), 2.38 (s, 3 H, CH₃); ¹³C NMR δ 165.4, 144.4, 141.9, 141.8, 140.5, 138.7, 135.7, 132.9, 132.9, 128.9, 127.5 (2), 127.3 (2), 127.2, 126.6, 123.5, 123.4, 45.6, 20.7. Anal. Calcd for C₂₀H₁₉N₃O₃S: C, 62.97; H, 5.02; N, 11.02. Found: C, 63.19; H, 5.05; N, 11.05%.

### Example 20

### Preparation of 4-((3-Fluorophenyisulfonamido)methyl)-N-(pyridin-3-yl)benzamide (29).

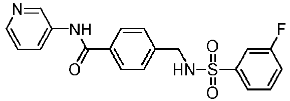

**Method C.** Reaction of amine salt **6** (400 mg, 1.0 mmol) and 3-fluorobenzenesulfonyl chloride (220 mg, 1.1 mmol) followed by column chromatography, eluting with EtOAc gave benzamide **29** (230 mg, 58%) as a white powder: mp (EtOAc) 181-183 °C; ¹H NMR δ 10.37 (s, 1 H, NHCO), 8.93 (d, *J* = 2.3 Hz, 1 H, H-2'), 8.40 (br s, 1 H, NHSO₂), 8.31 (dd, *J* = 4.7, 1.5 Hz, 1 H, H-6'), 8.18 (d, *J* = 8.3 Hz, 1 H, H-4'), 7.91 (d, *J =* 8.3 Hz, 2 H, H-2, H-6), 7.51-7.67 (m, 4 H, H-2", H-4", H-5", H-6"), 7.37-7.42 (m, 3 H, H-3, H-5, H-5), 4.14 (s, 2 H, CH₂N); ¹³C NMR δ 165.4, 161.6 (d, *J* = 248 Hz), 144.4, 142.7 (d, *J* = 7 Hz), 141.9, 141.5, 135.7, 133.0, 131.5 (d, *J* = 8 Hz), 127.6 (2), 127.3 (2), 127.2, 123.4, 122.6 (d, *J* = 3 Hz), 119.4 (d, *J* = 21 Hz), 113.4 (d, *J* = 24 Hz), 45.6. Anal. Calcd for C₁₉H₁₆FN₃O₃S: C, 59.21; H, 4.18; N, 10.90. Found: C, 59.24; H, 4.14; N, 10.73%.

### Example 21

### Preparation of 4-((3-Chlorophenylsulfonamido)methyl)-N-(pyridin-3-yl)benzamide (30).

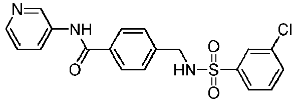

**Method C.** Reaction of amine salt **6** (798 mg, 2.1 mmol) and 3-chlorobenzenesulfonyl chloride (0.32 mL, 2.3 mmol) followed by column chromatography, eluting with a gradient (50-100%) of EtOAc/pet. ether, gave benzamide **30** (455 mg, 55%) as a white powder: mp (EtOAc) 181-183 °C; ¹H NMR δ 10.36 (s, 1 H, NHCO), 8.92 (d, *J* = 2.5 Hz, 1 H, H-2'), 8.42 (br s, 1 H, NHSO₂), 8.31 (dd, *J* = 4.7, 1.5 Hz, 1 H, H-6'), 8.18 (ddd, *J* = 8.3, 2.5, 1.5 Hz, 1 H, H-4'), 7.91 (br d, *J* = 8.3 Hz, 2 H, H-2, H-6), 7.76-7.79 (m, 2 H, H-2", H-4"), 7.71 (dt, *J* = 8.2, 1.6 Hz, 1 H, H-6"), 7.62 (br t, *J* = 8.2 Hz, 1 H, H-5"), 7.36-7.43 (m, 3 H, H-3, H-5, H-5'), 4.14 (s, 2 H, CH₂N); ¹³C NMR δ 165.34, 144.4, 142.5, 141.9, 141.4, 135.7, 133.6, 133.0, 132.2, 131.0, 127.6 (2), 127.4 (2), 127.2, 126.0, 125.0, 123.4, 45.6; MS *m*/*z* 402.3 (MH⁺, 100%). Anal. Calcd for C₁₉H₁₆ClN₃O₃S: C, 56.79; H, 4.01; N, 10.46. Found: C, 56.74; H, 4.18; N, 10.51%.

### Example 22

### Preparation of 4-((3-Bromophenylsulfonamido)methyl)-N-(pyridin-3-yl)benzamide (31).

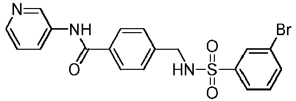

**Method C.** Reaction of amine salt **6** (392 mg, 1.0 mmol) and 3-bromobenzenesulfonyl chloride (0.16 mL, 1.1 mmol) followed by column chromatography, eluting with EtOAc, gave benzamide **31** (271 mg, 60%) as a white powder: mp (MeOH/EtOAc) 181-184 °C; ¹H NMR δ 10.36 (s, 1 H, NHCO), 8.92 (d, *J* = 2.4 Hz, 1 H, H-2'), 8.41 (br s, 1 H, NHSO₂), 8.31 (dd, *J* = 4.7, 1.5 Hz, 1 H, H-6'), 8.18 (ddd, *J* = 8.3, 2.4, 1.5 Hz, 1 H, H-4'), 7.89-7.93 (m, 3 H, H-2, H-6, H-2"), 7.78-7.85 (m, 2 H, H-4", H-6"), 7.55 (t, *J* = 7.9 Hz, 1 H, H-5"), 7.36-7.43 (m, 3 H, H-3, H-5, H-5'), 4.14 (s, 2 H, CH₂N); ¹³C NMR δ 165.3, 144.4, 142.6, 141.9, 141.4, 135.7, 135.1, 133.0, 131.3, 128.8, 127.6 (2), 127.4 (2), 127.2, 125.4, 123.4, 122.0, 45.6; MS *m*/*z* 446.1, 446.2 (MH⁺, 100%). Anal. Calcd for C₁₉H₁₆BrN₃O₃S: C, 51.13; H, 3.61; N, 9.41. Found: C, 51.40; H, 3.75; N, 9.48%.

### Example 23

### Preparation of N-(Pyridin-3-yl)-4-((3-(trifluoromethyl)phenylsulfonamido)methyl)benzamide (32).

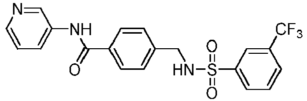

**Method C.** Reaction of amine salt **6** (588 mg, 1.5 mmol) and 3-trifluoromethylbenzenesulfonyl chloride (0.27 mL, 1. 7 mmol) followed by column chromatography, eluting with a gradient (50-100%) of EtOAc/pet. ether, gave benzamide **32** (280 mg, 43%) as a white powder: mp (MeOH/EtOAc) 200-201 °C; ¹H NMR δ 10.34 (s, 1 H, NHCO), 8.92 (d, *J* = 2.4 Hz, 1 H, H-2'), 8.52 (br s, 1 H, NHSO₂), 8.31 (dd, *J* = 4.7, 1.5 Hz, 1 H, H-6'), 8.18 (ddd, *J* = 8.4, 2.5, 1.5 Hz, 1 H, H-4'), 8.10 (br d, *J* = 7.9 Hz, 1 H, H-4"), 7.98-8.03 (m, 2 H, H-2", H-6"), 7.88 (br d, *J* = 8.3 Hz, 2 H, H-2, H-6), 7.83 (br t, *J* = 7.8 Hz, 1 H, H-5"), 7.39 (dd, *J* = 8.3, 0.6 Hz, 1 H, H-5'), 7.38 (br d, *J* = 8.3 Hz, 2 H, H-3, H-5), 4.17 (s, 2 H, CH₂N); ¹³C NMR δ 165.2, 144.2, 141.9, 141.8, 141.2, 135.6, 132.9, 130.7, 130.4, 129.7 (q, *J* = 33 Hz), 128.9 (q, *J* = 3 Hz), 127.6 (2), 127.4 (2), 127.2, 123.3, 123.2 (q, *J* = 273 Hz), 122.8 (q, *J* = 4 Hz), 45.6; MS *m*/*z* 436.5 (MH⁺, 100%). Anal. Calcd for C₂₀H₁₆F₃N₃O₃S: C, 55.17; H, 3.70; N, 9.65. Found: C, 55.40; H, 3.78; N, 9.69%.

### Example 24

### Preparation of 4-((3-Cyanophenylsulfonamido)methyl)-N-(pyridin-3-yl)benzamide (33).

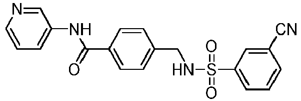

**Method C.** Reaction of amine salt **6** (419 mg, 1.1 mmol) and 3-cyanobenzenesulfonyl chloride (239 mg, 1.2 mmol) followed by column chromatography, eluting with EtOAc, gave benzamide **33** (266 mg, 63%) as a white powder: mp (MeOH/EtOAc) 214-217 °C; ¹H NMR δ 10.36 (s, 1 H, NHCO), 8.92 (d, *J* = 2.2 Hz, 1 H, H-2'), 8.49 (br s, 1 H, NHSO₂), 8.31 (dd, *J* = 4.7, 1.5 Hz, 1 H, H-6'), 8.18 (ddd, *J* = 8.3, 2.5, 1.5 Hz, 1 H, H-4'), 8.15 (t, *J* = 1.5 Hz, 1 H, H-2"), 8.07-8.12 (m, 2 H, H-4", H-6"), 7.90 (br d, *J* = 8.3 Hz, 2 H, H-2, H-6), 7.79 (dd, *J* = 8.0, 7.9 Hz, 1 H, H-5"), 7.36-7.41 (m, 3 H, H-3, H-5, H-5'), 4.17 (s, 2 H, CH₂N); ¹³C NMR δ 165.3, 144.4, 141.9 (2), 141.3, 135.8, 135.6, 133.0, 130.8, 130.6, 129.9, 127.6 (2), 127.4 (2), 127.2, 123.4, 117.4, 112.3, 45.6; MS *m*/*z* 393.4 (MH⁺, 100%). Anal. Calcd for C₂₀H₁₆N₄O₃S: C, 61.21; H, 4.11; N, 14.28. Found: C, 61.04; H, 4.19; N, 14.00%.

### Example 25

### Preparation of 4-((3-Nitrophenylsulfonamido)methyl)-N-(pyridin-3-yl)benzamide (34).

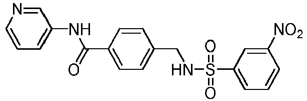

**Method C.** Reaction of amine salt **6** (940 mg, 2.4 mmol) and 3-nitrobenzenesulfonyl chloride (589 mg, 2.7 mmol) followed by column chromatography, eluting with a gradient (0-5%) of MeOH/EtOAc, gave benzamide **34** (588 mg, 59%) as a white powder: mp (MeOH/EtOAc) 228-230 °C; ¹H NMR δ 10.33 (s, 1 H, NHCO), 8.92 (d, *J* = 2.1 Hz, 1 H, H-2'), 8.63 (br s, 1 H, NHSO₂), 8.41-8.48 (m, 2 H, H-2", H-4"), 8.31 (dd, *J* = 4.7, 1.5 Hz, 1 H, H-6'), 8.15-8.21 (m, 2 H, H-4', H-6"), 7.83-7.90 (m, 3 H, H-2, H-6, H-5"), 7.36-7.41 (m, 3 H, H-3, H-5, H-3'), 4.18 (s, 2 H, CH₂N); ¹³C NMR δ 165.2, 147.7, 144.4, 142.3, 141.9, 141.0, 135.6, 133.0, 132.4, 131.1, 127.6 (2), 127.5 (2), 127.2, 126.8, 123.3, 121.6, 45.7; MS *m*/*z* 413.5 (MH⁺, 100%). Anal. Calcd for C₁₉H₁₆N₄O₅S: C, 55.33; H, 3.91; N, 13.58. Found: C, 55.57; H, 4.07; N, 13.54%.

### Example 26

### Preparation of 4-{[([1,1'-Biphenyl]-3-ylsulfonyl)amino]methyl}-N-(3-pyridinyl)benzamide (35).

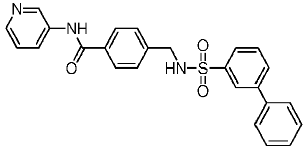

**Method C.** Reaction of amine salt **6** (150 mg, 0.39 mmol) and 3-phenylbenzenesulfonyl chloride (107 mg, 0.42 mmol) followed by column chromatography, eluting with a gradient (0-5%) of MeOH/DCM, gave benzamide **35** (88 mg, 52%) as a white powder: mp 180-182 °C; ¹H NMR δ 10.33 (s, 1 H, NHCO), 8.91 (d, *J* = 2.1 Hz, 1 H, H-2'), 8.31 (dd, *J* = 4.7, 1.5 Hz, 2 H, H-6', NHSO₂), 8.17 (ddd, *J* = 8.3, 2.5, 1.5 Hz, 1 H, H-4'), 8.02 (t, *J* = 1.7 Hz, 1 H, H-2"), 7.92 (ddd, *J* = 7.8, 1.7, 1.1 Hz, 1 H, H-4"), 7.89 (br d, *J* = 8.3 Hz, 2 H, H-2, H-6), 7.81 (ddd, *J* = 7.8, 1.7, 1.1 Hz, 1 H, H-6"), 7.66-7.70 (m, 3 H, H-5", H-2"', H-6"'), 7.51 (t, *J* = 7.5 Hz, 2 H, H-3"', H-5"'), 7.37-7.45 (m, 4 H, H-3, H-5, H-5', H-4"'), 4.15 (s, 2 H, CH₂N); ¹³C NMR δ 165.3, 144.4, 141.9, 141.7, 141.3, 141.0, 138.5, 135.6, 132.9, 130.5, 129.8, 129.0 (2), 128.1, 127.5 (2), 127.3 (2), 127.2, 126.8 (2), 125.2, 124.3, 123.3, 45.7; MS *m*/*z* 445.0 (MH⁺, 100%); HRMS (FAB⁺) calcd for C₂₅H₂₂N₃O₃S (MH⁺) *m*/*z* 444.1382, found 444.1381. Anal. calcd for C₂₅H₂₁N₃O₃S: C, 67.70; H, 4.77; N, 9.47. Found: C, 67.23; H, 4.81; N, 9.55%.

### Example 27

### Preparation of 4-[({[3-(2-Pyrimidinyl)phenyl]sulfonyl}amino)methyl]-N-(3-pyridinyl)benzamide (36).

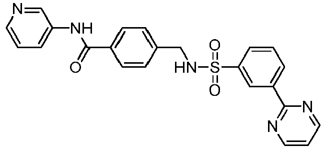

**Method C.** Reaction of amine salt **6** (175 mg, 0.45 mmol) and 3-pyrimidine-2-ylbenzenesulfonyl chloride (150 mg, 0.59 mmol) gave benzamide **36** (136 mg, 68%) as a dark yellow powder: mp 203-206 °C; ¹H NMR δ 10.31 (s, 1 H, NHCO), 8.96 (d, *J* = 4.9 Hz, 2 H, H-4"', H-6"'), 8.91 (d, *J =* 2.3 Hz, 1 H, H-2'), 8.82 (t, *J* = 1.7 Hz, 1 H, H-2"), 8.62 (dt, *J* = 8.0, 1.3 Hz, 1 H, H-6" or H-4"), 8.41 (t, *J* = 6.2 Hz, 1 H, NHSO₂), 8.31 (dd, *J* = 4.7, 1.4 Hz, 1 H, H-6'), 8.17 (ddd, *J* = 8.4, 2.5, 1.5 Hz, 1 H, H-4'), 7.97 (ddd, *J* = 7.8, 1.8, 1.1 Hz, 1 H, H-4" or H-6"), 7.87 (br d, *J* = 8.3 Hz, 2 H, H-2, H-6), 7.75 (t, *J* = 7.8 Hz, 1 H, H-5"), 7.52 (t, *J =* 4.9 Hz, 1 H, H-5"'), 7.37-7.42 (m, 3 H, H-3, H-5, H-5'), 4.14 (s, 2 H, CH₂N); ¹³C NMR δ 165.3, 161.8, 157.8 (2), 144.4, 141.9, 141.5, 141.3, 138.0, 135.6, 132.9, 131.0, 129.7, 128.5, 127.6 (2), 127.3 (2), 127.2, 125.5, 123.3, 120.5, 45.7; MS *m*/*z* 446.9 (MH⁺, 100%); HRMS (FAB⁺) calcd for C₂₃H₂₀N₅O₃S (MH⁺) *m*/*z* 446.1287, found 446.1286. Anal. calcd for C₂₃H₁₉N₅O₃S¼CH₃OH: C, 61.58; H, 4.45; N, 15.44. Found: C, 61.35; H, 4.45; N 15.58 %.

### Example 28

### Preparation of 4-[({[3-(1-Methyl-1H-pyrazol-3-yl)phenyl]sulfonyl}amino)methyl]-N-(3-pyridinyl)benzamide (37).

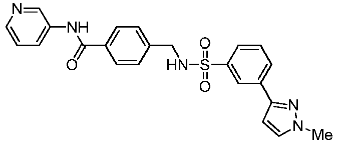

**Method C.** Reaction of amine salt **6** (160 mg, 0.41 mmol) and 3-(1-methyl-1*H-*pyrazol-3-yl)benzenesulfonyl chloride (137 mg, 0.54 mmol) followed by column chromatography, eluting with a gradient (0-5%) of MeOH/DCM, gave benzamide **44** (141 mg, 77%) as a white powder: mp 188-191 °C; ¹H NMR δ 10.34 (s, 1 H, NHCO), 8.92 (br s, 1 H, H-2'), 8.28-8.31 (m, 2 H, H-6', NHSO₂), 8.23 (t, *J* = 1.6 Hz, 1 H, H-2"), 8.18 (ddd, *J* = 8.4, 2.4, 1.5 Hz, 1 H, H-4'), 8.01 (dt, *J* = 8.0, 1.3 Hz, 1 H, H-4" or H-6"), 7.90 (br d, *J*= 8.3 Hz, 2 H, H-2, H-6), 7.78 (d, *J*= 2.3 Hz, 1 H, H-5"'), 7.72 (ddd, *J* = 7.8, 1.8, 1.1 Hz, 1 H, H-6" or H-4"), 7.61 (t, *J* = 7.8 Hz, 1 H, H-5"), 7.37-7.43 (m, 3 H, H-3, H-5, H-5'), 6.77 (d, *J* = 2.3 Hz, 1 Hz, H-4"'), 4.12 (s, 2 H, CH₂N), 3.91 (s, 3 H, CH₃); ¹³C NMR δ 165.4, 148.4, 144.3, 141.8, 141.7, 141.0, 135.7, 134.3, 132.9, 132.6, 129.5, 128.6, 127.6 (2), 127.3 (2), 127.2, 125.0, 123.4, 122.4, 102.9, 45.6, 38.6; MS *m*/*z* 449.0 (MH⁺, 100%); HRMS (FAB⁺) calcd for C₂₃H₂₂N₅O₃S (MH⁺) *m*/*z* 448.1443, found 448.1442. Anal. calcd for C₂₃H₂₁N₅O₃S·½CH₃OH: C, 60.89; H, 5.00; N, 15.11. Found: C, 60.93; H, 4.70; N 15.49 %

### Example 29

### Preparation of 4-[({[3-(5-Methyl-1,3,4-oxadiazol-2-yl)phenyl]sulfonyl}amino)methyl]-N-(3-pyridinyl)benzamide (38).

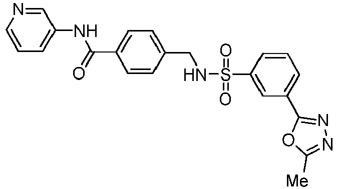

**Method C.** Reaction of amine salt **6** (150 mg, 0.39 mmol) and 3-(5-methyl-1,3,4-oxadiazol-2-yl)benzenesulfonyl chloride (150 mg, 0.58 mmol) followed by column chromatography, eluting with a gradient (0-10%) of MeOH/DCM, gave benzamide **38** (45 mg, 26%) as a white powder: mp (MeOH/DCM) 227-230 °C; ¹H NMR δ 10.30 (s, 1 H, NHCO), 8.91 (d, *J* = 2.0 Hz, 1 H, H-2'), 8.50 (t, *J* = 6.3 Hz, 1 H, NHSO₂), 8.31 (d, *J=* 3.8 Hz, 1 H, H-6'), 8.24 (t, *J=* 1.6 Hz, 1 H, H-2"), 8.15-8.19 (m, 2 H, H-4', H-4" or H-6"), 7.99-8.02 (m, 1 H, H-6" or H-4"), 7.86 (br d, *J* = 8.3 Hz, 2 H, H-2, H-6), 7.79 (t, *J =* 7.9 Hz, 1 H, H-5"), 7.37-7.43 (m, 3 H, H-3, H-5, H-5'), 4.16 (d, *J* = 6.3 Hz, 2 H, CH₂N), 2.59 (s, 3 H, CH₃); ¹³C NMR δ 165.2, 164.3, 144.3, 141.9, 141.8, 141.2, 132.8, 130.5, 129.7, 129.2, 127.5 (2), 127.4 (2), 127.3, 127.0, 124.3, 124.0, 123.4, 45.7, 10.5, one C not observed; MS *m*/*z* 450.9 (MH⁺, 100%); HRMS (FAB⁺) calcd for C₂₂H₂₀N₅O₄S (MH⁺) *m*/*z* 450.1236, found 450.1238. Anal. calcd for C₂₂H₁₉N₅O₄S·½CH₃OH: C, 58.06; H, 4.55; N, 15.04. Found: C, 58.13; H, 4.30; N 15.01%.

### Example 30

### Preparation of 4-[({[3-(5-Methyl-1,2,4-oxadiazol-3-yl)phenyl]sulfonyl}amino)methyl]-N-(3-pyridinyl)benzamide (39).

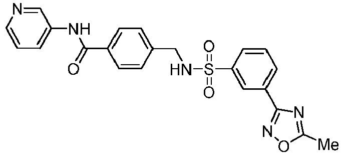

**Method C.** Reaction of amine salt **6** (163 mg, 0.42 mmol) and 3-(5-methyl-1,2,4-oxadiazol-3-yl)benzenesulfonyl chloride (130 mg, 0.50 mmol) followed by column chromatography, eluting with a gradient (0-6%) of MeOH/DCM, gave benzamide **39** (115 mg, 61%) as a cream powder: mp 182-185 °C; ¹H NMR δ 10.30 (s, 1 H, NHCO), 8.91 (d, *J* = 2.1 Hz, 1 H, H-2'), 8.47 (br s, 1 H, NHSO₂), 8.30-8.33 (m, 2 H, H-6', H-2"), 8.21 (m, 1 H, H-4" or H-6"), 8.17 (ddd, *J* = 8.3, 2.5, 1.5 Hz, 1 H, H-4'), 7.99 (ddd, *J* = 7.9, 1.8, 1.2 Hz, 1 H, H-6" or H-4"), 7.86 (br d, *J* = 8.3 Hz, 2 H, H-2, H-6), 7.77 (t, *J* = 7.8 Hz, 1 H, H-5"), 7.37-7.41 (m, 3 H, H-3, H-5, H-5'), 4.15 (s, 2 H, CH₂N), 2.67 (s, 3 H, CH₃); ¹³C NMR δ 177.8, 166.5, 165.2, 144.4, 141.9, 141.7, 141.3, 135.6, 132.8, 130.3, 130.2, 129.1, 127.5 (2), 127.4 (2), 127.2, 127.1, 124.7, 123.3, 45.7, 11.9; MS *m*/*z* 450.9 (MH⁺, 100%). Anal. calcd for C₂₂H₁₉N₅O₄S: C, 58.79; H, 4.26; N, 15.58. Found: C, 58.90; H, 4.54; N, 15.36%.

### Example 31

### Preparation of 4-[({[3-(2-Methyl-1,3-thiazol-4-yl)phenyl]sulfonyl}amino)methyl]-N-(3-pyridinyl)benzamide (40).

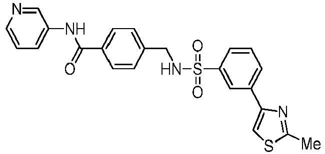

**Method C.** Reaction of amine salt **6** (165 mg, 0.42 mmol) and 3-(2-methyl-1,3-thiazol-4-yl)benzenesulfonyl chloride (150 mg, 0.55 mmol) followed by column chromatography, eluting with a gradient (0-10%) of MeOH/DCM, gave benzamide **40** (112 mg, 57%) as a white powder: mp 200-202 °C; ¹H NMR δ 10.33 (s, 1 H, NHCO), 8.92 (d, *J* = 2.4 Hz, 1 H, H-2'), 8.36 (t, *J* = 1.6 Hz, 1 H, H-2"), 8.30-8.32 (m, 2 H, H-6', NHSO₂), 8.16-8.19 (m, 2 H, H-4', H-4" or H-6"), 8.08 (s, 1 H, H-5"'), 7.89 (br d, *J* = 8.3 Hz, 2 H, H-2, H-6), 7.77 (m, 1 H, H-6" or H-4"), 7.64 (t, *J* = 7.8 Hz, 1 H, H-5"), 7.37-7.42 (m, 3 H, H-3, H-5, H-5'), 4.13 (s, 2 H, CH₂N), 2.73 (s, 3 H, CH₃); ¹³C NMR δ 166.0, 165.3, 152.0, 144.4, 141.9, 141.6, 141.1, 135.7, 134.9, 132.9, 129.6 (2), 129.3 (2), 127.6, 127.3, 127.2, 125.5, 123.7, 123.3, 115.4, 45.6, 18.8; MS *m*/*z* 466.0 (MH⁺, 100%). Anal. calcd for C₂₃H₂₀N₄O₃S₂: C, 59.46; H, 4.34; N, 12.06. Found: C, 59.39; H, 4.37; N 12.07%.

### Example 32

### Preparation of 4-((4-Aminophenylsulfonamido)methyl)-N-(pyridin-3-yl)benzamide (41).

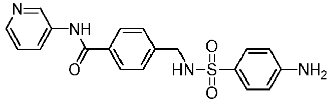

A mixture of nitrophenylbenzamide **60** (296 mg, 0.72 mmol) and Pd/C (50 mg, catalytic) in EtOH/EtOAc (1:1, 80 mL) was stirred under H₂ (50 psi) at 20 °C for 2 h. The mixture was filtered through Celite, the Celite was washed with EtOH (20 mL), and the solvent was evaporated. The residue was purified by column chromatography, eluting with a gradient (0-5%) of MeOH/EtOAc, to give benzamide **41** (210 mg, 76%) as a white powder: mp (EtOH/EtOAc) 219-221 °C; ¹H NMR δ 10.37 (s, 1 H, NHCO), 8.93 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 7.92 (br d, *J* = 8.3 Hz, 2 H, H-2, H-6), 7.72 (br t, *J* = 6.4 Hz, 1 H, NHSO₂), 7.47 (ddd, *J* = 8.7, 2.6, 1.8 Hz, 2 H, H-2", H-6"), 7.42 (br d, *J* = 8.3 Hz, 2 H, H-3, H-5), 7.38 (dd, *J* = 8.3, 4.7 Hz, 1 H, H-5'), 6.62 (ddd, *J* = 8.7, 2.6, 1.8 Hz, 2 H, H-3", H-5"), 5.92 (br s, 2 H, NH₂), 3.98 (d, *J* = 6.4 Hz, 2 H, CH₂N); ¹³C NMR δ 165.5, 152.4, 144.4, 142.4, 141.9, 135.7, 132.8, 128.4 (2), 127.5 (2), 127.3 (2), 127.2, 125.4, 123.3, 112.6 (2), 45.6; MS *m*/*z* 383.4 (MH⁺, 100%). Anal. Calcd for C₁₉H₁₈N₄O₃S·1½H₂O: C, 55.75; H, 5.17; N, 13.68. Found: C, 55.79; H, 4.71; N, 13.46%.

### Example 33

### Preparation of 4-((4-Methoxyphenylsulfonamido)methyl)-N-(pyridin-3-yl)benzamide (42).

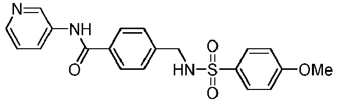

**Method C.** Reaction of amine salt **6** (400 mg, 1.0 mmol) and 4-methoxybenzenesulfonyl chloride (234 mg, 1.1 mmol) followed by column chromatography, eluting with EtOAc, gave benzamide **42** (311 mg, 76%) as a white powder: mp (EtOAc) 208-210 °C; ¹H NMR δ 10.37 (s, 1 H, NHCO), 8.93 (d, *J* = 2.2 Hz, 1 H, H-2'), 8.31 (dd, *J* = 4.6, 1.5 Hz, 1 H, H-6'), 8.18 (d, *J* = 8.3 Hz, 1 H, H-4'), 8.08 (br s, 1 H, NHSO₂), 7.90 (d, *J* = 8.4 Hz, 2 H, H-2, H-6), 7.75 (dd, *J* = 8.9, 3.0 Hz, 2 H, H-2", H-6"), 7.37-7.42 (m, 3 H, H-3, H-5, H-5'), 7.10 (dd, *J* = 8.9, 3.0 Hz, 2 H, H-3", H-5"), 4.05 (br s, 2 H, CH₂N), 3.83 (s, 3 H, OCH₃); ¹³C NMR δ 165.4, 162.0, 144.4, 141.9, 141.8, 135.7, 132.9, 132.2, 128.5 (2), 127.6 (2), 127.3 (2), 127.2, 123.4, 114.2 (2), 55.5, 45.6. Anal. Calcd for C₂₀H₁₉N₃O₄S: C, 60.44; H, 4.82; N, 10.57. Found: C, 60.73; H, 4.91; N, 10.65%.

### Example 34

### Preparation of 4-((4-Butoxyphenylsulfonamido)methyl)-N-(pyridin-3-yl)benzamide (43).

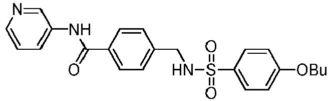

**Method C.** Reaction of amine salt **6** (395 mg, 1.0 mmol) and 4-butoxybenzenesulfonyl chloride (277 mg, 1.1 mmol) followed by column chromatography, eluting with a gradient (50-100%) of EtOAc/pet. ether, gave benzamide **43** (319 mg, 71%) as a white powder: mp (EtOAc/pet. ether) 189-191 °C; ¹H NMR δ 10.36 (s, 1 H, NHCO), 8.92 (d, *J* = 2.0 Hz, 1 H, H-2'), 8.31 (dd, *J* = 4.7, 1.5 Hz, 1 H, H-6'), 8.19 (ddd, *J* = 8.3, 2.5, 1.5 Hz, 1 H, H-4'), 8.07 (br s, 1 H, NHSO₂), 7.89 (br d, *J*= 8.3 Hz, 2 H, H-2, H-6), 7.71 (ddd, *J*= 8.9, 3.0, 2.0 Hz, 2 H, H-2", H-6"), 7.35-7.41 (m, 3 H, H-3, H-5, H-5'), 7.07 (ddd, *J* = 8.9, 3.0, 2.0 Hz, 2 H, H-3", H-5"), 4.00-4.06 (m, 4 H, CH₂O, CH₂N), 1.65-1.73 (m, 2 H, CH₂), 1.37-1.47 (m, 2 H, CH₂), 0.91 (t, *J* = 7.4 Hz, 3 H, CH₃); ¹³C NMR δ 165.4, 161.5, 144.4, 141.9, 140.7, 135.7, 132.9, 132.0, 128.5 (2), 127.5 (2), 127.3 (2), 127.1, 123.3, 114.6 (2), 67.6, 45.6, 30.4, 18.5, 13.5; MS *m*/*z* 440.6 (MH⁺, 100%). Anal. Calcd for C₂₃H₂₅N₃O₄S: C, 62.85; H, 5.73; N, 9.56. Found: C, 63.07; H, 5.84; N, 9.52%.

### Example 35

### Preparation of 4-((4-Phenoxyphenylsulfonamido)methyl)-N-(pyridin-3-yl)benzamide (44).

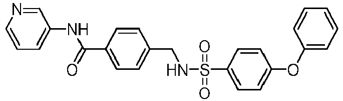

**Method C.** Reaction of amine salt **6** (318 mg, 0.82 mmol) and 4-phenoxyphenylsulfonyl chloride (241 mg, 0.90 mmol) followed by column chromatography, eluting with EtOAc, gave benzamide **44** (184 mg, 49%) as a white powder: mp (MeOH/EtOAc) 200-202 °C; ¹H NMR δ 10.37 (s, 1 H, NHCO), 8.93 (d, *J* = 2.3 Hz, 1 H, H-2'), 8.31 (dd, *J* = 4.7, 1.5 Hz, 1 H, H-6'), 8.22 (m, 1 H, NHSO₂), 8.19 (ddd, *J* = 8.3, 2.5, 1.5 Hz, 1 H, H-4'), 7.90 (br d, *J* = 8.3 Hz, 2 H, H-2, H-6), 7.76 (ddd, *J* = 8.9, 2.1, 2.0 Hz, 2 H, H-2", H-6"), 7.37-7.45 (m, 5 H, H-3, H-5, H-5', H-3"', H-5'"), 7.23 (dt, *J* = 7.4, 1.0 Hz, 1 H, H-4"'), 7.11 (dt, *J* = 7.6, 1.0 Hz, 2 H, H-2"', H-6"'), 7.07 (ddd, *J* = 8.9, 2.9, 2.0 Hz, 2 H, H-2", H-6"), 4.11 (s, 2 H, CH₂N); ¹³C NMR δ 165.4, 160.2, 154.8, 144.4, 141.9, 141.6, 135.7, 134.7, 132.8, 130.2 (2), 129.9 (2), 127.5 (2), 127.4 (2), 127.2, 124.7, 123.4, 119.8 (2), 117.5 (2), 45.6; MS *m*/*z* 460.6 (MH⁺, 100%). Anal. Calcd for C₂₅H₂₁N₃O₄S: C, 65.34; H, 4.61; N, 9.14. Found: C, 65.41; H, 4.55; N, 9.22%.

### Example 36

### Preparation of 4-((4-Methylphenylsulfonamido)methyl)-N-(pyridin-3-yl)benzamide (45).

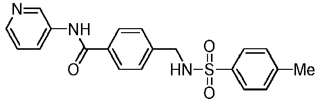

**Method C.** Reaction of amine salt **6** (530 mg, 1.4 mmol) and 4-toluenesulfonyl chloride (286 mg, 1.5 mmol) followed by column chromatography, eluting with a gradient (0-10%) of MeOH/EtOAc, gave benzamide **45** (218 mg, 42%) as a white powder: mp (EtOAc) 194-197 °C; ¹H NMR δ 10.37 (s, 1 H, NHCO), 8.92 (d, *J* = 2.2 Hz, 1 H, H-2'), 8.31 (dd, *J* = 4.7, 1.5 Hz, 1 H, H-6'), 8.19 (ddd, *J* = 8.3, 2.5, 1.5 Hz, 1 H, H-4'), 8.16 (br s, 1 H, NHSO₂), 7.90 (br d, *J* = 8.3 Hz, 2 H, H-2, H-6), 7.70 (br d, *J* = 8.3 Hz, 2 H, H-2", H-6"), 7.36-7.44 (m, 5 H, H-3, H-5, H-5', H-3", H-5"), 4.06 (s, 2 H, CH₂N), 2.39 (s, 3 H, CH₃); ¹³C NMR δ 165.4, 144.4, 142.5, 141.9 (2), 141.8, 137.6, 132.9, 129.5 (2), 127.5 (2), 127.3 (2), 127.2, 126.4 (2), 123.4, 45.6, 20.9; MS *m*/*z* 382.5 (MH⁺, 100%). Anal. Calcd for C₂₀H₁₉N₃O₃S: C, 62.97; H, 5.02; N, 11.02. Found: C, 62.23; H, 5.11; N, 11.16%.

### Example 37

### Preparation of 4-((4-Propylphenylsulfonamido)methyl)-N-(pyridin-3-yl)benzamide (46).

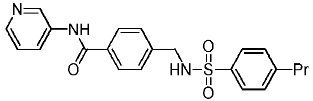

**Method C.** Reaction of amine salt **6** (400 mg, 1.0 mmol) and 4-propylbenzenesulfonyl chloride (247 mg, 1.1 mmol) followed by column chromatography, eluting with EtOAc, gave benzamide **46** (300 mg, 71%) as a white powder: mp (EtOAc) 190-191 °C; ¹H NMR δ 10.35 (s, 1 H, NHCO), 8.92 (d, *J* = 2.3 Hz, 1 H, H-2'), 8.31 (dd, *J*= 4.7, 1.4 Hz, 1 H, H-6'), 8.15-8.20 (m, 2 H, H-4', NHSO₂), 7.88 (d, *J*= 8.3 Hz, 2 H, H-2, H-6), 7.70 (d, *J*= 8.3 Hz, 2 H, H-2", H-6"), 7.36-7.40 (m, 5 H, H-3, H-5, H-5', H-3", H-5"), 4.09 (d, *J* = 6.4 Hz, 2 H, CH₂N), 2.62 (t, *J*= 7.6 Hz, 2 H, CH₂), 1.59 (s, *J*= 7.4 Hz, 2 H, CH₂), 0.87 (t, *J* = 7.3 Hz, 3 H, CH₃); ¹³C NMR δ 165.4, 146.9, 144.4, 141.9, 141.7, 138.0, 135.7, 132.8, 128.9 (2), 127.5 (2), 127.3 (2), 127.2 (2), 126.4, 123.3, 45.6, 36.8, 23.6, 13.4. Anal. Calcd for C₂₂H₂₃N₃O₃S: C, 64.53; H, 5.66; N, 10.26. Found: C, 64.46; H, 5.73; N, 10.16%.

### Example 38

### Preparation of 4-((4-tert-Butylphenylsulfonamido)methyl)-N-(pyridin-3-yl)benzamide (47).

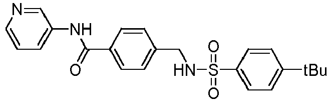

**Method C.** Reaction of amine salt **6** (740 mg, 1.9 mmol) and *4-tert-*butylbenzenesulfonyl chloride (490 mg, 2.1 mmol) followed by column chromatography, eluting with a gradient (50-100%) of EtOAc/pet. ether, gave benzamide **47** (560 mg, 70%) as a white powder: mp (EtOAc) 210-212 °C; ¹H NMR δ 10.35 (s, 1 H, NHCO), 8.90 (d, *J*= 2.3 Hz, 1 H, H-2'), 8.31 (dd, *J*= 4.7, 1.4 Hz, 1 H, H-6'), 8.13-8.20 (m, 2 H, NHSO₂, H-4'), 7.86 (br d, *J*= 8.3 Hz, 2 H, H-2, H-6), 7.68 (br d, *J =* 8.6 Hz, 2 H, H-2", H-6"), 7.54 (br d, *J =* 8.6 Hz, 2 H, H-3", H-5"), 7.35-7.40 (m, 3 H, H-3, H-5, H-5'), 4.09 (s, 2 H, CH₂N), 1.29 [s, 9 H, C(CH₃)₃]; ¹³C NMR δ 165.4, 155.2, 144.4, 141.9, 141.7, 137.7, 135.7, 132.8, 127.5 (2), 127.3 (2), 127.2, 126.2 (2), 125.8 (2), 123.4, 45.7, 34.6, 30.6 (3); MS *m*/*z* 424.5 (MH⁺, 100%). Anal. Calcd for C₂₃H₂₅N₃O₃S: C, 65.22; H, 5.95; N, 9.92. Found: C, 65.19; H, 6.10; N, 9.84%.

### Example 39

### Preparation of 4-[({[4-(1-Adamantyl)phenyl]sulfonyl}amino)methyl]-N(3-pyridinyl)benzamide (48).

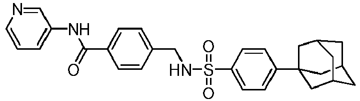

**Method C.** Reaction of amine salt **6** (200 mg, 0.51 mmol) and 4-adamantan-1-ylbenzenesulfonyl chloride (208 mg, 0.67 mmol) followed by column chromatography, eluting with a gradient (50-75%) of EtOAc/pet. ether gave benzamide **48** (73 mg, 28%) as a white powder: mp (H₂O) 220-222 °C; ¹H NMR δ 10.32 (s, 1 H, NHCO), 8.93 (d, *J* = 2.3 Hz, 1 H, H-2'), 8.30 (dd, *J* = 4.7, 1.5 Hz, 1 H, H-6'), 8.16-8.20 (m, 2 H, NHSO₂, H-4'), 7.83 (br d, *J*= 8.3 Hz, 2 H, H-2, H-6), 7.66 (br d, *J* = 8.6 Hz, 2 H, H-2", H-6"), 7.47 (br d, *J* = 8.6 Hz, 2 H, H-3", H-5"), 7.39 (dd, *J* = 8.4, 4.7, 1 H, H-5'), 7.35 (br d, *J* = 8.6 Hz, 2 H, H-3, H-5), 4.10 (d, *J* = 6.3 Hz, 2 H, CH₂N), 1.98-2.03 (m, 3 H, 3 × CH), 1.83 (br s, 6 H, 3 × CH₂), 1.75-1.84 (m, 6 H, 3 × CH₂); ¹³C NMR δ 165.3, 155.1, 144.4, 141.8, 141.5, 137.8, 135.7, 132.7, 127.4 (2), 127.3 (2), 127.1, 126.2 (2), 125.3 (2), 123.3, 45.7, 42.0 (3), 36.0, 35.8 (3), 28.0 (3); MS *m*/*z* 503.2 (MH⁺, 100%). Anal. calcd for C₂₉H₃₁N₃O₃S: C, 69.43; H, 6.23; N, 8.38. Found: C, 69.25; H, 6.30; N, 8.50%.

### Example 40

### Preparation of 4-[({[4-(3-chloro-1-adamantyl)phenyl]sulfonyl}amino)methyl]-N-(3-pyridinyl)benzamide (49).

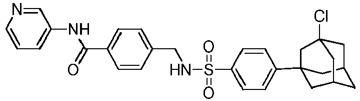

**Method C.** Also isolated from the above reaction was the chloride **49** (23 mg, 8%) as a white powder: mp (H₂O) 229-232 °C; ¹H NMR δ 10.32 (s, 1 H, NHCO), 8.93 (d, *J* = 2.3 Hz, 1 H, H-2'), 8.30 (dd, *J* = 4.7, 1.5 Hz, 1 H, H-6'), 8.17-8.23 (m, 2 H, NHSO₂, H-4'), 7.83 (br d, *J* = 8.3 Hz, 2 H, H-2, H-6), 7.67 (br d, *J* = 8.6 Hz, 2 H, H-2", H-6"), 7.50 (br d, *J* = 8.6 Hz, 2 H, H-3", H-5"), 7.38 (dd, *J* = 8.3, 4.7, 1 H, H-5'), 7.34 (br d, *J* = 8.6 Hz, 2 H, H-3, H-5), 4.11 (d, *J* = 6.3 Hz, 2 H, CH₂N), 2.22 (br s, 4 H, 2 × CH₂), 2.10 (br d, *J* = 2.5 Hz, 4 H, 2 × CH₂), 1.75-1.84 (m, 4 H, 2 × CH₂), 1.57-1.65 (m, 2 H, CH₂); ¹³C NMR δ 165.3, 152.9, 144.4, 141.8, 141.4, 138.3, 135.7, 132.7, 127.4 (2), 127.3 (2), 127.1, 127.0, 126.3 (2), 125.4 (2), 123.3, 69.5, 51.5 (2), 46.0 (2), 40.4, 40.0 (2), 33.6, 31.2 (2); MS *m*/*z* 533.3 (MH⁺, 100%). Anal. calcd for C₂₉H₃₀ClN₃O₃S: C, 64.97; H, 5.64; N, 7.84. Found: C, 64.97; H, 5.94; N, 7.65%.

### Example 41

### Preparation of Methyl 3-{4-[({4-[(3-Pyridinylamino)carbonyl]benzyl}amino)sulfonyl]phenyl}propanoate (50).

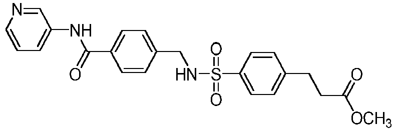

**Method C.** Reaction of amine salt **6** (500 mg, 1.29 mmol) and 3-(4-chlorosulfonyl)phenylpropionate (407 mg, 1.55 mmol) followed by column chromatography, eluting with a gradient (0-10%) of MeOH/DCM, gave benzamide **50** (427 mg, 73%) as a white powder: mp 181-182 °C; ¹H NMR δ 10.35 (s, 1 H, NHCO), 8.92 (d, *J* = 2.0 Hz, 1 H, H-2"'), 8.31 (dd, *J* = 4.7, 1.5 Hz, 1 H, H-6"'), 8.18 (ddd, *J* = 8.3, 2.5, 1.5 Hz, 2 H, H-4"', NHSO₂), 7.89 (br d, *J* = 8.3 Hz, 2 H, H-3", H-5"), 7.71 (br d, *J* = 8.4 Hz, 2 H, H-3', H-5'), 7.37-7.43 (m, 5 H, H-2', H-6', H-2", H-6", H-5"'), 4.08 (s, 2 H, CH₂N), 3.57 (s, 3 H, CH₃), 2.93 (t, *J* = 7.6 Hz, 2 H, H-3), 2.67 (t, *J* = 7.6 Hz, 2 H, H-2); ¹³C NMR δ 172.3, 165.4, 145.3, 144.4, 141.9, 141.7, 138.4, 135.7, 132.9, 128.9 (2), 127.5 (2), 127.3 (2), 127.2, 126.4 (2), 123.3, 51.2, 45.6, 34.1, 29.8; MS *m*/*z* 455.0 (MH⁺, 100%); Anal. Calcd for C₂₃H₂₃N₃O₅S: C, 60.91; H, 5.11; N, 9.27. Found: C, 60.97; H, 5.02; N,9.25%.

### Example 42

### Preparation of 4-((4-Acetamidophenylsulfonamido)methyl)-N-(pyridin-3-yl)benzamide (51).

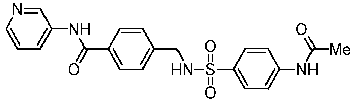

Acetic anhydride (0.10 mL, 1.1 mmol) was added dropwise to a stirred solution of 4-aminophenylsulfonamide **48** (210 mg, 0.55 mmol) in pyridine (10 mL) and the solution was stirred at 20 °C for 16 h. The solvent was evaporated and the residue suspended in water (20 mL) for 1 h. The precipitate was filtered, washed with water (5 mL) and dried. The crude solid was purified by column chromatography, eluting with a gradient (0-10%) of MeOH/EtOAc, to give benzamide **51** (150 mg, 64%) as a white powder: mp (MeOH/EtOAc) 229-231 °C; ¹H NMR δ 10.36 (br s, 1 H, NHCO), 10.32 (br s, 1 H, NHCO), 8.93 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.10 (br s, 1 H, NHSO₂), 7.91 (br d, *J* = 8.3 Hz, 2 H, H-2, H-6), 7.75 (br s, 4 H, H-3", H-5", H-2", H-6"), 7.37-7.43 (m, 3 H, H-3, H-5, H-5), 4.06 (s, 2 H, CH₂N), 2.08 (s, 3 H, COCH₃); ¹³C NMR δ 168.8, 165.4, 144.4, 142.7, 141.9, 141.8, 135.7, 134.1, 132.9, 127.6 (2), 127.5 (2), 127.3 (2), 127.1, 123.3, 118.5 (2), 45.6, 24.0; MS *m*/*z* 425.4 (MH⁺, 100%). Anal. Calcd for C₂₁H₂₀N₄O₄S: C, 59.42; H, 4.75; N, 13.20. Found: C, 59.63; H, 4.97; N, 12.89%.

### Example 43

### Preparation of 4-((4-Fluorophenylsulfonamido)methyl)-N-(pyridin-3-yl)benzamide (52).

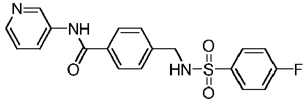

**Method C.** Reaction of amine salt **6** (453 mg, 1.2 mmol) and 4-flourobenzenesulfonyl chloride (249 mg, 1.3 mmol) followed by column chromatography, eluting with EtOAc, gave benzamide **52** (288 mg, 64%) as a white powder: mp (EtOAc/pet. ether) 189-191 °C; ¹H NMR δ 10.37 (s, 1 H, NHCO), 8.93 (d, *J* = 2.3 Hz, 1 H, H-2'), 8.27-8.31 (m, 2 H, NHSO₂, H-6'), 8.19 (ddd, *J* = 8.3, 2.5, 1.5 Hz, 1 H, H-4'), 7.91 (br d, *J* = 8.3 Hz, 2 H, H-2, H-6), 7.85-7.89 (m, 2 H, H-2", H-6"), 7.36-7.45 (m, 5 H, H-3, H-5, H-5', H-3", H-5"), 4.10 (s, 2 H, CH₂N); ¹³CNMR δ 165.4, 164.0 (d, *J* = 251 Hz), 144.4, 141.9, 141.6, 137.0 (d, *J* = 3 Hz), 135.7, 133.0, 129.4 (2, q, *J* = 10 Hz), 127.6 (2), 127.3 (2), 127.2, 123.4, 116.2 (2, q, *J* = 23 Hz), 45.6; MS *m*/*z* 436.5 (MH⁺, 100%). Anal. Calcd for C₁₉H₁₆FN₃O₃S·⅛ pet. ether: C, 59.88; H, 4.51; N, 10.65. Found: C, 59.79; H, 4.29; N, 10.86%.

### Example 44

### Preparation of 4-((4-Chlorophenyisulfonamido)methyl)-N-(pyridin-3-yl)benzamide (53).

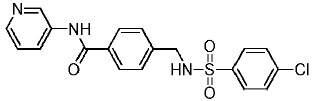

**Method C.** Reaction of amine salt **6** (400 mg, 1.0 mmol) and 4-chlorobenzenesulfonyl chloride (239 mg, 1.1 mmol) followed by column chromatography, eluting with a gradient (0-5%) of MeOH/EtOAc, gave the benzamide **53** (150 mg, 36%) as a cream coloured powder: mp (EtOAc) 229-231 °C; ¹H NMR δ 10.36 (s, 1 H, NHCO), 8.92 (d, *J* = 2.2 Hz, 1 H, H-2'), 8.36 (br t, *J* = 6.3 Hz, 1 H, NHSO₂ ), 8.31 (dd, *J* = 4.7, 1.5 Hz, 1 H, H-6'), 8.15-8.19 (m, 1 H, H-4'), 7.90 (d, *J* = 8.4 Hz, 2 H, H-2, H-6), 7.81 (d, *J* = 8.8 Hz, 2 H, H-2", H-6"), 7.66 (d, *J* = 8.8 Hz, 2 H, H-3", H-5"), 7.37-7.41 (m, 3 H, H-3, H-5, H-5'), 4.11 (d, *J* = 6.2 Hz, 2 H, CH₂N); ¹³C NMR δ 165.4, 144.4, 141.9, 141.5, 139.4, 137.1, 135.7, 133.0, 129.2 (2), 128.3 (2), 127.6 (2), 127.4 (2), 127.2, 123.4, 45.6. Anal. Calcd for C₁₉H₁₆ClN₃O₃S: C, 56.79; H, 4.01; N, 10.46. Found: C, 56.80; H, 3.93; N, 10.47%.

### Example 45

### Preparation of 4-((4-Bromophenylsulfonamido)methyl)-N-(pyridin-3-yl)benzamide (54).

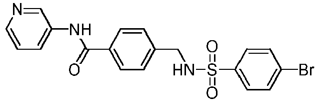

**Method C.** Reaction of amine salt **6** (353 mg, 0.91 mmol) and 4-bromobenzenesulfonyl chloride (278 mg, 1.1 mmol) followed by column chromatography, eluting with a gradient (0-10%) of MeOH/EtOAc, gave benzamide **54** (326 mg, 80%) as a white powder: mp (MeOH/EtOAc) 244-247 °C; ¹H NMR δ 10.37 (s, 1 H, NHCO), 8.92 (d, *J* = 2.3 Hz, 1 H, H-2'), 8.36 (br s, 1 H, NHSO₂), 8.31 (dd, *J* = 4.7, 1.4 Hz, 1 H, H-6'), 8.18 (ddd, *J* = 8.3, 2.5, 1.5 Hz, 1 H, H-4'), 7.91 (br d, *J* = 8.3 Hz, 2 H, H-2, H-6), 7.80 (ddd, *J* = 8.7, 2.2, 2.0 Hz, 2 H, H-2", H-6"), 7.73 (ddd, *J* = 8.7, 2.2, 2.0 Hz, 2 H, H-3", H-5"), 7.36-7.42 (m, 3 H, H-3, H-5, H-5'), 4.11 (s, 2 H, CH₂N); ¹³C NMR δ 165.4, 144.4, 141.9, 141.5, 139.8, 135.7, 133.0, 132.1 (2), 128.4 (2), 127.6 (2), 127.4 (2), 127.2, 126.1, 123.4, 45.6; MS *m*/*z* 446.4, 448.5 (MH⁺, 100%). Anal. Calcd for C₁₉H₁₆BrN₃O₃S: C, 51.13; H, 3.61; N, 9.41. Found: C, 50.90; H, 3.41; N, 9.20%.

### Example 46

### Preparation of N-(Pyridin-3-yl)-4-((4-(trifluoromethoxy)phenylsulfonamido)methyl)benzamide (55).

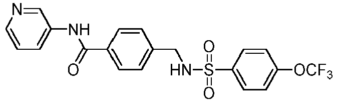

**Method C.** Reaction of amine salt **6** (400 mg, 1.0 mmol) and 4-trifluoromethoxybenzenesulfonyl chloride (0.19 mL, 1.1 mmol) followed by column chromatography, eluting with EtOAc, gave benzamide **55** (276 mg, 59%) as a white powder: mp (EtOAc/pet. ether) 221-223 °C; ¹H NMR δ 10.35 (s, 1 H, NHCO), 8.91 (d, *J* = 2.0 Hz, 1 H, H-2'), 8.42 (br s, 1 H, NHSO₂), 8.30 (dd, *J* = 4.7, 1.5 Hz, 1 H, H-6'), 8.18 (ddd, *J* = 8.3, 2.5, 1.5 Hz, 1 H, H-4'), 7.92 (ddd, *J* = 8.9, 3.0, 2.0 Hz, 2 H, H-2", H-6"), 7.88 (br d, *J* = 8.4 Hz, 2 H, H-2, H-6), 7.55 (br d, *J* = 8.9 Hz, 2 H, H-3", H-5"), 7.39 (m, 3 H, H-3, H-5, H-5'), 4.14 (s, 2 H, CH₂N); ¹³C NMR δ 165.3, 150.6 (q, *J* = 3 Hz), 144.4, 141.9, 141.4, 139.6, 135.7, 133.0, 128.9 (2), 127.6 (2), 127.4 (2), 127.2, 123.4, 121.3 (2), 119.7 (q, *J* = 258 Hz), 45.6; MS *m*/*z* 452.5 (MH⁺, 100%). Anal. Calcd for C₂₀H₁₆F₃N₃O₄S: C, 53.21; H, 3.57; N, 9.31. Found: C, 53.32; H, 3.74; N, 9.36%.

### Example 47

### Preparation of Methyl 4-(N-(4-(Pyridin-3-ylcarbamoyl)benzyl)sulfamoyl)benzoate (56).

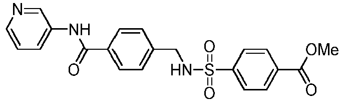

**Method C.** Reaction of amine salt **6** (1.69 g, 4.3 mmol) and methyl 4-(chlorosulfonyl)benzoate (1.02 g, 4.3 mmol) followed by column chromatography, eluting with a gradient (0-20%) of MeOH/EtOAc, gave benzoate **56** (553 mg, 30%) as a white powder: mp (MeOH/EtOAc) 212-215 °C; ¹H NMR δ 10.34 (s, 1 H, NHCO), 8.92 (d, *J* = 2.4 Hz, 1 H, H-2"), 8.47 (br s, 1 H, NHSO₂), 8.31 (dd, *J* = 4.7, 1.4 Hz, 1 H, H-6"), 8.18 (ddd, *J* = 8.3, 2.5, 1.5 Hz, 1 H, H-4"), 8.12 (ddd, *J* = 8.6, 1.9, 1.7 Hz, 2 H, H-3, H-5), 7.93 (ddd, *J* = 8.6, 1.9, 1.7 Hz, 2 H, H-2, H-6), 7.88 (brd, *J* = 8.3 Hz, 2 H, H-2', H-6'), 7.36-7.42 (m, 3 H, H-3', H-5', H-5"), 4.14 (s, 2 H, CH₂N), 3.88 (s, 3 H, CO₂CH₃). Anal. Calcd for C₂₁H₁₉N₃O₅S·½CH₃OH: C, 58.49; H, 4.80; N, 9.52. Found: C, 58.55; H, 4.51; N, 9.35%.

### Example 48

### Preparation of 4-((4-Acetylphenylsulfonamido)methyl)-N-(pyridin-3-yl)benzamide (57).

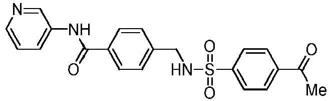

**Method C.** Reaction of amine salt **6** (400 mg, 1.0 mmol) and 4-acetylbenzenesulfonyl chloride (247 mg, 1.1 mmol) followed by column chromatography, eluting with a gradient (0-10%) of MeOH/EtOAc, gave benzamide **57** (229 mg, 54%) as a white powder: mp (MeOH/EtOAc) 229-231 °C; ¹H NMR δ 10.35 (s, 1 H, NHCO), 8.92 (d, *J* = 2.2 Hz, 1 H, H-2'), 8.45 (br s, 1 H, NHSO₂), 8.30 (dd, *J* = 4.7, 1.5 Hz, 1 H, H-6'), 8.18 (ddd, *J* = 8.3, 2.5, 1.5 Hz, 1 H, H-4'), 8.10 (ddd, *J* = 8.5, 1.9, 1.8 Hz, 2 H, H-3", H-5"), 7.92 (ddd, *J* = 8.5, 1.9, 1.8 Hz, 2 H, H-2", H-6"), 7.88 (br d, *J* = 8.3 Hz, 2 H, H-2, H-6), 7.36-7.42 (m, 3 H, H-3, H-5, H-5'), 4.13 (s, 2 H, CH₂N), 2.62 (s, 3 H, COCH₃); ¹³C NMR δ 197.2, 165.4, 144.4, 144.3, 141.9, 141.4, 139.3, 135.6, 133.0, 128.8 (2), 127.6 (2), 127.4 (2), 127.2, 126.7 (2), 123.4, 45.6, 26.8; MS *m*/*z* 410.5 (MH⁺, 100%). Anal. Calcd for C₂₁H₁₉N₃O₄S: C, 61.60; H, 4.68; N, 10.26. Found: C, 61.63; H, 4.84; N, 10.31%.

### Example 49

### Preparation of N-(Pyridin-3-yl)-4-((4-(trifluoromethyl)phenylsulfonamido)methyl)benzamide (58).

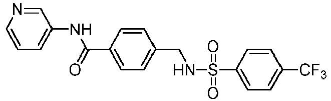

**Method C.** Reaction of amine salt **6** (397 mg, 1.0 mmol) and 4-triflouromethylbenzenesulfonyl chloride (275 mg, 1.1 mmol) followed by column chromatography, eluting with EtOAc, gave benzamide **58** (272 mg, 61 %) as a white powder: mp (EtOAc) 243-246 °C; ¹H NMR δ 10.35 (s, 1 H, NHCO), 8.92 (d, *J* = 2.5 Hz, 1 H, H-2'), 8.54 (br s, 1 H, NHSO₂), 8.31 (dd, *J* = 4.7, 1.5 Hz, 1 H, H-6'), 8.18 (ddd, *J* = 8.3, 2.5, 1.5 Hz, 1 H, H-4'), 7.99 (br d, *J* = 8.3 Hz, 2 H, H-3", H-5"), 7.94 (br d, *J* = 8.3 Hz, 2 H, H-2", H-6"), 7.88 (br d, *J* = 8.4 Hz, 2 H, H-2, H-6), 7.37-7.41 (m, 3 H, H-3, H-5, H-5'), 4.16 (s, 2 H, CH₂N); ¹³C NMR δ 165.3, 144.5, 144.4, 141.9, 141.3, 135.7, 133.0, 132.0 (q, *J* = 32 Hz), 127.6 (2), 127.4 (2), 127.3 (2), 127.2, 126.2 (2, q, *J* = 4 Hz), 123.4 (q, *J* = 272 Hz), 123.3, 45.6; MS *m*/*z* 436.5 (MH⁺, 100%). Anal. Calcd for C₂₀H₁₆F₃N₃O₃S: C, 55.17; H, 3.70; N, 9.65. Found: C, 55.39; H, 3.80; N, 9.58%.

### Example 50

### Preparation of 4-((4-Cyanophenylsulfonamido)methyl)-N-(pyridin-3-yl)benzamide (59).

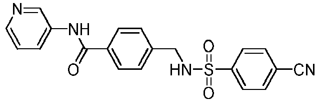

**Method C.** Reaction of amine salt **6** (424 mg, 1.1 mmol) and 4-cyanobenzenesulfonyl chloride (242 mg, 1.2 mmol) followed by column chromatography, eluting with a gradient (0-5%) of MeOH/EtOAc, gave benzamide **59** (201 mg, 47%) as a white powder: mp (MeOH/EtOAc) 249-251 °C; ¹H NMR δ 10.36 (s, 1 H, NHCO), 8.93 (d, *J* = 2.2 Hz, 1 H, H-2'), 8.56 (br s, 1 H, NHSO₂), 8.31 (dd, *J* = 4.7, 1.5 Hz, 1 H, H-6'), 8.18 (ddd, *J* = 8.3, 2.3, 1.5 Hz, 1 H, H-4'), 8.06 (ddd, *J* = 8.3, 2.3, 1.5 Hz, 2 H, H-3", H-5"), 7.95 (dd, *J* = 8.3, 1.9 Hz, 2 H, H-2", H-6"), 7.89 (br d, *J* = 8.3 Hz, 2 H, H-2, H-6), 7.37-7.42 (m, 3 H, H-3, H-5, H-5'), 4.15 (s, 2 H, CH₂N); ¹³C NMR δ 165.3, 144.7, 144.3, 141.9, 141.2, 135.6, 133.2 (2), 133.0, 127.6 (2), 127.4 (2), 127.2, 127.1 (2), 123.4, 117.6, 114.7, 45.6; MS *m*/*z* 393.4 (MH⁺, 100%). Anal. Calcd for C₂₀H₁₆N₄O₃S: C, 61.21; H, 4.11; N, 14.28. Found: C, 61.16; H, 4.09; N, 14.17%.

### Example 51

### Preparation of 4-((4-Nitrophenylsulfonamido)methyl)-N-(pyridin-3-yl)benzamide (60).

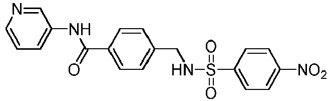

**Method C.** Reaction of amine salt **6** (984 mg, 2.5 mmol) and 4-nitrobenzenesulfonyl chloride (620 mg, 2.8 mmol) followed by column chromatography, eluting with a gradient (0-10%) of MeOH/EtOAc, gave benzamide **60** (687 mg, 66%) as a white powder: mp (MeOH/EtOAc) 241-243 °C; ¹H NMR δ 10.34 (s, 1 H, NHCO), 8.90 (d, *J* = 2.5 Hz, 1 H, H-2'), 8.64 (br s, 1 H, NHSO₂), 8.37 (ddd, *J* = 8.5, 1.9, 1.8 Hz, 2 H, H-3", H-5"), 8.31 (dd, *J* = 4.7, 1.5 Hz, 1 H, H-6'), 8.17 (ddd, *J* = 8.3, 2.5, 1.5 Hz, 1 H, H-4'), 8.10 (ddd, *J* = 8.5, 1.9, 1.8 Hz, 2 H, H-2", H-6"), 7.88 (br d, *J* = 8.3 Hz, 2 H, H-2, H-6), 7.35-7.41 (m, 3 H, H-3, H-5, H-5'), 4.13 (s, 2 H, CH₂N); ¹³C NMR δ 165.3, 149.4, 146.2, 144.4, 141.9, 141.1, 135.6, 133.0, 127.9 (2), 127.6 (2), 127.4 (2), 127.2, 124.4 (2), 123.3, 45.6; MS *m*/*z* 413.5 (MH⁺, 100%). Anal. Calcd for C₁₉H₁₆N₄O₅S: C, 55.33; H, 3.91; N, 13.58. Found: C, 55.58; H, 3.99; N, 13.57%.

### Example 52

### Preparation of 4-({[(4'-Methoxy[1,1'-biphenyl]-4-yl)sulfonyl]amino}methyl)-N-(3-pyridinyl)benzamide (61).

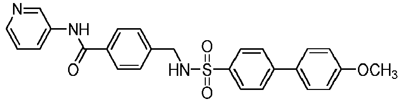

**Method C.** Reaction of amine salt **6** (200 mg, 0.51 mmol) and 4'-methoxy-(1,1'-biphenyl)-4-sulfonyl chloride (189 mg, 0.67 mmol) gave benzamide **61** (157 mg, 65%) as a cream powder: mp 244-247 °C; ¹H NMR δ 10.34 (s, 1 H, NHCO), 8.91 (d, *J* = 2.2 Hz, 1 H, H-2'), 8.31 (dd, *J* = 4.7, 1.5 Hz, 1 H, H-6'), 8.26 (br s, 1 H, NHSO₂), 8.17 (ddd, *J* = 8.4, 2.5, 1.5 Hz, 1 H, H-4'), 7.89 (br d, *J* = 8.3 Hz, 2 H, H-2, H-6), 7.78-7.84 (m, 4 H, H-2", H-3", H-5", H-6"), 7.68 (br d, *J* = 8.9 Hz, 2 H, H-2"', H-6"'), 7.37-7.43 (m, 3 H, H-3, H-5, H-5'), 7.03 (br d, *J* = 8.8 Hz, 2 H, H-3"', H-5"'), 4.12 (br s, 2 H, CH₂N), 3.80 (s, 3 H, OCH₃); ¹³C NMR δ 165.4, 159.5, 144.4, 143.4, 141.9, 141.7, 138.5, 135.6, 132.9, 130.7, 128.1 (2), 127.5 (2), 127.4 (2), 127.2, 127.0 (2), 126.5 (2), 123.3, 114.4 (2), 55.1, 45.7; MS *m*/*z* 475.0 (MH⁺, 100%). Anal. calcd for C₂₆H₂₃N₃O₄S: C, 65.94; H, 4.90; N, 8.87. Found: C, 65.63; H, 5.04; N, 8.79%.

### Example 53

### Preparation of 4-({[(4'-Methyl[1,1'-biphenyl]-4-yl)sulfonyl]amino}methyl)-N-(3-pyridinyl)benzamide (62).

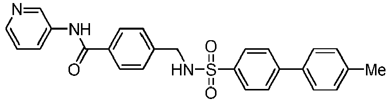

**Method C.** Reaction of amine salt **6** (200 mg, 0.51 mmol) and 4'-methyl(1,1'-biphenyl)-4-sulfonyl chloride (178 mg, 0.67 mmol) gave benzamide **62** (231 mg, 98%) as a yellow powder: mp 263-266 °C; ¹H NMR δ 10.33 (s, 1 H, NHCO), 8.91 (d, *J* = 2.4 Hz, 1 H, H-2'), 8.31 (dd, *J* = 4.7, 1.4 Hz, 1 H, H-6'), 8.28 (t, *J* = 6.4 Hz, 1 H, NHSO₂), 8.17 (ddd, *J* = 8.4, 2.4, 1.5 Hz, 1 H, H-4'), 7.89 (br d, *J* = 8.3 Hz, 2 H, H-2, H-6), 7.81-7.90 (m, 4 H, H-2", H-3", H-5", H-6"), 7.62 (br d, *J* = 8.1 Hz, 2 H, H-2"', H-6"'), 7.37-7.43 (m, 3 H, H-3, H-5, H-5'), 7.29 (br d, *J* = 8.0 Hz, 2 H, H-3"', H-5"'), 4.13 (d, *J* = 6.2 Hz, 2 H, CH₂N), 2.35 (s, 3 H, CH₃); ¹³C NMR δ 165.4, 144.4, 143.7, 141.9, 141.6, 139.0, 137.8, 135.6, 135.5, 132.9, 129.5 (2), 127.5 (2), 127.4 (2), 127.2, 127.0 (2), 126.9 (2), 126.7 (2), 123.3, 45.7, 20.5; MS *m*/*z* 459.0 (MH⁺, 100%); HRMS (FAB⁺) calcd for C₂₆H₂₄N₃O₃S (MH⁺) *m*/*z* 458.1538, found 458.1540. Anal. calcd for C₂₆H₂₃N₃O₃S·¼H₂O: C, 66.94; H, 5.19; N, 9.01. Found: C, 67.04; H, 5.29; N, 8.82%.

### Example 54

### Preparation of 4-((Biphenyl-4-ylsulfonamido)methyl)-N-(pyridin-3-yl)benzamide (63).

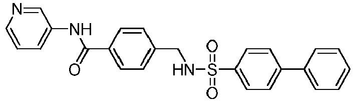

**Method C.** Reaction of amine salt **6** (305 mg, 0.78 mmol) and 4-biphenylsulfonyl chloride (238 mg, 0.94 mmol) followed by column chromatography, eluting with a gradient (0-10%) of MeOH/EtOAc, gave benzamide **63** (263 mg, 76%) as a white powder: mp (MeOH/EtOAc) 248-250 °C; ¹H NMR δ 10.36 (s, 1 H, NHCO), 8.92 (d, *J* = 1.7 Hz, 1 H, H-2'), 8.29-8.34 (m, 2 H, NHSO₂, H-6'), 8.17 (ddd, *J* = 8.3, 2.3, 1.4 Hz, 1 H, H-4'), 7.90 (br d, *J* = 8.4 Hz, 2 H, H-2, H-6), 7.83-7.87 (m, 4 H, H-2", H-3", H-5", H-6"), 7.71 (br d, *J* = 8.5 Hz, 2 H, H-2"', H-6"'), 7.46-7.51 (m, 2 H, H-3"', H-5"'), 7.36-7.44 (m, 4 H, H-3, H-5, H-5', H-4'"), 4.13 (d, *J* = 5.6 Hz, 2 H, CH₂N); ¹³C NMR δ 165.4, 144.4, 143.8, 141.9, 141.6, 139.3, 138.5, 135.7, 132.9, 128.9 (2), 128.3, 127.6 (2), 127.4 (2), 127.2 (2), 127.1, 127.0 (2), 126.9 (2), 123.3, 45.7; MS *m*/*z* 444.6 (MH⁺, 100%). Anal. Calcd for C₂₅H₂₁N₃O₃S·¼EtOAc: C, 67.08; H, 4.98; N, 9.03. Found: C, 67.11; H, 4.73; N, 9.32%.

### Example 55

### Preparation of 4-({[(4'-Fluoro[1,1'-biphenyl]-4-yl)sulfonyl]amino}methyl)-N-(3-pyridinyl)benzamide (64).

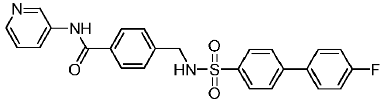

**Method C.** Reaction of amine salt **6** (200 mg, 0.51 mmol) and 4'-fluoro(1,1'-biphenyl)-4-sulfonyl chloride (181 mg, 0.668 mmol) gave benzamide **64** (159 mg, 67%) as a cream powder: mp (MeOH/DCM) 249-250 °C; ¹H NMR δ 10.34 (s, 1 H, NHCO), 8.91 (d, *J* = 2.5 Hz, 1 H, H-2'), 8.31 (dd, *J* = 4.7, 1.5 Hz, 2 H, H-6', NHSO₂), 8.17 (ddd, *J* = 8.3, 2.5, 1.5 Hz, 1 H, H-4'), 7.89 (br d, *J* = 8.3 Hz, 2 H, H-2, H-6), 7.82-7.87 (m, 4 H, H-2", H-3", H-5", H-6"), 7.77 (dd, *J* = 8.9, 5.4 Hz, 2 H, H-2"', H-6"'), 7.37-7.43 (m, 3 H, H-3, H-5, H-5'), 7.31 (t, *J* = 8.9 Hz, 2 H, H-3"', H-5"'), 4.13 (s, 2 H, CH₂N); ¹³C NMR δ 165.4, 162.3 (d, *J* = 246 Hz), 144.4, 142.7, 141.9, 141.6, 139.3, 135.6, 134.9 (d, *J* = 3 Hz), 132.9, 129.0 (2) (d, *J* = 8 Hz), 127.6 (2), 127.4 (2), 127.2 (2), 127.1, 127.0 (2), 123.3, 115.8 (2) (d, *J* = 22 Hz), 45.7; MS *m*/*z* 463.0 (MH⁺, 100%). Anal. calcd for C₂₅H₂₀FN₃O₃S: C, 65.06; H, 4.37; N, 9.10. Found: C, 64.66; H, 4.31; N, 9.01%.

### Example 56

### Preparation of 4-({[(4'-Chloro[1,1'-biphenyl]-4-yl)sulfonyl]amino}methyl)-N-(3-pyridinyl)benzamide (65).

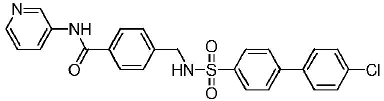

**Method C.** Reaction of amine salt **6** (200 mg, 0.51 mmol) and 4-(4'-chlorophenyl)benzenesulfonyl chloride (177 mg, 0.62 mmol) gave benzamide **65** (208 mg, 85%) as a white powder: mp (H₂O) 276-278 °C; ¹H NMR δ 10.33 (s, 1 H, NHCO), 8.91 (d, *J* = 2.4 Hz, 1 H, H-2'), 8.30-8.33 (m, 2 H, H-6', NHSO₂), 8.16 (ddd, *J* = 8.4, 2.5, 1.5 Hz, 1 H, H-4'), 7.89 (br d, *J* = 8.3 Hz, 2 H, H-2, H-6), 7.84-7.86 (m, 4 H, H-2", H-3", H-5", H-6"), 7.75 (br d, *J* = 8.6 Hz, 2 H, H-2"', H-6"'), 7.53 (br d, *J* = 8.6 Hz, 2 H, H-3"', H-5"'), 7.37-7.42 (m, 3 H, H-3, H-5, H-5'), 4.13 (d, *J* = 6.2 Hz, 2 H, CH₂N); ¹³C NMR δ 165.4, 144.4, 142.4, 141.9, 141.6, 139.7, 137.2, 135.6, 133.3, 132.9, 128.9 (2), 128.7 (2), 127.6 (2), 127.4 (2), 127.3 (2), 127.2, 127.1 (2), 123.3, 45.7; MS *m*/*z* 479.0 (MH⁺, 100%). Anal. calcd for C₂₅H₂₀ClN₃O₃S: C, 62.82; H, 4.22; N, 8.79. Found: C, 62.61; H, 4.36; N, 8.59%.

### Example 57

### Preparation of 4-[({[4-(2-Pyrimidinyl)phenyl]sulfonyl}amino)methyl]-N-(3-pyridinyl)-benzamide (66).

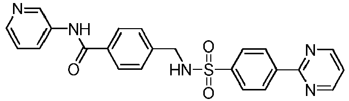

**Method C.** Reaction of amine salt **6** (180 mg, 0.46 mmol) and 4-pyrimidin-2-ylbenzenesulfonyl chloride (130 mg, 0.51 mmol) gave benzamide **66** (152 mg, 74%) as a yellow powder: mp (H₂O) 278-280 °C; ¹H NMR δ 10.33 (s, 1 H, NHCO), 8.96 (d, *J* = 4.9 Hz, 2 H, H-4"', H-6"'), 8.90 (d, *J* = 2.2 Hz, 1 H, H-2'), 8.56 (d, *J* = 8.6 Hz, 2 H, H-3", H-5"), 8.37 (br s, 1 H, NHSO₂), 8.31 (dd, *J* = 4.7, 1.4 Hz, 1 H, H-6'), 8.16 (ddd, *J* = 8.3, 2.5, 1.5 Hz, 1 H, H-4'), 7.97 (d, *J* = 8.6 Hz, 2 H, H-2", H-6"), 7.90 (br d, *J* = 8.3 Hz, 2 H, H-2, H-6), 7.52 (t, *J* = 4.9 Hz, 1 H, H-5"'), 7.42 (br d, *J* = 8.3 Hz, 2 H, H-3, H-5), 7.38 (dd, *J* = 8.3, 4.7, 1 H, H-5'), 4.15 (s, 2 H, CH₂N); ¹³C NMR δ 165.3, 161.9, 157.8 (2), 144.4, 142.4, 141.9, 141.6, 140.5, 135.6, 132.9, 128.2 (2), 127.6 (2), 127.3 (2), 127.2, 126.9 (2), 123.3, 120.5, 45.7; MS *m*/*z* 446.9 (MH⁺, 100%). Anal. calcd for C₂₃H₁₉N₅O₃S: C, 62.01; H, 4.30; N, 15.72. Found: C, 61.95; H, 4.46; N, 15.5%.

### Example 58

### Preparation of 4-[({[4-(1H-Pyrazol-1-yl)phenyl]sulfonyl}amino)methyl]-N-(3-pyridinyl)benzamide (67).

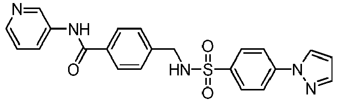

**Method C.** Reaction of amine salt **6** (170 mg, 0.44 mmol) and 4-(1*H*-pyrazol-1-yl)benzenesulfonyl chloride (159 mg, 0.66 mmol) gave benzamide **67** (167 mg, 88%) as a pale yellow powder: mp (H₂O) 243-245 °C; ¹H NMR δ 10.34 (s, 1 H, NHCO), 8.91 (d, *J* = 2.0 Hz, 1 H, H-2'), 8.61 (d, *J* = 2.3 Hz, 1 H, H-5"'), 8.31 (dd, *J* = 4.7, 1.4 Hz, 1 H, H-6'), 8.28 (br s, 1 H, NHSO₂), 8.17 (ddd, *J* = 8.4, 2.5, 1.5 Hz, 1 H, H-4'), 8.05 (br d, *J* = 8.9 Hz, 2 H, H-3", H-5"), 7.89-7.93 (m, 4 H, H-2, H-6, H-2", H-6"), 7.82 (d, *J* = 1.7 Hz, 1 H, H-3"'), 7.42 (br d, *J* = 8.4 Hz, 2 H, H-3, H-5), 7.39 (ddd, *J* = 8.4, 4.7, 0.6, 1 H, H-5'), 6.60 (dd, *J* = 2.5, 1.8 Hz, 1 H, H-4"'), 4.12 (s, 2 H, CH₂N); ¹³C NMR δ 165.4, 144.4, 142.1, 142.0, 141.9, 141.6, 137.6, 135.6, 133.0, 128.2, 128.1 (2), 127.6 (2), 127.4 (2), 127.2 (2), 123.3, 118.3, 108.6, 45.7; MS *m*/*z* 434.9 (MH⁺, 100%). Anal. calcd for C₂₂H₁₉N₅O₃S: C, 60.96; H, 4.42; N, 16.16. Found: C, 60.63; H, 4.57; N 15.84%.

### Example 59

### Preparation of 4-[({[4-(2-Methyl-1,3-thiazol-4-yl)phenyl]sulfonyl}amino)methyl]-N-(3-pyridinyl)benzamide (68).

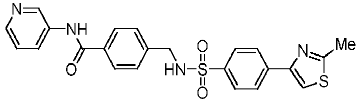

Method C. Reaction of amine salt 6 (154 mg, 0.40 mmol) and 4-(2-methyl-1,3-thiazol-4-yl)benzenesulfonyl chloride (130 mg, 0.48 mmol) followed purification by column chromatography, eluting with a gradient (0-10%) of MeOH/DCM gave benzamide 68 (114 mg, 62%) as a yellow powder: mp (MeOH/DCM) 221-223 °C; ¹H NMR δ 10.34 (s, 1 H, NHCO), 8.91 (d, *J* = 2.2 Hz, 1 H, H-2'), 8.31 (dd, *J* = 4.7, 1.4 Hz, 1 H, H-6'), 8.27 (br s, 1 H, NHSO₂), 8.17 (ddd, *J* = 8.4, 2.5, 1.5 Hz, 1 H, H-4'), 8.09-8.14 (m, 3 H, H-3", H-5", H-5"'), 7.90 (d, *J* = 8.3 Hz, 2 H, H-2, H-6), 7.86 (d, *J* = 8.6 Hz, 2 H, H-2", H-6"), 7.37-7.43 (m, 3 H, H-3, H-5, H-5'), 4.12 (s, 2 H, CH₂N), 2.72 (s, 3 H, CH₃); ¹³C NMR δ 166.0, 165.3, 152.1, 144.4, 141.9, 141.7, 139.4, 137.5, 135.6, 132.9, 127.6 (2), 127.3 (2), 127.2, 127.0 (2), 126.3 (2), 123.3, 116.4, 45.6, 18.8; MS *m*/*z* 465.9 (MH⁺, 100%). Anal. calcd for C₂₃H₂₀N₄O₃S₂: C, 59.46; H, 4.34; N, 12.06. Found: C, 59.69; H, 4.45; N, 12.03%.

### Example 60

### Preparation of 4-[({[4-(1,3-Oxazol-5-yl)phenyl]sulfonyl}amino)methyl]-N-(3-pyridinyl)benzamide (69).

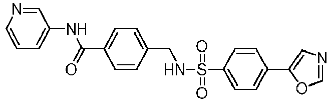

**Method C.** Reaction of amine salt **6** (160 mg, 0.41 mmol) and 4-(1,3-oazol-5-yl)benzenesulfonyl chloride (150 mg, 0.62 mmol) gave benzamide **69** (123 mg, 69%) as a dark yellow powder: mp (H₂O) 240-242 °C; ¹H NMR δ 10.33 (s, 1 H, NHCO), 8.91 (br s, 1 H, H-2'), 8.53 (s, 1 H, H-2"') 8.31-8.34 (m, 2 H, H-6', NHSO₂), 8.17 (m, 1 H, H-4'), 7.87-7.94 (m, 7 H, H-2, H-6, H-2", H-3", H-5", H-6", H-4"'), 7.37-7.42 (m, 3 H, H-3, H-5, H-5'), 4.12 (d, *J* = 6.3 Hz, 2 H, CH₂N); ¹³C NMR δ 165.4, 152.6, 149.1, 144.3, 141.7, 141.6, 140.0, 135.7, 132.9, 130.7, 127.6 (2), 127.4 (2), 127.3 (2), 124.5 (2), 124.1, 123.4, 45.6, one C not observed; MS *m*/*z* 435.9 (MH⁺, 100%); HRMS (FAB⁺) calcd for C₂₂H₁₉N₄O₄S (MH⁺) *m*/*z* 435.1127, found 435.1124. Anal. calcd for C₂₂H₁₈N₄O₄S·½H₂O: C, 59.58; H, 4.32; N, 12.63. Found: C, 59.55; H, 4.27; N, 12.45%.

### Example 61

### Preparation of 4-((3,4-Dimethoxyphenylsulfonamido)methyl)-N-(pyridin-3-yl)benzamide (70).

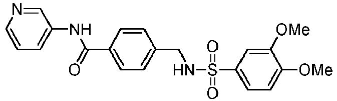

**Method C.** Reaction of amine salt **6** (407 mg, 1.1 mmol) and 3,4-dimethoxybenzenesulfonyl chloride (272 mg, 1.2 mmol) followed by column chromatography, eluting with EtOAc, gave benzamide **70** (295 mg, 66%) as a white powder: mp (EtOAc) 164-166 °C; ¹H NMR δ 10.36 (s, 1 H, NHCO), 8.93 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.06 (br s, 1 H, NHSO₂), 7.91 (br d, *J* = 8.3 Hz, 2 H, H-2, H-6), 7.41 (br d, 2 H, H-3, H-5), 7.35-7.40 (m, 2 H, H-5', H-6"), 7.30 (d, *J* = 2.1 Hz, 1 H, H-2"), 7.10 (d, *J* = 8.5 Hz, 1 H, H-5"), 4.04 (s, 2 H, CH₂N), 3.83 (s, 3 H, OCH₃), 3.81 (s, 3 H, OCH₃); ¹³C NMR δ 165.4, 151.8, 148.6, 144.4, 141.9, 141.8, 135.7, 132.9, 132.0, 127.5 (2), 127.3 (2), 127.2, 123.4, 120.1, 111.1, 109.4, 55.7, 55.6, 45.7; MS *m*/*z* 428.5 (MH⁺, 100%). Anal. Calcd for C₂₁H₂₁N₃O₅S: C, 59.00; H, 4.95; N, 9.83. Found: C, 59.27; H, 5.01; N, 9.93%.

### Example 62

### Preparation of 4-((3-tert-Butyl-4-methoxyphenylsulfonamido)methyl)-N-(pyridin-3-yl)benzamide (71).

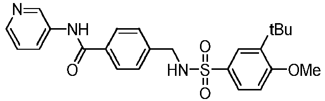

**Method C.** Reaction of amine salt **6** (391 mg, 1.0 mmol) and 3-*tert*-butyl-4-methoxybenzene sulfonyl chloride (290 mg, 1.1 mmol) followed by column chromatography, eluting with a gradient (70-100%) of EtOAc/pet. ether, gave benzamide **71** (221 mg, 49%) as a white powder: mp (EtOAc) 189-192 °C; ¹H NMR δ 10.33 (s, 1 H, NHCO), 8.91 (d, *J* = 2.3 Hz, 1 H, H-2'), 8.31 (dd, *J* = 4.7, 1.5 Hz, 1 H, H-6'), 8.17 (ddd, *J* = 8.3, 2.5, 1.5 Hz, 1 H, H-4'), 8.08 (br s, 1 H, NHSO₂), 7.88 (br d, *J* = 8.3 Hz, 2 H, H-2, H-6), 7.64 (dd, *J* = 8.5, 2.3 Hz, 1 H, H-6"), 7.60 (d, *J* = 2.3 Hz, 1 H, H-2"), 7.36-7.42 (m, 3 H, H-3, H-5, H-5'), 7.11 (d, *J* = 8.6 Hz, 1 H, H-5"), 4.07 (br s, 2 H, CH₂N), 3.88 (s, 3 H, OCH₃), 1.33 [s, 9 H, C(CH₃)₃]; ¹³C NMR δ 165.3, 160.9, 144.4, 141.9, 141.8, 137.8, 135.6, 132.7, 131.7, 127.5 (2), 127.3 (2), 127.2, 126.5, 124.6, 123.3, 111.7, 55.5, 45.6, 34.5, 29.0 (3); MS *m*/*z* 454.8 (MH⁺, 100%). Anal. Calcd for C₂₄H₂₇N₃O₄S: C, 63.56; H, 6.00; N, 9.26. Found: C, 63.53; H, 5.98; N, 9.34%.

### Example 63

### Preparation of 4-((2,3,4,5,6-Pentamethylphenylsulfonamido)methyl)-N-(pyridin-3-yl)benzamide (72).

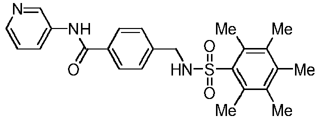

**Method C.** Reaction of amine salt **6** (329 mg, 0.85 mmol) and 2,3,4,5,6-pentamethylbenzene sulfonyl chloride (230 mg, 0.93 mmol) followed by column chromatography, eluting with EtOAc, gave benzamide **72** (191 mg, 51%) as a white powder: mp (EtOAc) 229-231 -C; ¹H NMR δ 10.32 (s, 1 H, NHCO), 8.92 (d, *J* = 2.3 Hz, 1 H, H-2'), 8.31 (dd, *J* = 4.7, 1.5 Hz, 1 H, H-6'), 8.17 (ddd, *J* = 8.3, 2.4, 1.5 Hz, 1 H, H-4'), 8.00 (br s, 1 H, NHSO₂), 7.85 (br d, *J* = 8.3 Hz, 2 H, H-2, H-6), 7.39 (ddd, J = 8.3, 4.7, 0.3 Hz, 1 H, H-5'), 7.31 (br d, *J* = 8.3 Hz, 2 H, H-3, H-5), 4.06 (s, 2 H, CH₂N), 2.48 (s, 6 H, 2 × CH₃), 2.20 (s, 3 H, CH₃), 2.15 (s, 6 H, 2 × CH₃); ¹³ CNMR8 165.3, 144.4, 141.9, 141.8, 138.5, 136.9, 135.7, 134.0, 133.2 (2), 132.6, 127.3 (2), 127.2 (2), 127.1 (2), 123.4, 45.6, 18.6 (2), 17.2, 16.5 (2); MS *m*/*z* 438.7 (MH⁺, 100%). Anal. Calcd for C₂₄H₂₇N₃O₃S: C, 65.88; H, 6.22; N, 9.60. Found: C, 65.92; H, 6.12; N, 9.70%.

### Example 64

### Preparation of 4-((2,4-Dimethylphenylsulfonamido)methyl)-N-(pyridin-3-yl)benzamide (73).

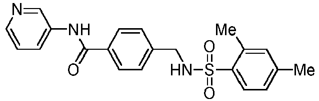

**Method C.** Reaction of amine salt **6** (333 mg, 0.86 mmol) and 2,4-dimethylbenzene sulfonyl chloride (193 mg, 0.94 mmol) followed by column chromatography, eluting with EtOAc, gave benzamide **73** (156 mg, 46%) as a white powder: mp (EtOAc) 180-182 °C; ¹H NMR δ 10.35 (s, 1 H, NHCO), 8.92 (d, *J* = 2.2 Hz, 1 H, H-2'), 8.31 (dd, *J* = 4.7, 1.5 Hz, 1 H, H-6'), 8.16-8.21 (m, 2 H, H-4', NHSO₂), 7.89 (br d, *J* = 8.3 Hz, 2 H, H-2, H-6), 7.70 (d, *J* = 8.0 Hz, 1 H, H-6"), 7.35-7.41 (m, 3 H, H-3, H-5, H-5'), 7.19 (br s, 1 H, H-3"), 7.15 (br d, *J* = 8.0 Hz, 1 H, H-5"), 4.07 (s, 2 H, CH₂N), 2.54 (s, 3 H, CH₃), 2.32 (s, 3 H, CH₃); ¹³C NMR δ 165.4, 144.4, 142.4, 142.0, 141.9, 136.2, 135.8, 135.6, 132.9, 132.8, 128.5, 127.5 (2), 127.2 (2), 127.1, 126.4, 123.4, 45.3, 20.5, 19.6; MS *m*/*z* 396.5 (MH⁺, 100%). Anal. Calcd for C₂₁H₂₁N₃O₃S: C, 63.78; H, 5.35; N, 10.63. Found: C, 64.07; H, 5.38; N, 10.73%.

### Example 65

### Preparation of 4-((3,4-Dimethylphenylsulfonamido)methyl)-N-(pyridin-3-yl)benzamide (74).

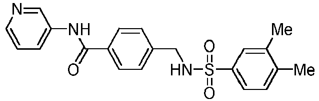

**Method C.** Reaction of amine salt **6** (284 mg, 0.73 mmol) and 3,4-dimethylbenzene sulfonyl chloride (164 mg, 0.83 mmol) followed by column chromatography, eluting with EtOAc, gave benzamide **74** (130 mg, 45%) as a white powder: mp (EtOAc) 181-183 °C; ¹H NMR δ 10.37 (s, 1 H, NHCO), 8.92 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.10 (br s, 1 H, NHSO₂), 7.90 (br d, *J* = 8.4 Hz, 2 H, H-2, H-6), 7.50-7.56 (m, 2 H, H-2", H-6"), 7.37-7.43 (m, 3 H, H-3, H-5, H-5'), 7.34 (d, *J* = 7.7 Hz, 1 H, H-5"), 4.07 (br d, *J* = 4.8 Hz, 2 H, CH₂N), 2.29 (s, 3 H, CH₃), 2.28 (s, 3 H, CH₃); ¹³C NMR δ 165.4, 144.4, 141.9, 141.8, 141.3, 137.8, 137.3, 135.7, 132.9, 129.9, 127.5 (2), 127.3 (2), 127.2, 127.1, 124.0, 123.4, 45.6, 19.3, 19.2; MS *m*/*z* 396.6 (MH⁺, 100%). Anal. Calcd for C₂₁ H₂₁ N₃O₃S: C, 63.78; H, 5.35; N, 10.63. Found: C, 64.01; H, 5.36; N, 10.65%.

### Example 66

### Preparation of 4-((3,5-Dimethylphenylsulfonamido)methyl)-N-(pyridin-3-yl)benzamide (75).

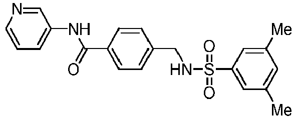

**Method C.** Reaction of amine salt **6** (404 mg, 1.0 mmol) and 3,5-dimethylbenzenesulfonyl chloride (234 mg, 1.1 mmol) followed by column chromatography, eluting with EtOAc, to give benzamide **75** (277 mg, 67%) as a white powder: mp (EtOAc/pet. ether) 181-183 -C; ¹H NMR δ 10.37 (s, 1 H, NHCO), 8.93 (d, *J* = 2.5 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.13 (br s, 1 H, NHSO₂), 7.91 (br d, *J* = 8.3 Hz, 2 H, H-2, H-6), 7.37-7.43 (m, 5 H, H-3, H-5, H-5', H-2", H-6"), 7.26 (br s, 1 H, H-4"), 4.08 (s, 2 H, CH₂N), 2.34 (s, 6 H, 2 x CH₃); ¹³C NMR δ 165.4, 144.4, 141.9, 141.8, 140.4, 138.5 (2), 135.7, 132.9, 127.5 (2), 127.3 (2), 127.2, 123.8 (2), 123.4, 45.6, 20.6 (2); MS *m*/*z* 396.4 (MH⁺, 100%). Anal. Calcd for C₂₁H₂₁N₃O₅S: C, 63.78; H, 5.35; N, 10.63. Found: C, 63.77; H, 5.41; N, 10.77%.

### Example 67

### Preparation of 4-((3-Fluoro-4-methylphenylsulfonamido)methyl)-N-(pyridin-3-yl)benzamide (76).

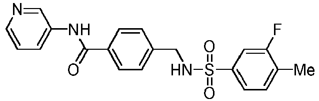

**Method C.** Reaction of amine salt **6** (392 mg, 1.0 mmol) and 3-fluoro-4-methylbenzene sulfonyl chloride (231 mg, 1.1 mmol) followed by column chromatography, eluting with EtOAc, gave benzamide **76** (192 mg, 48%) as a white powder: mp (EtOAc) 202-204 °C; ¹H NMR δ 10.36 (s, 1 H, NHCO), 8.93 (d, *J* = 2.3 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.28 (br s, 1 H, NHSO₂), 7.91 (br d, *J* = 8.3 Hz, 2 H, H-2, H-6), 7.48-7.57 (m, 3 H, H-2", H-5", H-6"), 7.36-7.42 (m, 3 H, H-3, H-5, H-5'), 4.11 (s, 2 H, CH₂N), 2.30 (d, *J* = 1.9 Hz, 3 H, CH₃); ¹³C NMR δ 165.4, 159 (d, *J* = 247 Hz), 144.4, 141.9, 141.5, 139.9 (d, *J* = 7 Hz), 135.6, 132.9, 132.3 (d, *J* = 5 Hz), 129.4 (d, *J* = 17 Hz), 127.6 (2), 127.4 (2), 127.2, 123.3, 122.3 (d, *J* = 3 Hz), 113.0 (d, *J* = 25 Hz), 45.6, 14.0 (d, *J* = 4 Hz); MS *m*/*z* 400.5 (MH⁺, 100%). Anal. Calcd for C₂₀H₁₈FN₃O₃S: C, 60.14; H, 4.54; N, 10.59. Found: C, 60.19; H, 4.53; N, 10.59%.

### Example 68

### Preparation of 4-((3-Chloro-2-methylphenylsulfonamido)methyl)-N-(pyridin-3-yl)benzamide (77)

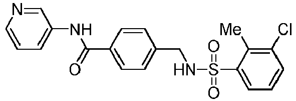

**Method C.** Reaction of amine salt **6** (400 mg, 1.0 mmol) and 3-chloro-2-methylbenzenesulfonyl chloride (255 mg, 1.1 mmol) followed by column chromatography, eluting with EtOAc, gave the benzamide **77** (274 mg, 64%) as a white powder: mp (EtOAc) 218-221 °C; ¹H NMR δ 10.35 (s, 1 H, NHCO), 8.93 (d, *J* = 2.3 Hz, 1 H, H-2'), 8.55 (br s, 1 H, NHSO₂), 8.31 (dd, *J* = 4.7, 1.4 Hz, 1 H, H-6'), 8.19 (d, *J* = 8.3 Hz, 1 H, H-4'), 7.89 (d, *J* = 8.3 Hz, 2 H, H-2, H-6), 7.84 (dd, *J* = 7.9, 1.0 Hz, 1 H, H-4"),* 7.70 (dd, *J* = 8.0, 0.8 Hz, 1 H, H-6"),* 7.35-7.41 (m, 4 H, H-3, H-5, H-5', H-5"), 4.16 (s, 2 H, CH₂N), 2.61 (s, 3 H, CH₃); ¹³C NMR δ 165.3, 144.4, 141.9, 141.7, 141.1, 135.7 (2), 133.9, 133.0, 132.9, 127.5 (2), 127.3 (2), 127.3 (2), 127.2, 123.4, 45.4, 16.4. Anal. Calcd for C₂₀H₁₈ClN₃O₃S: C, 57.76; H, 4.36; N, 10.10. Found: C, 57.79; H, 4.23; N, 10.04%. * assignments interchangeable

### Example 69

### Preparation of 4-((3-Chloro-4-methylphenylsulfonamido)methyl)-N-(pyridin-3-yl)benzamide (78).

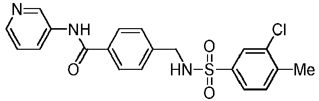

**Method C.** Reaction of amine salt **6** (400 mg, 1.0 mmol) and 3-chloro-4-methylbenzenesulfonyl chloride (255 mg, 1.1 mmol) followed by column chromatography, eluting with EtOAc, gave the sulfonamide **78** (267 mg, 63%) as a white powder: mp (EtOAc) 203-205 °C; ¹H NMR δ 10.36 (s, 1 H, NHCO), 8.93 (d, *J* = 2.3 Hz, 1 H, H-2'), 8.30-8.33 (m, 2 H, H-6', NHSO₂), 8.18 (d, *J* = 8.3 Hz, 1 H, H-4'), 7.90 (d, *J* = 8.3 Hz, 2 H, H-2, H-6), 7.73 (d, *J* = 1.8 Hz, 1 H, H-2"), 7.66 (dd, *J* = 8.0, 1.8 Hz, 1 H, H-6"), 7.55 (d, *J* = 8.0 Hz, 1 H, H-5"), 7.37-7.41 (m, 3 H, H-3, H-5, H-5'), 4.11 (d, *J* = 4.8 Hz, 2 H, CH₂N), 2.39 (s, 3 H, CH₃); ¹³C NMR δ 165.3, 144.4, 141.9, 141.4, 140.4, 139.8, 135.7, 133.7, 132.9, 131.8, 127.6 (2), 127.4 (2), 127.2, 126.5, 125.0, 123.4, 45.7, 19.5. Anal. Calcd for C₂₀H₁₈ClN₃O₃S: C, 57.76; H, 4.36; N, 10.10. Found: C, 57.97; H, 4.48; N, 10.13%.

### Example 70

### Preparation of 4-((3,4-Dichlorophenylsulfonamido)methyl)-N-(pyridin-3-yl)benzamide (79).

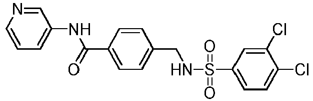

**Method C.** Reaction of amine salt **6** (379 mg, 0.97 mmol) and 3,4-dichlorobenzene sulfonyl chloride (263 mg, 1.1 mmol) followed by column chromatography, eluting with EtOAc, gave benzamide **79** (144 mg, 34%) as a white powder: mp (EtOAc) 219-221 °C; ¹H NMR δ 10.34 (s, 1H, NHCO), 8.92 (d, *J* = 2.4 Hz, 1 H, H-2'), 8.48 (br s, 1 H, NHSO₂), 8.30 (dd, *J* = 4.7, 1.5 Hz, 1 H, H-6'), 8.18 (ddd, *J* = 8.3, 2.5, 1.5 Hz, 1 H, H-4'), 7.88-7.93 (m, 3 H, H-2, H-6, H-2"), 7.85 (d, *J* = 8.4 Hz, 1H, H-5"), 7.75 (dd, *J* = 8.4, 2.2 Hz, 1 H, H-6"), 7.37-7.42 (m, 3 H, H-3, H-5, H-5'), 4.16 (s, 2 H, CH₂N); ¹³C NMR δ 165.2, 144.4, 141.9, 141.2, 141.0, 135.6, 135.3, 133.7, 131.9, 131.4, 128.2, 127.6 (2), 127.4 (2), 127.2, 126.5, 123.3, 45.6; MS *m*/*z* 436.6 (MH⁺, 100%), 438.6 (MH⁺, 70%). Anal. Calcd for C₁₉H₁₅N₃O₃S: C, 52.30; H, 3.47; N, 9.63. Found: C, 52.50; H, 3.46; N, 9.67%.

### Example 71

### Preparation of 4-((3-Cyano-4-fluorophenylsulfonamido)methyl)-N-(pyridin-3-yl)benzamide (80).

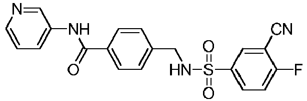

**Method C.** Reaction of amine salt **6** (422 mg, 1.1 mmol) and 3-cyano-4-fluorobenzene sulfonyl chloride (262 mg, 1.2 mmol) followed by column chromatography, eluting with EtOAc, gave benzamide **80** (149 mg, 34%) as a white powder: mp (EtOAc) 211-213 °C; ¹H NMR δ 10.35 (s, 1 H, NHCO), 8.91 (d, *J* = 2.4 Hz, 1 H, H-2'), 8.49 (br s, 1 H, NHSO₂), 8.31 (dd, *J* = 4.7, 1.4 Hz, 1 H, H-6'), 8.26 (dd, *J* = 6.0, 2.4 Hz, 1 H, H-2"), 8.18 (ddd, *J* = 8.3, 2.4, 1.5 Hz, 1 H, H-4'), 8.14 (ddd, *J* = 8.9, 5.0, 2.4 Hz, 1 H, H-6"), 7.91 (br d, *J* = 8.3 Hz, 2 H, H-2, H-6), 7.73 (t, *J* = 9.0 Hz, 1 H, H-5"), 7.37-7.42 (m, 3 H, H-3, H-5, H-5'), 4.18 (s, 2 H, CH₂N); ¹³C NMR δ 165.3, 164.0 (d, *J* = 262 Hz), 144.4, 141.9, 141.1, 138.1 (d, *J* = 3 Hz), 135.6, 134.2, (d, *J* = 10 Hz), 133.0, 132.5, 127.6 (2), 127.5 (2), 127.2, 123.3, 117.7 (d, *J* = 21 Hz), 112.7, 101.2 (d, *J* = 16 Hz), 45.6; MS *m*/*z* 411.6 (MH⁺, 100%). Anal. Calcd for C₂₀H₁₅FN₄O₃S: C, 58.53; H, 3.68; N, 13.65. Found: C, 58.59; H, 3.74; N, 13.58%.

### Example 72

### Preparation of 4-((Naphthalene-2-sulfonamido)methyl)-N-(pyridin-3-yl)benzamide (81).

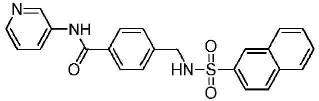

**Method C.** Reaction of amine salt **6** (400 mg, 1.0 mmol) and 2-naphthalenesulfonyl chloride (256 mg, 1.1 mmol) followed by column chromatography, eluting with EtOAc gave benzamide **81** (280 mg, 65%) as a white powder: mp (EtOAc) 210-213 -C; ¹H NMR δ 10.32 (s, 1 H, NHCO), 8.91 (d, *J* = 2.3 Hz, 1 H, H-2'), 8.45 (d, *J* = 1.5 Hz, 1 H, H-1"), 8.34 (br t, *J* = 6.1 Hz, 1 H, NHSO₂), 8.31 (dd, *J* = 4.7, 1.5 Hz, 1 H, H-6'), 8.12-8.16 (m, 3 H, H-4', H-3", H-4"), 8.04 (d, *J* = 7.8 Hz, 1 H, H-5"*), 7.84-7.89 (m, 3 H, H-2, H-6, H-8"*), 7.65-7.70 (m, 2 H, H-6", H-7"), 7.36-7.43 (m, 3 H, H-3, H-5, H-5'), 4.13 (d, *J* = 6.0 Hz, 2 H, CH₂N); ¹³C NMR δ 165.3, 144.4, 141.9, 141.7, 137.5, 135.6, 134.0, 132.9, 131.6, 129.3, 129.0, 128.5, 127.7, 127.5 (2), 127.4, 127.3 (2), 127.3, 127.2, 123.3, 122.1, 45.7. Anal. Calcd for C₂₃H₁₉N₃O₃S: C, 66.17; H, 4.59; N, 10.07. Found: C, 66.13; H, 4.77; N, 10.10%. * assignment interchangable

### Example 73

### Preparation of 4-((5-(Dimethylamino)naphthalene-1-sulfonamido)methyl)-N-(pyridin-3-yl)benzamide (82).

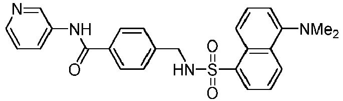

**Method C.** Reaction of amine salt **6** (417 mg, 1.1 mmol) and 5-(dimethylamino)naphthalene sulfonyl chloride (318 mg, 1.2 mmol) followed by column chromatography, eluting with EtOAc, gave benzamide **82** (20 mg, 4%) as a white powder: mp (EtOAc) 208-211 °C; ¹H NMR δ 10.28 (s, 1 H, NHCO), 8.90 (d, *J* = 2.3 Hz, 1 H, H-2'), 8.55 (m, 1 H, NHSO₂), 8.42 (d, *J* = 8.5 Hz, 1 H, H-2"), 8.32 (d, *J* = 9.0 Hz, 1 H, H-8"), 8.30 (dd, *J* = 4.7, 1.5 Hz, 1 H, H-6'), 8.15 (ddd, *J* = 8.3, 2.5, 1.5 Hz, 1 H, H-4'), 8.10 (dd, *J* = 7.3, 1.2 Hz, 1 H, H-4"), 7.77 (br d, *J* = 8.3 Hz, 2 H, H-2, H-6), 7.55-7.62 (m, 2 H, H-3", H-7"), 7.38 (ddd, *J* = 8.3, 4.7, 0.5 Hz, 1 H, H-5'), 7.28 (br d, *J* = 8.3 Hz, 2 H, H-3, H-5), 7.26 (d, *J* = 7.3 Hz, 1 H, H-6"), 4.13 (s, 2 H, CH₂N), 2.81 [s, 6 H, N(CH₃)₂]; ¹³C NMR δ 165.3, 151.3, 144.5, 141.9 (2), 141.8, 136.0, 135.6, 132.6, 129.4, 128.9, 128.3, 127.8, 127.3 (3), 127.2 (2), 123.4, 123.3, 119.0, 115.0, 45.5, 44.9 (2); MS *m*/*z* 461.8 (MH⁺, 100%). Anal. Calcd for C₂₅H₂₄N₄O₃S: C, 65.20; H, 5.25; N, 12.17. Found: C, 65.08; H, 5.31; N, 11.91%.

### Example 74

### Preparation of 4-((2,3-Dihydro-1H-indene-5-sulfonamido)methyl)-N-(pyridin-3-yl)benzamide (83).

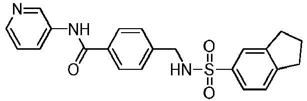

**Method C.** Reaction of amine salt **6** (438 mg, 1.1 mmol) and 2,3-dihydro-1H-indene-5-sulfonyl chloride (268 mg, 1.2 mmol) followed by column chromatography, eluting with EtOAc, gave benzamide **83** (229 mg, 50%) as a white powder: mp (EtOAc) 179-181 °C; ¹H NMR δ 10.35 (s, 1 H, NHCO), 8.92 (d, *J* = 2.3 Hz, 1 H, H-2'), 8.30 (dd, *J* = 4.7, 1.5 Hz, 1 H, H-6'), 8.18 (ddd, *J* = 8.3, 2.4, 1.5 Hz, 1 H, H-4'), 8.11 (br s, 1 H, NHSO₂), 7.89 (br d, *J* = 8.3 Hz, 2 H, H-2, H-6), 7.57-7.61 (m, 2 H, H-2", H-6"), 7.36-7.42 (m, 4 H, H-3, H-5, H-5', H-7"), 4.07 (s, 2 H, CH₂N), 2.88-2.93 (m, 4 H, H-1", H-3"), 2.04 (m, 2 H, H-2"); ¹³C NMR δ 165.4, 148.7, 144.8, 144.4, 141.9, 141.8, 138.5, 135.7, 132.8, 127.5 (2), 127.3 (2), 127.2, 124.6, 124.5, 123.3, 122.2, 45.7, 32.1, 31.9, 30.5; MS *m*/*z* 408.6 (MH⁺, 100%). Anal. Calcd for C₂₂H₂₁N₃O₃S: C, 64.85; H, 5.19; N, 10.31. Found: C, 64.78; H, 5.20; N, 10.29%.

### Example 75

### Preparation of 4-((2-(Dimethylamino)-2,3-dihydro-1H-indene-5-sulfonamido)methyl)-N-(pyridin-3-yl)benzamide (84).

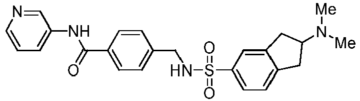

**Method C.** Reaction of amine salt **6** (142 mg, 0.36 mmol) and 2-(dimethylamino)-2,3-dihydro-1*H*-indene-5-sulfonyl chloride (100 mg, 0.38 mmol) followed by column chromatography, eluting with a gradient (0-15%) of MeOH/DCM, gave benzamide **84** (15 mg, 9%) as a white gum: ¹H NMR δ 10.36 (s, 1 H, NHCO), 8.94 (d, *J* = 2.4 Hz, 1 H, H-2'), 8.31 (dd, *J* = 4.7, 1.5 Hz, 1 H, H-6'), 8.19 (ddd, *J* = 8.3, 2.5, 1.5 Hz, 1 H, H-4'), 8.15 (br t, *J* = 6.4 Hz, 1 H, NHSO₂), 7.89 (br d, *J* = 8.3 Hz, 2 H, H-2, H-6), 7.59 (br d, *J* = 7.9 Hz, 1 H, H-7"), 7.55 (br s, 1 H, H-4"), 7.34-7.42 (m, 4 H, H-3, H-5, H-5', H-6"), 4.07 (d, *J* = 6.4 Hz, 2 H, CH₂N), 3.03-3.12 (m, 2 H, CH₂), 2.81-2.89 (m, 2 H, CH₂), 2.21-2.32 [m, 7 H, H-2", N(CH₃)₂]; ¹³C NMR δ 165.3, 146.2, 144.4, 142.4, 141.9, 141.7, 138.9, 135.7, 132.8, 127.5 (2), 127.3 (2), 127.1, 124.9, 124.7, 123.3, 122.2, 66.8, 45.6, 42.7 (2), 36.1, 35.9.

### Example 76

### Preparation of 4-((4-(4-Methylpiperazin-1-yl)phenylsulfonamido)methyl)-N-(pyridin-3-yl)benzamide (85).

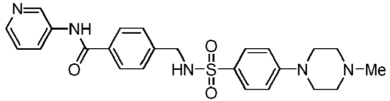

A mixture of fluoride **52** (696 mg, 1.8 mmol) and 4-methylpiperazine (3.0 mL) in DMSO (3 mL) was stirred in a sealed tube at 140 °C for 16 h. The solvent was evaporated and the residue was purified by column chromatography on neutral alumina, eluting with a gradient (0-5%) of MeOH/EtOAc, to give benzamide **85** (786 mg, 94%) as a white solid: mp (MeOH/EtOAc) 246-249 °C; ¹H NMR δ 10.39 (s, 1 H, NHCO), 8.91 (d, *J* = 2.4 Hz, 1 H, H-2'), 8.31 (dd, *J* = 4.7, 1.4 Hz, 1 H, H-6'), 8.19 (ddd, *J* = 8.3, 2.4, 1.5 Hz, 1 H, H-4'), 7.95 (t, *J* = 6.3 Hz, 1 H, NHSO₂), 6.90 (br d, *J* = 8.3 Hz, 2 H, H-2, H-6), 7.63 (d, *J* = 8.9 Hz, 2 H, H-2", H-6"), 7.36-7.42 (m, 3 H, H-3, H-5, H-5'), 7.08 (d, *J* = 8.9 Hz, 2 H, H-3", H-5"), 4.01 (d, *J* = 6.3 Hz, 2 H, CH₂N), 3.47 (br s, 4 H, 2 x CH₂N), 2.94 (br s, 4 H, 2 x CH₂N), 2.57 (s, 3 H, NCH₃); MS *m*/*z* 466.8 (MH⁺, 100%). The compound was recrystallized as the dihydrochloride salt. Anal. Calcd for C₂₄H₂₈ClN₅O₃S: C, 57.42; H, 5.62; N, 13.95. Found: C, 57.14; H, 5.62; N, 13.76%.

### Example 77

### Preparation of 4-[({4-[(Dimethylamino)methyl]phenyl}sulfonyl)amino]methyl-N-(3-pyridinyl)benzamide (86).

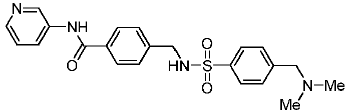

**Method C.** Reaction of amine salt **6** (300mg, 0.77 mmol) and 4-(bromomethyl)benzenesulfonyl chloride (249 mg, 0.93 mmol) gave the intermediate bromide. Dimethylamine (40% solution in H₂O, 2.0 mL, 39.5 mmol) was added and the mixture was warmed to 20 °C and stirred for a further 2 h. H₂O (50 mL) was added and the solvent evaporated and the residue triturated with H₂O (25 mL). The crude solid was purified by column chromatography, eluting with a gradient (0-10%) of MeOH/DCM followed by 1% cNH₃/10% MeOH/DCM, to give benzamide **86** (138 mg, 42%) as a pale yellow powder: mp 186-188 °C; ¹H NMR δ 10.36 (s, 1 H, NHCO), 8.93 (br s, 1 H, H-2'), 8.32-8.35 (m, 2 H, H-6', NHSO₂), 8.18 (ddd, *J* = 8.4, 2.3, 1.5 Hz, 1 H, H-4'), 7.88 (br d, *J* = 8.3 Hz, 2 H, H-2, H-6), 7.83 (br d, *J* = 8.1 Hz, 2 H, H-2", H-6"), 7.60 (br d, *J* = 7.8 Hz, 2 H, H-3", H-5"), 7.38-7.41 (m, 3 H, H-3, H-5, H-5'), 4.13 (d, *J* = 6.3 Hz, 2 H, CH₂N), CH₂, N(CH₃)₂ not observed; ¹³C NMR δ 165.3, 144.4, 141.9, 141.6, 139.9, 135.7, 132.8, 129.8 (2), 127.5 (2), 127.3 (2), 127.2, 126.5 (2), 123.4, 61.3, 45.6, 43.9 (2), one C not observed; MS *m*/*z* 425.9 (MH⁺, 100%). Anal. calcd for C₂₂H₂₄N₄O₃S·½CH₂Cl₂: C, 57.87; H, 5.40; N, 12.00. Found: C, 57.74; H, 5.61; N, 12.17%.

### Example 78

### Preparation of 4-{[({4-[(Diethylamino)methyl]phenyl}sulfonyl)amino]methyl}-N-(3-pyridinyl)benzamide (87).

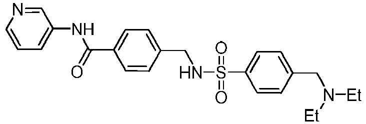

**Method C.** Reaction of amine salt **6** (300 mg, 0.77 mmol) and 4-(bromomethyl)benzenesulfonyl chloride (249 mg, 0.93 mmol) gave the intermediate bromide. Diethylamine (0.5 mL, 4.9 mmol) was added and the mixture was warmed to 20 °C and stirred for a further 5 h. The solvent was evaporated, H₂O (10 mL) added to the residue, the resulting precipitate triturated with H₂O (2 × 15 mL) and filtered. The crude solid was purified by column chromatography, eluting with a gradient (0-10%) of MeOH/DCM, to give benzamide **87** (66 mg, 19%) as a yellow powder: mp (MeOH/DCM) 159-162 °C; ¹H NMR δ 10.35 (s, 1 H, NHCO), 8.92 (d, *J* = 2.4 Hz, 1 H, H-2'), 8.31 (dd, *J* = 4.7, 1.4 Hz, 1 H, H-6'), 8.20 (br s, 1 H, NHSO₂), 8.18 (ddd, *J* = 8.4, 2.5, 1.5 Hz, 1 H, H-4'), 7.87 (br d, *J* = 8.3 Hz, 2 H, H-2, H-6), 7.73 (br s, 2 H, H-2", H-6"), 7.49 (br s, 2 H, H-3", H-5"), 7.37-7.41 (m, 3 H, H-3, H-5, H-5'), 4.11 (d, *J* = 5.9 Hz, 2 H, CH₂N), 3.58 (s, 2 H, CH₂N), 2.45 (br s, 4 H, 2 × CH₂), 0.96 (br s, 6 H, 2 × CH₃); ¹³C NMR δ 165.4, 144.4, 141.9, 141.7, 135.7, 132.8, 128.8 (2), 127.5 (2), 127.3 (2), 127.1, 126.4 (2), 123.4, 56.2, 55.9 (2), 45.6, 11.3 (2), two C not observed; MS *m*/*z454.1* (MH⁺, 100%); HRMS (FAB⁺) calcd for C₂₄H₂₉N₄O₃S (MH⁺) *m*/*z* 453.1960, found 453.1962. Anal. calcd for C₂₄H₂₈N₄O₃S·2H₂O: C, 59.00; H, 6.60, N, 11.47. Found: C, 58.67; H, 5.83; N, 11.26%.

### Example 79

### Preparation of 4-{[({4-[(Dipropylamino)methyl]phenyl}sulfonyl)amino]methyl}-N-(3-pyridinyl)benzamide (88).

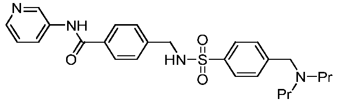

**Method C.** Reaction of amine salt **6** (150 mg, 0.39 mmol) and 4-(bomomethyl)benzenesulfonyl chloride (125 mg, 0.46 mmol) gave the intermediate bromide. Dipropylamine (0.21 mL, 1.5 mmol) was added and the mixture was warmed to 20 °C and stirred for a further 3 h. The solvent was evaporated and the residue was dissolved in DCM (50 mL), washed with H₂O (110 mL) and dried. The crude solid was purified by column chromatography, eluting with a gradient (0-10%) of MeOH/DCM and recrystallized from DCM/MeOH/iPr₂O. The solid was then triturated with H₂O (5 mL) and EtOAc (2 mL) to give benzamide **88** (42 mg, 23%) as a pale yellow powder: mp (MeOH/DCM) 184-187 °C; ¹H NMR δ 10.35 (s, 1 H, NHCO), 8.92 (d, *J* = 2.4 Hz, 1 H, H-2'), 8.31 (dd, *J* = 4.7, 1.4 Hz, 1 H, H-6'), 8.17-8.21 (m, 2 H, H-4', NHSO₂), 7.87 (br d, *J* = 8.3 Hz, 2 H, H-2, H-6), 7.73 (br d, *J* = 8.3 Hz, 2 H, H-2", H-6"), 7.48 (br d, *J* = 8.3 Hz, 2 H, H-3", H-5"), 7.37-7.40 (m, 3 H, H-3, H-5, H-5'), 4.10 (d, *J* = 6.1 Hz, 2 H, CH₂N), 3.58 (s, 2 H, CH₂N), 2.32 (t, *J* = 7.4 Hz, 4 H, 2 x CH₂N), 1.41 (sextet, *J* = 7.3 Hz, 4 H, 2 × CH₂), 0.81 (t, *J* = 7.4 Hz, 6 H, 2 × CH₃); ¹³C NMR δ 165.3, 145.2, 144.4, 141.8, 141.7, 138.8, 135.7, 132.8, 128.7 (2), 127.5 (2), 127.3 (2), 127.1, 126.2 (2), 123.3, 57.4, 55.3 (2), 45.6, 19.6 (2), 11.6 (2); MS *m*/*z* 482.1 (MH⁺, 100%); HRMS (FAB⁺) calcd for C₂₆H₃₃N₄O₃S (MH⁺) *m*/*z* 481.2273, found 481.2271. Anal. calcd for C₂₆H₃₂N₄O₃S·¼CH₃OH: C, 64.52; H, 6.81; N, 11.47. Found: C, 64.39; H, 6.82; N, 11.55%.

### Example 80

### Preparation of 4-[({[4-(1-Pyrrolidinylmethyl)phenyl]sulfonyl}amino)methyl]-N-(3-pyridinyl)-benzamide (89).

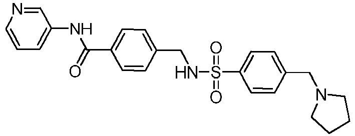

**Method C.** Reaction of amine salt **6** (300 mg, 0.77 mmol) and 4-(bromomethyl)benzenesulfonyl chloride (249 mg, 0.93 mmol) gave the intermediate bromide. Pyrrolidine (0.4 mL, 4.8 mmol) was added and the mixture was warmed to 20 °C and stirred for a further 5 h. The solvent was removed under reduced pressure, H₂O (10 mL) added to the residue, the resulting precipitate triturated with H₂O (2 × 15 mL) and filtered. The crude solid was purified by column chromatography, eluting with a gradient (0-10%) of MeOH/DCM, to give benzamide **89** (191 mg, 55%) as a yellow powder: mp (MeOH/DCM) 114-118 °C; ¹H NMR δ 10.36 (s, 1 H, NHCO), 8.93 (d, *J* = 2.3 Hz, 1 H, H-2'), 8.31 (dd, *J* = 4.7, 1.5 Hz, 2 H, H-6', NHSO₂), 8.18 (ddd, *J* = 8.4, 2.5, 1.5 Hz, 1 H, H-4'), 7.88 (br d, *J* = 8.3 Hz, 2 H, H-2, H-6), 7.79 (br d, *J* = 7.4 Hz, 2 H, H-2", H-6"), 7.59 (br s, 2 H, H-3", H-5"), 7.37-7.41 (m, 3 H, H-3, H-5, H-5'), 4.11 (d, *J* = 6.2 Hz, 2 H, CH₂N), 3.94 (s, 2 H, CH₂N), 2.73 (br s, 4 H, 2 × NCH₂), 1.80 (br s, 4 H, 2 × CH₂); ¹³C NMR δ 165.4, 144.4, 141.9, 141.6, 140.2, 135.7, 132.8, 129.8 (2), 127.5 (2), 127.3 (2), 127.2, 126.5 (2), 123.3, 57.5, 53.2 (2), 45.6, 22.7 (2), one C not observed; MS *mlz* 452.0 (MH⁺, 100%); MS *m*/*z* 452.0 (MH⁺, 100%); HRMS (FAB⁺) calcd for C₂₄H₂₇N₄O₃S (MH⁺) *mlz* 451.1804, found 451.1806.

### Example 81

### Preparation of 4-[({[4-(1-Piperidinylmethyl)phenyl]sulfonyl}amino)methyl]-N-(3-pyridinyl)benzamide (90).

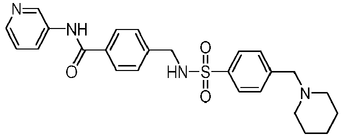

**Method C.** Reaction of amine salt **6** (150 mg, 0.39 mmol) and 4-(bromomethyl)benzenesulfonyl chloride (109 mg, 0.41 mmol) gave the intermediate bromide. Piperidine (0.20 mL, 1.9 mmol) was added and the mixture was warmed to 20 °C and stirred for 3 h. The solvent was evaporated and the residue was dissolved in DCM (50 mL), washed with H₂O (100 mL) and dried. The crude solid was purified by column chromatography, eluting with a gradient (0-10%) of MeOH/DCM, to give benzamide **90** (63 mg, 35%) as pale yellow crystals: mp (MeOH/DCM) 201-203 °C; ¹H NMR δ 10.35 (s, 1 H, NHCO), 8.92 (d, *J* = 2.3 Hz, 1 H, H-2'), 8.31 (dd, *J* = 4.7, 1.3 Hz, 1 H, H-6'), 8.16-8.23 (m, 2 H, H-4', NHSO₂), 7.87 (br d, *J* = 8.3 Hz, 2 H, H-2, H-6), 7.74 (br d, *J* = 8.3 Hz, 2 H, H-2", H-6"), 7.46 (br d, *J* = 8.3 Hz, 2 H, H-3", H-5"), 7.37-7.40 (m, 3 H, H-3, H-5, H-5'), 4.11 (d, *J* = 6.3 Hz, 2 H, CH₂N), 3.48 (s, 2 H, CH₂N), 2.30 (br s, 4 H, 2 × NCH₂), 1.48 (p, *J* = 5.5 Hz, 4 H, 2 × CH₂), 1.36 (m, 2 H, CH₂); ¹³C NMR δ 165.4, 144.4, 143.5, 141.9, 141.7, 139.1, 135.7, 132.8, 129.0 (2), 127.5 (2), 127.3 (2), 127.1, 126.3 (2), 123.3, 61.9, 53.8 (2), 45.6, 25.4 (2), 23.7; MS *m*/*z* 466.1 (MH⁺, 100%). Anal. calcd for C₂₅H₂₈N₄O₃S: C, 64.63; H, 6.07; N, 12.06. Found: C, 64.35; H, 6.21; N, 11.98%.

### Example 82

### Preparation of 4-[({[4-(1-Azepanylmethyl)phenyl]sulfonyl}amino)methyl]-N-(3-pyridinyl)benzamide (91).

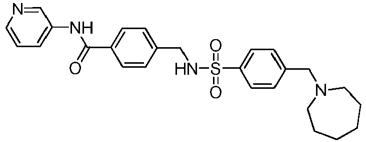

**Method C.** Reaction of amine salt **6** (150 mg, 0.39 mmol) and 4-(bromomethyl)benzenesulfonyl chloride (125 mg, 0.46 mmol) gave the intermediate bromide. Hexamethyleneimine (0.22 mL, 1.9 mmol) was added and the mixture was warmed to 20 °C and stirred for a further 4 h. The solvent was evaporated and H₂O (20 mL) added to the residue. The resulting solid was filtered and washed with H₂O (50 mL). The crude solid was purified by column chromatography, eluting with a gradient (0-10%) of MeOH/DCM, to give benzamide **91** (77 mg, 42%) as a cream powder: mp (MeOH/DCM) 170-172 °C; ¹H NMR δ 10.35 (s, 1 H, NHCO), 8.92 (d, *J* = 2.4 Hz, 1 H, H-2'), 8.31 (dd, *J* = 4.7, 1.4 Hz, 1 H, H-6'), 8.22 (br s, 1 H, NHSO₂), 8.18 (ddd, *J* = 8.4, 2.5, 1.5 Hz, 1 H, H-4'), 7.87 (br d, *J =* 8.3 Hz, 2 H, H-2, H-6), 7.74 (br d, *J* = 7.8 Hz, 2 H, H-2", H-6"), 7.49 (br d, *J* = 6.3 Hz, 2 H, H-3", H-5"), 7.37-7.41 (m, 3 H, H-3, H-5, H-5'), 4.12 (d, *J* = 6.3 Hz, 2 H, CH₂N), 3.66 (s, 2 H, CH₂N), 2.55 (br s, 4 H, 2 × NCH₂), 1.55 (br s, 8H, 4 × CH₂); ¹³C NMR δ 165.4, 144.4, 141.9, 141.7, 139.1, 135.7, 132.8, 128.9(2), 127.5(2), 127.3(2), 127.1, 126.3(2), 123.4, 60.9, 54.8 (2), 45.6, 27.5 (2), 26.3 (2), one C not observed; MS *m*/*z* 480.1 (MH⁺, 100%); HRMS (FAB⁺) calcd for C₂₆H₃₁N₄O₃S (MH⁺) *m*/*z* 479.2117, found 479.2113.

### Example 83

### Preparation of 4-[({[4-(4-Morpholinylmethyl)phenyl]sulfonyl}amino)methyl]-N-(3-pyridinyl)benzamide (92).

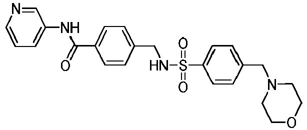

Method C. Reaction of amine salt 6 (150 mg, 0.39 mmol) and 4-(bromomethyl)benzenesulfonyl chloride (125 mg, 0.46 mmol) gave the intermediate bromide. Morpholine (0.20 mL, 2.3 mmol) was added and the mixture was warmed to 20 °C and stirred for a further 5 h. The solvent was evaporated and the residue dissolved in CH₂Cl₂ (60 mL), washed with H₂O (80 mL). The solvent was evaporated and the crude solid purified by column chromatography, eluting with a gradient (0-10%) of MeOH/DCM, to give benzamide **92** (122 mg, 68%) as a yellow powder: mp (MeOH/DCM) 158-160 °C; ¹H NMR δ 10.34 (s, 1 H, NHCO), 8.92 (d, *J* = 2.3 Hz, 1 H, H-2'), 8.31 (dd, *J* **=** 4.7, 1.5 Hz, 1 H, H-6'), 8.23 (br s, 1 H, NHSO₂), 8.18 (ddd, *J* **=** 8.3, 2.5, 1.5 Hz, 1 H, H-4'), 7.87 (br d, *J* = 8.3 Hz, 2 H, H-2, H-6), 7.75 (br d, *J* = 8.3 Hz, 2 H, H-2", H-6"), 7.48 (br d, *J* = 8.4 Hz, 2 H, H-3", H-5"), 7.36-7.41 (m, 3 H, H-3, H-5, H-5'), 4.11 (s, 2 H, CH₂NH), 3.57 (t, *J* = 4.6 Hz, 4 H, 2 × OCH₂), 3.52 (s, 2 H, CH₂N), 2.34 (t, *J* = 4.6 Hz, 4 H, 2 × NCH₂); ¹³C NMR δ 165.4, 144.4, 142.7, 141.8, 141.7, 139.3, 135.7, 132.8, 129.2 (2), 127.5 (2), 127.3 (2), 127.1, 126.3 (2), 123.4, 66.0 (2), 61.5, 53.0 (2), 45.6; MS *m*/*z* 468.0 (MH⁺, 100%). Anal. calcd for C₂₄H₂₆N₄O₄S C, 61.78; H, 5.62; N, 12.01. Found: C, 62.01; H, 5.74; N, 12.13%.

### Example 84

### Preparation of 4-{[({4-[(4-Methoxy-1-piperidinyl)methyl]phenyl}sulfonyl)amino]methyl}-N-(3-pyridinyl)benzamide (93).

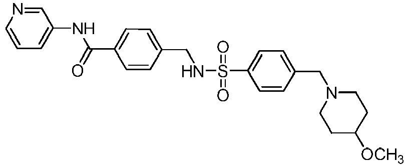

Method C. Reaction of amine salt 6 (340 mg, 0.87 mmol) and 4-(bromomethyl)benzenesulfonyl chloride (283 mg, 1.1 mmol) gave the intermediate bromide. 4-Methoxypiperidine hydrochloride (266 mg, 1.8 mmol) was added and the mixture was warmed to 20 °C and stirred for a further 150 mins. The solvent was evaporated, H₂O (30 mL) added to the residue and the resulting precipitate filtered. The aqueous layer was extracted with DCM (2 × 20 mL) and combined with the crude solid. The solvent was evaporated and the crude solid purified by column chromatography, eluting with a gradient (0-10%) of MeOH/DCM, to give benzamide **93** (67 mg, 16%) as a pale yellow powder: mp (MeOH/DCM) 182-184 °C; ¹H NMR δ 10.34 (s, 1 H, NHCO), 8.92 (d, *J* = 2.2 Hz, 1 H, H-2'), 8.30 (dd, *J* = 4.6, 1.2 Hz, 1 H, H-6'), 8.22 (t, *J* = 6.3 Hz, 1 H, NHSO₂) 8.18 (ddd, *J* = 8.4, 2.4, 1.5 Hz, 1 H, H-4'), 7.86 (br d, *J* = 8.3 Hz, 2 H, H-2, H-6), 7.73 (br d, *J* = 8.2 Hz, 2 H, H-2", H-6"), 7.46 (br d, *J* = 8.0 Hz, 2 H, H-3", H-5"), 7.36-7.40 (m, 3 H, H-3, H-5, H-5'), 4.11 (d, *J =* 6.3 Hz, 2 H, CH₂N), 3.50 (s, 2 H, CH₂N), 3.17 (s, 3 H, CH₃), 3.13 (m, 1 H, CH), 2.59 (m, 2 H, 2 × CH), 2.07 (m, 2 H, 2 × CH), 1.80 (m, 2 H, 2 × CH), 1.41 (m, 2 H, 2 × CH); ¹³C NMR δ 165.3, 144.4, 143.4, 141.8, 141.7, 139.2, 135.7, 132.8, 129.0 (2), 127.5 (2), 127.3 (2), 127.1, 126.3 (2), 123.3, 75.3, 61.1, 54.6, 50.3 (2), 45.6, 30.4 (2); MS *m*/*z* 496.1 (MH⁺, 100%); HRMS (FAB⁺) calcd for C₂₆H₃₀N₄O₄S (MH⁺) *m*/*z* 495.2066, found 495.2073. Anal. calcd for C₂₆H₃₀N₄O₄S·½CH₃OH: C, 62.33; H, 6.32; N, 10.97. Found: C, 62.28; H, 6.02; N 11.26%.

### Example 85

### Preparation of 4-{[({4-[(4-Methyl-1-piperazinyl)methyl]phenyl}sulfonyl)amino]methyl}-N-(3-pyridinyl)benzamide (94).

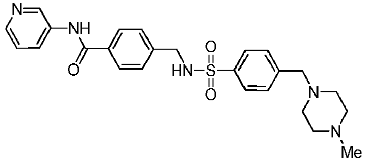

**Method C.** Reaction of amine salt **6** (250 mg, 0.64 mmol) and 4-(bromomethyl)benzenesulfonyl chloride (208 mg, 0.77 mmol) gave the intermediate bromide. 1-Methylpiperazine (0.36 mL, 3.3 mmol) was added and the mixture was warmed to 20 °C and stirred for 4 h. The solvent was evaporated, H₂O (25 mL) was added to the residue and the resulting precipitate filtered. The crude solid was purified by column chromatography, eluting with a gradient (0-10%) of MeOH/DCM, to give benzamide **94** (115 mg, 37%) as a pale tan powder: mp (MeOH/DCM) 158-162 °C,; ¹H NMR δ 10.36 (s, 1 H, NHCO), 8.93 (d, *J* = 2.4 Hz, 1 H, H-2'), 8.31 (dd, *J* = 4.7, 1.5 Hz, 1 H, H-6'), 8.25 (t, *J* = 6.4 Hz, 1 H, NHSO₂), 8.18 (ddd, *J* = 8.3, 2.5, 1.5 Hz, 1 H, H-4'), 7.87 (br d, *J* = 8.3 Hz, 2 H, H-2, H-6), 7.75 (br d, *J* = 8.3 Hz, 2 H, H-2", H-6"), 7.48 (br d, *J* = 8.2 Hz, 2 H, H-3", H-5"), 7.36-7.41 (m, 3 H, H-3, H-5, H-5'), 4.11 (d, *J =* 6.3 Hz, 2 H, CH₂N), 3.58 (s, 2 H, CH₂N), 2.80 (br s, 4 H, 2 × NCH₂), CH₃, 2 × NCH₂ not observed; ¹³C NMR δ 165.4, 144.4, 142.4, 141.9, 141.7, 139.4, 135.7, 132.8, 129.2 (2), 127.5 (2), 127.3 (2), 127.2, 126.4 (2), 123.3, 60.4, 53.3 (2), 50.3 (2), 45.6, 43.4; MS *m*/*z* 481.1 (MH⁺, 100%). Anal. calcd for C₂₅H₂₉N₅O₃S-CH₂Cl₂: C, 55.32; H, 5.53; N, 12.41. Found: C, 54.97; H, 5.91; N, 12.69%.

### Example 86

### Preparation of 4-tert-Butyl-N-4-(pyridin-3-ylcarbamoyl)benzyl)benzamide (95).

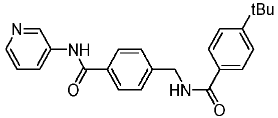

**Method A.** Reaction of 4-tert-butylbenzoic acid (207 mg, 1.2 mmol) and oxalyl chloride (0.15 mL, 1.7 mmol) with subsequent reaction with amine salt 6 (542 mg, 1.4 mmol), followed by column chromatography eluting with a gradient (0-10%) of MeOH/EtOAc, gave benzamide **95** (246 mg, 55%) as a white powder: mp (EtOAc) 182-184 °C; ¹H NMR δ 10.37 (s, 1 H, NHCO), 9.03 (t, *J* = 6.0 Hz, 1 H, NHCO), 8.92 (d, *J* = 2.2 Hz, 1 H, H-2'), 8.30 (dd, *J* = 4.7, 1.5 Hz, 1 H, H-6'), 8.18 (ddd, *J* = 8.3, 2.5, 1.5 Hz, 1 H, H-4'), 7.95 (br d, *J* = 8.3 Hz, 2 H, H-2, H-6), 7.86 (ddd, *J* = 8.5, 2.0, 1.8 Hz, 2 H, H-2", H-6"), 7.50 (ddd, *J* = 8.5, 2.0, 1.8 Hz, 2 H, H-3", H-5"), 7.47 (d, *J* = 8.3 Hz, 2 H, H-3, H-5), 7.38 (ddd, *J* = 8.3, 2.0, 0.5 Hz, 1 H, H-5'), 4.56 (d, *J* = 6.0 Hz, 2 H, CH₂N), 1.31 [s, 9 H, C(CH₃)₃]; ¹³C NMR δ 166.1, 165.6, 154.0, 144.4, 143.8, 141.9, 135.7, 132.6, 131.4, 127.6(2), 127.1, 127.0(2), 126.9(2), 125.0(2), 123.3, 42.2, 34.5, 30.8 (3); MS *m*/*z* 388.6 (MH⁺, 100%). Anal. Calcd for C₂₄H₂₅N₃O₂: C, 74.39; H, 6.50; N, 10.84. Found: C, 74.33; H, 6.52; N, 10.81%.

### Example 87

### Preparation of 3,5-Dimethyl-N-(4-(pyridin-3-ylcarbamoyl)benzyl)benzamide (96).

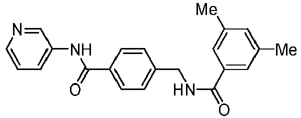

**Method A.** Reaction of 3,5-dimethylbenzoic acid (333 mg, 2.2 mmol) and oxalyl chloride (0.29 mL, 3.3 mmol) with subsequent reaction with amine salt **6** (1.04 g, 2.7 mmol), followed by column chromatography eluting with a gradient (0-10%) of MeOH/EtOAc, gave benzamide **96** (184 mg, 23%) as a white powder: mp (EtOAc) 241-243 °C; ¹H NMR δ 10.37 (s, 1 H, NHCO), 9.00 (t, *J* = 6.0 Hz, 1 H, NHCO), 8.94 (d, *J* = 2.3 Hz, 1 H, H-2'), 8.30 (dd, *J* = 4.7, 1.5 Hz, 1 H, H-6'), 8.18 (ddd, *J* = 8.3, 2.5, 1.5 Hz, 1 H, H-4'), 7.94 (br d, *J* = 8.3 Hz, 2 H, H-2, H-6), 7.52 (br s, 2 H, H-2", H-6"), 7.47 (br d, *J* = 8.3 Hz, 2 H, H-3, H-5), 7.38 (ddd, *J* = 8.3, 4.7, 0.5 Hz, 1 H, H-5'), 7.17 (s, 1 H, H-4"), 4.54 (d, *J* = 6.0 Hz, 2 H, CH₂N), 2.32 [s, 6 H, 2 × CH₃); ¹³C NMR δ 166.4, 165.6, 144.4, 143.8, 141.9, 137.3 (2), 135.7, 134.2, 132.7, 132.4, 127.7 (2), 127.1, 127.0 (2), 124.9 (2), 123.3, 42.3, 20.7 (2); MS *m*/*z* 360.5 (MH⁺, 100%). Anal. Calcd for C₂₂H₂₁N₃O₂: C, 73.52; H, 5.89; N, 11.69. Found: C, 73.22; H, 6.00; N, 11.59%.

### Example 88

### Preparation of 3,4-Dimethoxy-N-(4-(pyridin-3-ylcarbamoyl)benzyl)benzamide (97).

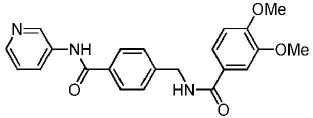

**Method A.** Reaction of 3,4-dimethoxybenzoic acid (247 mg, 1.4 mmol) and oxalyl chloride (0.18 mL, 2.0 mmol) with subsequent reaction with amine salt **6** (580 mg, 1.5 mmol), followed by column chromatography eluting with a gradient (0-10%) of MeOH/EtOAc, gave benzamide **97** (411 mg, 70%) as a white powder: mp (EtOAc) 219-220 °C; ¹H NMR δ 10.37 (s, 1 H, NHCO), 8.97 (t, *J* = 6.0 Hz, 1 H, NHCO), 8.92 (d, *J* = 2.3 Hz, 1 H, H-2'), 8.31 (dd, *J* = 4.7, 1.4 Hz, 1 H, H-6'), 8.18 (ddd, *J* = 8.3, 2.5, 1.5 Hz, 1 H, H-4'), 7.96 (br d, *J* = 8.3 Hz, 2 H, H-2, H-6), 7.55 (dd, *J* = 8.4, 2.0 Hz, 1 H, H-6"), 7.51 (d, *J* = 2.0 Hz, 1 H, H-2"), 7.48 (br d, *J* = 8.3 Hz, 2 H, H-3, H-5), 7.38 (dd, *J* = 8.3, 4.7 Hz, 1 H, H-5'), 7.03 (d, *J* = 8.4 Hz, 1 H, H 5"), 4.55 (br d, *J* = 6.0 Hz, 2 H, CH₂N), 3.82 (s, 3 H, OCH₃), 3.81 (s, 3 H, OCH₃); ¹³C NMR δ 165.7, 165.6, 151.3, 148.2, 144.4, 143.9, 141.9, 135.6, 132.7, 127.7 (2), 127.1, 127.0 (2), 126.4, 123.3, 120.4, 110.9, 110.6, 55.5, 55.4, 42.3; MS *m*/*z* 392.6 (MH⁺, 100%). Anal. Calcd for C₂₂H₂₁N₃O₄: C, 67.51; H, 5.41; N, 10.74. Found: C, 67.24; H, 5.36; N, 10.75%.

### Example 89

### Preparation of 4-[(4-tert-Butylphenylsulfonamido)methyl]-N-methyl-N-(3-pyridinyl)benzamide (99).

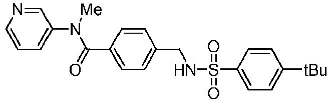

**4-(4-*tert-*Butylphenylsulfonamidomethyl)benzoic Acid (98).** 4-*tert-*Butylbenzenesulfonyl chloride (4.85 g, 20.8 mmol) was added dropwise to a stirred solution of 4-aminomethylbenzoic acid (3.0 g, 19.9 mmol) in 2 M NaOH (20 mL) at 20 °C. The mixture was stirred at 20° for 3 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The crude solid was purified by column chromatography, eluting with a gradient (5-20%) of MeOH/EtOAc, to give benzoic acid **98** (4.88 g, 71%) as a white powder: mp 290-291 °C; ¹H NMR δ 12.84 (br s, 1 H, CO₂H), 8.16 (br t, *J* = 6.1 Hz, 1 H, NHSO₂), 7.78 (br d, *J* = 8.2 Hz, 2 H, H-2, H-6), 7.68 (ddd, *J* = 8.6, 2.2, 1.9 Hz, 2 H, H-2', H-6'), 7.53 (ddd, *J* = 8.6, 2.2, 1.9 Hz, 2 H, H-3', H-5'), 7.27 (br d, *J* = 8.3 Hz, 2 H, H-3, H-5), 4.05 (br d, *J* = 6.1 Hz, 2 H, CH₂N), 1.29 [s, 9 H, C(CH₃)₃]. Anal. calcd for C₁₈H₂₁NO₄S: C, 62.23; H, 6.09; N, 4.03. Found: C, 62.32; H, 6.14; N, 4.06%.

**4-[(4-*tert-*Butylphenylsulfonamido)methyl]-*N-*methyl-*N*-(3-pyridinyl)benzamide (99). Method A.** Reaction of benzoic acid **98** (404 mg, 1.2 mmol) and oxalyl chloride (0.15 mL, 1.7 mmol) and subsequent reaction with 3-aminomethylpyridine (138 mg, 1.3 mmol), followed by column chromatography, eluting with EtOAc, gave benzamide **99** (137 mg, 27%) as a white powder: mp (EtOAc) 141-143 °C; ¹H NMR δ 8.34 (dd, *J* = 4.7, 1.4 Hz, 1 H, H-6'), 8.31 (d, *J* = 2.3 Hz, 1 H, H-2'), 8.05 (br t, *J* = 6.4 Hz, 1 H, NHSO₂), 7.66-7.71 (m, 3 H, H-4', H-2", H-6"), 7.57 (ddd, *J* = 8.7, 2.2, 1.8 Hz, 2 H, H-3", H-5"), 7.33 (ddd, *J* = 8.1, 4.7, 0.6 Hz, 1 H, H-5'), 7.19 (d, *J* = 8.3 Hz, 2 H, H-2, H-6), 7.11 (d, *J* = 8.3 Hz, 2 H, H-3, H-5), 3.93 (br d, *J =* 6.4 Hz, 2 H, CH₂N), 3.37 (s, 3 H, NCH₃), 1.30 [s, 9 H, C(CH)₃]; ¹³C NMR δ 169.3, 155.3, 148.1, 147.0, 141.0, 139.5, 137.9, 134.5, 134.1, 128.4 (2), 126.9 (2), 126.3 (2), 125.9 (2), 123.8, 45.5, 37.8, 34.8, 30.8 (3); MS *m*/*z* 438.6 (MH⁺, 100%). Anal. calcd for C₂₄H₂₇N₃O₃S·½EtOAc: C, 64.84; H, 6.49; N, 8.73. Found: C, 64.74; H, 6.36; N, 8.85%.

### Example 90

### Preparation of N-Methyl-4-{[(phenylsulfonyl)amino]methyl}-N-(3-pyridinyl)benzamide (100).

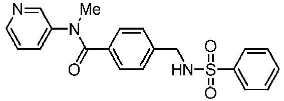

**Method A.** Reaction of benzoic acid **12** (406 mg, 1.4 mmol) and oxalyl chloride (0.18 mL, 2.1 mmol) and subsequent reaction with 3-aminomethylpyridine (165 mg, 1.5 mmol), followed by column chromatography, eluting with EtOAc, gave benzamide **100** (85 mg, 16%) as a white powder: mp (EtOAc) 170-172 °C; ¹H NMR δ 8.34 (dd, *J* = 4.7, 1.5 Hz, 1 H, H-6'), 8.32 (d, *J* = 2.3 Hz, 1 H, H-2'), 8.12 (br t, *J* = 6.3 Hz, 1 H, NHSO₂), 7.77 (ddd, *J* = 7.0, 2.1, 1.5 Hz, 2 H, H-2", H-6"), 7.69 (ddd, *J* = 8.2, 2.6, 1.5 Hz, 1 H, H-4'), 7.59-7.63 (m, 1 H, H-4"), 7.53-7.58 (m, 2 H, H-3", H-5"), 7.34 (ddd, *J* = 8.2, 4.7, 0.6 Hz, 1 H, H-5'), 7.19 (d, *J* = 8.3 Hz, 2 H, H-2, H-6), 7.11 (br d, *J* = 8.3 Hz, 2 H, H-3, H-5), 3.94 (d, *J* = 6.3 Hz, 2 H, CH₂N), 3.38 (s, 3 H, NCH₃); ¹³C NMR δ 169.3, 148.1, 147.1, 140.7, 139.4, 134.5, 134.1, 132.3, 129.1 (2), 128.3 (2), 126.9 (2), 126.4 (2), 123.4, 45.6, 37.8; MS *m*/*z* 381.6 (MH⁺, 100%). Anal. calcd for C₂₀H₁₉N₃O₃S.¼EtOAc: C, 62.52; H, 5.25; N, 10.41. Found: C, 62.36; H, 5.23; N, 10.57%.

### Example 91

### Preparation of 3-[(4-tert-Butylphenylsulfonamido)methyl]-N-(3-pyridinyl)benzamide (102).

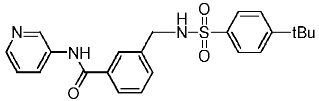

**3-(4-*tert*-Butylphenylsulfonamidomethyl)benzoic Acid (101).** 4-*tert-*Butylbenzenesulfonyl chloride (1.36 g, 5.9 mmol) was added in small portions to a stirred solution of 3-aminomethylbenzoic acid (1.0 g, 5.3 mmol) in 1 M NaOH (10 mL) at 20 °C. The mixture was stirred at 20 °C for 16 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The crude solid was purified by column chromatography, eluting with a gradient (0-5%) of MeOH/EtOAc, to give benzoic acid **101** (1.25 g, 65%) as a white powder: mp (MeOH/EtOAc) 191-193 °C; ¹H NMR δ 12.87 (br s, 1H, CO₂H), 8.15 (t, *J* = 6.4 Hz, 1 H, NHSO₂) 7.82 (br s, 1 H, H-2), 7.77 (br t, *J* = 7.7, 1.3 Hz, 1 H, H-6), 7.68 (ddd, *J* = 8.6, 2.2, 1.9 Hz, 2 H, H-2', H-6'), 7.54 (ddd, *J* = 8.6, 2.2, 1.9 Hz, 2 H, H-3', H-5'), 7.44 (br d, *J* = 7.9 Hz, 1 H, H-4), 7.37 (br t, *J* = 7.6 Hz, 1 H, H-5), 4.05 (br d, *J* = 6.1 Hz, 2 H, CH₂N), 1.29 [s, 9 H, C(CH₃)₃]; MS *m*/*z* 380.5 (MH⁺, 100%). Anal. calcd for C₁₈H₂₁NO₄S: C, 62.23; H, 6.09; N, 4.03. Found: C, 62.44; H, 6.23; N, 4.03%.

**3-[(4-*tert-*Butylphenylsulfonamido)methyl]-*N*-(3-pyridinyl)benzamide (102). Method A.** Reaction of benzoic acid **101** (0.35 g, 1.0 mmol) and oxalyl chloride (0.13 mL, 1.5 mmol) and subsequent reaction with 3-aminopyridine (105 mg, 1.1 mmol), followed by column chromatography, eluting with EtOAc, gave benzamide **102** (285 mg, 67%) as a white powder: mp (EtOAc) 183-186 °C; ¹H NMR δ 10.37 (s, 1 H, CONH), 8.92 (d, *J* = 2.2 Hz, 1 H, H-2'), 8.31 (dd, *J* = 4.6, 1.5 Hz, 1 H, H-6'), 8.13-8.20 (m, 2 H, H-5, H-4'), 7.83-7.86 (m, 2 H, H-2, NHSO₂), 7.73 (ddd, *J* = 8.6, 2.2, 1.9 Hz, 2 H, H-2", H-6"), 7.57 (ddd, *J* = 8.6, 2.2, 1.9 Hz, 2 H, H-3", H-5"), 7.44-7.49 (m, 2 H, H-4, H-6), 7.39 (br d, *J* = 8.7, 4.4 Hz, 1 H, H-5'), 4.09 (br d, *J =* 6.2 Hz, 2 H, CH₂N), 1.29 [s, 9 H, C(CH)₃]; ¹³C NMR δ 165.6, 155.3, 144.6, 142.0, 138.2, 137.8, 135.8, 134.2, 131.0, 128.3, 127.3, 127.1, 126.4 (2), 125.9 (2), 123.5, 45.9, 34.8, 30.8 (3), 1 C not observed; MS *m*/*z* 424.6 (MH⁺, 100%). Anal. calcd for C₂₃H₂₅N₃O₃S·¼CH₃OH: C, 64.71; H, 6.07; N, 9.73. Found: C, 64.76; H, 6.15; N, 9.71%.

### Example 92

### Preparation of 3-[(Phenylsulfonamido)methyl]-N-(3-pyridinyl)benzamide (104).

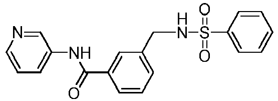

**3-(Phenylsulfonamidomethyl)benzoic Acid (103).** Benzenesulfonyl chloride (0.80 mL, 6.2 mmol) was added dropwise to a stirred solution of 3-aminomethylbenzoic acid (1.06 g, 5.7 mmol) in 1 M NaOH (12 mL) at 20 °C. The mixture was stirred at 20 °C for 16 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The crude solid was purified by column chromatography, eluting with a gradient (0-5%) of MeOH/EtOAc, to give benzoic acid 103 (0.96 g, 56%) as a white powder: mp (MeOH/EtOAc) 171-173 °C; ¹H NMR δ 12.91 (br s, 1 H, CO₂H), 8.23 (t, *J* = 6.3 Hz, 1 H, NHSO₂), 7.85 (br s, 1 H, H-2), 7.77-7.81 (m, 3 H, H-6, H-2', H-6'), 7.53-7.58 (m, 2 H, H-3', H-5'), 7.46 (br d, *J* = 7.8 Hz, 1 H, H-4), 7.39 (br t, *J* = 7.6 Hz, 1 H, H-5), 4.06 (d, *J* = 6.3 Hz, 2 H, CH₂N); MS *m*/*z* 292.5 (MH⁺, 100%). Anal. calcd for C₁₄H₁₃NO₄S: C, 57.72; H, 4.50; N, 4.81. Found: C, 57.77; H, 4.47; N, 4.78%.

**3-[(Phenylsulfonamido)methyl]-*N*-(3-pyridinyl)benzamide (104). Method A.** Reaction of benzoic acid **103** (0.56 g, 1.9 mmol) and oxalyl chloride (0.25 mL, 2.9 mmol) and subsequent reaction with 3-aminopyridine (200 mg, 2.1 mmol), followed by column chromatography, eluting with EtOAc, gave benzamide **104** (535 mg, 76%) as a white powder: mp (EtOAc) 74-78 °C; ¹H NMR δ 10.40 (s, 1 H, CONH), 8.93 (d, *J* = 2.3 Hz, 1 H, H-2'), 8.32 (dd, *J* = 4.6, 1.5 Hz, 1 H, H-6'), 8.23 (br t, *J* = 6.2 Hz, 1 H, NHSO₂), 8.18 (ddd, *J* = 8.3, 2.5, 1.5 Hz, 1 H, H-4'), 7.81-7.87 (m, 4 H, H-2, H-6, H-2", H-6"), 7.55-7.65 (m, 3 H, H-3", H-4", H-5"), 7.45-7.50 (m, 2 H, H-4, H-5), 7.39 (br dd, *J* = 8.3, 4.6 Hz, 1 H, H-5'), 4.09 (br d, *J* = 6.2 Hz, 2 H, CH₂N); ¹³C NMR δ 165.7, 144.6, 142.0, 140.6, 138.1, 135.7, 134.3, 132.4, 131.0, 129.2 (2), 128.3, 127.4, 127.1, 126.5 (2), 126.4, 123.5, 45.9; MS *m*/*z* 368.8 (MH⁺, 100%). Anal. calcd for C₁₉H₁₇N₃O₃S·¼EtOAc: C, 61.68; H, 4.92; N, 10.79. Found: C, 61.74; H, 4.90; N, 10.72%.

### Example 93

### Preparation of N-(1-Oxido-3-pyridinyl)-4-((phenylsulfonamido)methyl)-benzamide (105).

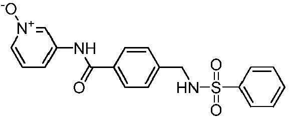

A mixture of MCPBA (223 mg, 0.65 mmol) and benzamide **10** (0.16 mL, 0.4 mol) in DCM (15 mL) was stirred at 20 °C for 16 h. The mixture was diluted with DCM (20 mL), washed with saturated aqueous KHCO₃ (2 × 5 mL), water (5 mL), brine (5 mL) and dried. The solvent was evaporated and the residue purified by column chromatography, eluting with a gradient (0-5%) of MeOH/DCM, to give *N-*oxide **105** (106 mg, 64%) as a white powder: mp (MeOH/EtOAc) 230-231 °C; ¹H NMR δ 10.52 br s, 1 H, NHCO), 8.92 (t, *J* = 1.7 Hz, 1 H, H-2"), 8.32 (br s, 1 H, NHSO₂), 8.00 (ddd, *J* = 6.4, 1.7, 0.8 Hz, 1 H, H-6"), 7.88 (br d, *J* = 8.3 Hz, 2 H, H-2, H-6), 7.82 (ddd, *J* = 6.9, 2.0, 1.4 Hz, 2 H, H-2', H-6'), 7.68 (ddd, *J* = 8.5, 1.7, 1.4 Hz, 1 H, H-4"), 7.57-7.66 (m, 3 H, H-3', H-4', H-5'), 7.37-7.43 (m, 3 H, H-3, H-5, H-5"), 4.08 (s, 2 H, CH₂N); ¹³C NMR δ 165.7, 142.2, 140.5, 138.4, 133.9, 132.5, 132.4, 130.8, 129.2 (2), 127.8 (2), 127.4 (2), 126.4 (2), 126.1, 116.7, 45.7; MS *m*/*z* 384.5 (MH⁺, 100%). Anal. calcd for C₁₉H₁₇N₃O₄S: C, 59.52; H, 4.47; N, 10.96. Found: C, 59.47; H, 4.36; N, 10.99%.

### Example 94

### Preparation of 4-((4-Iodophenylsulfonamido)methyl)-N-(pyridin-3-yl)benzamide (106).

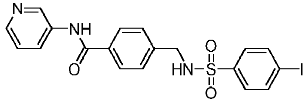

A mixture of 4-(aminomethyl)-*N*-(3-pyridinyl)benzamide **6** (727 mg, 3.2 mmol) and 4-iodobenzenesulfonyl chloride (970 mg, 3.2 mmol) in dry pyridine (10 mL) was stirred at 20 °C for 16 h. The solvent was evaporated and the residue stirred in water (20 mL) for 1 h. The precipitate was filtered, washed with water (5 mL) and dried. The crude solid was purified by column chromatography, eluting with a gradient (0-20%) of MeOH/EtOAc, to give benzamide **106** (1.47 g, 93%) as a cream powder: mp (MeOH/EtOAc) 249-251 °C; ¹H NMR δ 10.36 (s, 1 H, NHCO), 8.93 (d, *J* = 2.3 Hz, 1 H, H-2'), 8.34 (br s, 1 H, NHSO₂), 8.31 (dd, *J* = 4.7, 1.5 Hz, 1 H, H-6'), 8.18 (ddd, *J* = 8.3, 2.5, 1.5 Hz, 1 H, H-4'), 7.97 (ddd, *J* = 8.6, 2.2, 1.9 Hz, 2 H, H-2", H-6"), 7.90 (br d, *J* = 8.3 Hz, 2 H, H-2, H-6), 7.56 (ddd, *J* = 8.6, 2.2, 1.9 Hz, 2 H, H-3", H-5"), 7.37-7.42 (m, 3 H, H-3, H-5, H-5'), 4.09 (s, 2 H, CH₂N); ¹³C NMR δ 165.5, 144.5, 142.0, 141.6, 140.3, 138.1 (2), 135.8, 133.1, 128.2 (2), 127.7 (2), 127.5 (2), 127.3, 123.5, 100.3, 45.7; MS *m*/*z* 494.6 (MH⁺, 100%).

### Example 95

### Preparation of 4-((4-Ethynylphenylsulfonamido)methyl)-N-(pyridin-3-yl)benzamide (107).

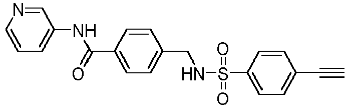

PdCl₂(PPh₃)₂ (47 mg, 66 □mol) was added to a stirred, degassed solution of iodide **106** (327 mg, 0.66 mmol), TMS-acetylene (0.93 mL, 6.6 mmol) and CuI (13 mg, 66 □mol) in Et₃N (3 mL) and DMF (3 mL), and the mixture was stirred in a sealed pressure vessel at 50 °C for 2 h. The mixture was cooled to 20 °C, diluted with EtOAc (150 mL) and washed with water (3 × 50 mL), washed with brine (50 mL) and dried. The solvent was evaporated and the residue suspended in MeOH (20 mL) and K₂CO₃ (110 mg, 0.72 mmol) was added. The mixture was stirred at 20 °C for 1 h. The solvent was evaporated and the residue purified by column chromatography, eluting with a gradient (0-5%) of MeOH/DCM, to give benzamide 107 (200 mg, 77%) as a tan powder: mp (EtOAc) 182-185 °C; ¹H NMR δ 10.36 (s, 1 H, NHCO), 8.92 (d, *J* = 2.2 Hz, 1 H, H-2'), 8.35 (br t, *J* = 4.0 Hz, 1 H, NHSO₂), 8.31 (br dd, *J* = 4.6, 1.2 Hz, 1 H, H-6'), 8.18 (ddd, *J* = 8.3, 2.4, 1.5 Hz, 1 H, H-4'), 7.90 (br d, *J* = 8.3 Hz, 2 H, H-2, H-6), 7.81 (ddd, *J* = 8.6, 1.9, 1.6 Hz, 2 H, H-2", H-6"), 7.68 (ddd, *J* = 8.6, 1.9, 1.6 Hz, 2 H, H-3", H-5"), 7.37-7.42 (m, 3 H, H-3, H-5, H-5'), 4.45 (s, 1 H, CH), 4.11 (br d, *J* = 4.0 Hz, 2 H, CH₂N); ¹³C NMR δ 165.5, 144.4, 142.0, 141.7, 140.7, 135.7, 133.1, 132.4 (2), 127.7 (2), 127.4 (2), 127.3 (2), 126.8, 125.7, 123.5, 83.8, 82.1, 47.7; MS *m*/*z* 392.5 (MH⁺, 100%). Anal. calcd for C₂₁H₁₇N₃O₃S: C, 64.43; H, 4.38; N, 10.73. Found: C, 64.26; H, 4.38; N, 10.43%.

### Example 96 (Reference Example)

### Preparation of 4-[(4-Bromophenylsulfonamido)methyl]-N-(4-pyridinyl)benzamide (109).

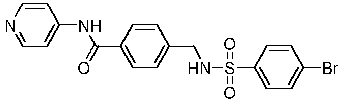

**4-((4-Bromophenylsulfonamido)methyl)benzoic Acid (108). 4-**Bromobenzenesulfonyl chloride (2.36 g, 10.14 mmol) was added to a stirred solution of 4-aminomethylbenzoic acid (1.73 g, 9.22 mmol) in 1 M NaOH (19 mL) at 20 °C. The mixture was stirred at 20 ° for 3 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The residue was purified by chromatography, eluting with a gradient (0-20%) of MeOH/EtOAc, to give benzoic acid **108** (2.44 g, 71%) as a white powder: mp 268-271 °C; ¹H NMR δ 12.82 (br s, 1 H, CO₂H), 8.34 (br t, *J* = 6.1 Hz, 1 H, NHSO₂), 7.85 (br d, *J* = 8.3 Hz, 2 H, H-2, H-6), 7.78 (ddd, *J* = 8.7, 2.2, 2.0 Hz, 2 H, H-2', H-6'), 7.70 (ddd, *J* = 8.7, 2.2, 2.0 Hz, 2 H, H-3', H-5'), 7.34 (br d, *J* = 8.3 Hz, 2 H, H-3, H-5), 4.10 (br d, *J* = 6.0 Hz, 2 H, CH₂N). Anal. calcd for C₁₄H₁₂BrNO₄S·¼CH₃OH: C, 45.23; H, 3.46; N, 3.70. Found: C, 44.95; H, 3.24; N, 3.68%.

**4-[(4-Bromophenylsulfonamido)methyl]-*N*-(4-pyridinyl)benzamide (109). Method** A. Reaction of benzoic acid **108** (920 mg, 2.5 mmol) and oxalyl chloride (0.33 mL, 3.7 mmol) and subsequent reaction with 4-aminopyridine (260 mg, 2.7 mmol), followed by column chromatography, eluting with EtOAc, gave benzamide **109** (146 mg, 13%) as a white powder: mp (EtOAc) 219-222 °C; ¹H NMR δ 10.52 (s, 1 H, NHCO), 8.48 (dd, *J* = 4.9, 1.5 Hz, 2 H, H-2', H-6'), 8.05 (br t, *J* = 6.3 Hz, 1 H, NHSO₂), 7.89 (br d, *J* = 8.3 Hz, 2 H, H-2, H-6), 7.77-7.81 (m, 4 H, H-3', H-5', H-2", H-6"), 7.78 (br ddd, *J* = 8.7, 2.0, 1.0 Hz, 2 H, H-3", H-5"), 7.40 (br d, *J* = 8.3 Hz, 2 H, H-3, H-5), 4.10 (d, *J* = 6.3 Hz, 2 H, CH₂N); ¹³C NMR δ 166.0, 150.1 (2), 146.0, 141.9, 139.9, 132.9, 132.3 (2), 128.5 (2), 127.9 (2), 127.5 (2), 126.2, 114.0 (2), 45.7; MS *m*/*z* 368.4/370.4 (MH⁺, 100%). Anal. calcd for C₁₉H₁₆BrN₃O₃S·¼H₂O: C, 50.12; H, 3.76; N, 9.23. Found: C, 50.12; H, 3.45; N, 8.33%.

### Example 97

### Preparation of 4-[(4-Fluorophenylsulfonamido)methyl]-N-(4-pyridinyl)benzamide (111).

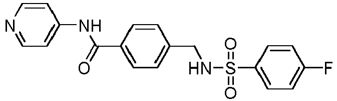

**4-(4-Fluorophenylsulfonamidomethyl)benzoic Acid (110).** 4-Fluorobenzenesulfonyl chloride (1.85 g, 9.49 mmol) was added to a stirred solution of 4-aminomethylbenzoic acid (1.62 g, 8.63 mmol) in 1 M NaOH (17 mL) at 20 °C. The mixture was stirred at 20° for 16 h. The pH of the mixture was adjusted to 2-3 with 6 M HCl and the precipitate filtered. The precipitate was washed with water (2 × 20 mL), ether (20 mL) and pet. ether (2 × 20 mL) and air-dried. The residue was purified by chromatography, eluting with a gradient (0-20%) of MeOH/EtOAc, to give benzoic acid **110** (2.23 g, 84%) as a white powder: mp 231-234 °C; ¹H NMR δ 8.28 (t, *J* = 6.3 Hz, 1 H, NHSO₂), 7.80-7.85 (m, 4 H, H-2, H-6, H-2', H-6'), 7.31-7.40 (m, 4 H, H-3, H-5, H-3', H-5'), 4.07 (d, *J* = 6.3 Hz, 2 H, CH₂N), CO₂H not observed. Anal. calcd for C₁₄H₁₂FNO₄S: C, 54.36; H, 3.91; N, 4.53. Found: C, 54.51; H, 3.81; N, 4.57%.

**4-[(4-Fluorophenylsulfonamido)methyl]-*N*-(4-pyridinyl)benzamide (111). Method A.** Reaction of benzoic acid **110** (840 mg, 2.7 mmol) and oxalyl chloride (0.36 mL, 4.1 mmol) and subsequent reaction with 4-aminopyridine (282 mg, 3.0 mmol), followed by column chromatography, eluting with EtOAc, gave benzamide **111** (406 mg, 39%) as a white powder: mp (EtOAc) 231-233 °C; ¹H NMR δ 10.50 (s, 1 H, NHCO), 8.47 (dd, *J* = 4.8, 1.5 Hz, 2 H, H-2', H-6'), 8.30 (t, *J* = 5.9 Hz, 1 H, NHSO₂), 7.85-7.91 (m, 4 H, H-2, H-6, H-2", H-6"), 7.78 (br ddd, *J* = 4.8, 1.5 Hz, 2 H, H-3', H-5'), 7.39-7.45 (m, 4 H, H-3, H-5, H-3", H-5"), 4.10 (d, *J* = 5.9 Hz, 2 H, CH₂N); ¹³C NMR δ 166.0, 164.1 (d, *J* = 250 Hz), 150.3 (2), 145.9, 142.0, 137.8 (d, *J* = 3 Hz), 133.0, 129.5 (2, d, *J* = 9 Hz), 127.8 (2), 127.5 (2), 116.3 (2, d, *J* = 22 Hz), 114.0 (2), 45.7; MS *m*/*z* 386.5 (MH⁺, 100%). Anal. calcd for C₁₉H₁₆FN₃O₃S: C, 59.21; H, 4.18; N, 10.90. Found: C, 59.34; H, 4.12; N, 10.78%.

### Example 98

### Preparation of 4-{[({4-[3-(Methyloxy)-1-propynyl]phenyl}sulfonyl)amino]methyl}-N-(3-pyridinyl)benzamide (112).

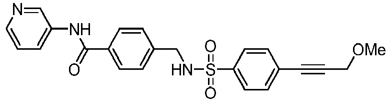

PdCl₂(PPh₃)₂ (29 mg, 42 µmol) was added to a stirred, degassed solution of iodide **106** (208 mg, 0.42 mmol), 3-methoxypropyne (53 µL, 0.63 mmol) and Cul (8 mg, 42 µmol) in Et₃N (2 mL) and DMF (2 mL), and the mixture was stirred in a sealed pressure vessel at 50 °C for 4 h. The mixture was cooled to 20 °C, diluted with EtOAc (150 mL) and washed with water (3 × 50 mL), washed with brine (50 mL) and dried. The solvent was evaporated and the residue purified by column chromatography, eluting with a gradient (0-5%) of MeOH/EtOAc, to give benzamide **112** (157 mg, 86%) as a cream powder: mp (MeOH/EtOAc) 200-201 °C; ¹H NMR δ 10.37 (s, 1 H, NHCO), 9.10 (br s, 1 H, H-2'), 8.50 (br s, 1 H, H-6'), 8.35 (br s, 1 H, NHSO₂), 8.20 (br d, *J* = 8.3 Hz, 1 H, H-4'), 7.59 (d, *J* = 8.3 Hz, 2 H, H-2, H-6), 7.60 (dd, *J* = 8.5, 1.8 Hz, 2 H, H-2", H-6"), 7.65 (dd, *J* = 8.5, 1.8 Hz, 2 H, H-3", H-5"), 7.50 (br s, 1 H, H-5'), 7.40 (d, *J* = 8.3 Hz, 2 H, H-3, H-5), 4.35 (s, 2 H, CH₂O), 4.11 (br s, 2 H, CH₂N), 3.29 (s, 3 H, OCH₃); ¹³C NMR δ 165.5, 146.7, 144.4, 142.1, 141.6, 140.5, 135.8, 133.1, 132.1 (2), 127.7 (2), 127.5 (2), 127.1, 126.8 (2), 125.9, 89.0, 84.5, 59.4, 57.0, 45.7; MS *m*/*z* 436.8 (MH⁺, 100%). Anal. calcd for C₂₃H₂₁N₃O₄S: C, 63.43; H, 4.86; N, 9.65. Found: C, 63.51; H, 4.91; N, 9.54%.

### Example 99 (Reference Example)

### Preparation of 4-[(4-Iodophenylsulfonamido)methyl]-N-methyl-N-(4-pyridinyl)benzamide (113).

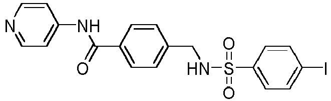

A mixture of 4-(aminomethyl)-*N*-(4-pyridinyl)benzamide **8** (520 mg, 2.3 mmol) and 4-iodobenzenesulfonyl chloride (690 mg, 2.3 mmol) in dry pyridine (20 mL) was stirred at 20 °C for 16 h. The solvent was evaporated and the residue stirred in water (20 mL) for 1 h. The precipitate was filtered, washed with water (5 mL) and dried. The crude solid was purified by column chromatography, eluting with a gradient (0-30%) of MeOH/EtOAc, to give benzamide **113** (906 mg, 80%) as a cream powder: mp (EtOAc) 270-273 °C; ¹H NMR δ 10.50 (s, 1 H, NHCO), 8.48 (br d, *J* = 4.8 Hz, 2 H, H-2', H-6'), 8.34 (br t, *J* = 6.2 Hz, 1 H, NHSO₂), 7.96 (ddd, *J* = 8.6, 2.2, 1.9 Hz, 2 H, H-2", H-6"), 7.89 (br d, *J* = 8.4 Hz, 2 H, H-2, H-6), 7.78 (br dd, *J* = 6.3, 1.4 Hz, 2 H, H-3', H-5'), 7.56 (ddd, *J* = 8.6, 2.2, 1.9 Hz, 2 H, H-3", H-5"), 7.40 (br d, *J* = 8.4 Hz, 2 H, H-3, H-5), 4.10 (d, *J* = 6.2 Hz, 2 H, CH₂N); ¹³C NMR δ 166.0, 150.3 (2), 145.9, 141.9, 140.2, 138.1 (2), 136.4, 133.0, 128.2 (2), 127.8 (2), 127.5 (2), 114.0, 100.3, 45.7; MS *m*/*z* 494.6 (MH⁺, 100%). Anal. calcd for C₁₉H₁₆IN₃O₃S: C, 46.26; H, 3.27; N, 8.52. Found: C, 46.49; H, 3.24; N, 8.46%.

### Example 100

### Preparation of 4-[({[4-(21-Amino-4,7,10,13,16,19-hexaoxahenicos-1-yn-1-yl)phenyl]sulfonyl}amino)methyl]-N-(3-pyridinyl)benzamide (116).

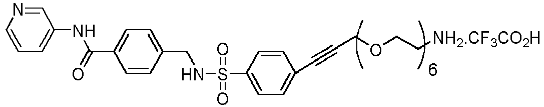

**tert-Butyl 3,6,9,12,15,18-Hexaoxahenicos-20-yn-1-ylcarbamate (114). Mesyl chloride** (1.78 mL, 23.0 mmol) was added dropwise to a stirred suspension of hexaethylene glycol (5.42 g, 19.2 mmol) and Ag₂O (4.67 g, 20.2 mmol) in dry DCM (50 mL) at 20 °C and the mixture was stirred at 20 °C for 3 days. The mixture was filtered through Celite^{®} and the solvent evaporated. The residue was purified by column chromatography, eluting with a gradient (0-10%) of MeOH/EtOAc, to give 17-hydroxy-3,6,9,12,15-pentaoxaheptadec-1-yl methanesulfonate (3.52 g, 51%) as a colourless oil: ¹H NMR (CDCl₃) δ 4.36-4.40 (m, 2 H, CH₂OSO₂), 3.76-3.78 (m, 2 H, CH₂O), 3.70-3.74 (m, 2 H, CH₂O), 3.64-3.67 (m, 16 H, 8 × CH₂O), 3.59-3.62 (m, 2 H, CH₂O), 3.09 (s, 3 H, SO₂CH₃), 2.80 (br s, 1 H, OH); MS *m*/*z* 361.6 (MH⁺, 100%). A mixture of the mesylate (3.52 g, 9.8 mmol) and NaN₃ (1.27 g, 19.5 mmol) in dry DMF (20 mL) was stirred at 110 °C for 2 h. The mixture was cooled to 20 °C and the solvent evaporated. The residue was purified by column chromatography, eluting with 10% MeOH/EtOAc, to give 17-azido-3,6,9,12,15-pentaoxaheptadecan-1-ol (2.98 g, 99%) as a colourless oil: ¹H NMR (CDCl₃) δ 3.71-3.74 (m, 2 H, CH₂O), 3.65-3.69 (m, 18 H, 9 × CH₂O), 3.59-3.62 (m, 2 H, CH₂O), 3.39 (br t, *J* = 5.2 Hz, 2 H, CH₂N₃), 2.82 (br s, 1 H, OH); MS *m*/*z* 308.5 (MH⁺, 100%). A mixture of azide (2.98 g, 9.7 mmol) and Pd/C (100 mg) in EtOH (50 mL) was stirred under H₂ (60 psi) for 1 h. The mixture was filtered through Celite^{®} and washed with EtOH (3 × 20 mL) and the solvent was evaporated. The crude residue was dissolved in DCM (50 mL) and di-*tert*-butyl dicarbonate (2.56 g, 11.7 mmol) in DCM (20 mL) was added dropwise and the solution was stirred at 20 °C for 16 h. The solvent was evaporated and residue was purified by column chromatography, eluting with 10% MeOH/EtOAc, to give *tert-*butyl 17-hydroxy-3,6,9,12,15-pentaoxaheptadec-1-ylcarbamate (2.97 g, 80%) as a colourless oil: ¹H NMR (CDCl₃) δ 5.17 (br s, 1 H, NHCO₂), 3.70-3.74 (m, 2 H, CH₂O), 3.60-3.68 (m, 18 H, 9 × CH₂O), 3.54 (br t, *J* = 5.1 Hz, 2 H, CH₂O), 3.31 (br q, *J* = 5.1 Hz, 2 H, CH₂N), 2.81 (br s, 1 H, OH), 1.44 [s, 9 H, C(CH₃)₃]; MS *m*/*z* 382.5 (MH⁺, 100%). NaH (343 mg, 8.56 mmol) was added in small portions to a stirred solution of alcohol (2.97 g, 7.8 mmol) in THF (50 mL) at 0 °C and the resulting mixture stirred at 0 °C for 30 min. Propargyl bromide (0.87 mL, 7.8 mmol) was added followed by tetrabutylammonium iodide (29 mg, 78 µmol) and the mixture was stirred at 20 °C for 16 h. The reaction was quenched with sat. aq. NH₄Cl and extracted with EtOAc (4 × 50 mL). The combined organic fraction was washed with brine (50 mL), dried and the solvent evaporated. The residue was purified by column chromatography, eluting with 80% EtOAc/pet. ether, to give the acetylene **114** (2.56 g, 79%) as a colourless oil: ¹H NMR (CDCl₃) δ 5.05 (br s, 1 H, NHCO₂), 4.20 (d, *J* = 2.4 Hz, 2 H, CH₂C≡C), 3.68-3.71 (m, 4 H, 2 × CH₂O), 3.64-3.67 (m, 12 H, 6 × CH₂O), 3.60-3.63 (m, 4 H, 2 × CH₂O), 3.54 (br t, *J* = 5.2 Hz, 2 H, CH₂O), 3.31 (br q, *J* = 5.2 Hz, 2 H, CH₂N), 2.42 (t, *J* = 2.4 Hz, 1 H, CH), 1.44 [s, 9 H, C(CH₃)₃]; MS *m*/*z* 420.7 (MH⁺, 100%); HRMS calcd for C₂₀H₃₈N₄O₈S (MH⁺) *m*/*z* 420.2592, found 420.2590 (0.4 ppm).

***tert*-Butyl 21-{4-[({4-[(3-Pyridinylamino)carbonyl]benzyl}amino)sulfonyl]phenyl}-3,6,9,12,15,18-hexaoxahenicos-20-yn-1-ylcarbamate (115).** PdCl₂(PPh₃)₂ (36 mg, 51 µmol) was added to a stirred, degassed solution of iodide **106** (250 mg, 510 µmol), acetylene **114** (320 mg, 770 µmol) and Cul (10 mg, 51 µmol) in Et₃N (3 mL) and DMF (3 mL), and the mixture was stirred in a sealed pressure vessel at 50 °C for 3 h. The mixture was cooled to 20 °C, diluted with EtOAc (150 mL) and washed with water (3 x 50 mL), washed with brine (50 mL) and dried. The solvent was evaporated and the residue purified by column chromatography, eluting with a gradient (0-5%) of MeOH/EtOAc, to give carbamate **115** (367 mg, 92%) as a tan oil: ¹H NMR (CDCl₃) δ 8.61 (br s, 1 H, H-2'), 8.41 (br s, 1 H, NHSO₂), 8.31 (d, *J* = 4.6 Hz, 1 H, H-6'), 8.25 (ddd, *J* = 8.3, 2.4, 1.4 Hz, 1 H, H-4'), 7.79-7.84 (m, 4 H, H-2, H-6, H-2", H-6"), 7.55 (dd, *J* = 8.6, 1.8 Hz, 2 H, H-3", H-5"), 7.28-7.34 (m, 3 H, H-3, H-5, H-5'), 5.78 (br s, 1 H, NHCO), 5.07 (br s, 1 H, NHCO₂), 4.42 (s, 2 H, CH₂O), 4.22 (br d, *J* = 6.0 Hz, 2 H, CH₂N), 3.74-3.77 (m, 2 H CH₂O), 3.68-3.71 (m, 2 H CH₂O), 3.55-3.66 (m, 18 H, 9 × CH₂O), 3.25 (br dd, *J* = 5.4, 5.2 Hz, 2 H, CH₂N), 1.43 [s, 9 H, C(CH₃)₃]; MS *m*/*z* 786.0 (MH⁺, 100%); HRMS calcd for C₃₉H₅₃N₄O₁₁S (MH⁺) *m*/*z* 785.3426, found 785.3410 (2.5 ppm).

**4-[({[4-(21-Amino-4,7,10,13,16,19-hexaoxahenicos-1-yn-1-yl)phenyl]sulfonyl}amino)methyl]-*N*-(3-pyridinyl)benzamide (116).** A solution of carbamate **115** (360 mg, 0.46 mmol) in HCl saturated MeOH (10 mL) was stood at 20 °C for 16 h. The solvent was evaporated and the crude oil purified by preparative HPLC [gradient elution 5-55% of (90%MeCN/H₂O)/(0.02% v/v aqueous CF₃CO₂H)] to give the amine as the trifluoracetate salt (270 mg, 73%) as a brown gum: ¹H NMR δ 11.34 (s, 1 H, NHCO), 9.42 (d, *J* = 2.2 Hz, 1 H, H-2'), 8.88 (d, *J* = 8.8 Hz, 1 H, H-4'), 8.64 (d, *J* = 4.9 Hz, 1 H, H-4'), 8.47 (br s, 1 H, NHSO₂), 8.04 (br d, *J* = 8.3 Hz, 2 H, H-2, H-6), 7.96 (m 4 H, NH₂·CF₃CO₂H, H-5'), 7.82 (dd, *J* = 8.5, 1.8 Hz, 2 H, H-2", H-6"), 7.65 (dd, *J* = 8.5, 1.8 Hz, 2 H, H-3", H-5"), 7.44 (br d, *J* = 8.3 Hz, 2 H, H-3, H-5), 4.43 (s, 2 H, CH₂O), 4.12 (br d, *J* = 6.2 Hz, 2 H, CH₂N), 3.51-3.65 (m, 22 H, 11 × CH₂O), 2.95 (br q, *J* = 5.5 Hz, 2 H, CH₂N); ¹³C NMR δ 165.9, 142.5, 140.5, 138.6, 137.0, 134.7, 133.5, 132.0 (2), 131.9, 129.1, 128.1 (2), 127.7 (2), 127.5 (2), 127.1, 126.8 (2), 125.8, 89.2, 84.3, 69.7 (3), 69.6 (2), 69.5, 68.8, 66.6, 58.0, 48.5, 45.7, 38.8; HRMS calcd for C₃₄H₄₅N₄O₉S (MH⁺) *m*/*z* 685.2902, found 685.2898 (1.2 ppm).

### Example 101

### Preparation of 4-[({[4-(3-Methoxypropyl)phenyl]sulfonyl}amino)methyl]-N-(3-pyridinyl)benzamide (117).

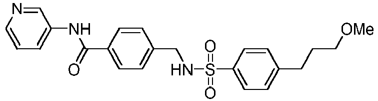

A mixture of acetylene **98** (103 mg, 0.24 mmol) and Pd/C (30 mg) in EtOH (50 mL) was stirred under H₂ (60 psi) for 1 h. The mixture was filtered through Celite, washed with EtOH. The solvent was evaporated and the crude solid was crystallized to give methyl ether **117** (62 mg, 59%) as a cream powder: mp (MeOH/EtOAc) 172-174 °C; ¹H NMR δ 10.36 (s, 1 H, NHCO), 8.92 (d, *J* = 2.2 Hz, 1 H, H-2'), 8.31 (br dd, *J* = 4.5, 1.5 Hz, 1 H, H-6'), 8.15-8.21 (m, 2 H, H-4', NHSO₂), 7.89 (br d, *J* = 8.2 Hz, 2 H, H-2, H-6), 7.71 (br d, *J* = 8.2 Hz, 2 H, H-2", H-6"), 7.35-7.42 (m, 5 H, H-3, H-5, H-5', H-3", H-5"), 4.09 (s, 2 H, CH₂N), 3.31 (t, *J* = 6.3 Hz, 2 H, CH₂O), 3.22 (s, 3 H, OCH₃), 2.65-2.70 (m, 2 H, CH₂), 1.76-1.83 (m, 2 H, CH₂); ¹³C NMR δ 165.5, 146.7, 144.5, 142.0, 141.8, 138.2, 135.8, 133.0, 129.0 (2), 127.6 (2), 127.4 (2), 127.3, 126.6 (2), 123.4, 70.9, 57.8, 45.7, 31.5, 30.4; MS *m*/*z* 440.7 (MH⁺, 100%). Anal. calcd for C₂₃H₂₅N₃O₄S·H₂O: C, 60.38; H, 5.95; N, 9.18. Found: C, 60.13; H, 5.60; N, 9.16%.

### Example 102

### Preparation of 4-[({[4-(1-benzyl-1H-1,2,3-triazol-4-yl)phenyl]sulfonyl}amino)methyl]-N-(3-pyridinyl)benzamide (118).

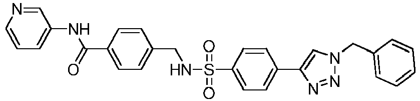

TBTA (28 mg, 53 µmol) was added to a suspension of CuSO₄·H₂O (13 mg, 53 µmol), sodium ascorbate (21 mg, 106 µmol), acetylene **107** (208 mg, 0.53 mmol) and benzyl azide (0.14 mL, 1.06 mmol) in water (1 mL) and DMSO (1 mL). The mixture was stirred at 20 °C for 24 h. The mixture was diluted with water (50 mL) and stirred at 20 °C for 30 min. The mixture was filtered, washed with water (3 × 5 mL) and dried. The precipitate was suspended in MeOH/EtOAc (1:1, 10 mL), filtered and washed with EtOAc to give benzamide **118** (240 mg, 86%) as a grey powder: mp (MeOH/EtOAc) 256-260 °C; ¹H NMR δ 10.37 (s, 1 H, NHCO), 8.79 (s, 1 H, H-2'), 8.25-8.30 (m, 2 H, H-6', NHSO₂), 8.20 (br d, *J* = 8.2 Hz, 1 H, H-4'), 8.06 (d, *J* = 8.4 Hz, 2 H, H-3", H-5"), 7.85-7.94 (m, 4 H, H-2, H-6, H-2", H-6"), 7.57 (br s, 1 H, H-5"'), 7.33-7.46 (m, 8 H, H-3, H-5, H-5', H-2"', H-3"', H-4"', H-5"', H-6"'), 5.67 (s, 2 H, CH₂N), 4.11 (br d, *J* = 6.1 Hz, 2 H, CH₂N); ¹³C NMR δ 165.5, 145.3, 144.3, 142.2, 141.8, 139.5, 135.8, 134.4, 133.1, 128.8 (2), 128.7, 128.2, 127.9 (2), 127.7 (2), 127.4 (2), 127.3 (2), 126.9, 125.6 (2), 122.8, 53.1, 47.7 (1 C not observed); MS *m*/*z* 525.6 (MH⁺, 100%). Anal. calcd for C₂₈H₂₄N₆O₃S·H₂O: C, 61.98; H, 4.83; N, 15.48. Found: C, 61.60; H, 4.33; N, 15.41%.

### Example 103

### Preparation of 4-[({[4-(3-Hydroxy-1-propynyl)phenyl]sulfonyl}amino)methyl]-N-(3-pyridinyl)benzamide (119).

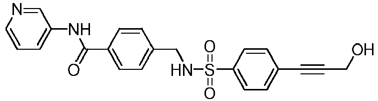

PdCl₂(PPh₃)₂ (105 mg, 149 µmol) was added to a stirred, degassed solution of iodide **106** (734 mg, 1.49 mmol), propargyl alcohol (132 µL, 2.23 mmol) and Cul (28 mg, 149 µmol) in Et₃N (3 mL) and DMF (3 mL), and the mixture was stirred in a sealed pressure vessel at 50 °C for 4 h. The mixture was cooled to 20 °C, diluted with EtOAc (150 mL) and washed with water (3 × 50 mL), washed with brine (50 mL) and dried. The solvent was evaporated and the residue purified by column chromatography, eluting with a gradient (0-5%) of MeOH/EtOAc, to give benzamide **119** (460 mg, 73%) as a cream powder: mp (MeOH/EtOAc) 207-210 °C; ¹H NMR δ 10.36 (s, 1 H, NHCO), 8.92 (d, *J* = 2.4 Hz, 1 H, H-2'), 8.34 (br s, 1 H, NHSO₂), 8.31 (dd, *J* = 4.6, 1.5 Hz, 1 H, H-6'), 8.18 (ddd, *J* = 8.4, 2.5, 1.5 Hz, 1 H, H-4'), 7.91 (d, *J* = 8.3 Hz, 2 H, H-2, H-6), 7.80 (dd, *J* = 8.5, 1.9 Hz, 2 H, H-2", H-6"), 7.62 (dd, *J* = 8.5, 1.9 Hz, 2 H, H-3", H-5"), 7.36-7.42 (m, 3 H, H-3, H-5, H-5'), 5.38 (br t, *J* = 5.9 Hz, 1 H, OH), 4.35 (br d, *J* = 5.8 Hz, 2 H, CH₂O), 4.11 (br s, 2 H, CH₂N); ¹³C NMR δ 165.5, 144.5, 142.0, 141.7, 140.2, 135.8, 133.1, 131.9 (2), 127.7 (2), 127.4 (2), 127.3, 126.8 (2), 126.4, 123.1, 93.0, 82.4, 49.4, 45.7; MS *m*/*z* 422.5 (MH⁺, 100%). Anal. calcd for C₂₂H₁₉N₃O₄S: C, 62.69; H, 4.54; N, 9.97. Found: C, 62.99; H, 4.38; N, 10.07%.

### Example 104

### Preparation of 4-[({[4-(3-Hydroxypropyl)phenyl]sulfonyl}amino)methyl]-N-(3-pyridinyl)benzamide (120).

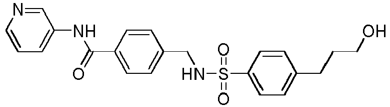

A mixture of acetylene **119** (405 mg, 0.96 mmol) and Pd/C (60 mg) in EtOH (150 mL) was stirred under H₂ (60 psi) for 1 h. The mixture was filtered through Celite, washed with EtOH. The solvent was evaporated and the crude solid was crystallized to give methyl ether 120 (295 mg, 72%) as a cream powder: mp (MeOH/EtOAc) 177-179 °C; ¹H NMR δ 10.36 (s, 1 H, NHCO), 8.92 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 8.14 (br s, 1 H, NHSO₂), 7.90 (br d, *J* = 8.3 Hz, 2 H, H-2, H-6), 7.72 (br d, *J* = 8.3 Hz, 2 H, H-2", H-6"), 7.37-7.42 (m, 5 H, H-3, H-5, H-5', H-3", H-5"), 4.48 (br s, 1 H, OH), 4.08 (s, 2 H, CH₂N), 3.38-3.41 (m, 2 H, CH₂O), 2.69 (dd, *J* = 7.9, 7.6 Hz, 2 H, CH₂), 1.68-1.77 (m, 2 H, CH₂); ¹³C NMR δ 165.5, 147.2, 144.5, 142.0, 141.9, 138.0, 135.8, 133.0, 129.0 (2), 127.6 (2), 127.4 (2), 127.3, 126.5 (2), 123.5, 59.9, 45.7, 33.8, 31.4; MS *m*/*z* 426.6 (MH⁺, 100%). Anal. calcd for C₂₂H₂₃N₃O₄S: C, 62.10; H, 5.45; N, 9.88. Found: C, 62.27; H, 5.41; N, 9.94%.

### Example 105 (Reference Example)

### Preparation of 4-[({[4-(21-Amino-4,7,10,13,16,19-hexaoxahenicos-1-yn-1-yl)phenyl]sulfonyl}amino)methyl]-N-(4-pyridinyl)benzamide (122).

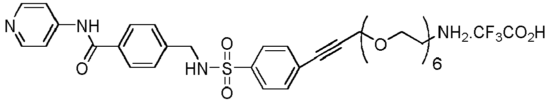

***tert*-Butyl 21-{4-[({4-[(4-Pyridinylamino)carbonyl]benzyl}amino)sulfonyl]phenyl}-3,6,9,12,15,18-hexaoxahenicos-20-yn-1-ylcarbamate (121).** PdCl₂(PPh₃)₂ (40 mg, 57 □mol) was added to a stirred, degassed solution of iodide **113** (284 mg, 570 □mol), acetylene **114** (362 mg, 863 □mol) and CuI (11 mg, 57 □mol) in Et₃N (3 mL) and DMF (3 mL), and the mixture was stirred in a sealed pressure vessel at 50 °C for 3 h. The mixture was cooled to 20 °C, diluted with EtOAc (150 mL) and washed with water (3 × 50 mL), washed with brine (50 mL) and dried. The solvent was evaporated and the residue purified by column chromatography, eluting with a gradient (0-10%) of MeOH/EtOAc, to give carbamate **121** (427 mg, 95%) as a tan oil: ¹H NMR (CDCl₃) δ 8.52 (dd, *J* = 6.3, 1.5 Hz, 2 H, H-2', H-6'), 8.42 (br s, 1 H, NHSO₂), 7.78-7.84 (m, 4 H, H-2, H-6, H-2", H-6"), 7.64 (dd, *J* = 6.3, 1.5 Hz, 2 H, H-3', H-5'), 7.54 (ddd, *J* = 8.6, 1.9, 1.6 Hz, 2 H, H-3", H-5"), 7.33 (br d, *J* = 8.3 Hz, 2 H, H-3, H-5), 5.45 (br s, 1 H, NHCO), 5.06 (br s, 1 H, NHCO₂), 4.42 (s, 2 H, CH₂O), 4.23 (d, *J* = 6.2 Hz, 2 H, CH₂N), 3.74-3.77 (m, 2 H CH₂O), 3.68-3.71 (m, 2 H CH₂O), 3.55-3.65 (m, 18 H, 9 × CH₂O), 3.24 (br d, *J* = 5.3 Hz, 2 H, CH₂N), 1.43 [s, 9 H, C(CH₃)₃]; MS *m*/*z* 786.0 (MH⁺, 100%); HRMS calcd for C₃₉H₅₃N₄O₁₁S (MH⁺) *m*/*z* 785.3426, found 785.3436 (-1.0 ppm).

**4-[({[4-(21-Amino-4,7,10,13,16,19-hexaoxahenicos-1-yn-1-yl)phenyl]sulfonyl}amino)methyl]-*N*-(4-pyridinyl)benzamide (122).** A solution of carbamate **121** (410 mg, 0.52 mmol) in HCl saturated MeOH (10 mL) was stood at 20 °C for 16 h. The solvent was evaporated and the crude oil was purified by preparative HPLC [gradient elution 5-55% of (90%MeCN/H₂O)/(0.02% v/v aqueous CF₃CO₂H)] to give the amine **122** as the trifluoracetate salt (217 mg, 52%) as a tan gum: ¹H NMR δ 11.42 (s, 1 H, NHCO), 8.76 (d, *J* = 7.1 Hz, 2 H, H-2', H-6'), 8.41 (br t, *J* = 6.3 Hz, 1 H, NHSO₂), 8.28 (d, *J* = 7.1 Hz, 2 H, H-3', H-5'), 7.97 (d, *J* = 8.4 Hz, 2 H, H-2, H-6), 7.81 (dd, *J* = 8.5, 1.8 Hz, 2 H, H-2", H-6"), 7.77 (br s, 3 H, NH₃⁺CF₃CO₂⁻), 7.65 (dd, *J* = 8.5, 1.8 Hz, 2 H, H-3", H-5"), 7.46 (d, *J* = 8.4 Hz, 2 H, H-3, H-5), 4.43 (s, 2 H, CH₂O), 4.13 (d, *J* = 6.3 Hz, 2 H, CH₂N), 3.63-3.66 (m, 2 H CH₂O), 3.51-3.61 (m, 20 H, 10 × CH₂O), 2.95-3.02 (m, 2 H, CH₂N); ¹³C NMR δ 166.8, 158.3, 152.8, 143.1, 153.0 (2), 140.4, 132.1 (2), 131.9, 128.3 (2), 127.6 (2), 126.8 (2), 125.9, 115.1 (2), 89.2, 84.3, 69.7 (br, 8), 69.6 (2), 69.5, 68.8, 66.6, 58.0, 45.6; MS *m*/*z* 685.2 (MH⁺, 100%); HRMS calcd for C₃₄H₄₅N₄O₉S (MH⁺) *m*/*z* 685.2902, found 685.2893 (1.9 ppm).

### Example 106 (Reference Example)

### Preparation of 4-((4-tert-Butylphenylsulfonamido)methyl)-N-(pyridin-4-yl)benzamide (123).

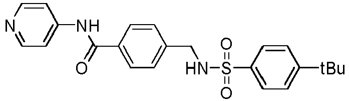

A mixture of 4-(aminomethyl)-*N*-(4-pyridinyl)benzamide dihydrobromide **8** (399 mg, 1.03 mmol) and 4-*tert*-butylbenzenesulfonyl chloride (263 mg, 1.13 mmol) in dry pyridine (10 mL) was stirred at 20 °C for 16 h. The solvent was evaporated and the residue stirred in water (20 mL) for 1 h. The precipitate was filtered, washed with water (5 mL) and dried. The crude solid was purified by column chromatography, eluting with a gradient (0-10%) of MeOH/EtOAc, to give benzamide **123** (189 mg, 43%) as a white powder: mp (MeOH/EtOAc) 261-263 °C; ¹H NMR δ 10.48 (s, 1 H, NHCO), 8.47 (dd, *J* = 4.8, 1.5 Hz, 2 H, H-2', H-6'), 8.19 (br t, *J* = 6.3 Hz, 1 H, NHSO₂), 7.86 (br d, *J* = 8.3 Hz, 2 H, H-2, H-6), 7.77 (dd, *J* = 4.8, 1.5 Hz, 2 H, H-3', H-5'), 7.70 (ddd, *J* = 8.6, 2.2, 2.0 Hz, 2 H, H-2", H-6"), 7.54 (ddd, *J* = 8.6, 2.2, 2.0 Hz, 2 H, H-3", H-5"), 7.38 (d, *J* = 8.3 Hz, 2 H, H-3, H-5), 4.09 (br d, *J* = 6.3 Hz, 2 H, CH₂N), 1.28 [s, 9 H, C(CH₃)₃]; ¹³C NMR δ 165.9, 155.2, 150.1 (2), 145.8, 142.0, 137.8, 132.7, 127.6 (2), 127.4 (2), 126.2 (2), 125.8 (2), 113.9 (2), 45.6, 34.6, 30.6 (3); MS *m*/*z* 424.5 (MH⁺, 100%). Anal. calcd for C₂₃H₂₅N₃O₃S: C, 65.23; H, 5.95; N, 9.92. Found: C, 65.29; H, 6.05; N, 9.88%.

### Example 107

### Preparation of 4-({[(4-tert-Butylphenyl)sulfonyl]amino}methyl)-N-(5-methyl-3-pyridinyl)benzamide (125).

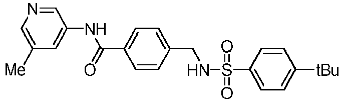

**4-({[(4-*tert*-Butylphenyl)sulfonyl]amino}methyl)benzoic Acid (124).** (4-*tert*-Butylphenyl)sulfonyl chloride 4.85 g, 20.84 mmol) was added in small portions to a stirred solution of 4-aminomethylbenzoic acid (3.0 g, 19.85 mmol) in 2 M NaOH solution (20 mL) and the mixture was stirred at 20 °C for 16 h. The pH was adjusted to 2 with 6 M HCl and the resulting precipitate was filtered, washed with water (2 × 10 mL), dried, washed with pet. ether (2 × 10 mL) and the material dried to give acid **124** (4.88 g, 71%) as a white powder: mp (MeOH) 290-291 °C; ¹H NMR δ 12.84 (br s, 1 H, CO₂H), 8.19 (t, *J* = 6.4 Hz, 1 H, NHSO₂), 7.79 (br d, *J* = 8.3 Hz, 2 H, H-2, H-6), 7.66 (ddd, *J* = 8.8, 2.2, 1.9 Hz, 2 H, H-2', H-6'), 7.52 (ddd, *J* = 8.8, 2.2, 1.9 Hz, 2 H, H-3', H-5'), 7.31 (br d, *J* = 8.3 Hz, 2 H, H-3, H-5), 4.07 (d, *J* = 6.4 Hz, 2 H, CH₂N). Anal. calcd for C₁₈H₂₁NO₄S: C, 62.23; H, 6.09; N, 4.03. Found: C, 62.32; H, 6.14; N, 4.06%.

**4-({[(4-*tert*-Butylphenyl)sulfonyl]amino}methyl)-*N*-(5-methyl-3-pyridinyl)benzamide (125).** Oxalyl chloride (94 µL, 1.1 mmol) was added dropwise to a stirred suspension of benzoic acid **124** (250 mg, 0.7 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 2-Methyl-3-pyridinylamine (86 mg, 0.8 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **125** (156 mg, 50%) as a white powder: mp (EtOAc) 189-191 °C; ¹H NMR δ 10.27 (s, 1 H, CONH), 8.71 (d, *J* = 2.3 Hz, 1 H, H-2'), 8.18 (br s, 1 H, NHSO₂), 8.16 (d, *J* = 1.2 Hz, 1 H, H-6'), 8.00-8.04 (m, 1 H, H-4'), 7.86 (br d, *J* = 8.4 Hz, 2 H, H-2, H-6), 7.70-7.74 (ddd, *J* = 8.6, 2.2, 1.9 Hz, 2 H, H-2", H-6"), 7.55 (ddd, *J* = 8.6, 2.2, 1.9 Hz, 2 H, H-3", H-5"), 7.37 (br d, *J* = 8.4 Hz, 2 H, H-3, H-5), 4.09 (br s, 2 H, CH₂N), 2.31 (s, 3 H, CH₃), 1.29 [s, 9 H, C(CH₃)₃]; ¹³C NMR δ 165.4, 155.3, 144.9, 141.7, 139.3, 137.9, 135.4, 133.0, 132.7, 127.7, 127.6 (2), 127.4 (2), 126.3 (2), 125.9 (2), 45.8, 34.8, 30.7 (3), 17.9; MS *m*/*z* 438.6 (MH⁺, 100%). Anal. calcd for C₂₄H₂₇N₃O₃S: C, 65.88; H, 6.22; N, 9.60. Found: C, 65.72; H, 6.37; N, 9.58%.

### Example 108

### Preparation of 4-({[(4-tert-Butylphenyl)sulfonyl]amino}methyl)-N-(2-methyl-3-pyridinyl)benzamide (126).

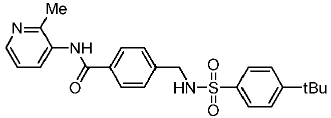

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 2-Methyl-3-pyridinylamine was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **126** (266 mg, 60%) as a white powder: mp (EtOAc) 188-190 °C; ¹H NMR δ 9.95 (s, 1 H, CONH), 8.34 (dd, *J* = 4.8, 1.6 Hz, 1 H, H-2'), 8.19 (br t, *J* = 6.4 Hz, 1 H, NHSO₂), 7.87 (br d, *J* = 8.3 Hz, 2 H, H-2, H-6), 7.68-7.74 (m, 3 H, H-4', H-2", H-6"), 7.56 (ddd, *J* = 8.6, 2.2, 2.0 Hz, 2 H, H-3", H-5"), 7.36 (br d, *J* = 8.4 Hz, 2 H, H-3, H-5), 7.27 (dd, *J* = 8.0, 4.8 Hz, 1 H, H-5'), 4.09 (d, *J* = 6.4 Hz, 2 H, CH₂N), 2.43 (s, 3 H, CH₃), 1.30 [s, 9 H, C(CH₃)₃]; ¹³C NMR δ 165.1, 155.3, 153.9, 146.1, 141.7, 137.9, 133.8, 132.7, 132.4, 127.5 (2), 127.4 (2), 126.3 (2), 125.9 (2), 121.4, 45.8, 34.8, 30.8 (3), 21.0; MS *m*/*z* 438.6 (MH⁺, 100%). Anal. calcd for C₂₄H₂₇N₃O₃S: C, 65.88; H, 6.22; N, 9.60. Found: C, 66.13; H, 6.40; N, 9.41%.

### Example 109

### Preparation of 4-({[(4-tert-Butylphenyl)sulfonyl]amino}methyl)-N-(6-methyl-3-pyridinyl)benzamide (127).

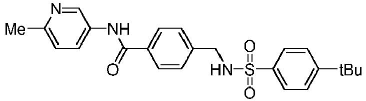

Oxalyl chloride (132 µL, 1.5 mmol) was added dropwise to a stirred suspension of benzoic acid **124** (348 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-Methyl-3-pyridinylamine (120 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **127** (322 mg, 74%) as a white powder: mp (EtOAc) 235-237 °C; ¹H NMR δ 10.25 (s, 1 H, CONH), 8.77 (d, *J* = 2.5 Hz, 1 H, H-2'), 8.19 (br t, *J* = 6.3 Hz, 1 H, NHSO₂), 8.04 (dd, *J* = 8.4, 2.5 Hz, 1 H, H-4'), 7.85 (br d, *J* = 8.3 Hz, 2 H, H-2, H-6), 7.70 (ddd, *J* = 8.6, 2.2, 1.9 Hz, 2 H, H-2", H-6"), 7.55 (ddd, *J* = 8.6, 2.2, 1.9 Hz, 2 H, H-3", H-5"), 7.37 (br d, *J* = 8.3 Hz, 2 H, H-3, H-5), 7.23 (d, *J* = 8.4 Hz, 1 H, H-5'), 4.09 (s, 2 H, CH₂N), 2.44 (s, 3 H, CH₃), 1.28 [s, 9 H, C(CH₃)₃]; ¹³C NMR δ 165.2, 155.2, 152.7, 141.6, 141.3, 137.8, 133.2, 133.0, 127.9, 127.5 (2), 127.4 (2), 126.3 (2), 125.8 (2), 122.5, 45.7, 34.7, 30.7 (3), 23.3; MS *m*/*z* 438.6 (MH⁺, 100%). Anal. calcd for C₂₄H₂₇N₃O₃S: C, 65.88; H, 6.22; N, 9.60. Found: C, 65.72; H, 6.23; N, 9.62%.

### Example 110

### Preparation of 4-({[(4-tert-Butylphenyl)sulfonyl]amino}methyl)-N-(6-methoxy-3-pyridinyl)benzamide (128).

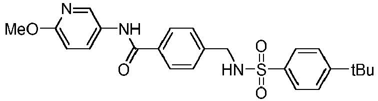

Triethylamine (0.4 mL, 3 mmol) was added dropwise to a stirred solution of benzoic acid **124** (250 mg, 0.72 mmol), EDCI (154 mg, 0.8 mmol), HOBt (108 mg, 0.8 mmol) and 6-methoxypyridin-3-amine (134 mg, 1.1 mmol) in anhydrous DCM (10 mL), and the reaction mixture was stirred at 20 ºC for 4 d. The solution was diluted with DCM (100 mL), washed with H₂O (2 × 50 mL), and washed with brine (50 mL). The combined organic phase was dried, filtered and the solvent evaporated. The residue was purified by column chromatography, eluting with 4% MeOH/DCM, to give benzamide **128** (71 mg, 22%) as a pink powder: mp (MeOH/DCM) 238-240 ºC; ¹H NMR δ 10.18 (s, 1 H, CONH), 8.49 (d, *J* = 2.5 Hz, 1 H, H-2'), 8.18 (t, *J* = 6.3 Hz, 1 H, NHSO₂), 8.02 (dd, *J* = 8.9, 2.7 Hz, 1 H, H-4'), 7.84 (br d, *J=* 8.3 Hz, 2 H, H-2, H-6), 7.69 (br d, *J =* 8.6 Hz, 2 H, H-2", H-6"), 7.55 (br d, *J =* 8.6 Hz, 2 H, H-3", H-5"), 7.36 (br d, *J* = 8.3 H, 2 H, H-3, H-5), 6.84 (d, *J* = 8.9 Hz, 1 H, H-5'), 4.08 (d, *J* = 6.3 Hz, 2 H, C*H*₂NHSO₂), 3.84 (s, 3 H, OCH₃), 1.29 [s, 9 H, C(CH₃)₃]; ¹³C NMR δ 165.0, 159.9, 155.3, 141.5, 138.9, 137.9, 133.0, 132.6, 129.9, 127.5 (2), 127.4 (2), 126.3 (2), 125.9 (2), 109.9, 53.1, 45.8, 34.7, 30.8 (3); MS *m*/*z* 455.3 (MH⁺, 100%). Anal. calcd for C₂₄H₂₇N₃O₄S: C, 63.56; H, 6.00; N, 9.26. Found: C, 63.29; H, 6.00; N, 9.13%.

### Example 111

### Preparation of 4-({[(4-tert-Butylphenyl)sulfonyl]amino}methyl)-N-(6-chloro-3-pyridinyl)benzamide (129).

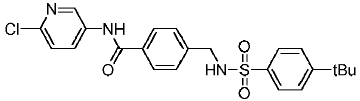

Oxalyl chloride (0.13 mL, 1.44 mmol) was added to a stirred solution of carboxylic acid **124** (250 mg, 0.72 mmol) and a catalytic amount of DMF (2 drops) in anhydrous THF (20 mL) and the solution was heated at reflux temperature for 2 h. The solvent was evaporated and the residue was dried under high vacuum. The residue was dissolved in anhydrous pyridine (10 mL), 6-chloropyridin-3-amine (102 mg, 0.8 mmol) was added, and the reaction mixture was stirred at 20 ºC for 16 h then at 60 ºC for 3 h. The solvent was evaporated and the residue was suspended in cold water (50 mL) and stirred at 0 ºC for 1 h. The solid was filtered, washed with water (20 mL) and dried. The residue was purified by column chromatography, eluting with 50% EtOAc/pet. ether to give benzamide **129** (60 mg, 19%) as a white solid: mp (EtOAc/pet. ether) 251-253 ºC; ¹H NMR δ 10.48 (s, 1 H, CONH), 8.78 (d, *J* = 2.5 Hz, 1 H, H-2'), 8.24 (dd, *J* = 8.7, 2.8 Hz, 1 H, H-4'), 8.19 (br t, *J* = 6.3 Hz, 1 H, NHSO₂), 7.86 (br d, *J =* 8.3 Hz, 2 H, H-2, H-6), 7.69 (dt, *J* = 8.6, 2.0 Hz, 2 H, H-2", H-6"), 7.55 (dt, *J* = 8.6, 2.0 Hz, 2 H, H-3", H-5"), 7.52 (d, *J* = 8.8 Hz, 1 H, H-5'), 7.38 (br d, *J* = 8.3 Hz, 2 H, H-3, H-5), 4.09 (d, *J =* 6.1 Hz, 2 H, C*H*₂NHSO₂), 1.28 [s, 9 H, C(CH₃)₃]; ¹³C NMR δ 165.5, 155.3, 144.0, 142.0, 141.5, 137.9, 135.4, 132.6, 130.9, 127.6 (2), 127.5 (2), 126.3 (2), 125.9 (2), 124.0, 45.8, 34.8, 30.8 (3); MS *m*/*z* 459.2 (MH⁺, 100%). Anal. calcd for C₂₃H₂₄ClN₃O₃S: C, 60.32; H, 5.28; N, 9.18. Found: C, 60.27; H, 5.28; N, 9.10%.

### Example 112

### Preparation of 4-({[(4-tert-Butylphenyl)sulfonyl]amino}methyl)-N-(4-chloro-3-pyridinyl)benzamide (130).

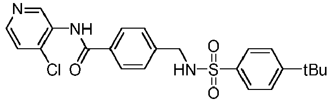

Oxalyl chloride (0.13 mL, 1.44 mmol) was added to a stirred solution of carboxylic acid **124** (250 mg, 0.72 mmol) and a catalytic amount of DMF (2 drops) in anhydrous THF (20 mL) and the solution was heated at reflux temperature for 2 h. The solvent was evaporated and the residue was dried under high vacuum. The residue was dissolved in anhydrous pyridine (10 mL), 4-chloropyridin-3-amine (102 mg, 0.8 mmol) was added, and the reaction mixture was stirred at 60 ºC for 3 h. The solvent was evaporated and the residue was suspended in cold H₂O (50 mL) and stirred at 0 ºC for 1 h. The solid was filtered, washed with H₂O (50 mL) and dried. The residue was purified by column chromatography, eluting with 4% MeOH/DCM, to give benzamide **130** (112 mg, 34%) as a white solid: mp (MeOH/DCM) 125-128 ºC; ¹H NMR δ 10.22 (s, 1 H, CONH), 8.67 (s, 1 H, H-2'), 8.44 (d, *J* = 5.2 Hz, 2 H, H-6'), 8.19 (t, *J =* 6.4 Hz, 1 H, NHSO₂), 7.88 (br d, *J* = 8.3 Hz, 2 H, H-2, H-6), 7.70 (br d, *J* = 8.6 Hz, 2 H, H-2", H-6"), 7.67 (d, *J* = 5.3 Hz, 1 H, H-5'), 7.55 (br d, *J* = 8.6 Hz, 2 H, H-3", H-5"), 7.38 (br d, *J* = 8.3 Hz, 2 H, H-3, H-5), 4.10 (d, *J* = 6.3 Hz, 2 H, C*H*₂NHSO₂), 1.30 [s, 9 H, C(CH₃)₃]; ¹³C NMR (DMSO-d₆) δ 165.3, 155.3, 149.3, 147.9, 142.0, 139.4, 137.9, 132.2, 132.0, 127.7 (2), 127.5 (2), 126.3 (2), 125.9 (2), 124.6, 45.7, 34.8, 30.8 (3); MS *m*/*z* 459.2 (MH⁺, 100%). Anal. calcd for C₂₃H₂₄ClN₃O₃S·¼CH₂Cl₂: C, 58.27; H, 5.15; N, 8.77. Found: C, 58.66; H, 5.20; N, 8.55%.

### Example 113

### Preparation of 4-({[(4-tert-Butylphenyl)sulfonyl]amino}methyl)-N-(2-chloro-3-pyridinyl)benzamide (131).

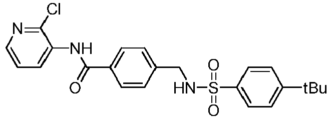

Oxalyl chloride (0.13 mL, 1.44 mmol) was added to a stirred solution of carboxylic acid **124** (250 mg, 0.72 mmol) and a catalytic amount of DMF (2 drops) in anhydrous THF (15 mL) and the solution was heated at reflux temperature for 2 h. The solvent was evaporated and the residue was dried under high vacuum. The residue was dissolved in anhydrous pyridine (10 mL), 2-chloropyridin-3-amine (102 mg, 0.8 mmol) was added, and the reaction mixture was stirred at 20 ºC for 16 h. The pyridine was evaporated and the residue was suspended in cold water (50 mL) and stirred at 0 ºC for 1 h. The solid was filtered, washed with water (10 mL) and dried. The residue was purified by column chromatography, eluting with 40% EtOAc/pet.ether followed by 2% MeOH/DCM, to give benzamide **131** (131 mg, 40%) as a white solid: mp (MeOH/DCM) 161-162 ºC; ¹H NMR δ 10.10 (s, 1 H, CONH), 8.31 (dd, *J* = 4.6, 1.8 Hz, 1 H, H-6'), 8.19 (t, *J =* 6.4 Hz, 1 H, NHSO₂), 8.05 (dd, *J =* 7.9, 1.8 Hz, 1 H, H-4'), 7.88 (br d, *J* = 8.3 Hz, 2 H, H-2, H-6), 7.70 (dt, *J* = 8.6, 2.0 Hz, 2 H, H-2", H-6"), 7.56 (dt, *J* = 8.7, 2.0 Hz, 2 H, H-3", H-5"), 7.50 (dd, *J* = 7.9, 4.7 Hz, 1 H, H-5'), 7.38 (br d, *J =* 8.3 Hz, 2 H, H-3, H-5), 4.10 (d, *J* = 6.3 Hz, 2 H, C*H*₂NHSO₂), 1.29 [s, 9 H, C(CH₃)₃]; ¹³C NMR δ 165.2, 155.3, 146.6, 146.2, 142.1, 137.9, 136.7, 132.2, 132.1, 127.6 (2), 127.5 (2), 126.3 (2), 125.9 (2), 123.4, 45.7, 34.8, 30.8 (3); MS *m*/*z* 459.2 (MH⁺, 100%). Anal. calcd for C₂₃H₂₄ClN₃O₃S: C, 60.32; H, 5.28; N, 9.18. Found: C, 60.46; H, 5.36; N, 8.95%.

### Example 114

### Preparation of 4-({[(4-tert-Butylphenyl)sulfonyl]amino}methyl)-N-(4-methyl-3-pyridinyl)benzamide (132).

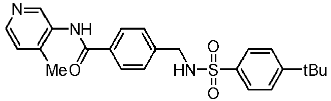

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 5-Methyl-3-pyridinylamine [prepared by reduction of 5-methyl-3-nitropyridine (152 mg, 1.1 mmol) in EtOH (50 mL) under H₂ (60 psi) for 2 h, filtering through Celite and evaporation of the solvent] was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-100%) of EtOAc/pet. ether, to give benzamide **132** (154 mg, 35%) as a white powder: mp (EtOAc) 183-185 °C; ¹H NMR δ 10.00 (s, 1 H, CONH), 8.45 (s, 1 H, H-2'), 8.31 (d, *J* = 4.8 Hz, 1 H, H-6'), 8.19 (br t, *J* = 6.3 Hz, 1 H, NHSO₂), 7.87 (d, *J* = 8.3 Hz, 2 H, H-2, H-6), 7.70 (ddd, *J* = 8.6, 2.2, 1.9 Hz, 2 H, H-2", H-6"), 7.55 (ddd, *J* = 8.6, 2.2, 1.9 Hz, 2 H, H-3", H-5"), 7.37 (br d, *J* = 8.3 Hz, 2 H, H-3, H-5), 7.32 (d, *J* = 4.8 Hz, 1 H, H-5'), 4.10 (d, *J* = 6.3 Hz, 2 H, CH₂N), 2.39 (s, 3 H, CH₃), 1.29 [s, 9 H, C(CH₃)₃]; ¹³C NMR δ 165.3, 155.3, 147.4, 146.7, 142.9, 141.7, 137.9, 133.5, 132.6, 127.6 (2), 127.4 (2), 126.3 (2), 125.9 (2), 125.3, 45.8, 34.8, 30.8 (3), 17.3; MS *m*/*z* 438.7 (MH⁺, 100%). Anal. calcd for C₂₄H₂₇N₃O₃S: C, 65.88; H, 6.22; N, 9.60. Found: C, 65.69; H, 6.35; N, 9.44%.

### Example 115

### Preparation of 4-({[(4-tert-Butylphenyl)sulfonyl]amino}methyl)-N-(5-chloro-3-pyridinyl)benzamide (133).

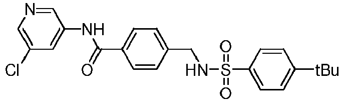

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 5-Chloro-3-pyridinylamine (143 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (30-50%) of EtOAc/pet. ether, to give benzamide **133** (243 mg, 53%) as a white powder: mp (EtOAc) 217-219 °C; ¹H NMR δ 10.98 (s, 1 H, CONH), 8.38 (d, *J* = 5.4 Hz, 1 H, H-6'), 8.29 (d, *J* = 2.0 Hz, 1 H, H-2'), 8.17 (br s, 1 H, NHSO₂), 7.92 (d, *J* = 8.4 Hz, 2 H, H-2, H-6), 7.70 (ddd, *J* = 8.6, 2.2, 1.9 Hz, 2 H, H-2", H-6"), 7.56 (ddd, *J* = 8.6, 2.2, 1.9 Hz, 2 H, H-3", H-5"), 7.35 (br d, *J =* 8.4 Hz, 2 H, H-3, H-5), 7.30 (dd, *J* = 5.4, 2.0 Hz, 1 H, H-4'), 4.08 (br s, 2 H, CH₂N), 1.29 [s, 9 H, C(CH₃)₃]; ¹³C NMR δ 166.0, 155.3, 153.4, 149.3, 143.9, 142.2, 137.9, 132.3, 128.0 (2), 127.3 (2), 126.3 (2), 125.9 (2), 119.7, 114.0, 45.7, 34.8, 30.8 (3); MS *m*/*z* 458.8 (MH⁺, 100%). Anal. calcd for C₂₃H₂₄ClN₃O₃S: C, 60.32; H, 5.28; N, 9.18. Found: C, 60.07; H, 5.28; N, 8.81%.

### Example 116

### Preparation of 4-({[(4-tert-Butylphenyl)sulfonyl]amino}methyl)-N-(2-nitro-3-pyridinyl)benzamide (134).

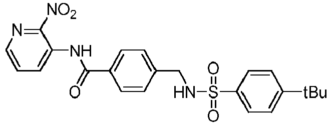

Oxalyl chloride (132 µL, 1.5 mmol) was added dropwise to a stirred suspension of benzoic acid **124** (355 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 2-Nitro-3-pyridinylamine (156 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-100%) of EtOAc/pet. ether, to give benzamide **134** (64 mg, 13%) as a cream powder: mp (EtOAc) 191-193 °C; ¹H NMR δ 10.78 (s, 1 H, CONH), 8.42 (dd, *J* = 4.5, 1.5 Hz, 1 H, H-6'), 8.28 (dd, *J* = 8.2, 1.5 Hz, 1 H, H-4'), 8.20 (br t, *J* = 6.4 Hz, 1 H, NHSO₂), 7.88 (dd, *J* = 8.2, 4.5 Hz, 1 H, H-5'), 7.85 (d, *J* = 8.4 Hz, 2 H, H-2, H-6), 7.71 (dd, *J* = 8.6, 1.8 Hz, 2 H, H-2", H-6"), 7.56 (dd, *J* = 8.6, 1.8 Hz, 2 H, H-3", H-5"), 7.40 (br d, *J =* 8.3 Hz, 2 H, H-3, H-5), 4.10 (d, *J* = 6.3 Hz, 2 H, CH₂N), 1.29 [s, 9 H, C(CH₃)₃]; ¹³C NMR δ 165.1, 155.4, 150.9, 144.1, 142.6, 137.8, 136.1, 131.6, 129.2, 127.7 (2), 127.6 (2), 127.3, 126.4 (2), 125.9 (2), 45.7, 34.8, 30.8 (3); MS *m*/*z* 469.8 (MH⁺, 100%). Anal. calcd for C₂₃H₂₄N₄O₄S: C, 58.96; H, 5.16; N, 11.96. Found: C, 59.17; H, 5.17; N, 12.08%.

### Example 117

### Preparation of 4-({[(4-tert-Butylphenyl)sulfonyl]amino}methyl)-N-[6-(4-morpholinyl)-3-pyridinyl]benzamide (135).

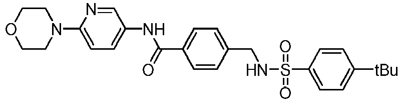

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-(4-morpholinyl)-3-pyridinylamine (200 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-100%) of EtOAc/pet. ether, to give benzamide **135** (262 mg, 51%) as a white powder: mp (EtOAc) 264-266 °C; ¹H NMR δ 10.05 (s, 1 H, CONH), 8.45 (d, *J* = 2.6 Hz, 1 H, H-2'), 8.17 (br t, *J* = 6.0 Hz, 1 H, NHSO₂), 7.91 (dd, *J* = 9.1, 2.6 Hz, 1 H, H-4'), 7.84 (br d, *J=* 8.3 Hz, 2 H, H-2, H-6), 7.69 (ddd, *J* = 8.6, 2.1, 1.8 Hz, 2 H, H-2", H-6"), 7.54 (ddd, *J* = 8.6, 2.1, 1.8 Hz, 2 H, H-3", H-5"), 7.34 (br d, *J* = 8.3 Hz, 2 H, H-3, H-5), 6.86 (d, *J* = 9.1 Hz, 1 H, H-5'), 4.08 (br d, *J* = 6.0 Hz, 2 H, CH₂N), 3.71 (br t, *J* = 4.8 Hz, 4 H, 2 × CH₂O), 3.40 (br t, *J* = 4.8 Hz, 4 H, 2 × CH₂N), 1.29 [s, 9 H, C(CH₃)₃]; ¹³C NMR δ 164.8, 156.1, 155.3, 141.3, 140.3, 137.9, 133.2, 131.1, 127.4 (2), 127.3 (2), 127.0, 126.3 (2), 125.9 (2), 106.6, 65.9 (2), 45.8, 45.6 (2), 37.8, 30.8 (3); MS *m*/*z* 509.8 (MH⁺, 100%). Anal. calcd for C₂₇H₃₂N₄O₄S: C, 63.76; H, 6.34; N, 11.02. Found: C, 63.77; H, 6.51; N, 10.81%.

### Example 118

### Preparation of 4-({[(4-tert-Butylphenyl)sulfonyl]amino}methyl)-N-[6-(trifluoromethyl)-3-pyridinyl]benzamide (136).

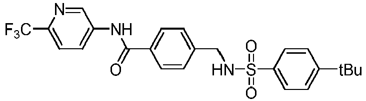

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-Trifluoromethyl-3-pyridinylamine (180 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-100%) of EtOAc/pet. ether, to give benzamide **136** (268 mg, 54%) as a white powder: mp (EtOAc) 249-252 °C; ¹H NMR δ 10.69 (s, 1 H, CONH), 9.09 (d, *J* = 2.4 Hz, 1 H, H-2'), 8.48 (dd, *J* = 8.6, 2.2 Hz, 1 H, H-4'), 8.23 (br s, 1 H, NHSO₂), 7.86-7.93 (m, 3 H, H-2, H-6, H-5'), 7.70 (ddd, *J* = 8.6, 2.2, 2.0 Hz, 2 H, H-2", H-6"), 7.55 (ddd, *J* = 8.6, 2.2, 1.9 Hz, 2 H, H-3", H-5"), 7.40 (d, *J* = 8.4 Hz, 2 H, H-3, H-5), 4.11 (br s, 2 H, CH₂N), 1.28 [s, 9 H, C(CH₃)₃]; ¹³C NMR δ 165.9, 155.3, 142.2, 141.7, 140.7 (q, *J* = 34.1 Hz), 138.8, 137.9, 132.4, 127.8 (2), 127.6, 127.5 (2), 126.3 (2), 125.9 (2), 121.8 (q, *J* = 273.0 Hz), 121.1 (q, *J* = 2.8 Hz), 45.7, 34.7, 30.7 (3); MS *m*/*z* 493.0 (MH⁺, 100%). Anal. calcd for C₂₄H₂₄F₃N₃O₃S: C, 58.65; H, 4.92; N, 8.55. Found: C, 58.82; H, 4.94; N, 8.41%.

### Example 119

### Preparation of N-[6-(Acetylamino)-3-pyridinyl]-4-({[(4-tert-butylphenyl)sulfonyl]amino}methyl)benzamide (137).

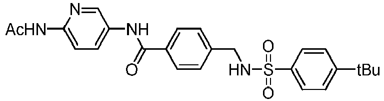

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-Acetyl-3-pyridinylamine (168 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (60-100%) of EtOAc/pet. ether, to give benzamide **137** (290 mg, 60%) as a white powder: mp (EtOAc) 240-243 °C; ¹H NMR δ 10.42 (s, 1 H, CONH), 10.27 (s, 1 H, CONH), 8.69 (t, *J* = 1.7 Hz, 1 H, H-2"), 8.18 (br s, 1 H, NHSO₂), 8.03-8.09 (m, 2 H, H-4", H-5"), 7.85 (br d, *J* = 8.4 Hz, 2 H, H-2, H-6), 7.70 (ddd, *J* = 8.6, 2.2, 2.0 Hz, 2 H, H-2', H-6'), 7.55 (ddd, *J* = 8.6, 2.2, 2.0 Hz, 2 H, H-3', H-5'), 7.37 (br d, *J* = 8.4 Hz, 2 H, H-3, H-5), 4.09 (br s, 2 H, CH₂N), 2.09 (s, 3 H, CH₃), 1.29 [s, 9 H, C(CH₃)₃]; ¹³C NMR δ 168.8, 165.1, 155.3, 147.9, 141.6, 140.0, 137.9, 133.0, 131.6, 130.0, 127.5 (2), 127.4 (2), 126.3 (2), 125.9 (2), 113.0, 45.8, 34.8, 30.6 (3), 23.8; MS *m*/*z* 481.8 (MH⁺, 100%). Anal. calcd for C₂₅H₂₈N₄O₄S: C, 62.48; H, 5.87; N, 11.66. Found: C, 62.51; H, 5.91; N, 11.88%.

### Example 120

### Preparation of 4-({[(4-tert-Butylphenyl)sulfonyl]amino}methyl)-N-(6-fluoro-3-pyridinyl)benzamide (138).

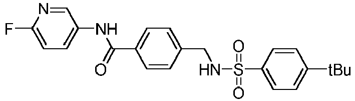

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-Fluoro-3-pyridinylamine (124 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-100%) of EtOAc/pet. ether, to give benzamide **138** (253 mg, 57%) as a white powder: mp (EtOAc) 254-257 °C; ¹H NMR δ 10.43 (s, 1 H, CONH), 8.57 (dd, *J* = 2.7,1.3 Hz, 1 H, H-2'), 8.31 (ddd, *J* = 8.9, 7.4, 2.8 Hz, 1 H, H-4'), 8.19 (br s, 1 H, NHSO₂), 7.86 (dd, *J* = 8.4, 1.7 Hz, 2 H, H-2, H-6), 7.70 (ddd, *J* = 8.6, 2.2, 1.9 Hz, 2 H, H-2", H-6"), 7.55 (ddd, *J* = 8.6, 2.2, 1.9 Hz, 2 H, H-3", H-5"), 7.38 (br d, *J* = 8.4 Hz, 2 H, H-3, H-5), 7.20 (dd, *J* = 8.9, 3.2 Hz, 1 H, H-5'), 4.09 (br s, 2 H, CH₂N), 1.29 [s, 9 H, C(CH₃)₃]; ¹³C NMR δ 165.3, 158.1 (d, *J* = 233.0 Hz), 155.3, 141.9, 138.9 (d, *J =* 15.6 Hz), 137.9, 134.1 (d, *J =* 4.5 Hz), 133.9 (d, *J* = 7.6 Hz), 132.7, 127.6 (2), 127.5 (2), 126.3 (2), 125.9 (2), 109.2 (d, *J =* 39.0 Hz), 47.8, 34.7, 30.8 (3); MS *m*/*z* 442.7 (MH⁺, 100%). Anal. calcd for C₂₃H₂₄FN₃O₃S: C, 62.57; H, 5.48; N, 9.52. Found: C, 62.64; H, 5.64; N, 9.56%.

### Example 121

### Preparation of 4-({[(4-tert-Butylphenyl)sulfonyl]amino}methyl)-N-(5-fluoro-3-pyridinyl)benzamide (139).

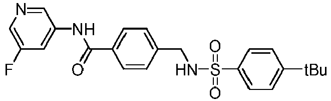

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 5-Fluoro-3-pyridinylamine (124 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (50-70%) of EtOAc/pet. ether, to give benzamide **139** (186 mg, 42%) as a white powder: mp (EtOAc) 214-217 °C; ¹H NMR δ 10.58 (s, 1 H, CONH), 8.78 (t, *J =* 1.6 Hz, 1 H, H-2'), 8.32 (d, *J* = 2.6 Hz, 1 H, H-6'), 8.16-8.21 (m, 2 H, NHSO₂, H-4'), 7.87 (br d, *J* = 8.4 Hz, 2 H, H-2, H-6), 7.70 (ddd, *J* = 8.6, 2.2, 1.9 Hz, 2 H, H-2", H-6"), 7.55 (ddd, *J* = 8.6, 2.2, 1.9 Hz, 2 H, H-3", H-5"), 7.40 (br d, *J* = 8.4 Hz, 2 H, H-3, H-5), 4.10 (br s, 2 H, CH₂N), 1.28 [s, 9 H, C(CH₃)₃]; ¹³C NMR δ 165.8, 158.6 (d, *J* = 253.0 Hz), 155.3, 142.1, 137.9 (d, *J* = 4.0 Hz), 137.0 (d, *J* = 6.2 Hz), 132.6, 132.0 (d, *J* = 22.6 Hz), 127.7 (2), 127.5 (2), 126.3 (2), 125.9 (2), 113.9 (d, *J* = 22.5 Hz), 45.7, 34.8, 30.7 (3); MS *m*/*z* 442.7 (MH⁺, 100%). Anal. calcd for C₂₃H₂₄FN₃O₃S: C, 62.57; H, 5.48; N, 9.52. Found: C, 62.48; H, 5.58; N, 9.52%.

### Example 122

### Preparation of 4-({[(4-tert-Butylphenyl)sulfonyl]amino}methyl)-N-[4-(trifluoromethyl)-3-pyridinyl]benzamide (140).

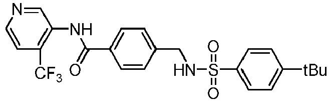

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 4-Trifluoromethyl-3-pyridinylamine (180 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **140** (265 mg, 53%) as a white powder: mp (EtOAc) 121-123 °C; ¹H NMR δ 10.29 (s, 1 H, CONH), 8.78 (dd, *J* = 5.1, 1.6 Hz, 1 H, H-6'), 8.76 (s, 1 H, H-2'), 8.19 (t, *J =* 6.2 Hz, 1 H, NHSO₂), 7.86 (br d, *J* = 8.4 Hz, 2 H, H-2, H-6), 7.84 (d, *J* = 5.1 Hz, 1 H, H-5'), 7.71 (ddd, *J* = 8.6, 2.2, 1.9 Hz, 2 H, H-2", H-6"), 7.56 (ddd, *J* = 8.6, 2.2, 1.9 Hz, 2 H, H-3", H-5"), 7.38 (br d, *J* = 8.4 Hz, 2 H, H-3, H-5), 4.10 (br s, 2 H, CH₂N), 1.30 [s, 9 H, C(CH₃)₃]; ¹³C NMR δ 166.3, 155.3, 152.0, 148.9, 142.1, 137.9, 133.7 (q, *J* = 31.2 Hz), 131.9, 130.9, 127.6 (2), 127.5 (2), 126.4 (2), 125.9 (2), 122.4 (d, *J* = 274.6 Hz), 120.2 (q, *J* = 4.4 Hz), 45.7, 34.8, 30.8 (3); MS *m*/*z* 492.8 (MH⁺, 100%). Anal. calcd for C₂₄H₂₄F₃N₃O₃S·H₂O: C, 56.57; H, 5.14; N, 8.24. Found: C, 56.76; H, 5.28; N, 8.30%.

### Example 123

### Preparation of 4-({[(4-tert-Butylphenyl)sulfonyl]amino}methyl)-N-(2-fluoro-3-pyridinyl)benzamide (141).

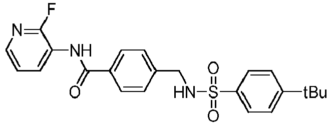

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 2-Fluoro-3-pyridinylamine (124 mg, 1.1 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (0-20%) of MeOH/EtOAc, to give benzamide **141** (276 mg, 62%) as a white powder: mp (EtOAc) 198-200 °C; ¹H NMR δ 10.18 (s, 1 H, CONH), 8.21 (t, *J* = 6.2 Hz, 1 H, NHSO₂), 8.16 (ddd, *J* = 9.6, 7.8, 1.2 Hz, 1 H, H-4'), 8.07 (dt, = 4.8, 1.5 Hz, 1 H, H-6'), 7.86 (br d, *J* = 8.4 Hz, 2 H, H-2, H-6), 7.69 (ddd, *J* = 8.6, 2.2, 1.9 Hz, 2 H, H-2", H-6"), 7.54 (ddd, *J* = 8.6, 2.2, 1.9 Hz, 2 H, H-3", H-5"), 7.40 (ddd, *J* = 7.8, 4.8, 1.2 Hz, 1 H, H-5'), 7.37 (br d, *J =* 8.4 Hz, 2 H, H-3, H-5), 4.10 (br s, 2 H, CH₂N), 1.29 [s, 9 H, C(CH₃)₃]; ¹³C NMR δ 165.3, 156.3 (d, *J =* 237.5 Hz), 155.3, 143.2 (d, *J* =14.3 Hz), 142.0, 137.9, 136.6 (d, *J =* 3.4 Hz), 132.1, 127.7 (2), 127.5 (2), 126.3 (2), 125.9 (2), 122.1 (d, *J* = 4.0 Hz), 121.2 (d, *J* = 27.6 Hz), 45.8, 34.7, 30.8 (3); MS *m*/*z* 442.6 (MH⁺, 100%). Anal. calcd for C₂₃H₂₄FN₃O₃S: C, 62.57; H, 5.48; N, 9.52. Found: C, 62.46; H, 5.27; N, 9.46%.

### Example 124

### Preparation of 4-({[(4-tert-Butylphenyl)sulfonyl]amino}methyl)-N-(4-methoxy-3-pyridinyl)benzamide (142).

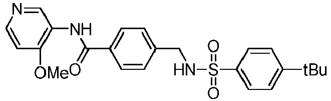

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 4-Methoxy-3-pyridinylamine [prepared by stirring 4-methoxy-3-nitropyridine (202 mg, 1.32 mmol) with Pd/C (50 mg) in EtOH under H₂ (60 psi) for 1 h, filtered through Celite and the solvent was evaporated] was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (0-20%) of MeOH/EtOAc, to give benzamide **142** (165 mg, 36%) as a white powder: mp (EtOAc) 231-233 °C; ¹H NMR δ 9.28 (s, 1 H, CONH), 8.76 (d, *J* = 2.2 Hz, 1 H, H-2'), 8.20 (t, *J* = 6.2 Hz, 1 H, NHSO₂), 7.77 (d, *J* = 8.2 Hz, 2 H, H-2, H-6), 7.67-7.73 (m, 3 H, H-6', H-2", H-6"), 7.54 (d, *J* = 8.6 Hz, 2 H, H-3", H-5"), 7.38 (d, *J* = 8.3 Hz, 2 H, H-3, H-5), 7.30 (d, *J* = 7.3 Hz, 1 H, H-5'), 4.08 (d, *J* = 6.2 Hz, 2 H, CH₂N), 3.77 (s, 3 H, OCH₃), 1.28 [s, 9 H, C(CH₃)₃]; ¹³C NMR δ 168.8, 163.9, 155.3, 142.0, 139.9, 137.9, 132.3, 128.6, 128.5, 126.9 (2), 126.9 (2), 126.3 (2), 125.9 (2), 112.5, 45.7, 43.9, 34.7, 30.7 (3); MS m/z 454.8 (MH⁺, 100%). Anal. calcd for C₂₄H₂₇N₃O4S·½CH₃OH: C, 62.67; H, 6.23; N, 8.95. Found: C, 62.65; H, 6.11; N, 9.17%.

### Example 125

### Preparation of N-(6-Bromo-3-pyridinyl)-4-({[(4-tert-butylphenyl)sulfonyl]amino}methyl)benzamide (143).

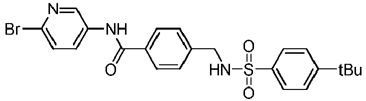

Oxalyl chloride (132 µL, 1.5 mmol) was added dropwise to a stirred suspension of benzoic acid **124** (350 mg, 1.0 mmol) and DMF (1 drop) in dry THF (20 mL) and the solution was stirred at 20 °C for 2 h, then at 66 °C for 1 h. The solution was cooled to 20 °C, then the solvent was evaporated and the residue dissolved in dry pyridine (10 mL). 6-Bromo-3-pyridinylamine (210 mg, 1.2 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with a gradient (0-20%) of MeOH/EtOAc, to give benzamide **143** (351 mg, 69%) as a white powder: mp (EtOAc) 242-244 °C; ¹H NMR δ 10.48 (s, 1 H, CONH), 8.78 (d, *J=* 2.8 Hz, 1 H, H-2"), 8.20 (br t, *J* = 6.0 Hz, 1 H, NHSO₂), 8.17 (dd, *J* = 8.7, 2.8 Hz, 1 H, H-4"), 7.86 (dd, *J* = 8.3, 1.6 Hz, 2 H, H-2, H-6), 7.69 (ddd, *J* = 8.6, 2.2, 1.9 Hz, 2 H, H-2', H-6'), 7.63 (d, *J* = 8.7 Hz, 1 H, H-5"), 7.54 (ddd, *J* = 8.6, 2.2, 1.9 Hz, 2 H, H-3', H-5'), 7.38 (d, *J =* 8.3 Hz, 2 H, H-3, H-5), 4.09 (d, *J* = 6.0 Hz, 2 H, CH₂N), 1.28 [s, 9 H, C(CH₃)₃]; ¹³C NMR δ 156.5, 155.3, 142.1, 142.0, 137.9, 135.8, 134.4, 132.6, 130.7, 127.8, 126.7 (2), 127.5 (2), 126.3 (2), 125.9 (2), 45.8, 34.8, 30.8 (3); MS *m*/*z* 502.5/504.5 (MH⁺, 100%). Anal. calcd for C₂₃H₂₄BrN₃O₃S: C, 54.98; H, 4.81; N, 8.36. Found: C, 55.05; H, 5.01; N, 8.28%.

### Example 126

### Preparation of 4-[({[3-(4-Morpholinyl)phenyl]sulfonyl}amino)methyl]-N-(3-pyridinyl)benzamide (144).

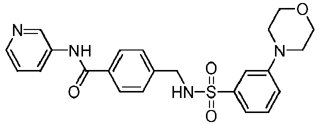

A mixture of fluoride **29** (110 mg, 0.29 mmol) and morpholine (2 mL) in DMSO (1 mL) was stirred in a sealed tube at 130 °C for 72 h. The solvent was evaporated and the residue was suspended in ice/water (50 mL) for 1 h. The precipitate was filtered, washed with water (5 mL) and dried. The crude solid was purified by column chromatography, eluting with a gradient (0-10%) of MeOH/EtOAc, to give benzamide **144** (113 mg, 86%) as a white powder: mp (EtOAc) 160-162 °C; ¹H NMR δ 10.36 (s, 1 H, CONH), 8.93 (d, *J* = 2.2 Hz, 1 H, H-2'), 8.31 (dd, *J* = 4.7, 1.4 Hz, 1 H, H-6'), 8.18 (ddd, *J* = 8.3, 2.4, 1.5 Hz, 1 H, H-4'), 8.14 (t, *J =* 6.0 Hz, 1 H, NHSO₂), 7.91 (br d, *J* = 8.3 Hz, 2 H, H-2, H-6), 7.37-7.45 (m, 4 H, H-3, H-5, H-3", H-5"), 7.13-7.26 (m 3 H, H-5', H-2", H-4"), 4.08 (d, *J* = 6.0 Hz, 2 H, CH₂N), 3.75 (br dd, *J* = 4.9, 4.7 Hz, 4 H, 2 × CH₂O), 3.15 (br dd, *J* = 4.9, 4.7 Hz, 4 H, 2 × CH₂N); ¹³C NMR δ 165.5, 151.2, 144.5, 142.0, 141.9, 141.4, 135.8, 133.0, 129.7, 127.7 (2), 127.4 (2), 127.3, 123.5, 118.5, 116.5, 111.9, 65.9 (2), 47.8 (2), 45.8; MS *m*/*z* 453.6 (MH⁺, 100%). Anal. calcd for C₂₃H₂₄N₄O₄S: C, 61.05; H, 5.35; N, 12.38. Found: C, 61.12; H, 5.49; N, 12.21%.

### Example 127

### Preparation of 4-[({[4-(4-Morpholinyl)phenyl]sulfonyl}amino)methyl]-N-(3-pyridinyl)benzamide (145).

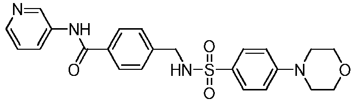

A mixture of fluoride **52** (108 mg, 0.28 mmol) and morpholine (2 mL) in DMSO (1 mL) was stirred in a sealed tube at 130 °C for 16 h. The solvent was evaporated and the residue was suspended in ice/water (50 mL) for 1 h. The precipitate was filtered, washed with water (5 mL) and dried. The crude solid was purified by column chromatography, eluting with a gradient (0-10%) of MeOH/EtOAc, to give benzamide **145** (110 mg, 87%) as a white powder: mp (EtOAc) 206-208 °C; ¹H NMR δ 10.36 (s, 1 H, CONH), 8.93 (d, *J* = 2.1 Hz, 1 H, H-2'), 8.31 (dd, *J* = 4.7, 1.5 Hz, 1 H, H-6'), 8.19 (ddd, *J* = 8.3, 2.5, 1.5 Hz, 1 H, H-4'), 7.94 (t, *J* = 6.0 Hz, 1 H, NHSO₂), 7.90 (br d, *J =* 8.3 Hz, 2 H, H-2, H-6), 7.61 (ddd, *J* = 9.0, 2.8, 1.9 Hz, 2 H, H-2", H-6"), 7.36-7.42 (m, 3 H, H-3, H-5, H-5'), 7.02 (ddd, *J* = 9.0, 2.8, 1.9 Hz, 2 H, H-3", H-5"), 4.02 (d, *J* = 6.0 Hz, 2 H, CH₂N), 3.72 (br dd, *J* = 5.0, 4.8 Hz, 4 H, 2 × CH₂O), 3.25 (br dd, *J* = 5.0, 4.8 Hz, 4 H, 2 × CH₂N); ¹³C NMR δ 165.4, 153.2, 144.4, 141.9, 141.8, 135.7, 132.8, 128.8, 128.0 (2), 127.5 (2), 127.3 (2), 127.2, 123.3, 113.4 (2), 65.7 (2), 49.6 (2), 45.6; MS *m*/*z* 453.6 (MH⁺, 100%). Anal. calcd for C₂₃H₂₄N₄O₄S: C, 61.05; H, 5.35; N, 12.38. Found: C, 61.11; H, 5.57; N, 12.49%.

### Example 128

### Preparation of 4-[({[4-(1-Piperidinyl)phenyl]sulfonyl}amino)methyl]-N-(3-pyridinyl)benzamide (146).

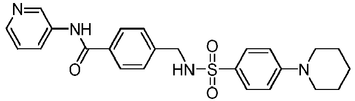

A mixture of fluoride **52** (107 mg, 0.28 mmol) and piperidine (2 mL) in DMSO (1 mL) was stirred in a sealed tube at 130 °C for 16 h. The solvent was evaporated and the residue was suspended in ice/water (50 mL) for 1 h. The precipitate was filtered, washed with water (5 mL) and dried. The crude solid was purified by column chromatography, eluting with a gradient (0-10%) of MeOH/EtOAc, to give benzamide **146** (97 mg, 77%) as a white powder: mp (EtOAc) 220-223 °C; ¹H NMR δ 10.35 (s, 1 H, CONH), 8.93 (d, *J* = 2.2 Hz, 1 H, H-2'), 8.31 (dd, *J* = 4.7, 1.5 Hz, 1 H, H-6'), 8.19 (ddd, *J* = 8.3, 2.5, 1.5 Hz, 1 H, H-4'), 7.86-7.91 (m, 3 H, H-2, H-6, NHSO₂), 7.55 (br d, *J* = 9.0 Hz, 2 H, H-2", H-6"), 7.36-7.41 (m, 3 H, H-3, H-5, H-5'), 6.96 (br d, *J* = 9.0 Hz, 2 H, H-3", H-5"), 4.02 (d, *J* = 5.6 Hz, 2 H, CH₂N), 3.28-3.32 (m, 4 H, 2 × CH₂N), 1.56 (br s, 6 H, 3 × CH₂); ¹³C NMR δ 165.4, 153.0, 144.4, 142.0, 141.8, 135.7, 132.7, 128.0, 127.5 (3), 127.3 (2), 127.2 (2), 127.1, 113.4 (2), 47.8 (2), 45.6, 24.6 (2), 23.7; MS *m*/*z* 451.7 (MH⁺, 100%). Anal. calcd for C₂₄H₂₆N₄O₃S: C, 63.98; H, 5.82; N, 12.44. Found: C, 63.76; H, 5.77; N, 12.62%.

### Example 129

### Preparation of 4-[({[4-(4-Methoxy-1-piperidinyl)phenyl]sulfonyl}amino)methyl]-N-(3-pyridinyl)benzamide (147).

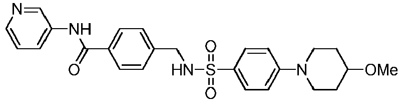

A mixture of fluoride **52** (119 mg, 0.31 mmol) and 4-methoxypiperidine (0.75 g) in DMSO (2 mL) was stirred in a sealed tube at 130 °C for 16 h. The mixture suspended in ice/water (50 mL) for 1 h. The precipitate was filtered, washed with water (5 mL) and dried. The crude solid was purified by column chromatography, eluting with a gradient (0-10%) of MeOH/EtOAc, to give benzamide **147** (124 mg, 83%) as a white powder: mp (EtOAc) 197-198 °C; ¹H NMR δ 10.35 (s, 1 H, CONH), 8.93 (d, *J* = 2.2 Hz, 1 H, H-2'), 8.30 (dd, *J* = 4.7, 1.5 Hz, 1 H, H-6'), 8.19 (ddd, *J* = 8.3, 2.4, 1.5 Hz, 1 H, H-4'), 7.89 (br d, *J* = 8.3 Hz, 2 H, H-2, H-6), 7.91 ( br s, 1 H, NHSO₂), 7.56 (br d, *J* = 9.0 Hz, 2 H, H-2", H-6"), 7.35-7.42 (m, 3 H, H-3, H-5, H-5'), 7.00 (br d, *J* = 9.0 Hz, 2 H, H-3", H-5"), 4.02 (br s, 2 H, CH₂N), 3.58-3.66 (m, 2 H, 2 × CHN), 3.34-3.40 (m, 1 H, OCH), 3.24 (s, 3 H OCH₃), 3.03-3.11 (m, 2 H, 2 × CHN), 1.84-1.92 (m, 2 H, 2 × CH), 1.42-1.52 (m, 2 H, 2 × CH); ¹³C NMR δ 165.5, 152.7, 144.5, 142.1, 142.0, 135.8, 132.9, 128.2 (2), 127.7, 127.6 (2), 127.4 (2), 127.2, 123.5, 113.7 (2), 75.0, 54.9, 45.8, 44.6 (2), 29.7 (2); MS m/z 481.6 (MH⁺, 100%). Anal. calcd for C₂₅H₂₈N₄O4S: C, 62.48; H, 5.87; N, 11.66. Found: C, 62.18; H, 5.91; N, 11.61 %.

### Example 130

### Preparation of 4-[({[4-(21-Amino-4,7,10,13,16,19-hexaoxahenicos-1-yl)phenyl]sulfonyl}amino)methyl]-N-(3-pyridinyl)benzamide (149).

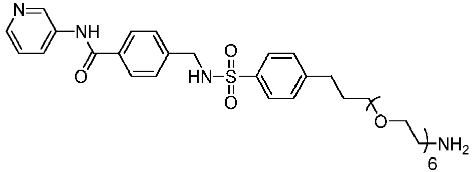

***tert*-Butyl 21-{4-[({4-[(3-Pyridinylamino)carbonyl]benzyl}amino)sulfonyl]phenyl} -3,6,9,12,15,18-hexaoxahenicos-1-ylcarbamate (148).** A mixture of alkyne **115** (743 mg, 0.95 mmol) and 10% Pd/C (250 mg, 0.1 mmol) in absolute EtOH (30 mL) was stirred at 20 ºC under of H₂ (60 psi) for 16 h. The mixture was filtered through Celite, washed with EtOH (100 mL), and the solvent was evaporated. The residue was purified by column chromatography, eluting with a gradient (3-5%) of MeOH/DCM, to give the carbamate **148** (650 mg, 87%) as a pale yellow oil: ¹H NMR δ 10.35 (s, 1 H, CONH), 8.92 (d, *J* = 2.4 Hz, 1 H, H-2'), 8.31 (dd, *J* = 4.7, 1.4 Hz, 1 H, H-6'), 8.17-8.20 (m, 2 H, NHSO₂, H-4'), 7.88 (br d, *J =* 8.3 Hz, 2 H, H-2, H-6), 7.88 (br d, *J* = 8.3 Hz, 2 H, H-2", H-6"), 7.37-7.40 (m, 5 H, H-5', H-3, H-5, H-3", H-5"), 6.69 (br s, 1 H, NHCO₂), 4.08 (d, *J* = 5.0 Hz, 2 H, CH₂N), 3.45-3.52 (m, 20 H, CH₂O), 3.37 (t, *J* = 6.2 Hz, 4 H, H-3"', H-20"'), 3.05 (q, *J =* 6.0 Hz, 2 H, H-21"'), 2.69 (t, *J* = 7.7 Hz, 2 H, H-1"'), 1.77-1.84 (m, 2 H, H-2"'), 1.37 [s, 9 H, C(CH₃)₃]; HRMS calcd for C₃₉H₅₆N₄O₁₁S (M⁺) *m*/*z* 789.3739; found 789.3723 (1.6 ppm).

**4-[({[4-(21-Amino-4,7,10,13,16,19-hexaoxahenicos-1-yl)phenyl]sulfonyl}amino) methyl]-*N*-(3-pyridinyl)benzamide (149)**. Trifluoroacetic acid (0.74 mL, 10 mmol) was added to a solution of carbamate **148** (313 mg, 0.4 mmol) in anhydrous DCM (5 mL) and the reaction mixture was stirred at 20 ºC for 2 h. The solvent was evaporated and the residue was purified by column chromatography, eluting with 8% MeOH/DCM containing 1% aqueous NH₃, to give the amine **149** (195 mg, 71%) as a colourless oil: ¹H NMR δ 10.35 (br s, 1 H, CONH), 8.92 (d, *J* = 2.2 Hz, 1 H, H-2'), 8.31 (dd, *J* = 4.7, 1.4 Hz, 1 H, H-6'), 8.18 (ddd, *J* = 8.3, 2.5, 1.5 Hz, 1 H, H-4'), 7.88 (br. d, *J* = 8.3 Hz, 2 H, H-2, H-6), 7.70 (br. d, *J* = 8.3 Hz, 2 H, H-2", H-6"), 7.37-7.40 (m, 5 H, H-5', H-3, H-5, H-3", H-5"), 4.08 (s, 2 H, C*H*₂NHSO₂), 3.45-3.52 (m, 20 H, CH₂O), 3.37 (t, *J* = 6.3 Hz, 2 H, H-3"'), 3.34 (t, *J* = 5.8 Hz, 2 H, H-20"'), 2.70 (t, *J =* 7.9 Hz, 2 H, H-1"'), 2.63 (t, *J =* 5.7 Hz, 2 H, H-21"'), 1.80 (tt, *J* = 6.4, 7.9 Hz, 2 H, H-2"'), NHSO₂ and NH₂ not observed; ¹³C NMR δ165.4, 146.6, 144.4, 141.9, 141.7, 138.0, 135.7, 132.8, 128.9 (2), 127.5 (2), 127.3 (2), 127.2, 126.4 (2), 123.3, 72.2, 69.69 (2), 69.66 (4), 69.62 (2), 69.4, 69.3, 69.1, 45.6, 40.9, 31.3, 30.4; H RMS calcd for C₃₄H₄₈N₄O₉S (M⁺) *m*/*z* 689.3215; found 389.3224 (-1.0 ppm).

### Example 131

### Preparation of 4-({[(4-{[3-(4-Morpholinyl)propyl]amino}phenyl)sulfonyl]amino}methyl)-N-(3-pyridinyl)benzamide (150).

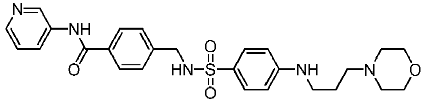

A mixture of fluoride **52** (113 mg, 0.29 mmol) and 3-(4-morpholinyl)propylamine (2.0 mL) in DMSO (1 mL) was stirred in a sealed tube at 130 °C for 16 h. The mixture suspended in ice/water (50 mL) for 1 h. The precipitate was filtered, washed with water (5 mL) and dried. The crude solid was purified by column chromatography, eluting with a gradient (5-10%) of MeOH/DCM followed by 1% aqueous NH₃/10% MeOH/DCM, to give benzamide **150** (135 mg, 91%) as a white powder: mp (EtOAc) 181-183 °C; ¹H NMR δ 10.36 (s, 1 H, CONH), 8.93 (d, *J* = 2.3 Hz, 1 H, H-2'), 8.31 (dd, *J* = 4.7, 1.4 Hz, 1 H, H-6'), 8.19 (ddd, *J* = 8.3, 2.4, 1.5 Hz, 1 H, H-4'), 7.90 (br d, *J* = 8.3 Hz, 2 H, H-2, H-6), 7.75 ( br s, 1 H, NHSO₂), 7.30 (br d, *J* = 8.8 Hz, 2 H, H-2", H-6"), 7.36-7.43 (m, 3 H, H-3, H-5, H-5'), 6.62 (br d, *J* = 8.8 Hz, 2 H, H-3", H-5"), 6.48 (br t, *J* = 5.4 Hz, 1 H, NH), 3.98 (br d, *J =* 6.4 Hz, 2 H, CH₂N), 3.58 (br t, *J* = 4.6 Hz, 4 H, 2 × CH₂N), 3.10 (dt, *J* = 6.5, 5.4 Hz, 2 H, CH₂N), 2.30-2.38 (m, 6 H, CH₂H, 2 × CH₂O), 1.68 (p, *J* = 6.9 Hz, 2 H, 2 × CH₂); ¹³C NMR δ 165.5, 152.0, 144.5, 142.3, 142.0, 135.8, 132.9, 128.4 (2), 127.6 (2), 127.4 (2), 127.2, 125.2, 123.4 (2), 110.8, 66.2 (2), 55.9, 53.4 (2), 45.7, 40.5, 25.3; MS *m*/*z* 510.8 (MH⁺, 100%). The compound was formulated as the HCl salt. Anal. calcd for C₂₆H₃₁N₅O₄S·HCl: C, 61.28; H, 6.13; N, 13.74. Found: C, 61.27; H, 6.15; N, 13.84%.

### Example 132

### Preparation of 4-[({[3-(4-Methyl-1-piperazinyl)phenyl]sulfonyl}amino)methyl]-N-(3-pyridinyl)benzamide (151).

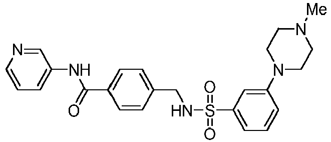

A mixture of fluoride **29** (110 mg, 0.29 mmol) and 4-methylpiperazine (2 mL) in DMSO (1 mL) was stirred in a sealed tube at 130 °C for 72 h. The solvent was evaporated and the residue was suspended in ice/water (50 mL) for 1 h. The precipitate was filtered, washed with water (5 mL) and dried. The crude solid was purified by column chromatography, eluting with a gradient (5-10%) of MeOH/DCM followed by 1% aqueous NH₃/10% MeOH/DCM, to give starting material **29** (67 mg, 60%) and benzamide **151** (45 mg, 33%) as a white powder: ¹H NMR δ 10.36 (s, 1 H, CONH), 8.93 (d, *J* = 2.4 Hz, 1 H, H-2'), 8.31 (dd, *J* = 4.7, 1.4 Hz, 1 H, H-6'), 8.18 (ddd, *J* = 8.3, 2.4, 1.5 Hz, 1 H, H-4'), 8.13 (t, *J* = 6.3 Hz, 1 H, NHSO₂), 7.91 (br d, *J* = 8.3 Hz, 2 H, H-2, H-6), 7.37-7.43 (m, 4 H, H-3, H-5, H-5', H-5"), 7.25 (br t, *J* = 1.9 Hz, 1 H, H-2"), 7.16-7.21 (m, 2 H, H-4", H-6"), 4.07 (d, *J* = 6.3 Hz, 2 H, CH₂N), 3.15-3.3.20 (m, 6 H, 3 × CH₂N), 2.48-2.52 (m, 2 H, CH₂N), 2.20 (s, 3 H, NCH₃); ¹³C NMR δ 165.5, 151.0, 144.5, 142.0, 141.9, 141.4, 135.8, 133.0, 129.7, 127.7 (2), 127.4 (2), 127.3, 123.5, 118.7, 116.1, 112.1, 54.2 (2), 43.7 (2), 45.8, 45.4; MS *m*/*z* 466.7 (MH⁺, 100%).

### Example 133

### Preparation of 4-({[(4-{[2-(Dimethylamino)ethyl]amino}phenyl)sulfonyl]amino}methyl)-N-(3-pyridinyl)benzamide (152).

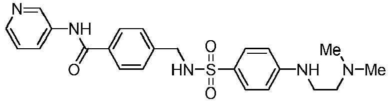

A mixture of fluoride **52** (170 mg, 0.44 mmol) and *N*,*N-*dimethylethylenediamine (2.0 mL) in DMSO (1 mL) was stirred in a sealed tube at 130 °C for 40 h. The mixture suspended in ice/water (50 mL) for 1 h. The precipitate was filtered, washed with water (5 mL) and dried. The crude solid was purified by column chromatography, eluting with a gradient (5-10%) of MeOH/DCM followed by 1%aqueous NH₃/10% MeOH/DCM, to give benzamide **152** (171 mg, 86%) as a white powder: mp (EtOAc) 166-168 °C; ¹H NMR δ 10.36 (s, 1 H, CONH), 8.93 (d, *J* = 2.2 Hz, 1 H, H-2'), 8.31 (dd, *J* = 4.7, 1.5 Hz, 1 H, H-6'), 8.18 (ddd, *J* = 8.3, 2.5, 1.5 Hz, 1 H, H-4'), 7.90 (br d, *J* = 8.3 Hz, 2 H, H-2, H-6), 7.76 (t, *J* = 6.4 Hz, 1 H, NHSO₂), 7.50 (d, *J* = 8.9 Hz, 2 H, H-2", H-6"), 7.35-7.43 (m, 3 H, H-3, H-5, H-5'), 6.66 (d, *J* = 8.9 Hz, 2 H, H-3", H-5"), 6.28 (br t, *J* = 5.3 Hz, 1 H, NH), 3.99 (d, *J* = 6.4 Hz, 2 H, CH₂N), 3.14 (dt, *J* = 6.5, 5.3 Hz, 2 H, CH₂N), 2.43 (t, *J* = 6.5 Hz, 2 H, CH₂N), 2.18 [s, 6 H, N(CH₃)₂]; ¹³C NMR δ 165.4, 151.7, 144.5, 142.1, 141.9, 135.8, 132.8, 128.3 (2), 127.5 (2), 127.3 (2), 127.1, 125.3, 123.3 (2), 110.8, 57.4, 45.6, 45.1 (2), 40.3; MS *m*/*z* 454.7 (MH⁺, 100%). Anal. calcd for C₂₃H₂₇N₅O₃S: C, 60.91; H, 6.00; N, 15.44. Found: C, 60.93; H, 6.00; N, 15.54%.

### Example 134

### Preparation of N-(3-Pyridinyl)-4-[({[3'-(trifluoromethyl)[1,1'-biphenyl]-4-yl]sulfonyl}amino)methyl]benzamide (153).

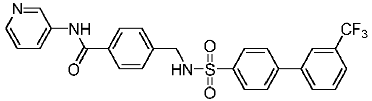

PdCl₂(dppf) (25 mg, 0.03 mmol) was added to a degassed solution of iodide **106** (150 mg, 0.3 mmol), 3-trifluoromethylboronic acid (75 mg, 0.39 mmol) and K₂CO₃ (415 mg, 3 mmol) in a mixture of toluene/ethanol/H₂O/DMF (5:3:2:2, 12 mL) and the mixture was heated at 90 ºC for 16 h. The mixture was cooled to 20 ºC, partitioned between EtOAc (200 mL) and H₂O (50 mL), and washed with brine (50 mL). The organic phase was dried, filtered and the solvent was evaporated. The residue was purified by column chromatography, eluting with a gradient (3-5%) of MeOH/DCM, to give benzamide **153** (147 mg, 96%) as a white powder: mp (MeOH/DCM) 227-229 ºC; ¹H NMR δ 10.33 (s, 1 H, CONH), 8.90 (d, *J* = 2.4 Hz, 1 H, H-2"'), 8.35 (t, *J* = 6.4 Hz, 1 H, NHSO₂), 8.31 (dd, *J* = 1.4, 4.6 Hz, 1 H, H-6"'), 8.16 (ddd, *J* = 1.6, 2.4, 8.3 Hz, 1 H, H-4"'), 8.05-8.03 (m, 2 H, H-2", H-4"), 7.95 (br d, *J* = 8.5 Hz, 2 H, H-2', H-6'), 7.89 (br. d, *J* = 8.7 Hz, 4 H, H-2, H-6, H-3', H-5'), 7.79 (br. d, *J* = 7.8 Hz, 1 H, H-6"), 7.72 (br. t, *J* = 8.0, 1 H, H-5"), 7.42 (br. d, *J* = 8.3 Hz, 2 H, H-3, H-5), 7.38 (dd, *J* = 4.7, 8.4 Hz, 1 H, H-5'"), 4.14 (d, *J* = 6.3 Hz, 2 H, C*H*₂NH); ¹³C NMR δ 165.4, 144.4, 142.1, 141.8, 141.5, 140.1, 139.5, 135.6, 132.9, 131.1, 130.0, 129.8 (q, *J* = 31.8 Hz), 127.7 (2), 127.6 (2), 127.4 (2), 127.1 (2), 124.9 (q, *J* = 3.7 Hz), 124.0 (q, *J* = 272.5 Hz), 123.4, (q, *J* = 3.9 Hz), 123.3, 45.7, 1 resonance not observed; MS *m*/*z* 513.4 (MH⁺, 100%). Anal. calcd for C₂₆H₂₀F₃N₃O₃S: C, 61.05; H, 3.94; N, 8.21. Found: C, 61.13; H, 3.84; N, 8.22%.

### Example 135

### Preparation of 4-({[(4-Benzylphenyl)sulfonyl]amino}methyl)-N-(3-pyridinyl)benzamide (154).

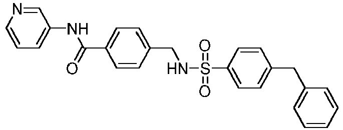

PdCl₂(dppf) (25 mg, 0.03 mmol) was added to a degassed solution of iodide **106** (150 mg, 0.3 mmol), 2-benzyl-4,4,5,5-tetramethyl-1,3,2-dioxaborolane (87 mg, 0.39 mmol) and K₂CO₃ (415 mg, 3 mmol) in a mixture of toluene/ethanol/H₂O/DMF (5:3:2:2, 12 mL) and the mixture was heated at 90 ºC for 16 h. The mixture was cooled to 20 ºC, partitioned between EtOAc (200 mL) and H₂O (50 mL), and washed with brine (50 mL). The organic phase was dried, filtered and the solvent evaporated. The residue was purified by column chromatography, eluting with 30% EtOAc/pet. ether, to give benzamide **154** (40 mg, 29%) as a white powder: mp (EtOAc/pet. ether) 198-200 ºC; ¹H NMR δ 10.36 (s, 1 H, CONH), 8.93 (d, *J* = 2.3 Hz, 1 H, H-2'), 8.31 (dd, *J* = 4.7, 1.4 Hz, 1 H, H-6'), 8.17-8.20 (m, 2 H, NHSO₂, H-4'), 7.88 (br d, *J =* 8.3 Hz, 2 H, H-2, H-6), 7.73 (br d, *J* = 8.3 Hz, 2 H, H-2", H-6"), 7.37-7.43 (m, 5 H, H-3, H-5, H-5', H-3", H-5"), 7.28-7.32 (m, 2 H, H-3'", H-5'"), 7.23-7.25 (m, 2 H, H-2'", 6'"), 7.17-7.21 (m, 1 H, H-4'"), 4.07 (s, 2 H, C*H*₂NH), 4.03 (s, 2 H, CH₂Ph); ¹³C NMR δ165.4, 146.0, 144.4, 141.9, 141.7, 140.1, 138.3, 135.7, 132.9, 129.2 (2), 128.6 (2), 128.4 (2), 127.5 (2), 127.3 (2), 127.1, 126.6 (2), 126.1, 123.3, 45.6, 40.6; MS m/z 459.3 (MH⁺, 100%). Anal. calcd for C₂₆H₂₃N₃O₃S: C, 68.25; H, 5.07; N, 9.18; Found: C, 68.14; H, 4.98; N, 9.17%.

### Example 136

### Preparation of 4-{[({4-[3-(4-Morpholinyl)-1-propynyl]phenyl}sulfonyl)amino]methyl}-N-(3-pyridinyl)benzamide (155).

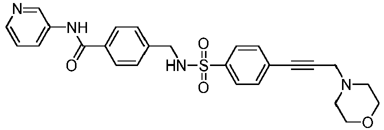

MsCl (22 µL, 0.29 mmol) was added to a solution of alcohol **119** (100 mg, 0.24 mmol) and NEt₃ (67 µL, 0.48 mmol) in anhydrous THF (10 mL) at -20 ºC and the reaction mixture was stirred at -20 ºC for 1 h. The mixture was diluted with EtOAc (100 mL), washed with H₂O (30 mL) and then washed with brine (30 mL). The organic phase was dried, filtered and the solvent was evaporated. Morpholine (0.44 mL, 5.0 mmol) was added to a solution of crude mesylate in anhydrous THF (20 mL) and the solution was stirred at 50 ºC for 2 h. The solution was cooled to 20 ºC, partitioned between EtOAc (200 mL) and saturated aqueous NaHCO₃ solution (50 mL), and the organic fraction was washed with NaHCO₃ (50 mL). The organic phase was dried, filtered and the solvent was evaporated. The residue was purified by column chromatography, eluting with 5% MeOH/DCM containing 0.5% aqueous NH₃, to give benzamide **155** (56 mg, 47%) as a white powder: mp (MeOH/DCM) 209-211 ºC; ¹H NMR δ10.36 (s, 1 H, CONH), 8.92 (d, *J* = 2.3 Hz, 1 H, H-2'), 8.34 (d, *J* = 6.3 Hz, 1 H, NHSO₂), 8.31 (dd, *J* = 1.3, 4.7 Hz, 1 H, H-6'), 8.18 (ddd, *J* = 8.3, 2.4, 1.5 Hz, 1 H, H-4'), 7.90 (br d, *J* = 8.3 Hz, 2 H, H-2, H-6), 7.78 (br d, *J* = 8.5 Hz, 2 H, H-2", H-6"), 7.62 (br d, *J =* 8.5 Hz, 2 H, H-3", H-5"), 7.37-7.41 (m, 3 H, H-3, H-5, H-5'), 4.10 (d, *J* = 6.3 Hz, 2 H, C*H*₂NH), 3.61 (t, *J* = 4.5 Hz, 4 H, 2 × CH₂O), 3.54 (s, 2 H, CH₂C≡C), 2.51 (m, 4 H, 2 × CH₂N); ¹³C NMR δ 165.4, 144.4, 141.9, 141.5, 140.0, 135.7, 133.0, 131.9 (2), 127.6 (2), 127.3 (2), 127.2, 126.7 (2), 126.2, 123.3, 88.4, 83.7, 65.9 (2), 51.6 (2), 46.8, 45.6; MS *m*/*z* 492.4 (MH⁺, 100%); Anal. calcd for C₂₆H₂₆N₄O₄S: C, 63.66; H, 5.34; N, 11.42; Found: C, 63.65; H, 5.33; N, 11.37%.

### Example 137

### Preparation of 4-{[({4-[3-(Dimethylamino)-1-propynyl]phenyl}sulfonyl)amino]methyl}-N-(3-pyridinyl)benzamide (156).

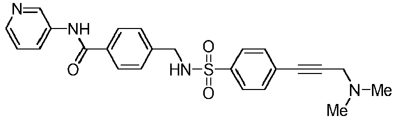

MsCl (41 µL, 0.53 mmol) was added to a solution of alcohol **119** (185 mg, 0.44 mmol) and Et₃N (0.13 mL, 0.88 mmol) in anhydrous THF (10 mL) at -20 ºC and the reaction mixture was stirred at -20 ºC for 1 h. The solution was then diluted with EtOAc (100 mL), washed with H₂O (30 mL) then brine (30 mL). The organic phase was dried, filtered and the solvent was evaporated. A 2 M solution of dimethylamine in THF (2.2 mL, 4.4 mmol) was added to a solution of crude mesylate in anhydrous DMF (10 mL) and the reaction mixture was stirred at 50 ºC for 2 h. The solution was cooled to 20 ºC, then partitioned between EtOAc (200 mL) and saturated aqueous NaHCO₃ solution (50 mL), and washed with NaHCO₃ (50 mL). The organic phase was dried, filtered and the solvent was evaporated. The residue was purified by column chromatography, eluting with 5% MeOH/DCM containing 0.5% aqueous NH₃, to give benzamide **156** (93 mg, 47%) as a pale yellow solid: mp (MeOH/DCM) 200-202 ºC; ¹H NMR δ 10.35 (s, 1 H, CONH), 8.93 (d, *J* = 2.3 Hz, 1 H, H-2'), 8.30-8.35 (m, 2 H, NHSO₂, H-6'), 8.18 (ddd, *J* = 8.3, 2.4, 1.5 Hz, 1 H, H-4'), 7.90 (br. d, *J* = 8.3 Hz, 2 H, H-2, H-6), 7.78 (br. d, *J* = 8.5 Hz, 2 H, H-2", H-6"), 7.62 (br d, *J* = 8.5 Hz, 2 H, H-3", H-5"), 7.36-7.41 (m, 3 H, H-5', H-3, H-5), 4.10 (d, *J=* 6.2 Hz, 2 H, C*H*₂NH), 3.48 (s, 2 H, C*H*₂N(CH₃)₂), 2.23 [s, 6 H, N(CH₃)₂]; ¹³C NMR δ 165.4, 144.4, 141.9, 141.5, 139.9, 135.7, 133.0, 131.9 (2), 127.6 (2), 127.3 (2), 127.1, 126.7 (2), 126.4, 123.3, 88.6, 83.6, 47.5, 45.6, 43.6 (2); MS *m*/*z* 450.2 (MH⁺, 100%). Anal. calcd for C₂₄H₂₄N₄O₃S: C, 64.27; H, 5.39; N, 12.49; Found: C, 64.41; H, 5.38; N, 12.42%.

### Example 138

### Preparation of 4-{[[(4-tert-Butylphenyl)sulfonyl](methyl)amino]methyl}-N-(3-pyridinyl) benzamide (157).

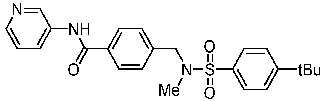

Mel (8 µL, 0.12 mmol) was added to a stirred suspension of benzamide **47** (50 mg, 0.12 mmol) and Cs₂CO₃ (80 mg, 0.24 mmol) in anhydrous DMF (2 mL), and the reaction mixture was stirred at 20 ºC for 16 h. The mixture was partitioned between EtOAc (100 mL) and H₂O (30 mL), and the organic fraction was washed with brine (30 mL). The organic phase was dried, filtered and the solvent was evaporated. The residue was purified by column chromatography, eluting with 5% MeOH/DCM, to give benzamide **157** (30 mg, 57%) as a white powder. mp (MeOH/DCM) = 215-218 ºC; ¹H NMR δ 10.41 (s, 1 H, CONH), 8.92 (d, *J* = 2.3, 1 H, H-2'), 8.31 (dd, *J* = 4.6, 1.4 Hz, 1 H, H-6'), 8.18 (ddd, *J* = 8.3, 2.5, 1.5 Hz, 1 H, H-4'), 7.97 (d, *J=* 8.3 Hz, 2 H, H-2, H-6), 7.79 (dt, *J* = 8.6, 1.7 Hz, 2 H, H-2", H-6"), 7.68 (dt, *J* = 8.6, 1.8 Hz, 2 H, H-3", H-5"), 7.48 (d, *J =* 8.3 Hz, 2 H, H-3, H-5), 7.39 (ddd, *J* = 8.3, 4.6, 0.4 Hz, 1 H, H-5'), 4.25 (s, 2 H, CH₂N), 2.60 (s, 3 H, NCH₃), 1.34 [s, 9 H, C(CH₃)₃]; ¹³C NMR δ165.6, 156.1, 144.2, 141.6, 140.3, 135.9, 134.2, 133.6, 128.1 (2), 128.0 (2), 127.6, 127.1 (2), 126.3 (2), 123.6, 52.9, 34.9, 34.7, 30.8 (3); MS *m*/*z* 439.2 (MH⁺, 100%); Anal. calcd for C₂₄H₂₇N₃O₃S: C, 65.88; H, 6.22; N, 9.60; Found: C, 65.78; H, 6.31; N, 9.69%.

### Example 139

### Preparation of 4-{[[(4-tert-Butylphenyl)sulfonyl](ethyl)amino]methyl}-N-(3-pyridinyl)benzamide (158).

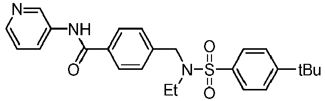

Ethyl iodide (38 µL, 0.47 mmol) was added to a stirred suspension of benzamide **47** (200 mg, 0.47 mmol) and Cs₂CO₃ (308 mg, 0.94 mmol) in anhydrous DMF (5 mL), and the reaction mixture was stirred at 20 ºC for 24 h. The mixture was partitioned between EtOAc (150 mL) and H₂O (50 mL), and the organic fraction was washed with brine (50 mL). The organic phase was dried, filtered and the solvent was evaporated. The residue was purified by column chromatography, eluting with 30% EtOAc/pet. ether to give benzamide **158** (63 mg, 30%) as a white solid; mp (EtOAc/pet. ether) 163-165 ºC; ¹H NMR δ 10.41 (s, 1 H, CONH), 8.93 (d, *J* = 2.4 Hz, 1 H, H-2'), 8.31 (dd, *J* = 4.7, 1.4 Hz, 1 H, H-6'), 8.18 (ddd, *J* = 8.3, 2.5, 1.5 Hz, 1 H, H-4'), 7.95 (br d, *J =* 8.3 Hz, 2 H, H-2, H-6), 7.80 (dt, *J* = 8.6, 2.0 Hz, 2 H, H-2", H-6"), 7.65 (dt, *J* = 8.6, 1.9 Hz, 2 H, H-3", H-5"), 7.49 (br d, *J =* 8.3 Hz, 2 H, H-3, H-5), 7.39 (dd, *J* = 8.8, 4.7 Hz, 1 H, H-5'), 4.41 (s, 2 H, C*H*₂NEtSO₂), 3.16 (q, *J* = 7.2 Hz, 2 H, C*H*₂CH₃), 1.33 [s, 9 H, C(CH₃)₃], 0.88 (t, *J* = 7.1 Hz, 3 H, CH₂C₃); ¹³C NMR δ 165.5, 155.7, 144.4, 141.9, 141.4, 136.4, 135.6, 133.3, 127.8 (2), 127.7 (2), 127.2, 126.7 (2), 126.1 (2), 123.3, 50.4, 43.0, 34.7, 30.7 (3), 13.6; MS *m*/*z* 453.3 (MH⁺, 100%). Anal. calcd for C₂₅H₂₉N₃O₃S: C, 66.49; H, 6.47; N, 9.31. Found: C, 66.66; H, 6.67; N, 9.25%.

### Example 140

### Preparation of 4-{[[(4-tert-Butylphenyl)sulfonyl](propyl)amino]methyl}-N-(3-pyridinyl)benzamide (159).

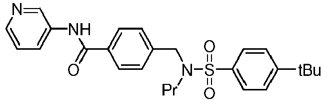

Propyl iodide (46 µL, 0.47 mmol) was added to a stirred suspension of benzamide **47** (200 mg, 0.47 mmol) and Cs₂CO₃ (308 mg, 0.94 mmol) in anhydrous DMF (5 mL), and the reaction mixture was stirred at 20 ºC for 24 h. The mixture was partitioned between EtOAc (150 mL) and H₂O (50 mL), and the organic fraction was washed with brine (50 mL). The organic phase was dried, filtered and the solvent was evaporated. The residue was purified by column chromatography, eluting with 40% EtOAc/pet. ether to give benzamide **159** (132 mg, 60%) as a white solid; mp (EtOAc/pet. ether) 177-179 ºC; ¹H NMR δ 10.41 (s, 1 H, CONH), 8.92 (d, *J* = 2.3 Hz, 1 H, H-2'), 8.31 (dd, *J* = 4.7, 1.4 Hz, 1 H, H-6'), 8.18 (ddd, *J* = 8.3, 2.5, 1.5 Hz, 1 H, H-4'), 7.94 (br. d, *J* = 8.3 Hz, 2 H, H-2, H-6), 7.79 (dt, *J* = 8.6, 1.9 Hz, 2 H, H-2", H-6"), 7.64 (dt, *J* = 8.6, 1.9 Hz, 2 H, H-3", H-5"), 7.48 (br d, *J =* 8.3 Hz, 2 H, H-3, H-5), 7.39 (ddd, *J* = 8.3, 4.7, 0.4 Hz, 1 H, H-5'), 4.39 (s, 2 H, C*H*₂NPrSO₂), 3.06 (br t, *J* = 7.5 Hz, 2 H, NC*H*₂CH₂CH₃), 1.33 [s, 9 H, C(CH₃)₃], 1.33-1.27 (m, 2 H, NCH₂C*H*₂CH₃), 0.67 (t, *J=* 7.4 Hz, 3 H, NCH₂CH₂C*H*₃); ¹³C NMR δ 165.5,155.7, 144.4, 141.9, 141.5, 136.2, 135.7, 133.3, 127.71 (2), 127.70 (2), 127.2, 126.7 (2), 126.1 (2), 123.4, 51.3, 50.5, 34.8, 30.7 (3), 21.3, 10.8; MS *m*/*z* 467.3 (MH⁺, 100%). Anal. calcd for C₂₆H₃₁N₃O₃S: C, 67.07; H, 6.71; N, 9.02. Found: C, 66.78; H, 6.78; N, 8.93%.

### Example 141

### Preparation of 4-{[({4-[3-(4-Morpholinyl)propyl]phenyl}sulfonyl)amino]methyl}-N-(3-pyridinyl)benzamide (160).

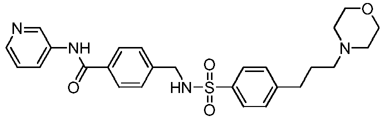

A mixture of alkyne **155** (190 mg, 0.39 mmol) and 10% Pd/C (70 mg, 0.06 mmol) in MeOH (20 mL) was stirred at 20 ºC under of H₂ (60 psi) for 4 h. The mixture was filtered through Celite, the pad was washed with MeOH (100 mL), and the solvent was evaporated. The residue was purified by column chromatography, eluting with 6% MeOH/DCM containing 0.5% aqueous NH₃, to give the benzamide **160** (50 mg, 26%) as a white powder: mp (MeOH/DCM) 170-171 ºC; ¹H NMR δ 10.35 (s, 1 H, CONH), 8.92 (d, *J* = 2.3 Hz, 1 H, H-2'), 8.31 (dd, *J* = 4.7, 1.4 Hz, 1 H, H-6'), 8.20-8.16 (m, 2 H, NHSO₂, H-4'), 7.88 (br d, *J* = 8.3 Hz, 2 H, H-2, H-6), 7.69 (br d, *J* = 8.3 Hz, 2 H, H-2", H-6"), 7.40-7.37 (m, 5 H, H-3, H-5, H-5', H-3", H-5"), 4.08 (d, *J* = 6.3 Hz, 2 H, CH₂NHSO₂), 3.54 (t, *J* = 4.6 Hz, 4 H, H-2"", H-6""), 2.66 (t, *J* = 7.4 Hz, 2 H, H-1"'), 2.31 (t, *J* = 4.3 Hz, 4 H, H-3"", H-5""), 2.25 (t, *J* = 7.2 Hz, 2 H, H-3"'), 1.73 (q, *J* = 7.4 Hz, 2 H, H-2"'); ¹³C NMR δ 165.5, 147.1, 144.5, 142.0, 141.8, 138.1, 135.8, 133.0, 129.0 (2), 127.6 (2), 127.4 (2), 127.2, 126.5 (2), 126.5, 66.2 (2), 57.4, 53.2 (2), 45.8, 32.6, 27.4; MS *m*/*z* 496.4 (MH⁺, 100%). Anal. calcd for C₂₆H₃₀N₄O₄S: C, 63.14; H, 6.11; N, 11.33. Found: C, 63.10; H, 6.19; N, 11.36%.

### Example 142

### Preparation of 4-{[({4-[3-(Dimethylamino)propyl]phenyl}sulfonyl)amino]methyl}-N-(3-pyridinyl)benzamide (161).

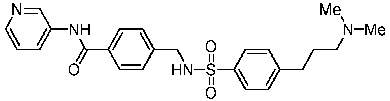

A mixture of alkyne **156** (250 mg, 0.56 mmol) and 10% Pd/C (80 mg, 0.07 mmol) in MeOH (20 mL) was stirred at 20 ºC under of H₂ (60 psi) for 4 h. The mixture was filtered through Celite, the pad was washed with MeOH (100 mL), and the solvent was evaporated. The residue was purified by column chromatography, eluting with 6% MeOH/DCM containing 0.5% aqueous NH₃, to give benzamide **161** (100 mg, 40%) as a white powder: mp (MeOH/DCM) 169-170 ºC; ¹H NMR δ 10.35 (s, 1 H, CONH), 8.92 (d, *J* = 2.2 Hz, 1 H, H-2'), 8.31 (dd, *J* = 1.5 Hz, 1 H, H-6'), 8.16-8.20 (m, 2 H, NHSO₂, H-4'), 7.88 (br d, *J* = 8.3 Hz, 2 H, H-2, H-6), 7.70 (br d, *J* = 8.3 Hz, 2 H, H-2", H-6"), 7.38-7.40 (m, 5 H, H-3, H-5, H-5', H-3", H-5"), 4.08 (d, *J* = 6.1 Hz, 2 H, CH₂N*H*SO₂), 2.65 (t, *J* = 7.4 Hz, 2 H, H-1"'), 2.18 (t, *J* = 7.2 Hz, 2 H, H-3"'), 2.11 [s, 6 H, N(CH₃)₂], 1.69 (br q, *J* = 7.3 Hz, 2 H, H-2"'); ¹³C NMR δ 165.5, 147.1, 144.5, 142.0, 141.8, 138.1, 135.8, 133.0, 129.0 (2), 127.6 (2), 127.4 (2), 127.3, 126.5 (2), 123.4, 58.3, 45.7, 45.1 (2), 32.6, 28.5; MS *m*/*z* 454.3 (MH⁺, 100%). Anal. calcd for C₂₄H₂₈N₄O₃S·H₂O: C, 61.26; H, 6.43; N, 11.91. Found: C, 61.61; H, 6.17; N, 11.90%.

### Example 143

### Preparation of 4-[({[3-(Propionylamino)phenyl]sulfonyl}amino)methyl]-N-(3-pyridinyl)benzamide (162).

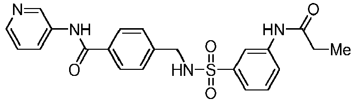

Propionyl chloride (25 µL, 0.29 mmol) was added dropwise to a stirred solution of aniline **26*** (106 mg, 0.28 mmol) and iPr₂NEt₂ (54 µL, 0.31 mmol) in dry THF (10 mL) and the solution was stirred at 20 °C for 16 h. The solvent was evaporated and the residue was suspended in ice/water (30 mL) for 1 h. The precipitate was filtered, washed with water (10 mL) and dried. The residue was purified by column chromatography, eluting with a gradient (0-10%) of MeOH/EtOAc, to give benzamide **162** (78 mg, 64%) as a white powder: mp (EtOH/EtOAc) 215-218 °C; ¹H NMR δ 10.38 (s, 1 H, CONH), 10.17 (s, 1 H, CONH), 8.93 (d, *J* = 2.2 Hz, 1 H, H-2'), 8.31 (dd, *J* = 4.7, 1.5 Hz, 1 H, H-6'), 8.28 (t, *J* = 6.3 Hz, 1 H, NHSO₂), 8.16-8.21 (m, 2 H, H-4', H-2"), 7.91 (br d, *J* = 8.3 Hz, 2 H, H-2, H-6), 7.77 (dt, *J* = 8.0, 1.7 Hz, 1 H, H-6"), 7.51 (t, *J* = 7.8 Hz, 1 H, H-5"), 7.47 (dt, *J* = 7.8, 1.6 Hz, 1 H, H-4"), 7.37-7.44 (m, 3 H, H-3, H-5, H-5'), 4.08 (s, 2 H, CH₂N), 2.35 (q, *J* = 7.5 Hz, 2 H, CH₂CO), 1.09 (t, *J* = 7.5 Hz, 3 H, CH₃); ¹³C NMR δ 172.4, 165.5, 144.5, 142.0, 141.9, 141.0, 139.9, 135.8, 133.0, 129.7, 127.7 (2), 127.4 (2), 127.3 123.5, 122.3, 120.6, 116.7, 45.7, 29.5, 9.5; MS *m*/*z* 439.5 (MH⁺, 100%). Anal. calcd for C₂₂H₂₂N₄O₄S: C, 60.26; H, 5.06; N, 12.78. Found: C, 59.97; H, 5.18; N, 12.39%.

### Example 144

### Preparation of 4-[({[3-(Acryloylamino)phenyl]sulfonyl}amino)methyl]-N-(3-pyridinyl)benzamide (163).

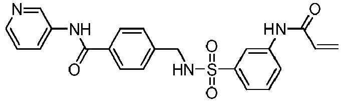

Acryloyl chloride (50 µL, 0.62 mmol) was added dropwise to a stirred solution of aniline **26** (225 mg, 0.59 mmol) and iPr₂NEt₂ (113 µL, 0.65 mmol) in dry THF (10 mL) and the solution was stirred at 20 °C for 16 h. The solvent was evaporated and the residue was suspended in ice/water (30 mL) for 1 h. The precipitate was filtered, washed with water (10 mL) and dried. The residue was purified by column chromatography, eluting with a gradient (0-10%) of MeOH/EtOAc, to give benzamide **163** (76 mg, 30%) as a white powder: mp (EtOH/EtOAc) 178-180 °C,; ¹H NMR δ 10.44 (s, 1 H, CONH), 10.36 (s, 1 H, CONH), 8.92 (d, *J* = 2.3 Hz, 1 H, H-2'), 8.25-8.33 (m, 3 H, NHSO₂, H-6', H-2"), 8.18 (ddd, *J* = 8.4, 2.3, 1.6 Hz, 1 H, H-4'), 7.91 (br d, *J* = 8.2 Hz, 2 H, H-2, H-6), 7.87 (dt, *J* = 7.5, 1.7 Hz, 1 H, H-6"), 7.50-7.58 (m, 2 H, H-4", H-5"), 7.36-7.45 (m, 3 H, H-3, H-5, H-5'), 6.44 (dd, *J* = 17.0, 10.0 Hz, 1 H, =CH₂), 6.30 (dd, *J* = 17.0, 2.0 Hz, 1 H, =CH₂), 5.80 (dd, *J* = 10.0, 2.0 Hz, 1 H, =CH), 4.08 (d, *J* = 6.2 Hz, 2 H, CH₂N); ¹³C NMR δ 165.5, 163.5, 144.5, 142.0, 141.8, 141.1, 139.6, 135.8, 133.0, 131.5, 129.8, 127.6 (2), 127.5, 127.4 (2), 127.3, 123.5, 122.7, 121.2, 117.1, 45.8; MS *m*/*z* 437.6 (MH⁺, 100%). Anal. calcd for C₂₂H₂₀N₄O₄S: C, 60.54; H, 4.62; N, 12.84. Found: C, 60.27; H, 4.67; N, 12.70%.

### Example 145

### Preparation of 4-({[(4-tert-Butylphenyl)sulfonyl]amino}methyl)-2-methyl-N-(3-pyridinyl)benzamide (167).

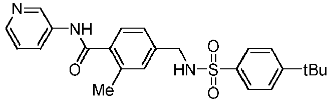

**4-Cyano-2-methylbenzoic acid (164).** A solution of nBuLi in THF (2.5 M, 2.24 mL, 5.6 mmol) was added dropwise to a stirred solution of 4-bromo-3-methylbenzonitrile (1.0 g, 5.1 mmol) in dry THF (50 mL) at -78 °C and the solution stirred at -78 °C for 1 h. A stream of dry CO₂ was bubbled through the solution for 10 min and the mixture warmed to 20 °C. The mixture was diluted with water (100 ml) and washed with Et₂O (3 × 20 mL). The aqueous phase was acidified to pH 2 with cHCl and extracted with CHCl₃ (3 × 50 mL) and the organic fraction was dried and the solvent evaporated to give crude acid **164** (0.67 g, 82%) as a tan powder: mp (CHCl₃) 193-195 °C; ¹H NMR δ 13.43 (br s, 1 H, CO₂H), 7.90 (d, *J* = 8.0 Hz, 1 H, H-6), 7.82 (br s, 1 H, H-3), 7.77 (dd, *J* = 8.0, 1.0 Hz, 1 H, H-5), 2.53 (s, 3 H, CH₃).

**4-Cyano-2-methyl-*N*-(3-pyridinyl)benzamide (165).** Oxalyl chloride (720 µL, 8.32 mmol) was added dropwise to a stirred suspension of benzoic acid **164** (670 mg, 4.16 mmol) and DMF (1 drop) in dry THF (25 mL) and the solution was stirred at 20 °C for 2 h, then at 66 °C for 1 h. The solution was cooled to 20 °C, then the solvent was evaporated and the residue dissolved in dry pyridine (10 mL). 3-Pyridinylamine (430 mg, 4.60 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide 165 (608 mg, 62%) as a white powder: mp (EtOAc) 193-195 °C; ¹H NMR δ 10.70 (s, 1 H, CONH), 8.86 (d, *J=* 2.3 Hz, 1 H, H-2'), 8.33 (dd, *J* = 4.7, 1.5 Hz, 1 H, H-6'), 8.16 (ddd, *J* = 8.3, 2.4, 1.5 Hz, 1 H, H-4'), 7.85 (br s, 1 H, H-3), 7.81 (br dd, *J* = 7.9, 1.0 Hz, 1 H, H-5), 7.69 (d, *J* = 7.9 Hz, 1 H, H-6), 7.41 (dd, *J* = 8.3, 4.7 Hz, 1 H, H-5'), 2.42 (s, 3 H, CH₃); MS *m*/*z* 238.4 (MH⁺, 100%). Anal. calcd for C₁₄H₁₁N₃O: C, 70.87; H, 4.67; N, 11.71. Found: C, 70.68; H, 4.75; N, 17.52%.

**4-(Aminomethyl)-2-methyl-N-(3-pyridinyl)benzamide (166).** A mixture of benzamide **165** (410 mg, 1.72 mmol) and 10% Pd/C (50 mg) and cHCl (0.43 mL, 5.2 mmol) in EtOH (100 mL) was stirred under H₂ (60 psi) at 20 °C for 16 h. The mixture was filtered through Celite, the Celite was washed with EtOH (20 mL), and the solvent was evaporated. The residue was partitioned between dilute aqueous NH₃ solution (30 mL) and CHCl₃ (3 × 30 mL) and the organic fraction dried and the solvent evaporated to give crude amine **166** (338 mg, 81%) as a gum: ¹H NMR δ 10.43 (s, 1 H, CONH), 8.87 (d, *J* = 2.2 Hz, 1 H, H-2'), 8.29 (dd, *J* = 4.7, 1.5 Hz, 1 H, H-6'), 8.37 (br d, *J =* 8.3 Hz, 1 H, H-4'), 7.45 (br d, *J =* 7.7 Hz, 1 H, H-6), 7.37 (ddd, *J* = 8.3, 4.7, 0.5 Hz, 1 H, H-5'), 7.24-7.28 (m, 2 H, H-3, H-5), 3.73 (s, 2 H, CH₂N), 2.38 (s, 3 H, CH₃), 2.05 (s, 2 H, NH₂); MS *m*/*z* 242.4 (MH⁺, 100%).

**4-({[(4-*tert*-Butylphenyl)sulfonyl]amino}methyl)-2-methyl-*N*-(3-pyridinyl)benzamide (167).** A mixture of benzamide **166** (334 mg, 1.38 mmol) and 4-*tert*-butylbenzenesulfonyl chloride (354 mg, 1.52 mmol) in dry pyridine (10 mL) was stirred at 20 °C for 16 h. The solvent was evaporated and the residue stirred in water (40 mL) for 1 h. The precipitate was filtered, washed with water (5 mL) and dried. The crude solid was purified by column chromatography, eluting with EtOAc, to give benzamide **167** (369 mg, 61%) as a white powder: mp (EtOAc) 162-163 °C; ¹H NMR δ 10.43 (s, 1 H, CONH), 8.86 (d, *J* = 2.2 Hz, 1 H, H-2'), 8.30 (dd, *J* = 4.7, 1.5 Hz, 1 H, H-6'), 8.13-8.18 (m, 2 H, NHSO₂, H-4'), 7.73 (ddd, *J* = 8.6, 2.2, 1.9 Hz, 2 H, H-2", H-6"), 7.60 (br d, *J* = 8.6, 2.2, 1.9 Hz, 2 H, H-3", H-5"), 7.43 (d, *J* = 7.8 Hz, 1 H, H-6), 7.37 (dd, *J* = 8.3, 4.7 Hz, 1 H, H-5'), 7.18 (br d, *J =* 7.8 Hz, 1 H, H-5), 7.12 (br s, 1 H, H-3), 4.08 (s, 2 H, CH₂N), 2.32 (s, 3 H, CH₃), 1.31 [s, 9 H, C(CH₃)₃]; ¹³C NMR δ 167.9, 155.2, 144.3, 141.2, 149.7, 137.9, 135.8, 135.4, 135.0, 129.6, 127.3, 126.4, 126.3 (2), 125.9 (2), 124.6, 123.5, 45.6, 34.7, 30.7 (3), 19.3; MS *m*/*z* 438.6 (MH⁺, 100%). Anal. calcd for C₂₄H₂₇N₃O₃S: C, 65.88; H, 6.22; N, 9.60. Found: C, 65.95; H, 6.40; N, 9.33%.

### Example 146

### Preparation of 4-({[(4-tert-Butylphenyl)sulfonyl]amino}methyl)-2-fluoro-N-(3-pyridinyl)benzamide (170).

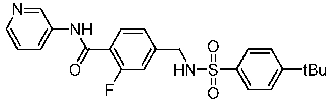

**4-Cyano-2-fluoro-*N*-(3-pyridinyl)benzamide (168).** Oxalyl chloride (0.79 mL, 9.08 mmol) was added dropwise to a stirred suspension of 4-cyano-2-fluorobenzoic acid (1.00 g, 6.06 mmol) and DMF (1 drop) in dry THF (40 mL) and the solution was stirred at 20 °C for 2 h, then at 66 °C for 1 h. The solution was cooled to 20 °C, then the solvent was evaporated and the residue dissolved in dry pyridine (10 mL). 3-Pyridinylamine (0.627 g, 6.67 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **168** (1.36 g, 93%) as a white powder: mp (EtOAc) 140-142 °C; ¹H NMR δ 10.86 (s, 1 H, CONH), 8.85 (d, *J=* 2.2 Hz, 1 H, H-2'), 8.36 (dd, *J* = 4.7, 1.5 Hz, 1 H, H-6'), 8.14 (ddd, *J* = 8.3, 2.5, 1.5 Hz, 1 H, H-4'), 8.08 (dd, *J* =1 0.0, 1.1 Hz, 1 H, H-3), 7.85-7.93 (m, 2 H, H-5, H-6), 7.42 (ddd, *J* = 8.3, 4.7, 0.4 Hz, 1 H, H-5'); MS *m*/*z* 242.3 (MH⁺, 100%). Anal. calcd for C₁₃H₈FN₃O·½H₂O: C, 62.40; H, 3.63; N, 16.79. Found: C, 62.53; H, 3.50; N, 16.92%.

**4-(Aminomethyl)-2-fluoro-*N*-(3-pyridinyl)benzamide (169).** A mixture of benzamide **168** (163 mg, 0.68 mmol) and 10% Pd/C (30 mg) and cHCl (0.17 mL, 2.0 mmol) in EtOH (50 mL) was stirred under H₂ (60 psi) at 20 °C for 16 h. The mixture was filtered through Celite, the Celite was washed with EtOH (20 mL), and the solvent was evaporated. The residue was partitioned between dilute aqueous NH₃ solution (30 mL) and CHCl₃ (3 × 30 mL) and the organic fraction dried and the solvent evaporated to give crude amine **169** (127 mg, 54%) as a white powder: mp (CHCl₃) 90-91 -C: ¹H NMR δ 10.51 (s, 1 H, CONH), 8.86 (d, *J* = 2.3 Hz, 1 H, H-2'), 8.31 (dd, *J* = 4.7, 1.5 Hz, 1 H, H-6'), 8.16 (br d, *J =* 8.3 Hz, 1 H, H-4'), 7.63 (t, *J* = 7.7 Hz, 1 H, H-6), 7.39 (ddd, *J* = 8.2, 4.7, 0.6 Hz, 1 H, H-5'), 7.34 (d, *J* =11.7 Hz, 1 H, H-3), 7.28 (dt, *J* = 7.8, 0.7 Hz, 1 H, H-5), 3.78 (s, 2 H, CH₂N), 2.01 (s, 2 H, NH₂); MS *m*/*z* 246.4 (MH⁺, 100%). Anal. calcd for C₁₃H₁₂FN₃O: C, 63.66; H, 4.93; N, 17.13. Found: C, 63.54; H, 5.01; N, 17.01%.

**4-({[(4-*tert*-Butylphenyl)sulfonyl]amino}methyl)-2-fluoro-*N*-(3-pyridinyl)benzamide (170).** A mixture of benzamide **169** (116 mg, 0.34 mmol) and 4-*tert*-butylbenzenesulfonyl chloride (86 mg, 0.37 mmol) in dry pyridine (10 mL) was stirred at 20 °C for 16 h. The solvent was evaporated and the residue stirred in water (40 mL) for 1 h. The precipitate was filtered, washed with water (5 mL) and dried. The crude solid was purified by column chromatography, eluting with EtOAc, to give benzamide **170** (111 mg, 74%) as a white powder: mp (EtOAc) 185-187 °C; ¹H NMR δ 10.52 (s, 1 H, CONH), 8.85 (d, *J* = 2.3 Hz, 1 H, H-2'), 8.32 (dd, *J* = 4.7, 1.5 Hz, 1 H, H-6'), 8.27 (br s, 1 H, NHSO₂), 8.13 (br d, *J* = 8.8 Hz, 1 H, H-4'), 7.70 (ddd, *J* = 8.6, 2.2, 1.9 Hz, 2 H, H-2", H-6"), 7.55-7.61 (m, 3 H, H-6, H-3", H-5"), 7.39 (ddd, *J* = 8.3, 4.7, 0.4 Hz, 1 H, H-5'), 7.20 (br dd, *J* = 8.0, 1.3 Hz, 1 H, H-5), 7.15 (br d, *J* = 11.4 Hz, 1 H, H-3), 4.10 (s, 2 H, CH₂N), 1.29 [s, 9 H, C(CH₃)₃]; ¹³C NMR δ 162.9, 158.8 (d, *J* = 249.9 Hz), 155.4, 144.8, 143.7 (d, *J* = 7.7 Hz), 141.4, 137.8, 135.5, 129.8 (d, *J* = 2.7 Hz), 126.8, 126.3 (2), 126.0 (2), 123.6, 123.4, 122.7 (d, *J* = 14.5 Hz), 114.9 (d, *J* = 22.8 Hz), 45.2, 34.8, 30.7 (3); MS *m*/*z* 442.6 (MH⁺, 100%). Anal. calcd for C₂₃H₂₄FN₃O₃S: C, 62.57; H, 5.48; N, 9.52. Found: C, 62.56; H, 5.57; N, 9.46%.

### Example 147

### Preparation of 4-({[(4-tert-Butylphenyl)sulfonyl]amino}methyl)-3-methyl-N-(3-pyridinyl)benzamide (*).

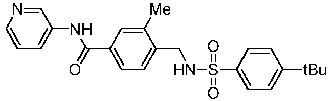

**4-Cyano-3-methylbenzoic acid (171).** A solution of nBuLi in THF (2.5 M, 4.49 mL, 11.2 mmol) was added dropwise to a stirred solution of 4-bromo-2-methylbenzonitrile (2.0 g, 10.2 mmol) in dry THF (100 mL) at -78 °C and the solution stirred at -78 °C for 1 h. A stream of dry CO₂ was bubbled through the solution for 10 min and the mixture warmed to 20 °C. The mixture was diluted with water (100 ml) and washed with Et₂O (3 × 20 mL). The aqueous phase was acidified to pH 2 with cHCl and extracted with CHCl₃ (3 × 50 mL) and the organic fraction was dried and the solvent evaporated to give crude acid **171** (1.05 g, 64%) as a tan powder: mp (EtOAc) 215-217 °C; ¹H NMR δ 13.49 (br s, 1 H, CO₂H), 7.99 (br s, 1 H, H-2), 7.91 (br d, *J* = 7.9 Hz, 1 H, H-5), 7.87 (br d, *J =* 8.0 Hz, 1 H, H-6), 2.55 (s, 3 H, CH₃).

**4-Cyano-3-methyl-*N*-(3-pyridinyl)benzamide (172).** Oxalyl chloride (763 µL, 8.75 mmol) was added dropwise to a stirred suspension of benzoic acid **171** (940 mg, 5.83 mmol) and DMF (1 drop) in dry THF (40 mL) and the solution was stirred at 20 °C for 2 h, then at 66 °C for 1 h. The solution was cooled to 20 °C, then the solvent was evaporated and the residue dissolved in dry pyridine (10 mL). 3-Pyridinylamine (603 mg, 6.41 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **172** (1.43 g, 99%) as a white powder: mp (EtOAc) 189-190 °C; ¹H NMR δ 10.64 (s, 1 H, CONH), 8.92 (d, *J* = 2.4 Hz, 1 H, H-2'), 8.34 (dd, *J* = 4.7, 1.5 Hz, 1 H, H-6'), 8.18 (ddd, *J* = 8.3, 2.5, 1.5 Hz, 1 H, H-4'), 8.02 (br s, 1 H, H-2), 7.97 (d, *J =* 8.1 Hz, 1 H, H-5), 7.91 (dd, *J* = 8.1, 1.0 Hz, 1 H, H-6), 7.41 (dd, *J* = 8.3, 4.7 Hz, 1 H, H-5'), 2.59 (s, 3 H, CH₃); MS *m*/*z* 238.4 (MH⁺, 100%). Anal. calcd for C₁₄H₁₁N₃O: C, 70.87; H, 4.67; N, 11.71. Found: C, 70.95; H, 4.67; N, 17.68.

**4-({[(4-*tert*-Butylphenyl)sulfonyl]amino}methyl)-3-methyl-*N*-(3-pyridinyl)benzamide (173).** A mixture of benzamide **172** (240 mg, 1.00 mmol) and 10% Pd/C (50 mg) and cHCl (0.25 mL, 3.0 mmol) in EtOH (50 mL) was stirred under H₂ (60 psi) at 20 °C for 16 h. The mixture was filtered through Celite, the Celite was washed with EtOH (20 mL), and the solvent was evaporated. The residue was partitioned between dilute aqueous NH₃ solution (30 mL) and CHCl₃ (3 × 30 mL) and the organic fraction dried and the solvent evaporated to give crude amine as a gum which was used directly. A mixture of crude amine (288 mg, 1.19 mmol) and 4-*tert-*butylbenzenesulfonyl chloride (306 mg, 1.31 mmol) in dry pyridine (10 mL) was stirred at 20 °C for 16 h. The solvent was evaporated and the residue stirred in water (40 mL) for 1 h. The precipitate was filtered, washed with water (5 mL) and dried. The crude solid was purified by column chromatography, eluting with EtOAc, to give benzamide **173** (44 mg, 10%) as a white powder: mp (EtOAc) 203-205 °C; ¹H NMR δ 10.32 (s, 1 H, CONH), 8.91 (d, *J* = 2.3 Hz, 1 H, H-2'), 8.30 (dd, *J* = 4.7, 1.4 Hz, 1 H, H-6'), 8.17 (ddd, *J* = 8.3, 2.5, 1.5 Hz, 1 H, H-4'), 8.06 (t, J = 6.2 Hz, 1 H, NHSO₂), 7.68-7.75 (m, 4 H, H-2, H-6, H-2", H-6"), 7.57 (ddd, *J* = 8.6, 2.2, 1.9 Hz, 2 H, H-3", H-5"), 7.33-7.40 (m, 2 H, H-5, H-5'), 4.04 (d, *J* = 6.2 Hz, 2 H, CH₂N), 2.30 (s, 3 H, CH₃), 1.30 [s, 9 H, C(CH₃)₃]; MS *m*/*z* 438.6 (MH⁺, 100%).

### Example 148

### Preparation of 4-({[(4-tert-Butylphenyl)sulfonyl]amino}methyl)-3-fluoro-N-(3-pyridinyl)benzamide (175).

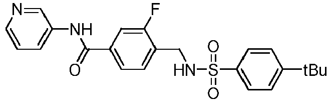

**4-Cyano-3-fluoro-N-(3-pyridinyl)benzamide (174).** Oxalyl chloride (0.79 mL, 9.08 mmol) was added dropwise to a stirred suspension of 4-cyano-3-fluorobenzoic acid (1.00 g, 6.06 mmol) and DMF (1 drop) in dry THF (40 mL) and the solution was stirred at 20 °C for 2 h, then at 66 °C for 1 h. The solution was cooled to 20 °C, then the solvent was evaporated and the residue dissolved in dry pyridine (10 mL). 3-Pyridinylamine (627 mg, 6.67 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **174** (1.19 g, 81%) as a white powder: mp (EtOAc) 189-191 °C; ¹H NMR δ 10.71 (s, 1 H, CONH), 8.92 (d, *J* = 2.4 Hz, 1 H, H-2'), 8.36 (dd, *J* = 4.7, 1.5 Hz, 1 H, H-6'), 8.13-8.20 (m, 2 H, H-5, H-4'), 8.06 (dd, *J* =10.0, 1.4 Hz, 1 H, H-2), 7.97 (dd, *J* = 8.0, 1.5 Hz, 2 H, H-6), 7.43 (ddd, *J* = 8.3, 4.7, 0.7 Hz, 1 H, H-5'); MS *m*/*z* 242.3 (MH⁺, 100%). Anal. calcd for C₁₃H₈FN₃O: C, 64.73; H, 3.34; N, 17.42. Found: C, 64.51; H, 3.31; N, 17.05%.

**4**-**({[(4-*tert*-Butylphenyl)sulfonyl]amino}methyl)-3-fluoro-*N*-(3-pyridinyl)benzamide (175).** A mixture of benzamide **174** (330 mg, 1.37 mmol) and 10% Pd/C (30 mg) and cHCl (0.34 mL, 4.1 mmol) in EtOH (30 mL) was stirred under H₂ (60 psi) at 20 °C for 16 h. The mixture was filtered through Celite, the Celite was washed with EtOH (20 mL), and the solvent was evaporated. The residue was partitioned between dilute aqueous NH₃ solution (30 mL) and CHCl₃ (3 × 30 mL) and the organic fraction dried and the solvent evaporated to give crude amine which was used directly. A mixture of amine (106 mg, 0.43 mmol) and 4-*tert-*butylbenzenesulfonyl chloride (111 mg, 0.48 mmol) in dry pyridine (10 mL) was stirred at 20 °C for 16 h. The solvent was evaporated and the residue stirred in water (40 mL) for 1 h. The precipitate was filtered, washed with water (5 mL) and dried. The crude solid was purified by column chromatography, eluting with EtOAc, to give benzamide **175** (45 mg, 7%) as a white powder: mp (EtOAc) 198-200 °C; ¹H NMR δ 10.40 (s, 1 H, CONH), 8.90 (d, *J =* 2.3 Hz, 1 H, H-2'), 8.33 (dd, *J* = 4.7, 1.2 Hz, 1 H, H-6'), 8.23 (t, *J =* 5.6 Hz, 1 H, NHSO₂), 8.15 (ddd, *J* = 8.4, 2.5, 1.5 Hz, 1 H, H-4'), 7.71 (br d, *J* = 8.0 Hz, 1 H, H-6), 7.63-7.68 (m, 3 H, H-5, H-2", H-6"), 7.52 (br d, *J* = 8.5 Hz, 2 H, H-3", H-5"), 7.46 (br t, *J* = 7.8 Hz, 1 H, H-2), 7.40 (br dd, *J* = 8.3, 4.7 Hz, 1 H, H-5'), 4.12 (d, *J* = 5.6 Hz, 2 H, CH₂N), 1.27 [s, 9 H, C(CH₃)₃]; MS *m*/*z* 442.6 (MH⁺, 100%).

### Example 149

### Preparation of 4-(1-{[(4-tert-Butylphenyl)sulfonyl]amino}ethyl)-N-(3-pyridinyl)benzamide (177).

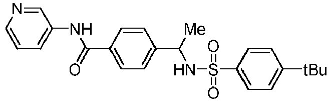

**4-Acetyl-*N*-(3-pyridinyl)benzamide (176).** Oxalyl chloride (0.67 mL, 7.68 mmol) was added dropwise to a stirred suspension of 4-acetylbenzoic acid (0.97 g, 5.12 mmol) and DMF (1 drop) in dry THF (40 mL) and the solution was stirred at 20 °C for 2 h, then at 66 °C for 1 h. The solution was cooled to 20 °C, then the solvent was evaporated and the residue dissolved in dry pyridine (10 mL). 3-Pyridinylamine (505 mg, 5.38 mmol) was added and the solution stirred at 20 °C for 16 h. The solvent was evaporated and the residue suspended in ice/water (50 mL) for 1 h. The precipitate was filtered, washed with water (5 mL) and dried. The crude solid was purified by column chromatography, eluting with EtOAc, to give benzamide **176** (1.05 g, 85%) as a white powder: mp (EtOAc) 169-171 °C; ¹H NMR δ 10.59 (s, 1 H, CONH), 8.94 (d, *J* = 2.2 Hz, 1 H, H-2'), 8.34 (dd, *J* = 4.7, 1.5 Hz, 1 H, H-6'), 8.20 (ddd, *J* = 8.3, 2.5, 1.5 Hz, 1 H, H-4'), 8.07-8.13 (m, 4 H, H-2, H-3, H-5, H-6), 7.42 (ddd, *J* = 8.3, 4.7, 0.6 Hz, 1 H, H-5'), 2.26 (s, 3 H, COCH₃); MS *m*/*z* 241.3 (MH⁺, 100%). Anal. calcd for C₁₄H₁₂N₂O₂: C, 69.99; H, 5.03; N, 11.66. Found: C, 69.69; H, 5.05; N, 11.72%.

**4-(1-{[(4-*tert*-Butylphenyl)sulfonyl]amino}ethyl)-*N*-(3-pyridinyl)benzamide (177).** NaCNBH₃ (40 mg, 0.63 mmol) was added to a stirred solution of benzamide **176** (217 mg, 0.90 mmol) and NH₄OAc (0.70 g, 9.0 mmol) in dry MeOH (10 mL) and the mixture was stirred at 20 °C for 16 h. The solvent was evaporated and the residue was partitioned between dilute aqueous NH₃ solution (30 mL) and CHCl₃ (3 × 30 mL). The combined organic fraction was dried and the solvent evaporated to give crude 4-(1-aminoethyl)-*N*-(3-pyridinyl)benzamide (210 mg, 98%) as a white foam: ¹H NMR δ 10.34 (s, 1 H, CONH), 8.93 (d, *J* = 2.2 Hz, 1 H, H-2'), 8.30 (dd, *J* = 4.7, 1.5 Hz, 1 H, H-6'), 8.19 (ddd, *J* = 8.3, 2.5, 1.5 Hz, 1 H, H-4'), 7.92 (br d, *J* = 8.3 Hz, 2 H, H-2, H-6), 7.53 (br d, *J* =8.3 Hz, 2 H, H-3, H-5), 7.39 (ddd, *J* = 8.3, 4.7, 0.5 Hz, 1 H, H-5'), 4.07 (q, *J* = 6.6 Hz, 2 H, CHN), 1.27 (s, 3 H, CH₃). A mixture of amine (210 mg, 0.87 mmol) and 4-*tert*-butylbenzenesulfonyl chloride (213 mg, 0.91 mmol) in dry pyridine (10 mL) was stirred at 20 °C for 16 h. The solvent was evaporated and the residue stirred in water (40 mL) for 1 h. The precipitate was filtered, washed with water (5 mL) and dried. The crude solid was purified by column chromatography, eluting with EtOAc, to give benzamide **177** (163 mg, 43 %) as a white powder: ¹H NMR δ 10.34 (s, 1 H, CONH), 8.90 (d, *J* = 2.4 Hz, 1 H, H-2'), 8.31 (dd, *J* = 4.7, 1.4 Hz, 1 H, H-6'), 8.21 (d, *J =* 8.4 Hz, 1 H, NHSO₂), 8.15 (ddd, *J* = 8.3, 2.5, 1.5 Hz, 1 H, H-4'), 7.74 (br d, *J* = 8.3 Hz, 2 H, H-2, H-6), 7.51 (ddd, *J* = 8.5, 2.1, 1.8 Hz, 2 H, H-2", H-6"), 7.36-7.41 (m, 3 H, H-5', H-3", H-5"), 7.28 (br d, *J =* 8.3 Hz, 2 H, H-3, H-5), 4.39-4.49 (m, 2 H, CH₂N), 1.28 (d, *J* = 7.0 Hz, 3 H, CH₃), 1.27 [s, 9 H, C(CH₃)₃]; ³C NMR δ 165.3, 155.8, 146.9, 144.4, 141.9, 138.2, 135.7, 132.4, 127.3 (2), 127.2, 126.1 (2), 126.0 (2), 125.4 (2), 123.4, 52.6, 34.5, 30.6 (3), 23.4; MS *m*/*z* 438.6 (MH⁺, 100%).

### Example 150

Using procedures similar to those described herein, the following compounds may be prepared and tested:
4-((4-(tert-butyl)phenylsulfonamido)methyl)-3-nitro-N-(pyridin-3-yl)benzamide;
(S)-4-(1-(4-(tert-butyl)phenylsulfonamido)ethyl)-N-(pyridin-3-yl)benzamide and (R)-4-(1-(4-(tert-butyl)phenylsulfonamido)ethyl)-N-(pyridin-3-yl)benzamide and mixtures thereof;
4-(N-phenylsulfamoylmethyl)-N-(pyridin-3-yl)benzamide; 4-((N-(4-fluorophenyl)sulfamoyl)methyl)-N-(pyridin-3-yl)benzamide; 4-((N-(4-tert-butylphenyl)sulfamoyl)methyl)-N-(pyridin-3-yl)benzamide; 4-((N-(4-(4-methylpiperazin-1-yl)phenyl)sulfamoyl)methyl)-N-(pyridin-3-yl)benzamide.)

### Example 151

### Biological Methods

**Cell culture.** RCC4 parental and RCC4 with VHL-reintroduced (RCC4/VHL), SN12C and SN12C-CSCG-VHL shRNA were maintained in DMEM supplemented with 10% FCS.

**IC₅₀ Assays.** IC₅₀ values for compounds were determined by XTT assay. For 2,3-bis[2-methoxy-4-nitro-5-sulfophenyl]-2H-tetrazolium-5-carboxanilide inner salt (XTT) assays, five thousand cells were plated in 96-well plates. The next day, vehicle or drug was added to each well and incubated for four days. Media was aspirated and phenol red-free medium with 0.3 mg/mL XTT and 2.65 ug/mL N-methyl dibenzopyrazine methyl sulfate was added. This was incubated at 37 °C for 1-2 hours and absorbance was read at 450 nm. IC₅₀ values for each compound were calculated using linear interpolation.

**Clonogenic assay.** Three hundred cells were plated into 60-mm tissue culture dishes in DMEM. The next day, cells were treated with vehicle or drug and were further incubated for an additional 10 days. After 10 days, the media was removed and colonies were fixed and stained in 95% ethanol and 0.1% crystal violet for 15 minutes. The stain was removed and plates were washed in deionized water. Colonies were quantified. All conditions were measured in triplicate and all experiments were performed in triplicate.

**Glucose uptake.** One hundred thousand cells were plated into 6-well plates. The following day, the cells were treated with vehicle or drug and incubated for the indicated time. Cells were washed twice in phosphate buffered saline and low glucose media was added for 30 minutes. Cells were then incubated with 0.5 microCi of tritiated-2-deoxyglucose and incubated for an hour at 37 °C. Cells were washed twice in PBS and then lysed in 0.2 N NaOH and 0.2% SDS. Lysates were transferred to scintillation tubes with scintillation fluid and quantified by scintillation counter.

**In vivo experiments.** Five million cells were injected into the flanks of nu/nu mice (4-6 weeks old males) and allowed to grow to approximately 50 mm³. The mice were injected daily by intra-peritoneal to deliver either vehicle or drug. Tumors were measured every other day and tumor volume was calculated as 0.5 length by width squared.

IC50values and selectivity ratios for certain exemplary compounds are described below. The designation A reflects an IC50 of <1 µM; B reflects an IC50 ranging from 1 to 20 µM; and C is an IC50 of > 20 µM. The designation "a" reflects a ratio of RCC4/RCC-VHL+ ranging from 1 to 10; "b" reflects from 10 to 100; "c" reflects a ratio of > 100, and "nd" represents "not determined".

**Table 1**

| **Example** | **IC₅₀ RCC4 µM** | **Ratio (RCC4:RCC/VHL)** |
|---|---|---|
| Example 1 | C | nd |
| Example 2 | B | a |
| Example 3 | C | nd |
| Example 4 | C | nd |
| Example 5 | C | nd |
| Example 6 | C | nd |
| Example 7 | C | nd |
| Example 8 | C | nd |
| Example 9 | C | nd |
| Example 10 | B | a |
| Example 11 | B | a |
| Example 12 | B | a |
| Example 13 | B | a |
| Example 14 | C | nd |
| Example 15 | C | nd |
| Example 16 | C | a |
| Example 17 | B | b |
| Example 18 | B | b |
| Example 19 | A | b |
| Example 20 | A | b |
| Example 21 | A | b |
| Example 22 | A | b |
| Example 23 | A | b |
| Example 24 | B | a |
| Example 25 | B | b |
| Example 26 | B | b |
| Example 27 | A | b |
| Example 28 | A | c |
| Example 29 | B | b |
| Example 30 | A | c |
| Example 31 | B | b |
| Example 32 | B | b |
| Example 33 | B | b |
| Example 34 | A | b |
| Example 35 | B | b |
| Example 36 | B | b |
| Example 37 | A | b |
| Example 38 | A | c |
| Example 39 | nd | nd |
| Example 40 | nd | nd |
| Example 41 | C | nd |
| Example 42 | B | a |
| Example 43 | B | a |
| Example 44 | B | b |
| Example 45 | B | b |
| Example 46 | B | b |
| Example 47 | C | nd |
| Example 48 | A | b |
| Example 49 | B | a |
| Example 50 | B | a |
| Example 51 | C | nd |
| Example 52 | B | b |
| Example 53 | B | a |
| Example 54 | B | b |
| Example 55 | A | b |
| Example 56 | B | b |
| Example 57 | A | b |
| Example 58 | B | a |
| Example 59 | B | b |
| Example 60 | A | b |
| Example 61 | A | C |
| Example 62 | A | C |
| Example 63 | A | b |
| Example 64 | B | a |
| Example 65 | A | C |
| Example 66 | A | C |
| Example 67 | A | C |
| Example 68 | C | nd |
| Example 69 | A | b |
| Example 70 | B | b |
| Example 71 | nd | nd |
| Example 72 | B | b |
| Example 73 | A | c |
| Example 74 | A | c |
| Example 75 | A | c |
| Example 76 | A | c |
| Example 77 | A | b |
| Example 78 | A | c |
| Example 79 | A | b |
| Example 80 | A | c |
| Example 81 | A | b |
| Example 82 | A | c |
| Example 83 | A | c |
| Example 84 | A | c |
| Example 85 | A | c |
| Example 86 | C | nd |
| Example 87 | C | nd |
| Example 88 | C | nd |
| Example 89 | B | a |
| Example 90 | B | a |
| Example 91 | C | nd |
| Example 92 | C | nd |
| Example 93 | C | nd |
| Example 94 | B | a |
| Example 95 | B | b |
| Example 96 | C | ND |
| Example 97 | C | ND |
| Example 98 | B | a |
| Example 99 | C | ND |
| Example 100 | B | a |
| Example 101 | B | ND |
| Example 102 | C | a |
| Example 103 | C | nd |
| Example 104 | A | c |
| Example 105 | C | nd |
| Example 107 | C | nd |
| Example 108 | C | nd |
| Example 109 | C | nd |
| Example 110 | C | nd |
| Example 111 | C | nd |
| Example 112 | C | a |
| Example 113 | C | a |
| Example 114 | C | nd |
| Example 115 | C | nd |
| Example 116 | C | nd |
| Example 117 | C | a |
| Example 118 | C | nd |
| Example 119 | C | nd |
| Example 120 | C | nd |
| Example 121 | C | nd |
| Example 122 | B | a |
| Example 123 | B | a |
| Example 124 | C | nd |
| Example 125 | C | nd |
| Example 126 | B | b |
| Example 127 | A | b |
| Example 128 | A | c |
| Example 129 | A | c |
| Example 130 | B | a |
| Example 131 | A | b |
| Example 132 | A | b |
| Example 133 | A | c |
| Example 134 | C | nd |
| Example 135 | A | b |
| Example 136 | A | b |
| Example 137 | A | b |
| Example 138 | B | a |
| Example 139 | C | a |
| Example 140 | C | nd |
| Example 141 | A | c |
| Example 142 | A | b |
| Example 143 | nd | nd |
| Example 144 | nd | nd |
| Example 145 | nd | nd |
| Example 146 | nd | nd |
| Example 147 | nd | nd |
| Example 148 | nd | nd |
| Example 149 | nd | nd |
| Compound A | C | a |
| Compound B | B | a |
| Compound C | C | nd |
| Compound D | C | nd |
| Compound E | B | b |

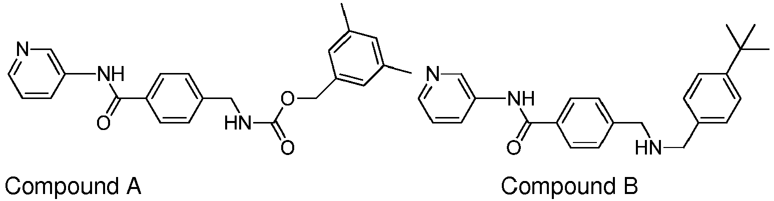

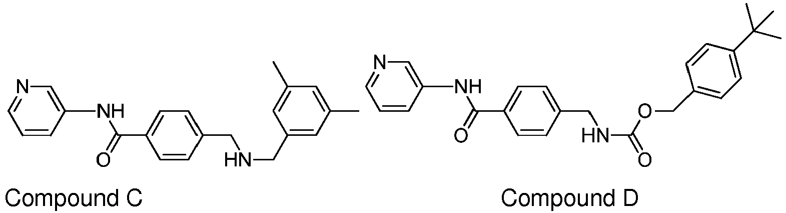

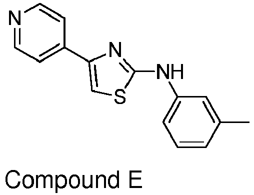

### Example 152 - Synthetic Lethal Targeting of Glucose Metabolism in Renal Carcinoma

### Methods

**Cell Culture and Reagents.** All cells were grown in DMEM +10% FCS. ACHN and ACHN shVHL were a kind gift from George V. Thomas (UCLA). HIF overexpressing clones were described previously. Transfection of RNA oligos were performed with DnarmaFECT Reagent 1 (Dharmcon), according to manufacturer's directions. ON-TARGETplus SMART pools against HIF-1β/ARNT were purchased from Dharmacon. Glut1 was detected with anti-GLUT1 antibody from NeoMarkers/LabVision/Fisher. Pyruvate/lactate levels and hexokinase activity were both measured by fluorometric assay (BioVision and Sigma-Aldrich, respectively). ATP levels were measured by bioluminescence assay (ATP Determination Kit from Molecular Probes/Invitrogen). In vitro kinase activities were performed by Millipore KinaseProfiler. Affi-Gel 10 (BioRad) activated affinity media was coupled to analogs to generate immobilized affinity linkers.

**Cell Viability Assays.** For 2,3-bis[2-methoxy-4-nitro-5-sulfophenyl]-2H-tetrazolium-5-carboxanilide (XTT) assays, five thousand cells were plated in 96-well plates. The next day, vehicle (DMSO) or drug was added by serial dilution. Four days later, media were aspirated, XTT solution (0.3 mg/ml of XTT (Sigma), 2.65 mg/ml N-methyl dibenxopyrazine methyl sulfate (Simga) in phenol red-free media) was added, and the plates were incubated at 37°C for 1-2 hours. Metabolism of XTT was quantified by measuring the absorbance at 450 nm. IC₅₀s were calculated using linear interpolation. For clonogenic survival assays, three hundred cells were plated per 60 mm tissue culture dish. The cells were allowed to attach overnight and then treated with vehicle or drug for 14 days. Colonies were fixed and stained with crystal violet (0.1% crystal violet in 95% ethanol). All conditions were measured in triplicate and each experiment was done in duplicate or triplicate. To determine necrosis, cells were treated with drug for a given time point. Media and cells were collected, centrifugated, and resuspended in 0.4% trypan blue (Invitrogen). Live and dead cells were counted on a hematocytometer.

**Glucose Uptake.** One hundred thousand cells were plated per well in a six-well plate. The next day, cells were treated with the indicated concentration of drug and incubated for the indicated time. Cells were then washed twice with phosphate-buffered saline, incubated in low-glucose medium for 30 minutes, and ³H-2-deoxyglucose (0.5 µCi) was added in 1 ml of glucose-free media for an additional hour. Cells were washed twice in PBS and lysed (0.2 N NaOH and 0.2% sodium dodecyl sulfate). Glucose uptake was quantified with a scintillation counter.

**Oxygen Consumption.** Following treatment with vehicle or drug, cells were trypsinized, suspended at 5 million cells per ml in DMEM +10% FCS, and oxygen consumption was measured in 0.5 ml volume using an Oxytherm electrode unit (Hansatech).

**Quantitative Real-time RT-PCR.** Total RNA was extracted from cells (TRIzol, Invitrogen) as per manufacturer's directions. Total RNA (1.5 µg) was reversed transcribed with random hexamers and MMLV-RT. Power SYBR Green PCR reactions were performed in triplicate for each sample and analyzed using the ABI Prism 7900HT sequence detection system. Data were normalized to TBP levels.

**In Vivo Studies and Immunohistochemistry.** All experiments were approved by Stanford's Administrative Panel on Laboratory Animal Care (APLAC) and in accordance with both institutional and national guidelines. Five million cells were implanted subcutaneously into the flanks of nude mice (4-6 weeks old)(Charles River Laboratories). Tumors were measured with calipers. Volume was calculated by the following formula: width² x 0.5 length. Once tumors reached an average size of >20 mm³, mice were randomized into vehicle (DMSO diluted in 16% cremaphor EL/PBS) or treated groups. Mice were treated with compound **85** (11.6 mg/kg for the first 3 days, followed by 7.8 mg/kg for the 7-9 days). Five-micron sections were cut for immunohistochemistry. Sections were counterstained with hematoxylin and eosin. For 2-[¹⁸F]-fluoro-2-deoxy-glucose-positron emission tomography imaging, mice bearing tumors were fasted overnight. The next day, the mice were anesthetized with 2% isoflurane and injected intraperitoneally with 250 µCi of FDG. Mice were imaged for 10 minutes at one hour post-injection, using a Rodent R4 microPET system (Concorde Microsystems). Data were reconstructed into three-dmensional volumes using an ordered subset expectation maximization algorithm and were calibrated into units of percent injected dose per gram.

**Statistical Analyses.** Student's t test was used to determine significance. All error bars represent the standard error of the mean.

**Primers**
Glut1/SLC2A1:
   Forward: 5'-GGCCAAGAGTGTGCTAAAGAA-3'
   Reverse: 5'-ACAGCGTTGATGCCAGACAG-3'
Glut2/SLC2A2 :
   Forward: 5'-GTCACTGGGACCCTGGTTTTC-3'
   Reverse: 5'-AGTTGTTGATAGCTTTTCGGTCA-3'
HK1:
   Forward: 5'-TGGCCTATTACTTCACGGAGC-3'
   Reverse: 5'-GGAATGGACCTTACGAATGTTGG-3'
H K2:
   Forward: 5'-TTTGACCACATTGCCGAATGC-3'
   Reverse: 5'-GGTCCATGAGACCAGGAAACT-3'
PAI-1/Serpine1:
   Forward: 5'-CATCCCCCATCCTACGTGG-3'
   Reverse: 5'-CCCCATAGGGTGAGAAAACCA-3'
PDK:
   Forward: 5'-CTGTGATACGGATCAGAAACCG-3'
   Reverse: 5'-TCCACCAAACAATAAAGAGTGCT-3'
PGK:
   Forward: 5'-CCTGGGCGGAGCTAAAGTTG-3'
   Reverse: 5'-TCTCAGCTTTGGACATTAGGTCT-3'
VEGF:
   Forward: 5'-CAACATCACCATGCAGATTATGC-3'
   Reverse: 5'-CCCACAGGGATTTTCTTGTCTT-3'

### Results

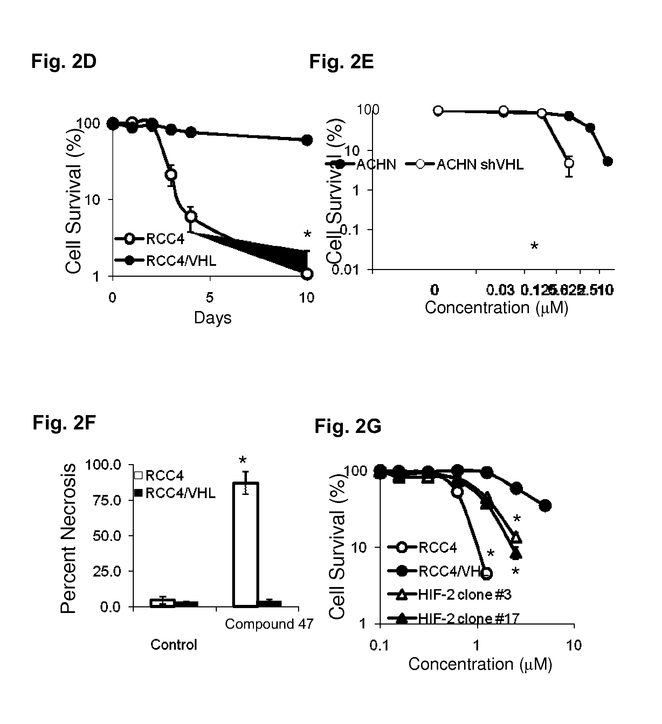

In order to discover classes of drugs that would selectively target RCC, we screened approximately 64,000 compounds to identify small molecules that function in a synthetic lethal manner to the loss of *VHL.* We employed multiple RCC cell lines with naturally occurring *VHL* mutations and, as a negative control, their genetically matched counterparts with reintroduced wild-type *VHL.* These matched cell lines, engineered to stably express enhanced yellow fluorescent protein, were treated with a small molecule library at a concentration of 10-20 µM for four days. Fluorescence was measured on day four as a surrogate marker for viability and growth. From this fluorescent-based cell assay, two classes of drugs exhibited toxicity to cells that had lost *VHL,* but were relatively non-toxic to cells with functional VHL. Here we characterize the selective cytotoxicity of a second class, which includes compound **27** and compound **47** (i.e., 4-((4-(tert-butyl)phenylsulfonamido)methyl)-N-(pyridin-3-yl)benzamide), members of a family of 4-(*p*henylsulfonamido)-*N*-(*p*yridin-3-yl)benzamides (PPBs). Both short-term metabolic assays and long-term survival assays were used to validate the primary screen (Fig. 2A and 2B). Metabolic activity was measured by 2,3-bis[2-methoxy-4-nitro-5-sulfophenyl]-2*H*-tetrazolim-5-carboxanilide (XTT) after four days of treatment with compound **27** and compound **47.** We observed a significant decrease in the number of RCC4 cells that had lost *VHL* compared to their wild-type counterparts (RCC4/VHL) in a concentration-dependent manner (Fig. 2A). Clonogenic survival confirmed that these PPBs were specifically toxic to RCC4 cells while the RCC4/VHL cells were relatively unaffected (Fig. 1B, 1C, and 7A). Approximately 80% of RCC4 cells treated with compound **47** were killed following treatment whereas RCC4 cells treated under the same conditions were largely able to recover (Fig. 2D). To corroborate the *VHL-*dependence of PPB resistance, we examined a cell line, ACHN, which normally maintains functional VHL. We found that only the ACHN renal carcinoma cells where VHL expression was silenced by shRNA were sensitive to compound **47** (Fig. 2E). Thus, our chemical synthetic screening using a fluorescent, cell-based assay has identified compounds that are specifically cytotoxic to cells that have impaired VHL function.

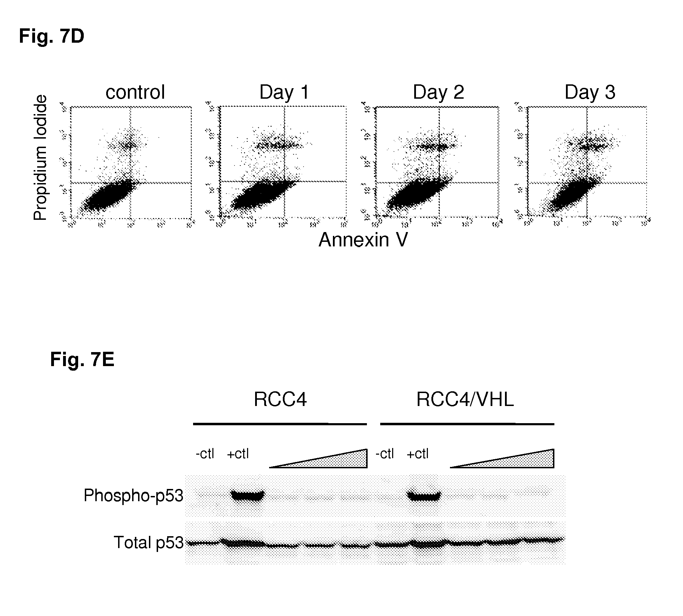

Having previously demonstrated a selective sensitivity of *VHL*-deficient cells to autophagic cell death, we next sought to determine whether compound **47** acts by the same mechanism or whether this small molecule targets a different pathway. Treatment with compound **47** did not induce any morphologic or biochemical features of autophagy, such as intracellular accumulation of vacuoles (data not shown) or LC3 processing (Fig. 7B). Incubation of *VHL*-deficient and isogenic matched wild-type *VHL* RCCs with compound **47** showed no nuclear condensation in either cell line (Fig. 7C), nor an increase in either propidium iodide or annexin V staining (Fig. 7D), suggesting that compound **47** is not killing these cells by apoptosis. Compound **47** did not increase total p53 or phospho-p53 levels, also indicating that compound **47** does not induce a DNA damage response in treated cells (Fig. 7E). However, RCC cells without VHL undergo a necrotic cell death in response to compound **47** as measured by the ability of the cells to exclude trypan blue, an indicator of cell membrane integrity. Treatment with compound **47** resulted in greater than 80% of RCC4 cells exhibiting necrotic cell death, while RCC4/VHL cells were relatively insensitive (Fig. 2F). Taken together, these results indicate that compound **47** is synthetic lethal to the loss of *VHL* by causing a necrotic cell death. These results also demonstrate that compound **47** acts in a manner distinct from the autophagic cell death pathway we previously described for 4-(pyridin-4-yl)-N-(m-tolyl)thiazol-2-amine.

As the hypoxia-inducible factor family of transcription factors are the best-characterized VHL targets, we next examined whether toxicity was HIF-dependent. A non-degradable, constitutively active HIF was overexpressed in RCC4/VHL cells. Two individual HIF-overexpressing clones were tested for their sensitivity to compound **47.** Ectopic expression of HIF in cells with wild-type VHL sensitized these cells to compound **47** treatment, suggesting that deregulated HIF expression in *VHL-*deficient cells is responsible for their selective cytotoxicty to compound **47** (Fig. 2G). These data suggest that compound **47** represents a new class of drugs that function in a synthetic lethal manner to *VHL* mutation, preferentially targeting *VHL*-deficient cells. Furthermore, the sensitivity of RCCs that lack functional VHL to compound **47** is directly linked to the aberrant upregulation of HIF.

As the central mediator of oxygen homeostasis, HIF plays an important role in the cellular adaptation to low oxygen conditions through the regulation of genes involved in metabolism and energy production. Inactivation of *VHL* results in an increase in the half-life of HIF protein. In turn, HIF directs the transcription of many genes, including those involved in glucose metabolism (Fig. 8A). We hypothesized that compound **47** might have an effect on metabolic pathways, which if inhibited, would lead to necrotic cell death. This possibility, along with the increased expression of glucose transporters in *VHL*-deficient RCCs, directed us to investigate how compound **47** affects glucose metabolism. To examine whether this compound alters the rate of glycolysis in *VHL*-deficient cells, we measured the intracellular production of lactate, which is rapidly converted from pyruvate, the end-product of glycolysis. Treatment with compound **47** significantly inhibited lactate production in *VHL*-deficient cells by approximately 60% compared to control-treated cells (Fig. 3A). Baseline levels of lactate production were lower in wild-type VHL cells compared to *VHL*-deficient cells, likely due to the constitutive expression of HIF and subsequent overexpression of glucose transporters and glyolytic enzymes. However, treatment with compound **47** did not affect glycolysis in cells with wild-type VHL cells.

We then examined whether this decrease in glycolysis in response to compound **47** was due to a decrease in glucose uptake or whether compound **47** inhibited a particular glycolytic enzyme. To test this, we measured glucose uptake using 2-deoxy-D-[³H] glucose, a non-hydrolyzable, radioactive glucose analog, following two days of treatment with compound **47.** Compound **47** impaired glucose uptake in RCC4 and 786-O cells but not in the matched isogenic cells expressing wild-type VHL (Fig. 3B and 8B). RCC4/VHL cells had lower baseline levels of glucose uptake compared to RCC4 cells and were unaffected by treatment with compound **47.** Furthermore, compound **47** inhibited glucose uptake in RCC4 cells in a dose-dependent manner, but glucose levels in RCC4/VHL cells were relatively stable with increasing concentrations of compound **47** (Fig. 3C). Because the phosphorylation of glucose to glucose-6-phosphate is important for preventing glucose efflux from the cell, we asked whether compound **47** might function by inhibiting the phosphorylation of glucose by hexokinase. Hexokinase activity was inhibited by compound **47** only after three days of treatment in VHL-deficient RCC4 cells but hexokinase activity of RCC4/VHL cells with wild-type VHL was unchanged by compound **47** (Fig. 3D). Again, the baseline activity of hexokinase is higher in RCC4 cells, consistent with *VHL*-deficient RCCs having higher rates of glycolysis, and that the hexokinase gene is a HIF target (Fig. 3A). The decrease in hexokinase activity occurred subsequent to changes in glucose uptake, indicating that inhibition of hexokinase is not directly responsible for the differential cytotoxicity of compound **47** in cells with and without VHL. Furthermore, inhibitors of hexokinase did not result in selective cytotoxicity to *VHL*-deficient cells (data not shown). These data indicate that compound **47** decreases glycolysis by decreasing glucose transport and not by inhibiting a particular glycolytic step or enzyme per se.

To further investigate the relationship between HIF and compound **47** toxicity, we silenced HIF-1β in RCC4 cells and assessed its affect on glucose uptake. Transiently inhibiting HIF-1β, the constitutively expressed binding partner of HIF-1α and HIF-2α reduces HIF activity in RCC4 cells to the levels found in wild-type VHL cells. Glucose uptake was insensitive to treatment with compound **47** when the HIF-1β was silenced in RCC4 cells, further supporting the concept that the HIF-dependent glucose uptake was responsible for the differential toxicity of compound **47** to *VHL*-deficient renal carcinomas (Fig. 3E).

We next investigated how a decrease in glycolysis could lead to selective necrotic cell death. One possibility is that the reduction in glycolysis lowers the availability of pyruvate, the essential precursor for the generation of acetyl-CoA. As previous studies have indicated that RCCs have decreased oxygen consumption because of constitutive HIF expression and the subsequent induction of genes, such as *PDK1* and *MXI1,* that inhibit the conversion of pyruvate to acetyl-CoA, we hypothesized that compound **47** may be inhibiting oxidative phosphorylation and the use of pyruvate. We therefore examined oxygen consumption as a marker of oxidative phosphorylation and ATP production in treated and untreated cells. While there was a difference in oxygen consumption between *VHL*-deficient and wild-type *VHL* cells, there was no difference in oxygen consumption between cells treated with compound **47** and those that were not treated (Fig. 3F). This finding demonstrates that the mitochondria and the oxidative pathway remain unaffected by compound **47.** However, the decrease in glucose uptake in response to treatment with compound **47** in *VHL*-deficient cells results in a 75% decrease in ATP levels (Fig, 3G). Furthermore, inhibition of ATP production in response to compound **47** treatment is dose-dependent (Fig. 3H). Taken together, these data suggest that loss of *VHL* is associated with reduced oxidative phosphorylation and greater dependence on glycolysis for ATP production. By disrupting glycolysis, compound **47** functions in a synthetic lethal manner to *VHL* mutation, ultimately killing *VHL*-deficient cells by inhibiting their primary mechanism of energy production.

These data support an emerging model that renal cells with defective *VHL,* like a range of other cancers, are highly dependent on aerobic glycolysis for energy production. We further examined this conditional genetic interaction of glucose dependency and VHL interaction by depriving the cells of glucose in a growth curve assay. RCC4 cells and 786-O cells lacking functional VHL were sensitive to changes in glucose levels, while the isogenically matched cells with wild-type *VHL* continued to grow despite the absence of glucose (Fig. 8C and Fig. 8D). Conversely, when cells were deprived of pyruvate, cells with and without VHL were relatively unaffected. These results suggest that *VHL*-deficient cells are more sensitive than cells with VHL to changes in glucose. The addition of pyruvate was unable to overcome deprivation of glucose and the inhibition of glycolysis because of the increased expression of *PDK* and *MXI1* that inhibit the conversion of pyruvate to acetyl-CoA. Together, these data demonstrate that *VHL*-deficient cells are unable to utilize oxidative phosphorylation to overcome their dependence on glycolysis for energy production.

We next wanted to investigate the differential glucose uptake between RCCs with and without *VHL* treated with compound **47** that subsequently lead to the selective death of *VHL*-deficient cells. We first examined the message levels of the two main glucose transporters, GLUT1 and GLUT2 by quantitative real-time PCR. GLUT1 is an inducible, high-affinity glucose transporter, while GLUT2 is the glucose transporter responsible for basal glucose uptake. Other family members, such as GLUT3 and GLUT4, are not expressed in renal cells. GLUT1 was highly expressed in cells lacking *VHL,* while cells with *VHL* had very low levels of GLUT1 (Fig. 3I). In contrast, GLUT2 was highly expressed in cells with wild-type VHL. Cells deficient in *VHL* had very low levels of GLUT2 that could barely be detected (Fig. 3J). The expression of the two different glucose transporters suggests that compound **47** kills cells with mutant *VHL* by inhibiting the higher affinity glucose transporter, depriving *VHL*-deficient cells of glucose and consequently, energy needed to sustain the cells. In order to more directly test this, we performed binding assays to see whether compound **47** was directly interacting with GLUT1. An analog of compound **47,** compound **116** was synthesized and linked to an immobilized linker (Affi-gel 10). Cell lysates from both RCC4 and RCC4/VHL were incubated with the Affi-gel-compound **47.** Following washing of the resin-bound compound **47,** this affinity column was eluted with several fractions of increasing salt concentration with a final elution of urea. These elution fractions were then subjected to immunoblotting for GLUT1. GLUT1 bound to compound **116,** an analog of compound **47,** in RCC4 cells but not RCC4/VHL cells (Fig. 3K). Importantly, GLUT1 did not bind a similarly prepared resin linked to 4-{2-[1-(6-aminohexyl)-1H-1,2,3-triazol-4-yl]-4-pyridinyl}-N-(3-methylphenyl)-1,3-thiazol-2-amine, an analog of 4-(pyridin-4-yl)-N-(m-tolyl)thiazol-2-amine, the compound implicated in autophagic cell death, in either RCC4 or RCC4/VHL cells, indicating the specificity of the interaction between GLUT1 and compound **47.** Thus, binding of compound **47** to the high affinity glucose transporter, GLUT1, prevents glucose uptake in *VHL*-deficient cells leading to an inhibition of glycolysis and ATP production. This impairment of GLUT1 activity results in necrotic cell death in cells that lack VHL. We also investigated whether the small molecule compound **47** functioned as a kinase inhibitor. In vitro testing of a broad range of 50 different kinases demonstrated no significant decrease in any of the kinases examined (Table 2).

**Table 2**

| | | | |
|---|---|---|---|
| Abl | 108 | IRAK1 | 105 |
| AMPK | 92 | JAK2 | 113 |
| ASK1 | 129 | JNK1α1 | 101 |
| Aurora-A | 108 | MAPKAP-K2 | 87 |
| Axl | 87 | MEK1 | 99 |
| CaMKI | 87 | Met | 105 |
| CDK1/cyclinB | 96 | MKK4 | 128 |
| CDK6/cyclinD3 | 105 | MLK1 | 97 |
| CHK1 | 114 | MSK1 | 107 |
| CK1γ1 | 85 | mTOR | 101 |
| cKit(D816H) | 99 | NEK2 | 103 |
| CSK | 99 | PAK2 | 97 |
| c-RAF | 103 | PDK1 | 105 |
| cSRC | 104 | PI3K | 97 |
| DAPK1 | 92 | Pim-2 | 112 |
| DYRK2 | 94 | PKA | 96 |
| EphA1 | 102 | PKBα | 99 |
| FGFR1 | 107 | PKCδ | 116 |
| Flt3 | 111 | Plk3 | 104 |
| Fyn | 91 | ROCK-1 | 74 |
| GSK3α | 134 | Rsk1 | 120 |
| Hck | 84 | SAPK2a | 127 |
| IGF-1R | 108 | Syk | 94 |
| IKKα | 102 | Tie2 | 111 |
| IR | 121 | TrkA | 124 |

To determine a pharmacological structure-activity relationship (SAR), analogs of the selective cytotoxin compound **47** were synthesized and tested in a 4-day viability assay using paired RCC lines with and without VHL (See Table 1). All analogs of compound **47** that selectively killed *VHL*-deficient RCCs inhibited glucose uptake, whereas all inactive analogs that did not kill *VHL*-deficient cells did not inhibit glucose uptake (Fig. 4). To determine whether this assay reflected a specific inhibition of glucose uptake rather than broad toxicity, we also investigated cytotoxins that are known to act by a different mechanism. The compounds (e.g. 4-(pyridin-4-yl)-N-(m-tolyl)thiazol-2-amine), which induced VHL-dependent, HIF-independent autophagic cell death, did not decrease glucose uptake in this assay, indicating that compound **47** cytotoxicity is dependent on glucose metabolism (Table 1 and Fig. 4). These data suggest that compound **47** is specifically cytotoxic to cells that have elevated HIF levels due to their increased rate and dependence on glucose uptake and glycolysis.

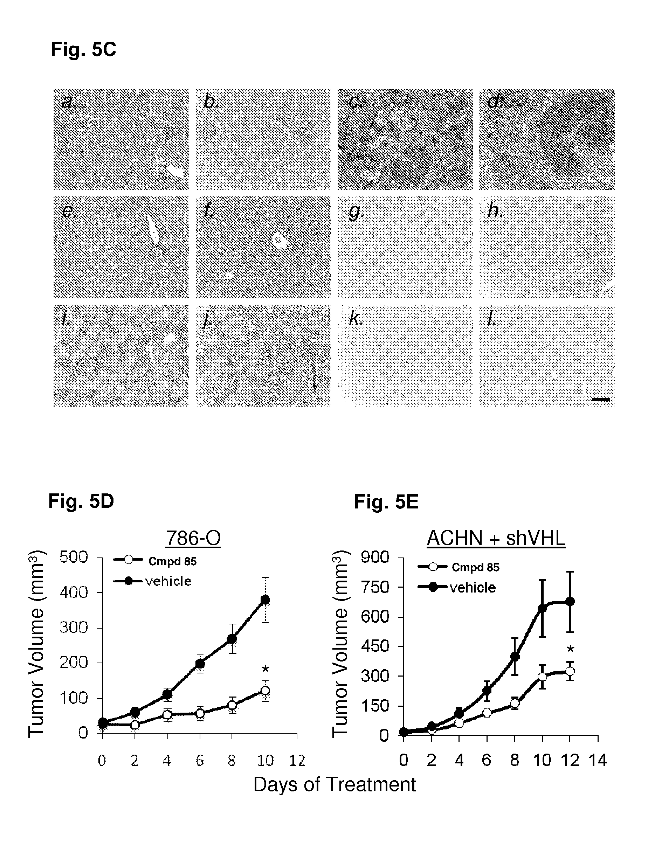

The high utilization of glucose by cancer cells compared to normal cells is the basis of fluoro-deoxyglucose positron emission tomography (FDG-PET) in the diagnosis of cancer. We hypothesized that if compound **47** was functioning by inhibiting glucose uptake, we could monitor the effects of compound **47** by FDG-PET. Pre-treatment scans of animals inoculated with subcutaneous *VHL*-deficient human renal cell carcinomas revealed a high glucose uptake within the tumors (Fig. 5A). Following three daily doses of compound **85,** a more soluble analog of compound **47,** subsequent scanning revealed a striking decrease in glucose uptake within the tumors (Fig. 5A). Despite a variation in initial tumor FDG uptake, treatment with compound **85** consistently decreased FDG uptake, suggesting that the inhibition of glucose uptake by compound **85** may lead to tumor control (Fig. 5B). Importantly, animals treated with compound **85** exhibited no normal tissue toxicity (Fig. 5C). Control animals that were given either vehicle or 4-(pyridin-4-yl)-N-(m-tolyl)thiazol-2-amine did not have a decrease in glucose uptake (data not shown). Moreover, these results demonstrate that the effectiveness of compound **47** and its analog compound **85** can be directly monitored by clinically by FDG-PET.

We next tested whether the PPBs are effective at treating tumors in a xenograft model of RCC. Daily systemic treatment of mice with *VHL*-deficient xenografts with compound **85** for ten to fourteen days markedly delayed tumor growth in two renal cell carcinoma model systems: 786-O with a naturally occurring *VHL* mutation and ACHN expressing short hairpin RNA to VHL (Fig. 5D and 5E). In both of these models, treatment with compound **85** delayed tumor growth compared to tumors treated with vehicle alone. Importantly, ACHN tumors with wild-type *VHL* grew at similar rates as those treated with compound **85** or treated with vehicle control, indicating that compound **85** is differentially cytotoxic to tumors that have lost *VHL* function, a common and frequent event in renal cell carcinoma (Fig. 8A). Taken together, we have identified an agent that is selectively toxic to a particular genotype found in the vast majority of kidney cancers. Furthermore, through its mechanism of action of inhibiting glucose metabolism, we are able to follow its effectiveness with FDG-PET, a clinically utilized imaging modality.

Compound **47** represents the second class of small molecules that we have identified that selectively kill RCCs lacking functional VHL. However, compound **47** is distinct from the previous class in its mechanism of killing RCC. Ccompound **47** and other compounds of Formula I, IA, or II, or pharmaceutically acceptable salts thereof, act by disrupting glucose uptake and utilization. The selective cytotoxicity of this effect provides direct evidence to support an emerging model of dependence on glycolysis in many cancer cell types, including the majority of RCCs. In this model, the disruption of VHL or other regulators of HIF leads to active inhibition of mitochondrial activity through the HIF-mediated induction of *PDK1,* a kinase that blocks the activity of pyruvate dehydrogenase and the production of acetyl-CoA (Fig. 6). Thus, *VHL*-deficient RCCs are selectively sensitive to compound **47** because aberrant HIF stabilization results in diminished mitochondrial activity, causing these cells to become highly dependent on glucose uptake for glycolysis and ATP production. By inhibiting glucose uptake and retention, compound **47** specifically targets the Achilles' heel of RCCs. Cells with an intact VHL pathway are not strictly dependent on glycolysis for viability and therefore insensitive to compound **47** toxicity. Our findings indicate that the differential metabolism of cancer cells can be exploited for the preferential targeting of these cells by small molecules.

Our results have a number of implications for the development of new cancer therapeutics. Firstly, our method of screening for compounds that are synthetically lethal to the loss of *VHL* should be adaptable to other tumor types with distinct genotypes, such as the loss-of-function of a particular tumor suppressor gene or gain-of-function of a specific oncogene. Secondly, the selective cytotoxicity of compound **47** may not be restricted only to *VHL*-deficient tumors alone. It is likely that a number of other cancer types possess genetic or epigenetic alterations that make them highly dependent on aerobic glycolysis for energy production and therefore sensitive to PPBs. This is currently an active area of research. Similarly, cells with wild-type *VHL* could be sensitized to compound **47** by inactivating VHL. It should also be noted that targeting GLUT1 in human renal cell cancers is feasible as *Glut1* heterozygous knockout mice are viable and recapitulate the human GLUT1 deficiency syndrome, which is effectively treated by a ketogenic diet. It is important to reiterate here that we did not observe any normal tissue toxicity, including brain, in these studies. Finally, our data show that the effectiveness of compound **47** can be monitored by in vivo imaging. This property offers the potential advantages of enabling dosage optimization and more importantly, identification of which kidney cancers will respond best to compound **47** treatment in Phase I clinical trials. Being able to track the response of a particular tumor is both cost-effective and lends itself to personalized medicine, which are two of the primary objectives of future cancer therapy.

### Example 153 - A Broad Range of Cancer Cells Are Sensitive to Glut1 Inhibition

A broad range of cancer types were tested and shown to be sensitive to GLUT1 inhibition. (Fig. 9.)
## Claims

1. A compound of Formula I: 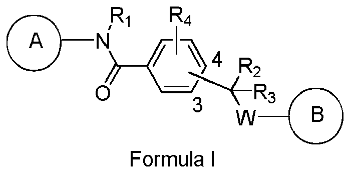 or a pharmaceutically acceptable salt thereof,
wherein:
A is a 3-pyridinyl which is optionally substituted; 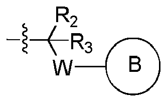 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen or C₁-C₈ alkyl;
W is -NRSO₂-, wherein R is hydrogen or C₁-C₈ alkyl; and
B is an optionally substituted aryl ring,
provided that if R₁, R₂, and R₃ are each hydrogen, and W is -NHSO₂-, then B is not 3-methoxyphenyl, 3,4-dimethylphenyl, 2,3,4-trifluorophenyl, 2,3,5,6-tetramethylphenyl, 2,5-dimethylphenyl, 3-chlorophenyl, 3-trifluoromethylphenyl, 4-methoxyphenyl, 4-tertbutylphenyl, 4-fluorophenyl, or 4-acetylphenyl;
wherein "aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic.

2. The compound according to claim 1, wherein R₂ and R₃ are each independently hydrogen or optionally substituted alkyl.

3. The compound according to claim 1, wherein R₄ is hydrogen or methyl.

4. The compound according to claim 1, wherein B is an optionally substituted phenyl ring.

5. The compound according to claim 4, wherein B is phenyl optionally substituted with one or more substituents selected from the group consisting of heterocycloalkyl, halo, alkyl, alkenyl, alkynyl, cycloalkyl, aryl, heteroaryl, hydroxyl, alkoxy, aryloxy, acyl, carboxy, alkoxycarbonyl, NO₂, optionally substituted amino, and CN, wherein each of said alkyl, alkenyl, alkynyl, alkoxy, cycloalkyl, aryl, heteroaryl, heterocycloalkyl, alkoxy, and aryloxy groups may be optionally independently substituted with one or more substituents selected from the group consisting of alkyl, halo, hydroxyl, alkoxy, carboxy, alkoxycarbonyl, heterocycloalkyl, and optionally substituted amino.

6. The compound according to claim 4, or a pharmaceutically acceptable salt thereof, wherein B is selected from the group consisting of 4-(4-methylpiperazin-1-yl)phenyl, phenyl, 2-methylphenyl, 2-fluorophenyl, 2-chlorophenyl, 2-bromophenyl, 2-methoxycarbonylphenyl, 2-trifluoromethylphenyl, 2-cyanophenyl, 3-aminophenyl, 3-methoxyphenyl, 3-methylphenyl, 3-fluorophenyl, 3-chlorophenyl, 3-bromophenyl, 3-trifluoromethylphenyl, *tert*-butylphenyl, 4-ethynylphenyl, 3-cyanophenyl, 3-nitrophenyl, 3-phenylphenyl, 3-(2-pyrimidinyl)phenyl, 3-(1-methyl-1*H*-pyrazol-3-yl)phenyl, 3-(5-methyl-1,3,4-oxadiazol-2-yl)phenyl, 3-(5-methyl-1,2,4-oxadiazol-2-yl)phenyl, 3-(2-methyl-1,3-thiazol-4-yl)phenyl, 4-aminophenyl, 4-methoxyphenyl, 4-butoxyphenyl, 4-phenoxyphenyl, 4-methylphenyl, 4-propylphenyl, 4-tert-butylphenyl, 4-(1-adamantyl)phenyl, 4-(3-chloro-1-adamantyl)phenyl, 4-methoxycarbonylethylphenyl, 4-acetamidophenyl, 4-fluorophenyl, 4-chlorophenyl, 4-bromophenyl, 4-iodophenyl, 4-trifluoromethoxyphenyl, 4-methoxycarbonylphenyl, 4-acetylphenyl, 4-trifluoromethylphenyl, 4-cyanophenyl, 4-nitrophenyl, 4'-methoxy[1,1'-biphenyl]-4-yl, 4'-methyl[1,1'-biphenyl]-4-yl, 4-phenylphenyl, 4'-fluoro[1,1'-biphenyl]-4-yl, 4'-chloro[1,1'-biphenyl]-4-yl, 4-(2-pyrimidinyl)phenyl, 4-(1*H-*pyrazol-1-yl)phenyl, 4-(2-methyl-1,3-thiazol-4-yl)phenyl, 4-(1,3-oxazol-5-yl)phenyl, 3,4-dimethoxyphenyl, 3-tert-butyl-4-methoxyphenyl, 2,3,4,5,6-pentamethylphenyl, 2,4-dimethylphenyl, 3,4-dimethylphenyl, 3,5-dimethylphenyl, 3-fluoro-4-methylphenyl, 3-chloro-2-methylphenyl, 3-chloro-4-methylphenyl, 3,4-dichlorophenyl, 3-cyano-4-fluorophenyl, 2-naphthalenyl, 5-(dimethylamino)-2-naphthalenyl, 2,3-dihydro-5-indeneyl, 2-(dimethylamino)-2,3-dihydro-5-indeneyl, 4-(dimethylamino)methylphenyl, 4-(diethylamino)methylphenyl, 4-(dipropylamino)methylphenyl, 4-(1-pyrrolidinylmethyl)phenyl, 4-(1-piperidinylmethyl)phenyl, 4-(1-azepanylmethyl)phenyl, 4-(4-morpholinylmethyl)phenyl, 4-(4-methoxy-1-piperidinyl)methylphenyl, 4-(4-methyl-1-piperazinyl)methylphenyl, 4-(3-hydroxypropyl)phenyl, 3-morpholinophenyl, 4-morpholinophenyl, (1-piperidinyl)phenyl, (4-methoxy-1-piperidinyl)phenyl, (21-amino-4,7,10,13,16,19-hexaoxahenicos-1-yl)phenyl, {[3-(4-morpholinyl)propyl]amino}phenyl, 3-(4-methyl-1-piperazinyl)phenyl, 4-{[2-(dimethylamino)ethyl]amino}phenyl, 3'-(trifluoromethyl)[1,1'-biphenyl], 4-benzylphenyl, 4-[3-(4-morpholinyl)-1-propynyl]phenyl, 4-[3-(dimethylamino)-1-propynyl]phenyl, 4-[3-(4-morpholinyl)propyl]phenyl, 4-[3-(dimethylamino)propyl]phenyl, 3-(propionylamino)phenyl, and 3-(acryloylamino)phenyl.

7. A compound selected from the group consisting of
4-((4-(4-Methylpiperazin-1-yl)phenylsulfonamido)methyl)-*N*-(pyridin-3-yl)benzamide;
4-(Phenylsulfonamidomethyl)-*N*-(pyridin-3-yl)benzamide;
4-((2-Methylphenylsulfonamido)methyl)-*N*-(pyridin-3-yl)benzamide;
4-((2-Fluorophenylsulfonamido)methyl)-*N*-(pyridin-3-yl)benzamide;
4-((2-Chlorophenylsulfonamido)methyl)-*N*-(pyridin-3-yl)benzamide;
4-((2-Bromophenylsulfonamido)methyl)-*N*-(pyridin-3-yl)benzamide;
Methyl 2-(*N*-(4-(Pyridin-3-ylcarbamoyl)benzyl)sulfamoyl)benzoate;
*N*-(Pyridin-3-yl)-4-((2-(trifluoromethyl)phenylsulfonamido)methyl)benzamide;
4-((2-Cyanophenylsulfonamido)methyl)-*N*-(pyridin-3-yl)benzamide;
4-((3-Aminophenylsulfonamido)methyl)-*N*-(pyridin-3-yl)benzamide;
4-((3-Methylphenylsulfonamido)methyl)-*N*-(pyridin-3-yl)benzamide;
4-((3-Fluorophenylsulfonamido)methyl)-*N*-(pyridin-3-yl)benzamide;
4-((3-Bromophenylsulfonamido)methyl)-*N*-(pyridin-3-yl)benzamide;
4-((3-Cyanophenylsulfonamido)methyl)-*N*-(pyridin-3-yl)benzamide;
4-((3-Nitrophenylsulfonamido)methyl)-*N*-(pyridin-3-yl)benzamide;
4-{[([1,1'-Biphenyl]-3-ylsulfonyl)amino]methyl}-*N*-(3-pyridinyl)benzamide;
4-[({[3-(2-Pyrimidinyl)phenyl]sulfonyl}amino)methyl]-*N*-(3-pyridinyl)benzamide;
4-[({[3-(1-Methyl-1*H*-pyrazol-3-yl)phenyl]sulfonyl}amino)methyl]-*N*-(3-pyridinyl)benzamide;
4-[({[3-(5-Methyl-1,3,4-oxadiazol-2-yl)phenyl]sulfonyl}amino)methyl]-*N*-(3-pyridinyl)benzamide;
4-[({[3-(5-Methyl-1,2,4-oxadiazol-3-yl)phenyl]sulfonyl}amino)methyl]-*N*-(3-pyridinyl)benzamide;
4-[({[3-(2-Methyl-1,3-thiazol-4-yl)phenyl]sulfonyl}amino)methyl]-*N*-(3-pyridinyl)benzamide;
4-((4-Aminophenylsulfonamido)methyl)-*N*-(pyridin-3-yl)benzamide;
4-((4-Butoxyphenylsulfonamido)methyl)-*N*-(pyridin-3-yl)benzamide;
4-((4-Phenoxyphenylsulfonamido)methyl)-*N*-(pyridin-3-yl)benzamide;
4-((4-Methylphenylsulfonamido)methyl)-*N*-(pyridin-3-yl)benzamide;
4-((4-Propylphenylsulfonamido)methyl)-*N*-(pyridin-3-yl)benzamide;
4-[({[4-(1-Adamantyl)phenyl]sulfonyl}amino)methyl]-*N*-(3-pyridinyl)benzamide;
4-[({[4-(3-Chloro-1-adamantyl)phenyl]sulfonyl}amino)methyl]-*N*-(3-pyridinyl)benzamide;
Methyl 3-{4-[({4-[(3 Pyridinylamino)carbonyl]benzyl}amino)sulfonyl]phenyl} propanoate;
4-((4-Acetamidophenylsulfonamido)methyl)-*N*-(pyridin-3-yl)benzamide;
4-((4-Chlorophenylsulfonamido)methyl)-*N*-(pyridin-3-yl)benzamide;
4-((4-Bromophenylsulfonamido)methyl)-*N*-(pyridin-3-yl)benzamide;
*N*-(Pyridin-3-yl)-4-((4-(trifluoromethoxy)phenylsulfonamido)methyl)benzamide;
Methyl 4-(*N*-(4-(Pyridin-3-ylcarbamoyl)benzyl)sulfamoyl)benzoate;
*N*-(Pyridin-3-yl)-4-((4-(trifluoromethyl)phenylsulfonamido)methyl)benzamide;
4-((4-Cyanophenylsulfonamido)methyl)-*N*-(pyridin-3-yl)benzamide;
4-((4-Nitrophenylsulfonamido)methyl)-*N*-(pyridin-3-yl)benzamide;
4-((Biphenyl-4-ylsulfonamido)methyl)-*N*-(pyridin-3-yl)benzamide;
4-({[(4'-Methoxy[1,1'-biphenyl]-4-yl)sulfonyl]amino}methyl)-*N*-(3-pyridinyl)benzamide;
4-({[(4'-M ethyl[1,1'-biphenyl]-4-yl)sulfonyl]amino}methyl)-*N*-(3-pyridinyl)benzamide;
4-({[(4'-Fluoro[1,1'-biphenyl]-4-yl)sulfonyl]amino}methyl)-*N*-(3-pyridinyl)benzamide;
4-({[(4'-Chloro[1,1'-biphenyl]-4-yl)sulfonyl]amino}methyl)-*N*-(3-pyridinyl)benzamide;
4-[({[4-(2-Pyrimidinyl)phenyl]sulfonyl}amino)methyl]-*N*-(3-pyridinyl)-benzamide;
4-[({[4-(1*H*-Pyrazol-1-yl)phenyl]sulfonyl}amino)methyl]-*N*-(3-pyridinyl)benzamide;
4-[({[4-(2-Methyl-1,3-thiazol-4-yl)phenyl]sulfonyl}amino)methyl]-*N*-(3-pyridinyl)benzamide;
[({[4-(1,3-Oxazol-5-yl)phenyl]sulfonyl}amino)methyl]-*N*-(3-pyridinyl)benzamide;
4-((3,4-Dimethoxyphenylsulfonamido)methyl)-*N*-(pyridin-3-yl)benzamide;
4-((3-*tert*-Butyl-4-methoxyphenylsulfonamido)methyl)-*N*-(pyridin-3-yl)benzamide;
4-((2,3,4,5,6-Pentamethylphenylsulfonamido)methyl)-*N*-(pyridin-3-yl)benzamide;
4-((2,4-Dimethylphenylsulfonamido)methyl)-*N*-(pyridin-3-yl)benzamide;
4-((3,4-Dimethylphenylsulfonamido)methyl)-*N*-(pyridin-3-yl)benzamide;
4-((3,5-Dimethylphenylsulfonamido)methyl)-*N*-(pyridin-3-yl)benzamide;
4-((3-Fluoro-4-methylphenylsulfonamido)methyl)-*N*-(pyridin-3-yl)benzamide;
4-((3-Chloro-2-methylphenylsulfonamido)methyl)-*N*-(pyridin-3-yl)benzamide;
4-((3-Chloro-4-methylphenylsulfonamido)methyl)-*N*-(pyridin-3-yl)benzamide;
4-((3,4-Dichlorophenylsulfonamido)methyl)-*N*-(pyridin-3-yl)benzamide;
4-((3-Cyano-4-fluorophenylsulfonamido)methyl)-*N*-(pyridin-3-yl)benzamide;
4-((Naphthalene-2-sulfonamido)methyl)-*N*-(pyridin-3-yl)benzamide;
4-((5-(Dimethylamino)naphthalene-1-sulfonamido)methyl)-*N*-(pyridin-3-yl)benzamide;
4-((2,3-Dihydro-1*H*-indene-5-sulfonamido)methyl)-*N*-(pyridin-3-yl)benzamide;
4-((2-(Dimethylamino)-2,3-dihydro-1*H*-indene-5-sulfonamido)methyl)-*N*-(pyridin-3-yl)benzamide;
4-[({4-[(Dimethylamino)methyl]phenyl}sulfonyl)amino]methyl-*N*-(3-pyridinyl)benzamide;
4-{[({4-[(Diethylamino)methyl]phenyl}sulfonyl)amino]methyl}-*N*-(3-pyridinyl)benzamide,
4-{[({4-[(Dipropylamino)methyl]phenyl}sulfonyl)amino]methyl}-*N*-(3-pyridinyl)benzamide;
4-[({[4-(1-Pyrrolidinylmethyl)phenyl]sulfonyl}amino)methyl]-*N*-(3-pyridinyl)-benzamide;
4-[({[4-(1-Piperidinylmethyl)phenyl]sulfonyl}amino)methyl]-*N*-(3-pyridinyl)benzamide;
4-[({[4-(1-Azepanylmethyl)phenyl]sulfonyl}amino)methyl]-*N*-(3-pyridinyl)benzamide;
4-[({[4-(4-Morpholinylmethyl)phenyl]sulfonyl}amino)methyl]-*N*-(3-pyridinyl)benzamide;
4-{[({4-[(4-Methoxy-1-piperidinyl)methyl]phenyl}sulfonyl)amino]methyl}-*N*-(3-pyridinyl)benzamide;
4-{[({4-[(4-Methyl-1-piperazinyl)methyl]phenyl}sulfonyl)amino]methyl}-*N*-(3-pyridinyl)benzamide;
4-*tert*-Butyl-N-(4-(pyridin-3-ylcarbamoyl)benzyl)benzamide;
4-((4-*tert*-Butylphenylsulfonamido)methyl)-N-methyl-N-(pyridin-3-yl)benzamide;
*N*-Methyl-4-(phenylsulfonamidomethyl)-N-(pyridin-3-yl)benzamide;
3-((4-*tert*-Butylphenylsulfonamido)methyl)-N-(pyridin-3-yl)benzamide;
3-(Phenylsulfonamidomethyl)-*N*-(pyridin-3-yl)benzamide;
3-(4-(Phenylsulfonamidomethyl)benzamido)pyridine 1-oxide;
4-((4-Iodophenylsulfonamido)methyl)-N-(pyridin-3-yl)benzamide;
4-((4-Ethynylphenylsulfonamido)methyl)-N-(pyridin-3-yl)benzamide;
4-((4-Bromophenylsulfonamido)methyl)-N-(pyridin-3-yl)benzamide;
4-((4-Fluorophenylsulfonamido)methyl)-N-(pyridin-3-yl)benzamide;
3,5-Dimethyl-*N*-(4-(pyridin-3-ylcarbamoyl)benzyl)benzamide;
3,4-Dimethoxy-*N*-(4-(pyridin-3-ylcarbamoyl)benzyl)benzamide,
4-{[({4-[3-(Methyloxy)-1-propynyl]phenyl}sulfonyl)amino]methyl}-*N*-(3-pyridinyl)benzamide;
4-[({[4-(21-Amino-4,7,10,13,16,19-hexaoxahenicos-1-yn-1-yl)phenyl]sulfonyl}amino)methyl]-*N*-(3-pyridinyl)benzamide;
4-[({[4-(3-Methoxypropyl)phenyl]sulfonyl}amino)methyl]-*N*-(3-pyridinyl)benzamide;
4-[({[4-(1-benzyl-1*H*-1,2,3-triazol-4-yl)phenyl]sulfonyl}amino)methyl]-*N*-(3-pyridinyl)benzamide;
4-[({[4-(3-Hydroxy-1-propynyl)phenyl]sulfonyl}amino)methyl]-*N*-(3-pyridinyl)benzamide;
4-[({[4-(3-Hydroxypropyl)phenyl]sulfonyl}amino)methyl]-*N*-(3-pyridinyl)benzamide;
4-({[(4-*tert*-Butylphenyl)sulfonyl]amino}methyl)-*N*-(5-methyl-3-pyridinyl)benzamide;
4-({[(4-*tert*-Butylphenyl)sulfonyl]amino}methyl)-*N*-(2-methyl-3-pyridinyl)benzamide;
4-({[(4-*tert*-Butylphenyl)sulfonyl]amino}methyl)-*N*-(6-methyl-3-pyridinyl)benzamide;
4-({[(4-*tert*-Butylphenyl)sulfonyl]amino}methyl)-*N*-(6-methoxy-3-pyridinyl)benzamide;
4-({[(4-*tert*-Butylphenyl)sulfonyl]amino}methyl)-*N*-(6-chloro-3-pyridinyl)benzamide;
4-({[(4-*tert*-Butylphenyl)sulfonyl]amino}methyl)-*N*-(4-chloro-3-pyridinyl)benzamide;
4-({[(4-*tert*-Butylphenyl)sulfonyl]amino}methyl)-*N*-(2-chloro-3-pyridinyl)benzamide;
4-({[(4-*tert*-Butylphenyl)sulfonyl]amino}methyl)-*N*-(4-methyl-3-pyridinyl)benzamide;
4-({[(4-*tert*-Butylphenyl)sulfonyl]amino}methyl)-*N*-(5-chloro-3-pyridinyl)benzamide;
4-({[(4-*tert*-Butylphenyl)sulfonyl]amino}methyl)-*N*-(2-nitro-3-pyridinyl)benzamide;
4-({[(4-*tert*-Butylphenyl)sulfonyl]amino}methyl)-*N*-[6-(4-morpholinyl)-3-pyrid inyl]benzamide;
4-({[(4-*tert*-Butylphenyl)sulfonyl]amino}methyl)-N-[6-(trifluoromethyl)-3-pyridinyl]benzamide;
*N*-[6-(Acetylamino)-3-pyridinyl]-4-({[(4-*tert-*butylphenyl)sulfonyl]amino}methyl)benzamide;
4-({[(4-*tert*-Butylphenyl)sulfonyl]amino}methyl)-*N*-(6-fluoro-3-pyridinyl)benzamide;
4-({[(4-*tert*-Butylphenyl)sulfonyl]amino}methyl)-*N*-(5-fluoro-3-pyridinyl)benzamide;
4-({[(4-*tert*-Butylphenyl)sulfonyl]amino}methyl)-*N*-[4-(trifluoromethyl)-3-pyridinyl]benzamide;
4-({[(4-*tert*-Butylphenyl)sulfonyl]amino}methyl)-*N*-(2-fluoro-3-pyridinyl)benzamide;
4-({[(4-*tert*-Butylphenyl)sulfonyl]amino}methyl)-*N*-(4-methoxy-3-pyridinyl)benzamide;
*N*-(6-Bromo-3-pyridinyl)-4-({[(4-*tert*-butylphenyl)sulfonyl]amino}methyl)benzamide;
4-[({[3-(4-Morpholinyl)phenyl]sulfonyl}amino)methyl]-*N*-(3-pyridinyl)benzamide;
4-[({[4-(4-Morpholinyl)phenyl]sulfonyl}amino)methyl]-*N*-(3-pyridinyl)benzamide;
4-[({[4-(1-Piperidinyl)phenyl]sulfonyl}amino)methyl]-*N*-(3-pyridinyl)benzamide;
4-[({[4-(1-Piperidinyl)phenyl]sulfonyl}amino)methyl]-*N*-(3-pyridinyl)benzamide;
4-[({[4-(21 -Amino-4,7,10,13,16,19-hexaoxahenicos-1 -yl)phenyl]sulfonyl}amino) methyl]-*N*-(3-pyridinyl)benzamide;
4-({[(4-{[3-(4-Morpholinyl)propyl]amino}phenyl)sulfonyl]amino}methyl)-*N*-(3-pyridinyl)benzamide;
4-[({[3-(4-Methyl-1-piperazinyl)phenyl]sulfonyl}amino)methyl]-*N*-(3-pyridinyl)benzamide;
4-({[(4-{[2-(Dimethylamino)ethyl]amino}phenyl)sulfonyl]amino}methyl)-*N*-(3-pyridinyl)benzamide;
*N*-(3-Pyridinyl)-4-[({[3'-(trifluoromethyl)[1,1'-biphenyl]-4-yl]sulfonyl}amino)methyl]benzamide;
4-({[(4-Benzylphenyl)sulfonyl]amino}methyl)-*N*-(3-pyridinyl)benzamide;
4-{[({4-[3-(4-Morpholinyl)-1-propynyl]phenyl}sulfonyl)amino]methyl}-*N*-(3-pyridinyl)benzamide;
4-{[({4-[3-(Dimethylamino)-1-propynyl]phenyl}sulfonyl)amino]methyl}-*N*-(3-pyridinyl)benzamide;
4-{[[(4-*tert*-Butylphenyl)sulfonyl](methyl)amino]methyl}-*N*-(3-pyridinyl) benzamide;
4-{[[(4-*tert*-Butylphenyl)sulfonyl](ethyl)amino]methyl}-*N*-(3-pyridinyl)benzamide;
4-{[[(4-*tert*-Butylphenyl)sulfonyl](propyl)amino]methyl}-*N*-(3-pyridinyl)benzamide;
4-{[({4-[3-(4-Morpholinyl)propyl]phenyl}sulfonyl)amino]methyl}-*N*-(3-pyridinyl)benzamide;
4-{[({4-[3-(Dimethylamino)propyl]phenyl}sulfonyl)amino]methyl}-*N*-(3-pyridinyl)benzamide;
4-[({[3-(Propionylamino)phenyl]sulfonyl}amino)methyl]-*N*-(3-pyridinyl)benzamide;
4-[({[3-(Acryloylamino)phenyl]sulfonyl}amino)methyl]-*N*-(3-pyridinyl)benzamide;
4-({[(4-*tert*-Butylphenyl)sulfonyl]amino}methyl)-2-methyl-*N*-(3-pyridinyl)benzamide;
4-({[(4-*tert*-Butylphenyl)sulfonyl]amino}methyl)-2-fluoro-*N*-(3-pyridinyl)benzamide;
4-({[(4-*tert*-Butylphenyl)sulfonyl]amino}methyl)-3-methyl-*N*-(3-pyridinyl)benzamide;
4-({[(4-*tert*-Butylphenyl)sulfonyl]amino}methyl)-3-fluoro-*N*-(3-pyridinyl)benzamide;
4-(1-{[(4-*tert*-Butylphenyl)sulfonyl]amino}ethyl)-*N*-(3-pyridinyl)benzamide;
4-[(anilinosulfonyl)methyl]-*N*-(3-pyridinyl)benzamide;
4-{[(4-*tert*-butylanilino)sulfonyl]methyl}-N-(3-pyridinyl)benzamide;
4-{[(4-fluoroanilino)sulfonyl]methyl}-*N*-(3-pyridinyl)benzamide;
4-({[4-(4-methyl-1-piperazinyl)anilino]sulfonyl}methyl)-*N*-(3-pyridinyl)benzamide,
4-((4-(tert-butyl)phenylsulfonamido)methyl)-2-methyl-N-(pyridin-3-yl)benzamide;
4-((4-(tert-butyl)phenylsulfonamido)methyl)-3-methyl-N-(pyridin-3-yl)benzamide;
4-((4-(tert-butyl)phenylsulfonamido)methyl)-2-fluoro-N-(pyridin-3-yl)benzamide;
4-((4-(tert-butyl)phenylsulfonamido)methyl)-3-fluoro-N-(pyridin-3-yl)benzamide;
4-((4-(tert-butyl)phenylsulfonamido)methyl)-3-nitro-N-(pyridin-3-yl)benzamide;
4-(1-(4-(tert-butyl)phenylsulfonamido)ethyl)-N-(pyridin-3-yl)benzamide;
4-(N-phenylsulfamoylmethyl)-N-(pyridin-3-yl)benzamide;
4-((N-(4-fluorophenyl)sulfamoyl)methyl)-N-(pyridin-3-yl)benzamide;
4-((N-(4-tert-butylphenyl)sulfamoyl)methyl)-N-(pyridin-3-yl)benzamide; and
4-((N-(4-(4-methylpiperazin-1-yl)phenyl)sulfamoyl)methyl)-N-(pyridin-3-yl)benzamide; or a pharmaceutically acceptable salt thereof.

8. A pharmaceutical composition comprising at least one compound of Formula IA: 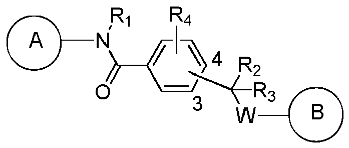 or a pharmaceutically acceptable salt thereof,
wherein:
A is 3-pyridinyl which is optionally substituted; 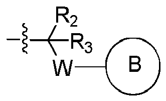 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen or C₁-C₈ alkyl;
W is -NRSO₂-, wherein R is hydrogen or C₁-C₈ alkyl; and
B is an optionally substituted aryl ring,
wherein "aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic;
and at least one pharmaceutically acceptable carrier.

9. A pharmaceutical composition as defined in claim 8 in which the compound or salt is as defined in any one of claims 1 to 7.

10. A compound or pharmaceutically acceptable salt thereof, which is specifically cytotoxic to cells that have elevated HIF levels due to their increased rate and dependence on glucose uptake and glycolysis, for use in a method for treating a disease mediated by defective pVHL protein, comprising administering to a subject at least one said compound or pharmaceutically acceptable salt;
wherein the compound is a compound of formula I 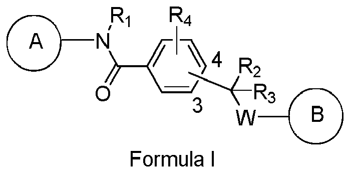 or a pharmaceutically acceptable salt thereof, wherein:
A is 3-pyridinyl which is optionally substituted; 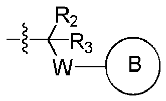 is attached to the phenyl ring at either the 3 or 4 position;
R₁, R₂, and R₃ are each independently selected from the group consisting of hydrogen, optionally substituted alkyl, and optionally substituted alkenyl;
R₄ is hydrogen, hydroxy, optionally substituted alkyl, optionally substituted alkenyl, optionally substituted alkoxy, optionally substituted cycloalkyl, optionally substituted aryl, optionally substituted heterocycloalkyl, optionally substituted heteroaryl, halo, carboxy, nitro, sulfonyl, sulfinyl, or optionally substituted amino;
W is -NRSO₂-, -SO₂NR-, or -NRCO-, wherein each R is independently selected from the group consisting of hydrogen, alkyl, alkenyl, alkynyl, cycloalkyl, aryl, heterocycloalkyl, and heteroaryl, each of which, except for hydrogen, is optionally substituted; and
B is an optionally substituted aryl ring,
provided that if R₁, R₂, and R₃ are each hydrogen, and W is -NHSO₂-, then B is not 3-methoxyphenyl, 3,4-dimethylphenyl, 2,3,4-trifluorophenyl, 2,3,5,6-tetramethylphenyl, 2,5-dimethylphenyl, 3-chlorophenyl, 3-trifluoromethylphenyl, 4-methoxyphenyl, 4-tertbutylphenyl, 4-fluorophenyl, or 4-acetylphenyl;
wherein "cycloalkyl" refers to a saturated hydrocarbon ring group which may be a bridged or caged saturated ring group; and
"aryl" refers to a 5- or 6-membered carbocyclic aromatic ring; a bicyclic ring system wherein at least one ring is carbocyclic and aromatic; or a tricyclic ring system wherein at least one ring is carbocyclic and aromatic.

11. A compound or pharmaceutically acceptable salt thereof for use as defined in claim 10 in which the compound or salt is as defined in any one of claims 1 to 8.

## Patentansprüche

1. Verbindung der Formel I: 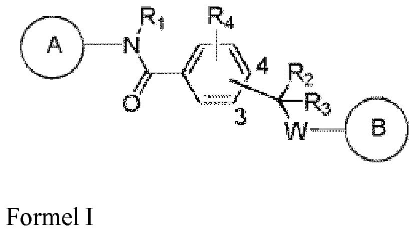 oder ein pharmazeutisch akzeptables Salz davon, wobei:
A ein 3-Pyridinyl ist, das optional substituiert ist; 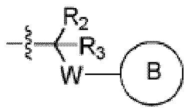 an dem Phenylring entweder an Position 3 oder 4 gebunden ist;
R₁, R₂, und R₃ jeweils unabhängig aus der Gruppe ausgewählt sind, die aus Folgendem besteht: Wasserstoff, optional substituiertes Alkyl und optional substituiertes Alkenyl;
R₄ Wasserstoff oder C₁-C₈-Alkyl ist;
W -NRSO₂- ist, wobei R Wasserstoff oder C₁-C₈-Alkyl ist; und
B ein optional substituierter Arylring ist,
vorausgesetzt, dass wenn R₁, R₂, und R₃ jeweils Wasserstoff sind und W -NHSO₂- ist, dann ist B nicht 3-Methoxyphenyl, 3,4-Dimethylphenyl, 2,3,4-Trifluorphenyl, 2,3,5,6-Tetramethylphenyl, 2,5-Dimethylphenyl, 3-Chlorophenyl, 3-Trifluormethylphenyl, 4-Methoxyphenyl, 4- Tertbutylphenyl, 4-Fluorphenyl oder 4-Acetylphenyl;
wobei sich "Aryl" auf Folgendes bezieht: einen 5- oder 6-gliedrigen carbocyclischen aromatischen Ring; ein bicyclisches Ringsystem, wobei mindestens ein Ring carbocyclisch und aromatisch ist; oder ein tricyclisches Ringsystem, wobei mindestens ein Ring carbocyclisch und aromatisch ist.

2. Verbindung nach Anspruch 1, wobei R₂ und R₃ jeweils unabhängig voneinander Wasserstoff oder optional substituiertes Alkyl sind.

3. Verbindung nach Anspruch 1, wobei R₄ Wasserstoff oder Methyl ist.

4. Verbindung nach Anspruch 1, wobei B ein optional substituierter Phenylring ist.

5. Verbindung nach Anspruch 4, wobei B Phenyl ist, das optional mit einem oder mehreren Substituenten substituiert ist, die aus der Gruppe ausgewählt, die aus Folgendem besteht: Heterocycloalkyl, Halogen, Alkyl, Alkenyl, Alkynyl, Cycloalkyl, Aryl, Heteroaryl, Hydroxyl, Alkoxy, Aryloxy, Acyl, Carboxy, Alkoxycarbonyl, NO₂, optional substituiertes Amino und CN, wobei jede der Alkyl-, Alkenyl-, Alkynyl-, Alkoxy-, Cycloalkyl-, Aryl-, Heteroaryl-, Heterocycloalkyl-, Alkoxy- und Aryloxygruppen optional unabhängig mit einem oder mehreren Substituenten substituiert sein können, die aus Gruppe ausgewählt sind, die aus Folgendem besteht: Alkyl, Halogen, Hydroxyl, Alkoxy, Carboxy, Alkoxycarbonyl, Heterocycloalkyl und optional substituiertes Amino.

6. Verbindung nach Anspruch 4 oder ein pharmazeutisch akzeptables Salz davon, wobei B aus der Gruppe ausgewählt wird, die aus Folgendem besteht: 4-(4-Methylpiperazin-1-yl)phenyl, Phenyl, 2-Methylphenyl, 2-Fluorphenyl, 2-Chlorophenyl, 2-Bromophenyl, 2-Methoxycarbonylphenyl, 2-Trifluormethylphenyl, 2-Cyanophenyl, 3-Aminophenyl, 3-Methoxyphenyl, 3-Methylphenyl, 3-Fluorphenyl, 3-Chlorophenyl, 3-Bromophenyl, 3- Trifluormethylphenyl, Tert-butylphenyl, 4-Ethynylphenyl, 3-Cyanophenyl, 3-Nitrophenyl, 3-Phenylphenyl, 3-(2-Pyrimidinyl)phenyl, 3-(1-Methyl-1 H-pyrazol-3-yl)phenyl, 3-(5-Methyl- 1,3,4-Oxadiazol-2-yl)phenyl, 3-(5-Methyl-1,2,4-oxadiazol-2-yl)phenyl, 3-(2-Methyl-1,3- thiazol-4-yl)phenyl, 4-Aminophenyl, 4-Methoxyphenyl, 4-Butoxyphenyl, 4-Phenoxyphenyl, 4-Methylphenyl, 4-Propylphenyl, 4-Tert-butylphenyl, 4-(1-Adamantyl)phenyl, 4-(3-Chloro-1-adamantyl)phenyl, 4-Methoxycarbonylethylphenyl, 4-Acetamidophenyl, 4-Fluorphenyl, 4- Chlorophenyl, 4-Bromophenyl, 4-Iodophenyl, 4-Trifluormethoxyphenyl, 4- Methoxycarbonylphenyl, 4-Acetylphenyl, 4-Trifluormethylphenyl, 4-Cyanophenyl, 4-Nitrophenyl, 4'-Methoxy[1,1'-biphenyl]-4-yl, 4'-Methyl[1,1'-biphenyl]-4-yl, 4-Phenylphenyl, 4'-Fluor[1,1'-biphenyl]-4-yl, 4'-Chlor[1,1'-biphenyl]-4-yl, 4-(2-Pyrimidinyl)phenyl, 4-(1H-Pyrazol-1-yl)phenyl, 4-(2-Methyl-1,3-thiazol-4-yl)phenyl, 4-(1,3-Oxazol-5-yl)phenyl, 3,4- Dimethoxyphenyl, 3-Tert-butyl-4-methoxyphenyl, 2,3,4,5,6-Pentamethylphenyl, 2,4-Dimethylphenyl, 3,4-Dimethylphenyl, 3,5-Dimethylphenyl, 3-Fluor-4-methylphenyl, 3- Chlor-2-methylphenyl, 3-Chlor-4-methylphenyl, 3,4-Dichlorophenyl, 3-Cyano-4-fluorphenyl, 2-naphthalenyl, 5-(Dimethylamino)-2-naphthalenyl, 2,3-Dihydro-5-indeneyl, 2-(Dimethylamino)-2,3-dihydro-5-indeneyl, 4-(Dimethylamino)methylphenyl, 4-(Diethylamino)methylphenyl, 4-(Dipropylamino)methylphenyl, 4-(1-Pyrrolidinylmethyl)phenyl, 4-(1-Piperidinylmethyl)phenyl, 4-(1-Azepanylmethyl)phenyl, 4- (4-Morpholinylmethyl)phenyl, 4-(4-Methoxy-1-piperidinyl)methylphenyl, 4-(4-Methyl-1- piperazinyl)methylphenyl, 4-(3-Hydroxypropyl)phenyl, 3-Morpholinophenyl, 4- Morpholinophenyl, (1-Piperidinyl)phenyl, (4-Methoxy-1-piperidinyl)phenyl, (21-Amino- 4,7,10,13,16,19-hexaoxahenicos-1-yl)phenyl, {[3-(4-Morpholinyl)propyl]amino}phenyl, 3-(4-Methyl-1-piperazinyl)phenyl, 4-{[2-(Dimethylamino)ethyl]amino}phenyl, 3'- (Trifluormethyl)[1,1'-biphenyl], 4-Benzylphenyl, 4-[3-(4-Morpholinyl)-1-propynyl] phenyl, 4- [3-(Dimethylamino)-1-propynyl]phenyl, 4-[3-(4-Morpholinyl)propyl] phenyl, 4-[3-(Dimethylamino)propyl] Phenyl, 3-(Propionylamino)phenyl und 3-(Acryloylamino)phenyl.

7. Verbindung, die aus der Gruppe ausgewählt wird, die aus Folgendem besteht:
4-((4-(4-Methylpiperazin-1-yl)phenylsulfonamido)methyl)-N-(pyridin-3-yl)benzamid;
4-(Phenylsulfonamidomethyl)-N-(pyridin-3-yl)benzamid;
4-((2-Methylphenylsulfonamido)methyl)-N-(pyridin-3-yl)benzamid;
4-((2-Fluorphenylsulfonamido)methyl)-N-(pyridin-3-yl)benzamid;
4-((2-Chlorophenylsulfonamido)methyl)-N-(pyridin-3-yl)benzamid;
4-((2-Bromophenylsulfonamido)methyl)-N-(pyridin-3-yl)benzamid;
Methyl 2-(N-(4-(Pyridin-3-ylcarbamoyl)benzyl)sulfamoyl)benzoat;
N-(Pyridin-3-yl)-4-((2-(trifluormethyl)phenylsulfonamido)methyl)benzamid;
4-((2-Cyanophenylsulfonamido)methyl)-N-(pyridin-3-yl)benzamid;
4-((3-Aminophenylsulfonamido)methyl)-N-(pyridin-3-yl)benzamid;
4-((3-Methylphenylsulfonamido)methyl)-N-(pyridin-3-yl)benzamid;
4-((3-Fluorphenylsulfonamido)methyl)-N-(pyridin-3-yl)benzamid;
4-((3-Bromophenylsulfonamido)methyl)-N-(pyridin-3-yl)benzamid;
4-((3-Cyanophenylsulfonamido)methyl)-N-(pyridin-3-yl)benzamid;
4-((3-Nitrophenylsulfonamido)methyl)-N-(pyridin-3-yl)benzamid;
4-{[([1,1'-Biphenyl]-3-ylsulfonyl)amino]methyl}-N-(3-pyridinyl)benzamid;
4-[({[3-(2-Pyrimidinyl)phenyl]sulfonyl}amino)methyl]-N-(3-pyridinyl)benzamid;
4-[({[3-(1-Methyl-1H-pyrazol-3-yl)phenyl]sulfonyl}amino)methyl]-N-(3-pyridinyl)benzamid;
4-[({[3-(5-Methyl-1,3,4-oxadiazol-2-yl)phenyl]sulfonyl}amino)methyl]-N-(3-pyridinyl)benzamid;
4-[({[3-(5-Methyl-1,2,4-oxadiazol-3-yl)phenyl]sulfonyl}amino)methyl]-N-(3-pyridinyl)benzamid;
4-[({[3-(2-Methyl-1,3-thiazol-4-yl)phenyl]sulfonyl}amino)methyl]-N-(3-pyridinyl)benzamid;
4-((4-Aminophenylsulfonamido)methyl)-N-(pyridin-3-yl)benzamid;
4-((4-Butoxyphenylsulfonamido)methyl)-N-(pyridin-3-yl)benzamid;
4-((4-Phenoxyphenylsulfonamido)methyl)-N-(pyridin-3-yl)benzamid;
4-((4-Methylphenylsulfonamido)methyl)-N-(pyridin-3-yl)benzamid;
4-((4-Propylphenylsulfonamido)methyl)-N-(pyridin-3-yl)benzamid;
4-[({[4-(1-Adamantyl)phenyl]sulfonyl}amino)methyl]-N-(3-pyridinyl)benzamid;
4-[({[4-(3-Chloro-1-adamantyl)phenyl]sulfonyl}amino)methyl]-N-(3-pyridinyl)benzamid;
Methyl 3-{4-[({4-[(3 Pyridinylamino)carbonyl] benzyl} amino)sulfonyl]phenyl}propanoate;
4-((4-Acetamidophenylsulfonamido)methyl)-N-(pyridin-3-yl)benzamid;
4-((4-Chlorophenylsulfonamido)methyl)-N-(pyridin-3-yl)benzamid;
4-((4-Bromophenylsulfonamido)methyl)-N-(pyridin-3-yl)benzamid;
N-(Pyridin-3-yl)-4-((4-(trifluormethoxy)phenylsulfonamido)methyl)benzamid;
Methyl 4-(N-(4-(Pyridin-3-ylcarbamoyl)benzyl)sulfamoyl)benzoat;
N-(Pyridin-3-yl)-4-((4-(trifluormethyl)phenylsulfonamido)methyl)benzamid;
4-((4-Cyanophenylsulfonamido)methyl)-N-(pyridin-3-yl)benzamid;
4-((4-Nitrophenylsulfonamido)methyl)-N-(pyridin-3-yl)benzamid;
4-((Biphenyl-4-ylsulfonamido)methyl)-N-(pyridin-3-yl)benzamid;
4-({[(4'-Methoxy[1,1'-biphenyl]-4-yl)sulfonyl]amino}methyl)-N-(3-pyridinyl)benzamid;
4-({[(4'-Methyl[1,1'-biphenyl]-4-yl)sulfonyl]amino}methyl)-N-(3-pyridinyl)benzamid;
4-({[(4'-Fluor[1,1'-biphenyl]-4-yl)sulfonyl]amino}methyl)-N-(3-pyridinyl)benzamid;
4-({[(4'-Chlor[1,1'-biphenyl]-4-yl)sulfonyl]amino}methyl)-N-(3-pyridinyl)benzamid;
4-[({[4-(2-Pyrimidinyl)phenyl]sulfonyl}amino)methyl]-N-(3-pyridinyl)-benzamid;
4-[({[4-(1H-Pyrazol-1-yl)phenyl]sulfonyl}amino)methyl]-N-(3-pyridinyl)benzamid;
4-[({[4-(2-Methyl-1,3-thiazol-4-yl)phenyl]sulfonyl}amino)methyl]-N-(3-pyridinyl)benzamid;
[({[4-(1,3-0xazol-5-yl)phenyl]sulfonyl}amino)methyl]-N-(3-pyridinyl)benzamid;
4-((3,4-Dimethoxyphenylsulfonamido)methyl)-N-(pyridin-3-yl)benzamid;
4-((3-tert-Butyl-4-methoxyphenylsulfonamido)methyl)-N-(pyridin-3-yl)benzamid;
4-((2,3,4,5,6-Pentamethylphenylsulfonamido)methyl)-N-(pyridin-3-yl)benzamid;
4-((2,4-Dimethylphenylsulfonamido)methyl)-N-(pyridin-3-yl)benzamid;
4-((3,4-Dimethylphenylsulfonamido)methyl)-N-(pyridin-3-yl)benzamid;
4-((3,5-Dimethylphenylsulfonamido)methyl)-N-(pyridin-3-yl)benzamid;
4-((3-Fluor-4-methylphenylsulfonamido)methyl)-N-(pyridin-3-yl)benzamid;
4-((3-Chloro-2-methylphenylsulfonamido)methyl)-N-(pyridin-3-yl)benzamid;
4-((3-Chloro-4-methylphenylsulfonamido)methyl)-N-(pyridin-3-yl)benzamid;
4-((3,4-Dichlorophenylsulfonamido)methyl)-N-(pyridin-3-yl)benzamid;
4-((3-Cyano-4-fluorphenylsulfonamido)methyl)-N-(pyridin-3-yl)benzamid;
4-((Naphthalene-2-sulfonamido)methyl)-N-(pyridin-3-yl)benzamid;
4-((5-(Dimethylamino)naphthalen-1-sulfonamido)methyl)-N-(pyridin-3-yl)benzamid;
4-((2,3-Dihydro-1H-indene-5-sulfonamido)methyl)-N-(pyridin-3-yl)benzamid;
4-((2-(Dimethylamino)-2,3-dihydro-1H-indene-5-sulfonamido)methyl)-N-(pyridin-3-yl)benzamid;
4-[({4-[(Dimethylamino)methyl]phenyl}sulfonyl)amino]methyl-N-(3-pyridinyl)benzamid;
4-{[({4-[(Diethylamino)methyl]phenyl}sulfonyl)amino]methyl}-N-(3-pyridinyl)benzamid,
4-{[({4-[(Dipropylamino)methyl]phenyl}sulfonyl)amino]methyl}-N-(3-pyridinyl)benzamid;
4-[({[4-(1-Pyrrolidinylmethyl)phenyl]sulfonyl}amino)methyl]-N-(3-pyridinyl)-benzamid;
4-[({[4-(1-Piperidinylmethyl)phenyl]sulfonyl}amino)methyl]-N-(3-pyridinyl)benzamid;
4-[({[4-(1-Azepanylmethyl)phenyl]sulfonyl}amino)methyl]-N-(3-pyridinyl)benzamid;
4-[({[4-(4-Morpholinylmethyl)phenyl]sulfonyl}amino)methyl]-N-(3-pyridinyl)benzamid;
4-{[({4-[(4-Methoxy-1-piperidinyl)methyl]phenyl}sulfonyl)amino]methyl}-N-(3-pyridinyl)benzamid;
4-{[({4-[(4-Methyl-1-piperazinyl)methyl]phenyl}sulfonyl)amino]methyl}-N-(3-pyridinyl)benzamid;
4-tert-Butyl-N-(4-(pyridin-3-ylcarbamoyl)benzyl)benzamid;
4-((4-tert-Butylphenylsulfonamido)methyl)-N-methyl-N-(pyridin-3-yl)benzamid;
N-Methyl-4-(phenylsulfonamidomethyl)-N-(pyridin-3-yl)benzamid;
3-((4-tert-Butylphenylsulfonamido)methyl)-N-(pyridin-3-yl)benzamid;
3-(Phenylsulfonamidomethyl)-N-(pyridin-3-yl)benzamid;
3-(4-(Phenylsulfonamidomethyl)benzamido)pyridine 1-oxid;
4-((4-lodophenylsulfonamido)methyl)-N-(pyridin-3-yl)benzamid;
4-((4-Ethynylphenylsulfonamido)methyl)-N-(pyridin-3-yl)benzamid;
4-((4-Bromophenylsulfonamido)methyl)-N-(pyridin-3-yl)benzamid;
4-((4-Fluorphenylsulfonamido)methyl)-N-(pyridin-3-yl)benzamid;
3,5-Dimethyl-N-(4-(pyridin-3-ylcarbamoyl)benzyl)benzamid;
3,4-Dimethoxy-N-(4-(pyridin-3-ylcarbamoyl)benzyl)benzamid,
4-{[({4-[3-(Methyloxy)-1-propynyl]phenyl}sulfonyl)amino]methyl}-N-(3-pyridinyl)benzamid;
4-[({[4-(21-Amino-4,7,10,13,16,19-hexaoxahenicos-1-yn-1-yl)phenyl]sulfonyl}amino)methyl]-N-(3-pyridinyl)benzamid;
4-[({[4-(3-Methoxypropyl)phenyl]sulfonyl}amino)methyl]-N-(3-pyridinyl)benzamid;
4-[({[4-(1-benzyl-1H-1,2,3-triazol-4-yl)phenyl]sulfonyl}amino)methyl]-N-(3-pyridinyl)benzamid;
4-[({[4-(3-Hydroxy-1-propynyl)phenyl]sulfonyl}amino)methyl]-N-(3-pyridinyl)benzamid;
4-[({[4-(3-Hydroxypropyl)phenyl]sulfonyl}amino)methyl]-N-(3-pyridinyl)benzamid;
4-({[(4-tert-Butylphenyl)sulfonyl]amino}methyl)-N-(5-methyl-3-pyridinyl)benzamid;
4-({[(4-tert-Butylphenyl)sulfonyl]amino}methyl)-N-(2-methyl-3-pyridinyl)benzamid;
4-({[(4-tert-Butylphenyl)sulfonyl]amino}methyl)-N-(6-methyl-3-pyridinyl)benzamid;
4-({[(4-tert-Butylphenyl)sulfonyl]amino}methyl)-N-(6-methoxy-3-pyridinyl)benzamid;
4-({[(4-tert-Butylphenyl)sulfonyl]amino}methyl)-N-(6-chlor-3-pyridinyl)benzamid;
4-({[(4-tert-Butylphenyl)sulfonyl]amino}methyl)-N-(4-chlor-3-pyridinyl)benzamid;
4-({[(4-tert-Butylphenyl)sulfonyl]amino}methyl)-N-(2-chlor-3-pyridinyl)benzamid;
4-({[(4-tert-Butylphenyl)sulfonyl]amino}methyl)-N-(4-methyl-3-pyridinyl)benzamid;
4-({[(4-tert-Butylphenyl)sulfonyl]amino}methyl)-N-(5-chlor-3-pyridinyl)benzamid;
4-({[(4-tert-Butylphenyl)sulfonyl]amino}methyl)-N-(2-nitro-3-pyridinyl)benzamid;
4-({[(4-tert-Butylphenyl)sulfonyl]amino}methyl)-N-[6-(4-morpholinyl)-3-pyridinyl]benzamid;
4-({[(4-tert-Butylphenyl)sulfonyl]amino}methyl)-N-[6-(trifluormethyl)-3-pyridinyl]benzamid;
N-[6-(Acetylamino)-3-pyridinyl]-4-({[(4-tert-butylphenyl)sulfonyl]amino}methyl)benzamid;
4-({[(4-tert-Butylphenyl)sulfonyl]amino}methyl)-N-(6-fluor-3-pyridinyl)benzamid;
4-({[(4-tert-Butylphenyl)sulfonyl]amino}methyl)-N-(5-fluor-3-pyridinyl)benzamid;
4-({[(4-tert-Butylphenyl)sulfonyl]amino}methyl)-N-[4-(trifluormethyl)-3-pyridinyl]benzamid;
4-({[(4-tert-Butylphenyl)sulfonyl]amino}methyl)-N-(2-fluor-3-pyridinyl)benzamid;
4-({[(4-tert-Butylphenyl)sulfonyl]amino}methyl)-N-(4-methoxy-3-pyridinyl)benzamid;
N-(6-Bromo-3-pyridinyl)-4-({[(4-tert-butylphenyl)sulfonyl]amino}methyl)benzamid;
4-[({[3-(4-Morpholinyl)phenyl]sulfonyl}amino)methyl]-N-(3-pyridinyl)benzamid;
4-[({[4-(4-Morpholinyl)phenyl]sulfonyl}amino)methyl]-N-(3-pyridinyl)benzamid;
4-[({[4-(1-Piperidinyl)phenyl]sulfonyl}amino)methyl]-N-(3-pyridinyl)benzamid;
4-[({[4-(1-Piperidinyl)phenyl]sulfonyl}amino)methyl]-N-(3-pyridinyl)benzamid;
4-[({[4-(21-Amino-4,7,10,13,16,19-hexaoxahenicos-1-yl)phenyl]sulfonyl}amino)methyl]-N-(3-pyridinyl)benzamid;
4-({[(4-{[3-(4-Morpholinyl)propyl]amino}phenyl)sulfonyl]amino}methyl)-N-(3-pyridinyl)benzamid;
4-[({[3-(4-Methyl-1-piperazinyl)phenyl]sulfonyl}amino)methyl]-N-(3-pyridinyl)benzamid;
4-({[(4-{[2-(Dimethylamino)ethyl]amino}phenyl)sulfonyl]amino}methyl)-N-(3-pyridinyl)benzamid;
N-(3-Pyridinyl)-4-[({[3'-(trifluormethyl)[1,1'-biphenyl]-4-yl]sulfonyl}ami no)methyl]benzamid;
4-({[(4-Benzylphenyl)sulfonyl]amino}methyl)-N-(3-pyridinyl)benzamid;
4-{[({4-[3-(4-Morpholinyl)-1-propynyl]phenyl}sulfonyl)amino]methyl}-N-(3-pyridinyl)benzamid;
4-{[({4-[3-(Dimethylamino)-1-propynyl]phenyl}sulfonyl)amino]methyl}-N-(3-pyridinyl)benzamid;
4-{[[(4-tert-Butylphenyl)sulfonyl](methyl)amino]methyl}-N-(3-pyridinyl) benzamid;
4-{[[(4-tert-Butylphenyl)sulfonyl](ethyl)amino]methyl}-N-(3-pyridinyl)benzamid;
4-{[[(4-tert-Butylphenyl)sulfonyl](propyl)amino]methyl}-N-(3-pyridinyl)benzamid;
4-{[({4-[3-(4-Morpholinyl)propyl]phenyl}sulfonyl)amino]methyl}-N-(3-pyridinyl)benzamid;
4-{[({4-[3-(Dimethylamino)propyl]phenyl}sulfonyl)amino]methyl}-N-(3-pyridinyl)benzamid;
4-[({[3-(Propionylamino)phenyl]sulfonyl}amino)methyl]-N-(3-pyridinyl)benzamid;
4-[({[3-(Acryloylamino)phenyl]sulfonyl}amino)methyl]-N-(3-pyridinyl)benzamid;
4-({[(4-tert-Butylphenyl)sulfonyl]amino}methyl)-2-methyl-N-(3-pyridinyl)benzamid;
4-({[(4-tert-Butylphenyl)sulfonyl]amino}methyl)-2-fluor-N-(3-pyridinyl)benzamid;
4-({[(4-tert-Butylphenyl)sulfonyl]amino}methyl)-3-methyl-N-(3-pyridinyl)benzamid;
4-({[(4-tert-Butylphenyl)sulfonyl]amino}methyl)-3-fluor-N-(3-pyridinyl)benzamid;
4-(1-{[(4-tert-Butylphenyl)sulfonyl]amino}ethyl)-N-(3-pyridinyl)benzamid;
4-[(anilinosulfonyl)methyl]-N -(3-pyridinyl)benzamid;
4-{[(4-tert-butylanilino)sulfonyl]methyl}-N-(3-pyridinyl)benzamid;
4-{[(4-fluoranilino)sulfonyl]methyl}-N-(3-pyridinyl)benzamid;
4-({[4-(4-methyl-1-piperazinyl)anilino]sulfonyl}methyl)-N-(3-pyridinyl)benzamid,
4-((4-(tert-butyl)phenylsulfonamido)methyl)-2-methyl-N-(pyridin-3-yl)benzamid;
4-((4-(tert-butyl)phenylsulfonamido)methyl)-3-methyl-N-(pyridin-3-yl)benzamid;
4-((4-(tert-butyl)phenylsulfonamido)methyl)-2-fluor-N-(pyridin-3-yl)benzamid;
4-((4-(tert-butyl)phenylsulfonamido)methyl)-3-fluor-N-(pyridin-3-yl)benzamid;
4-((4-(tert-butyl)phenylsulfonamido)methyl)-3-nitro-N-(pyridin-3-yl)benzamid;
4-(1-(4-(tert-butyl)phenylsulfonamido)ethyl)-N-(pyridin-3-yl)benzamid;
4-(N-phenylsulfamoylmethyl)-N-(pyridin-3-yl)benzamid;
4-((N-(4-fluorphenyl)sulfamoyl)methyl)-N-(pyridin-3-yl)benzamid;
4-((N-(4-tert-butylphenyl)sulfamoyl)methyl)-N-(pyridin-3-yl)benzamid; und
4-((N-(4-(4-methylpiperazin-1-yl)phenyl)sulfamoyl)methyl)-N-(pyridin-3-yl)benzamid; oder ein pharmazeutisch akzeptables Salz davon.

8. Pharmazeutische Zusammensetzung, die mindestens eine Verbindung der Formel IA umfasst: 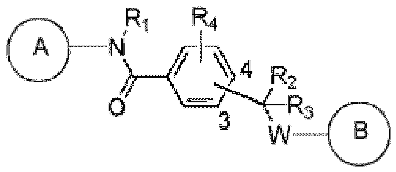 oder ein pharmazeutisch akzeptables Salz davon, wobei:
A 3-Pyridinyl ist, das optional substituiert ist; 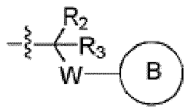 an dem Phenylring entweder an Position 3 oder 4 gebunden ist;
R₁, R₂, und R₃ jeweils unabhängig aus der Gruppe ausgewählt sind, die aus Folgendem besteht: Wasserstoff, optional substituiertes Alkyl und optional substituiertes Alkenyl;
R₄ Wasserstoff oder C₁-C₈-Alkyl ist;
W -NRSO₂- ist, wobei R Wasserstoff oder C₁-C₈-Alkyl ist; und
B ein optional substituierter Arylring ist,
wobei sich "Aryl" auf Folgendes bezieht: einen 5- oder 6-gliedrigen carbocyclischen aromatischen Ring; ein bicyclisches Ringsystem, wobei mindestens ein Ring carbocyclisch und aromatisch ist; oder ein tricyclisches Ringsystem, wobei mindestens ein Ring carbocyclisch und aromatisch ist;
und mindestens einen pharmazeutisch akzeptablen Träger.

9. Pharmazeutische Zusammensetzung nach Anspruch 8, wobei die Verbindung oder das Salz nach einem der Ansprüche 1 bis 7 definiert ist.

10. Verbindung oder pharmazeutisch akzeptables Salz davon, das spezifisch cytotoxisch gegenüber Zellen ist, die erhöhte HIF-Niveaus aufgrund ihrer erhöhten Rate und Abhängigkeit von Glukoseaufnahme und Glykolyse aufweisen, zur Verwendung in einem Verfahren zur Behandlung einer Krankheit, die durch fehlerhaftes pVHL-Protein vermittelt wird, umfassend das Verabreichen mindestens einer Verbindung oder eines pharmazeutisch verträglichen Salzes an eine Person;
wobei die Verbindung eine Verbindung der Formel I 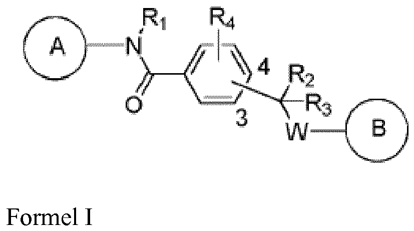 oder ein pharmazeutisch akzeptables Salz davon ist, wobei:
A ein 3-Pyridinyl ist, das optional substituiert ist; 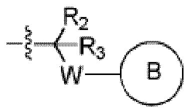 an dem Phenylring entweder an Position 3 oder 4 gebunden ist;
R₁, R₂, und R₃ jeweils unabhängig aus der Gruppe ausgewählt sind, die aus Folgendem besteht: Wasserstoff, optional substituiertes Alkyl und optional substituiertes Alkenyl;
R₄ Folgendes ist: Wasserstoff, Hydroxy, optional substituiertes Alkyl, optional substituiertes Alkenyl, optional substituiertes Alkoxy, optional substituiertes Cycloalkyl, optional substituiertes Aryl, optional substituiertes Heterocycloalkyl, optional substituiertem Heteroaryl, Halogen, Carboxy, Nitro, Sulfonyl, Sulfinyl, oder optional substituiertes Amino;
W -NRSO₂-, -SO₂NR- oder -NRCO- ist, wobei jedes R unabhängig ausgewählt aus der Gruppe ausgewählt wird, die aus Folgendem besteht: Wasserstoff, Alkyl, Alkenyl, Alkynyl, Cycloalkyl, Aryl, Heterocycloalkyl und Heteroaryl, von denen jedes, außer Wasserstoff, optional substituiert ist; und
B ein optional substituierter Arylring ist,
vorausgesetzt, dass wenn R₁, R₂, und R₃ jeweils Wasserstoff sind und W -NHSO₂- ist, dann ist B nicht 3-Methoxyphenyl, 3,4-Dimethylphenyl, 2,3,4-Trifluorphenyl, 2,3,5,6-Tetramethylphenyl, 2,5-Dimethylphenyl, 3-Chlorophenyl, 3-Trifluormethylphenyl, 4-Methoxyphenyl, 4- Tertbutylphenyl, 4-Fluorphenyl oder 4-Acetylphenyl;
wobei sich "Cycloalkyl" auf eine gesättigte Kohlenwasserstoffringgruppe bezieht, die eine überbrückte oder eingeschlossene gesättigte Ringgruppe sein kann; und
sich "Aryl" auf Folgendes bezieht: einen 5- oder 6-gliedrigen carbocyclischen aromatischen Ring; ein bicyclisches Ringsystem, wobei mindestens ein Ring carbocyclisch und aromatisch ist; oder ein tricyclisches Ringsystem, wobei mindestens ein Ring carbocyclisch und aromatisch ist.

11. Verbindung oder pharmazeutisch akzeptables Salz davon zur Verwendung nach Anspruch 10, wobei die Verbindung oder das Salz nach einem der Ansprüche 1 bis 8 definiert ist.

## Revendications

1. Composé de formule I : 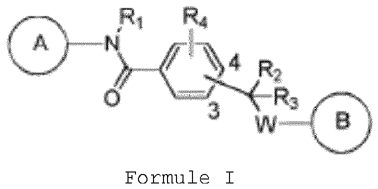 ou sel pharmaceutiquement acceptable de celui-ci, dans lequel :
A est un groupe 3-pyridinyle qui est éventuellement substitué ; 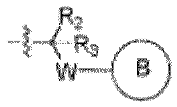 est attaché au cycle phényle en position 3 ou 4 ;
R₁, R₂ et R₃ sont chacun indépendamment choisis dans le groupe constitué par un atome d'hydrogène, un groupe alkyle éventuellement substitué, et un groupe alcényle éventuellement substitué ;
R₄ est un atome d'hydrogène ou un groupe alkyle en C₁ à C₈ ;
W est -NRSO₂-, où R est un atome d'hydrogène ou un groupe alkyle en C₁ à C₈ ; et
B est un cycle aryle éventuellement substitué,
à condition que si R₁, R₂ et R₃ sont chacun un atome d'hydrogène, et W est -NHSO₂-, B ne soit pas un groupe 3-méthoxyphényle, 3,4-diméthylphényle, 2,3,4-trifluorophényle, 2,3,5,6-tétraméthylphényle, 2,5-diméthylphényle, 3-chlorophényle, 3-trifluorométhylphényle, 4-méthoxyphényle, 4-tertbutylphényle, 4-fluorophényle, ou 4-acétylphényle ;
où « aryle » fait référence à un cycle aromatique carbocyclique à 5 ou 6 chaînons ; un système de cycle bicyclique dans lequel au moins un cycle est carbocyclique et aromatique ; ou un système de cycle tricyclique dans lequel au moins un cycle est carbocyclique et aromatique.

2. Composé selon la revendication 1, dans lequel R₂ et R₃ sont chacun indépendamment un atome d'hydrogène ou un groupe alkyle éventuellement substitué.

3. Composé selon la revendication 1, dans lequel R₄ est un atome d'hydrogène ou un groupe méthyle.

4. Composé selon la revendication 1, dans lequel B est un cycle phényle éventuellement substitué.

5. Composé selon la revendication 4, dans lequel B est un groupe phényle éventuellement substitué par un ou plusieurs substituants choisis dans le groupe constitué par les groupes hétérocycloalkyle, halogéno, alkyle, alcényle, alcynyle, cycloalkyle, aryle, hétéroaryle, hydroxyle, alcoxy, aryloxy, acyle, carboxy, alcoxycarbonyle, NO₂, amino éventuellement substitué, et CN, où chacun desdits groupes alkyle, alcényle, alcynyle, alcoxy, cycloalkyle, aryle, hétéroaryle, hétérocycloalkyle, alcoxy, et aryloxy peut être éventuellement indépendamment substitué par un ou plusieurs substituants choisis dans le groupe constitué par les groupes alkyle, halogéno, hydroxyle, alcoxy, carboxy, alcoxycarbonyle, hétérocycloalkyle, et amino éventuellement substitué.

6. Composé selon la revendication 4, ou sel pharmaceutiquement acceptable de celui-ci, dans lequel B est choisi dans le groupe constitué par les groupes 4-(4-méthylpipérazin-1-yl)phényle, phényle, 2-méthylphényle, 2-fluorophényle, 2-chlorophényle, 2-bromophényle, 2-méthoxycarbonylphényle, 2-trifluorométhylphényle, 2-cyanophényle, 3-aminophényle, 3-méthoxyphényle, 3-méthylphényle, 3-fluorophényle, 3-chlorophényle, 3-bromophényle, 3-trifluorométhylphényle, *tert*-butylphényle, 4-éthylylphényle, 3-cyanophényle, 3-nitrophényle, 3-phénylphényle, 3-(2-pyrimidinyl)phényle, 3-(1-méthyl-1*H-*pyrazol-3-yl)phényle, 3-(5-méthyl-1,3,4-oxadiazol-2-yl)phényle, 3-(5-méthyl-1,2,4-oxadiazol-2-yl)phényle, 3-(2-méthyl-1,3-thiazol-4-yl)phényle, 4-aminophényle, 4-méthoxyphényle, 4-butoxyphényle, 4-phénoxyphényle, 4-méthylphényle, 4-propylphényle, 4-tert-butylphényle, 4-(1-adamantyl)phényle, 4-(3-chloro-1-adamantyl)phényle, 4-méthoxycarbonyléthylphényle, 4-acétamidophényle, 4-fluorophényle, 4-chlorophényle, 4-bromophényle, 4-iodophényle, 4-trifluorométhoxyphényle, 4-méthoxycarbonylphényle, 4-acétylphényle, 4-trifluorométhylphényle, 4-cyanophényle, 4- nitrophényle, 4'-méthoxy[1,1'-biphényl]-4-yle, 4'-méthyl[1,1'-biphényl]-4-yle, 4-phénylphényle, 4'-fluoro[1,1'-biphényl]-4-yle, 4'-chloro[1,1'-biphényl]-4-yle, 4-(2-pyrimidinyl)phényle, 4-(1*H-*pyrazol-1-yl)phényle, 4-(2-méthyl-1,3-thiazol-4-yl)phényle, 4-(1,3-oxazol-5-yl)phényle, 3,4-diméthoxyphényle, 3-*tert-*butyl-4-méthoxyphényle, 2,3,4,5,6-pentaméthylphényle, 2,4-diméthylphényle, 3,4-diméthylphényle, 3,5-diméthylphényle, 3-fluoro-4-méthylphényle, 3-chloro-2-méthylphényle, 3-chloro-4-méthylphényle, 3,4-dichlorophényle, 3-cyano-4-fluorophényle, 2-naphthalényle, 5-(diméthylamino)-2-naphthalényle, 2,3-dihydro-5-indénéyle, 2-(diméthylamino)-2,3-dihydro-5-indénéyle, 4-(diméthylamino)méthylphényle, 4-(diéthylamino)méthylphényle, 4-(dipropylamino)méthylphényle, 4-(1-pyrrolidinylméthyl)phényle, 4-(1-pipéridinylméthyl)phényle, 4-(1-azépanylméthyl)phényle, 4-(4-morpholinylméthyl)phényle, 4-(4-méthoxy-1-pipéridinyl)méthylphényle, 4-(4-méthyl-1-pipérazinyl)méthylphényle, 4-(3-hydroxypropyl)phényle, 3-morpholinophényle, 4-morpholinophényle, (1-pipéridinyl)phényle, (4-méthoxy-1-pipéridinyl)phényle, (21-amino-4,7,10,13,16,19-hexaoxahénicos-1-yl)phényle, {[3-(4-morpholinyl)propyl]amino}phényle, 3-(4-méthyl-1-pipérazinyl)phényle, 4-{[2-(diméthylamino)éthyl]amino}phényle, 3'-(trifluorométhyl)[1,1'-biphényle], 4-benzylphényle, 4-[3-(4-morpholinyl)-1-propynyl]phényle, 4-[3-(diméthylamino)-1-propynyl]phényle, 4-[3-(4-morpholinyl)propyl]phényle, 4-[3-(diméthylamino)propyl]phényle, 3-(propionylamino)phényle, et 3-(acryloylamino)phényle.

7. Composé choisi dans le groupe constitué par les éléments suivants :
4-((4-(4-méthylpipérazin-1-yl)phénylsulfonamido)méthyl)-*N-*(pyridin-3-yl)benzamide ;
4-(phénylsulfonamidométhyl)-*N*-(pyridin-3-yl)benzamide ;
4-((2-méthylphénylsulfonamido)méthyl)-*N*-(pyridin-3-yl)benzamide ;
4-((2-fluorophénylsulfonamido)méthyl)-*N*-(pyridin-3-yl)benzamide ;
4-((2-chlorophénylsulfonamido)méthyl)-*N*-(pyridin-3-yl)benzamide ;
4-((2-bromophénylsulfonamido)méthyl)-*N*-(pyridin-3-yl)benzamide ;
méthyl 2-(*N*-(4-(pyridin-3-ylcarbamoyl)benzyl)sulfamoyl)benzoate ;
*N*-(pyridin-3-yl)-4-((2-(trifluorométhyl)phénylsulfonamido)méthyl)benzamide ;
4-((2-cyanophénylsulfonamido)méthyl)-*N*-(pyridin-3-yl)benzamide ;
4-((3-aminophénylsulfonamido)méthyl)-*N*-(pyridin-3-yl)benzamide ;
4-((3-méthylphénylsulfonamido)méthyl)-*N*-(pyridin-3-yl)benzamide ;
4-((3-fluorophénylsulfonamido)méthyl)-*N*-(pyridin-3-yl)benzamide ;
4-((3-bromophénylsulfonamido)méthyl)-*N*-(pyridin-3-yl)benzamide ;
4-((3-cyanophénylsulfonamido)méthyl)-*N*-(pyridin-3-yl)benzamide ;
4-((3-nitrophénylsulfonamido)méthyl)-*N*-(pyridin-3-yl)benzamide ;
4-{[([1,1'-biphényll-3-ylsulfonyl)amino]méthyl}-*N*-(3-pyridinyl)benzamide ;
4-[({[3-(2-pyrimidinyl)phényl]sulfonyl}amino)méthyl]-*N*-(3-pyridinyl)benzamide ;
4-[({[3-(1-méthyl-1*H*-pyrazol-3-yl)phényl]sulfonyl}amino)méthyl]-*N*-(3-pyridinyl)benzamide ;
4-[({[3-(5-méthyl-1,3,4-oxadiazol-2-yl)phényl]sulfonyl}amino)méthyl]-*N*-(3-pyridinyl)benzamide ;
4-[({[3-(5-méthyl-1,2,4-oxadiazol-3-yl)phényl]sulfonyl}amino)méthyl]-*N*-(3-pyridinyl)benzamide ;
4-[({[3-(2-méthyl-1,3-thiazol-4-yl)phényl]sulfonyl}amino)méthyl]-*N*-(3-pyridinyl)benzamide ;
4-((4-aminophénylsulfonamido)méthyl)-*N*-(pyridin-3-yl)benzamide ;
4-((4-butoxyphénylsulfonamido)méthyl)-*N*-(pyridin-3-yl)benzamide ;
4-((4-phénoxyphénylsulfonamido)méthyl)-*N*-(pyridin-3-yl)benzamide ;
4-((4-méthylphénylsulfonamido)méthyl)-*N*-(pyridin-3-yl)benzamide ;
4-((4-propylphénylsulfonamido)méthyl)-*N*-(pyridin-3-yl)benzamide ;
4-[({[4-(1-adamantyl)phényl]sulfonyl}amino)méthyl]-*N*-(3-pyridinyl)benzamide ;
4-[({[4-(3-chloro-1-adamantyl)phényl]sulfonyl}amino)méthyl]-*N*-(3-pyridinyl)benzamide ;
méthyl 3-{4-[({4-[(3-pyridinylamino)carbonyl]benzyl}amino)sulfonyl]phényl} propanoate ;
4-((4-acétamidophénylsulfonamido)méthyl)-*N*-(pyridin-3-yl)benzamide ;
4-((4-chlorophénylsulfonamido)méthyl)-*N*-(pyridin-3-yl)benzamide ;
4-((4-bromophénylsulfonamido)méthyl)-*N*-(pyridin-3-yl)benzamide ;
*N*-(pyridin-3-yl)-4-((4-(trifluorométhoxy)phénylsulfonamido)méthyl)benzamide ;
méthyl-4-(*N*-(4-(pyridin-3-ylcarbamoyl)benzyl)sulfamoyl)benzoate ;
*N*-(pyridin-3-yl)-4-((4-(trifluorométhyl)phénylsulfonamido)méthyl)benzamide ;
4-((4-cyanophénylsulfonamido)méthyl)-*N*-(pyridin-3-yl)benzamide ;
4-((4-nitrophénylsulfonamido)méthyl)-*N*-(pyridin-3-yl)benzamide ;
4-((biphényl-4-ylsulfonamido)méthyl)-*N*-(pyridin-3-yl)benzamide ;
4-({[(4'-méthoxy[1,1'-biphényl]-4-yl)sulfonyl]amino}méthyl)-*N*-(3-pyridinyl)benzamide ;
4-({[(4'-méthyl[1,1'-biphényl]-4-yl)sulfonyl]amino}méthyl)-*N*-(3-pyridinyl)benzamide ;
4-({[(4'-fluoro[1,1'-biphényl]-4-yl)sulfonyl]amino}méthyl)-N-(3-pyridinyl)benzamide ;
4-({[(4'-chloro[1,1'-biphényl]-4-yl)sulfonyl]amino}méthyl)-*N*-(3-pyridinyl)benzamide ;
4-[({[4-(2-pyrimidinyl)phényl]sulfonyl}amino)méthyl]-*N*-(3-pyridinyl)-benzamide ;
4-[({[4-(1*H*-pyrazol-1-yl)phényl]sulfonyl}amino)méthyl]-*N-*(3-pyridinyl)benzamide ;
4-[({[4-(2-méthyl-1,3-thiazol-4-yl)phényl]sulfonyl}amino)méthyl]-*N*-(3-pyridinyl)benzamide ;
[({[4-(1,3-oxazol-5-yl)phényl]sulfonyl}amino)méthyl]-*N*-(3-pyridinyl)benzamide ;
4-((3,4-diméthoxyphénylsulfonamido)méthyl)-*N*-(pyridin-3-yl)benzamide ;
4-((3-*tert*-butyl-4-méthoxyphénylsulfonamido)méthyl)-*N-*(pyridin-3-yl)benzamide ;
4-((2,3,4,5,6-pentaméthylphénylsulfonamido)méthyl)-*N-*(pyridin-3-yl)benzamide ;
4-((2,4-diméthylphénylsulfonamido)méthyl)-*N*-(pyridin-3-yl)benzamide ;
4-((3,4-diméthylphénylsulfonamido)méthyl)-*N*-(pyridin-3-yl)benzamide ;
4-((3,5-diméthylphénylsulfonamido)méthyl)-*N*-(pyridin-3-yl)benzamide ;
4-((3-fluoro-4-méthylphénylsulfonamido)méthyl)-*N*-(pyridin-3-yl)benzamide ;
4-((3-chloro-2-méthylphénylsulfonamido)méthyl)-*N*-(pyridin-3-yl)benzamide ;
4-((3-chloro-4-méthylphénylsulfonamido)méthyl)-*N*-(pyridin-3-yl)benzamide ;
4-((3,4-dichlorophénylsulfonamido)méthyl)-*N*-(pyridin-3-yl)benzamide ;
4-((3-cyano-4-fluorophénylsulfonamido)méthyl)-*N*-(pyridin-3-yl)benzamide ;
4-((naphthalène-2-sulfonamido)méthyl)-*N*-(pyridin-3-yl)benzamide ;
4-((5-(diméthylamino)naphthalène-1-sulfonamido)méthyl)-*N-*(pyridin-3-yl)benzamide ;
4-((2,3-dihydro-1*H*-indene-5-sulfonamido)méthyl)-*N-*(pyridin-3-yl)benzamide ;
4-((2-(diméthylamino)-2,3-dihydro-1*H*-indène-5-sulfonamido)méthyl)-*N*-(pyridin-3-yl)benzamide ;
4-[({4-[(diméthylamino)méthyl]phényl}sulfonyl)amino]méthyl-*N*-(3-pyridinyl)benzamide ;
4-{[({4-[(diéthylamino)méthyl]phényl}sulfonyl)amino]méthyl}-*N*-(3-pyridinyl)benzamide ;
4-{[({4-[(dipropylamino)méthyl]phényl}sulfonyl)amino]méthyl}-*N*-(3-pyridinyl)benzamide ;
4-[({[4-(1-pyrrolidinylméthyl)phényl]sulfonyl}amino)méthyl]-*N*-(3-pyridinyl)-benzamide ;
4-[({[4-(1-pipéridinylméthyl)phényl]sulfonyl}amino)méthyl]-*N*-(3-pyridinyl)benzamide ;
4-[({[4-(1-azépanylméthyl)phényl]sulfonyl}amino)méthyl]-*N-*(3-pyridinyl)benzamide ;
4-[({[4-(4-morpholinylméthyl)phényl]sulfonyl}amino)méthyl]-*N*-(3-pyridinyl)benzamide ;
4-{[({4-[(4-méthoxy-1-pipéridinyl)méthyl]phényl}sulfonyl)amino]méthyl}-*N*-(3-pyridinyl)benzamide ;
4-{[({4-[(4-méthyl-1-pipérazinyl)méthyl]phényl}sulfonyl)amino]méthyl}-*N*-(3-pyridinyl)benzamide ;
4-tert-butyl-*N*-(4-(pyridin-3-ylcarbamoyl)benzyl)benzamide ;
4-((4-*tert*-butylphénylsulfonamido)méthyl)-*N*-méthyl-*N-*(pyridin-3-yl)benzamide ;
*N*-méthyl-4-(phénylsulfonamidométhyl)-*N*-(pyridin-3-yl)benzamide ;
3-((4-*tert*-butylphénylsulfonamido)méthyl)-*N*-(pyridin-3-yl)benzamide ;
3-(phénylsulfonamidométhyl)-*N*-(pyridin-3-yl)benzamide ;
1-oxyde de 3-(4-(phénylsulfonamidométhyl)benzamido)pyridine ;
4-((4-iodophénylsulfonamido)méthyl)-*N*-(pyridin-3-yl)benzamide ;
4-((4-éthynylphénylsulfonamido)méthyl)-*N*-(pyridin-3-yl)benzamide ;
4-((4-bromophénylsulfonamido)méthyl)-*N*-(pyridin-3-yl)benzamide ;
4-((4-fluorophénylsulfonamido)méthyl)-*N*-(pyridin-3-yl)benzamide ;
3,5-diméthyl-*N*-(4-(pyridin-3-ylcarbamoyl)benzyl)benzamide ;
3,4-diméthoxy-*N*-(4-(pyridin-3-ylcarbamoyl)benzyl)benzamide ;
4-{[({4-[3-(méthyloxy)-1-propynyl]phényl}sulfonyl)amino]méthyl}-*N*-(3-pyridinyl)benzamide ;
4-[({[4-(21-amino-4,7,10,13,16,19-hexaoxahénicos-1-yn-1-yl)phényl]sulfonyl}amino)méthyl]-*N*-(3-pyridinyl)benzamide ;
4-[({[4-(3-méthoxypropyl)phényl]sulfonyl}amino)méthyl]-*N-*(3-pyridinyl)benzamide ;
4-[({[4-(1-benzyl-1*H*-1,2,3-triazol-4-yl)phényl]sulfonyl}amino)méthyl]-*N*-(3-pyridinyl)benzamide ;
4-[({[4-(3-hydroxy-1-propynyl)phényl]sulfonyl}amino)méthyl]-*N*-(3-pyridinyl)benzamide ;
4-[({[4-(3-hydroxypropyl)phényl]sulfonyl}amino)méthyl]-*N-*(3-pyridinyl)benzamide ;
4-({[(4-*tert*-butylphényl)sulfonyl]amino}méthyl)-*N*-(5-méthyl-3-pyridinyl)benzamide ;
4-({[(4-*tert*-butylphényl)sulfonyl]amino}méthyl)-*N*-(2-méthyl-3-pyridinyl)benzamide ;
4-({[(4-*tert*-butylphényl)sulfonyl]amino}méthyl)-*N*-(6-méthyl-3-pyridinyl)benzamide ;
4-({[(4-*tert*-butylphényl)sulfonyl]amino}méthyl)-*N*-(6-méthoxy-3-pyridinyl)benzamide ;
4-({[(4-*tert*-butylphényl)sulfonyl]amino}méthyl)-*N*-(6-chloro-3-pyridinyl)benzamide ;
4-({[(4-*tert*-butylphényl)sulfonyl]amino}méthyl)-*N*-(4-chloro-3-pyridinyl)benzamide ;
4-({[(4-*tert*-butylphényl)sulfonyl]amino}méthyl)-*N*-(2-chloro-3-pyridinyl)benzamide ;
4-({[(4-*tert*-butylphényl)sulfonyl]amino}méthyl)-*N*-(4-méthyl-3-pyridinyl)benzamide ;
4-({[(4-*tert*-butylphényl)sulfonyl]amino}méthyl)-*N*-(5-chloro-3-pyridinyl)benzamide ;
4-({[(4-*tert*-butylphényl)sulfonyl]amino}méthyl)-*N*-(2-nitro-3-pyridinyl)benzamide ;
4-({[(4-*tert*-butylphényl)sulfonyl]amino}méthyl)-*N*-[6-(4-morpholinyl)-3-pyridinyl]benzamide ;
4-({[(4-*tert*-butylphényl)sulfonyl]amino}méthyl)-*N*-[6-(trifluorométhyl)-3-pyridinyl]benzamide ;
*N*-[6-(acétylamino)-3-pyridinyl]-4-({[(4-*tert-*butylphényl)sulfonyl]amino}méthyl)benzamide ;
4-({[(4-*tert*-butylphényl)sulfonyl]amino}méthyl)-*N*-(6-fluoro-3-pyridinyl)benzamide ;
4-({[(4-*tert*-butylphényl)sulfonyl]amino}méthyl)-*N*-(5-fluoro-3-pyridinyl)benzamide ;
4-({[(4-*tert*-butylphényl)sulfonyl]amino}méthyl)-*N*-[4-(trifluorométhyl)-3-pyridinyl]benzamide ;
4-({[(4-*tert*-butylphényl)sulfonyl]amino}méthyl)-*N*-(2-fluoro-3-pyridinyl)benzamide ;
4-({[(4-*tert*-butylphényl)sulfonyl]amino}méthyl)-*N*-(4-méthoxy-3-pyridinyl)benzamide ;
*N*-(6-bromo-3-pyridinyl)-4-({[(4-*tert-*butylphényl)sulfonyl]amino}méthyl)benzamide ;
4-[({[3-(4-morpholinyl)phényl]sulfonyl}amino)méthyl]-*N*-(3-pyridinyl)benzamide ;
4-[({[4-(4-morpholinyl)phényl]sulfonyl}amino)méthyl]-*N*-(3-pyridinyl)benzamide ;
4-[({[4-(1-pipéridinyl)phényl]sulfonyl}amino)méthyl]-*N*-(3-pyridinyl)benzamide ;
4-[({[4-(1-pipéridinyl)phényl]sulfonyl}amino)méthyl]-*N*-(3-pyridinyl)benzamide ;
4-[({[4-(21-amino-4,7,10,13,16,19-hexaoxahénicos-1-yl)phényl]sulfonyl}amino)méthyl]-*N*-(3-pyridinyl)benzamide ;
4-({[(4-{[3-(4-morpholinyl)propyl]amino}phényl)sulfonyl]amino}méthyl)-*N*-(3-pyridinyl)benzamide ;
4-[({[3-(4-méthyl-1-pipérazinyl)phényl]sulfonyl}amino)méthyl]-*N*-(3-pyridinyl)benzamide ;
4-({[(4-{[2-(diméthylamino)éthyl]amino}phényl)sulfonyl]amino}méthyl)-*N*-(3-pyridinyl)benzamide ;
*N*-(3-pyridinyl)-4-[({[3'-(trifluorométhyl)[1,1'-biphényl]-4-yl]sulfonyl}amino)méthyl]benzamide ;
4-({[(4-benzylphényl)sulfonyl]amino}méthyl)-*N*-(3-pyridinyl)benzamide ;
4-{[({4-[3-(4-morpholinyl)-1-propynyl]phényl}sulfonyl)amino]méthyl}-*N*-(3-pyridinyl)benzamide ;
4-{[({4-[3-(diméthylamino)-1-propynyl]phényl}sulfonyl)amino]méthyl}-*N*-(3-pyridinyl)benzamide ;
4-{[[(4-*tert*-butylphényl)sulfonyl](méthyl)amino]méthyl}-*N-*(3-pyridinyl)benzamide ;
4-{[[(4-*tert*-butylphényl)sulfonyl](éthyl)amino]méthyl}-*N-*(3-pyridinyl)benzamide ;
4-{[[(4-*tert*-butylphényl)sulfonyl](propyl)amino]méthyl}-*N-*(3-pyridinyl)benzamide ;
4-{[({4-[3-(4-morpholinyl)propyl]phényl}sulfonyl)amino]méthyl}-*N*-(3-pyridinyl)benzamide ;
4-{[({4-[3-(diméthylamino)propyl]phényl}sulfonyl)amino]méthyl}-*N*-(3-pyridinyl)benzamide ;
4-[({[3-(propionylamino)phényl]sulfonyl}amino)méthyl]-*N-*(3-pyridinyl)benzamide ;
4-[({[3-(acryloylamino)phényl]sulfonyl}amino)méthyl]-*N*-(3-pyridinyl)benzamide ;
4-({[(4-*tert*-butylphényl)sulfonyl]amino}méthyl)-2-méthyl-*N*-(3-pyridinyl)benzamide ;
4-({[(4-*tert*-butylphényl)sulfonyl]amino}méthyl)-2-fluoro-*N*-(3-pyridinyl)benzamide ;
4-({[(4-*tert*-butylphényl)sulfonyl]amino}méthyl)-3-méthyl-*N*-(3-pyridinyl)benzamide ;
4-({[(4-*tert*-butylphényl)sulfonyl]amino}méthyl)-3-fluoro-*N*-(3-pyridinyl)benzamide ;
4-(1-{[(4-*tert*-butylphényl)sulfonyl]amino}éthyl)-*N*-(3-pyridinyl)benzamide ;
4-[(anilinosulfonyl)méthyl]-*N*-(3-pyridinyl)benzamide ;
4-{[(4-*tert*-butylanilino)sulfonyl]méthyl}-*N*-(3-pyridinyl)benzamide ;
4-{[(4-fluoroanilino)sulfonyl]méthyl}-*N*-(3-pyridinyl)benzamide ;
4-({[4-(4-méthyl-1-pipérazinyl)anilino]sulfonyl}méthyl)-*N-*(3-pyridinyl)benzamide ;
4-((4-(*tert*-butyl)phénylsulfonamido)méthyl)-2-méthyl-*N-*(pyridin-3-yl)benzamide ;
4-((4-(*tert*-butyl)phénylsulfonamido)méthyl)-3-méthyl-*N-*(pyridin-3-yl)benzamide ;
4-((4-(*tert*-butyl)phénylsulfonamido)méthyl)-2-fluoro-*N-*(pyridin-3-yl)benzamide ;
4-((4-(*tert*-butyl)phénylsulfonamido)méthyl)-3-fluoro-*N-*(pyridin-3-yl)benzamide ;
4-((4-(*tert*-butyl)phénylsulfonamido)méthyl)-3-nitro-*N-*(pyridin-3-yl)benzamide ;
4-(1-(4-(*tert*-butyl)phénylsulfonamido)éthyl)-*N*-(pyridin-3-yl)benzamide ;
4-(*N*-phénylsulfamoylméthyl)-*N*-(pyridin-3-yl)benzamide ;
4-((*N*-(4-fluorophényl)sulfamoyl)méthyl)-*N*-(pyridin-3-yl)benzamide ;
4-((*N*-(4-*tert*-butylphényl)sulfamoyl)méthyl)-*N*-(pyridin-3-yl)benzamide ; et
4-((*N*-(4-(4-méthylpipérazin-1-yl)phényl)sulfamoyl)méthyl)-*N*-(pyridin-3-yl)benzamide ; ou sel pharmaceutiquement acceptable de celui-ci.

8. Composition pharmaceutique comprenant au moins un composé de formula IA : 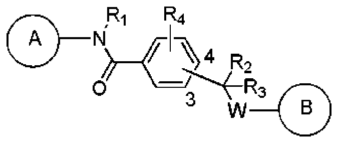 ou sel pharmaceutiquement acceptable de celui-ci, dans lequel :
A est un groupe 3-pyridinyle qui est éventuellement substitué ; 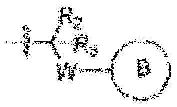 est attaché au cycle phényle en position 3 ou 4 ;
R₁, R₂ et R₃ sont chacun indépendamment choisis dans le groupe constitué par un atome d'hydrogène, un groupe alkyle éventuellement substitué, et un groupe alcényle éventuellement substitué ;
R₄ est un atome d'hydrogène ou un groupe alkyle en C₁ à C₈ ;
W est -NRSO₂-, où R est un atome d'hydrogène ou un groupe alkyle en C₁ à C₈ ; et
B est un cycle aryle éventuellement substitué, où « aryle » fait référence à un cycle aromatique carbocyclique à 5 ou 6 chaînons ; un système de cycle bicyclique dans lequel au moins un cycle est carbocyclique et aromatique ; ou un système de cycle tricyclique dans lequel au moins un cycle est carbocyclique et aromatique ;
et au moins un support pharmaceutiquement acceptable.

9. Composition pharmaceutique telle que définie dans la revendication 8, dans laquelle le composé ou sel est tel que défini dans l'une quelconque des revendications 1 à 7.

10. Composé ou sel pharmaceutiquement acceptable de celui-ci, qui est spécifiquement cytotoxique pour les cellules présentant un taux de HIF élevé du fait de leur vitesse d'absorption du glucose et de glycolyse et de leur dépendance à celles-ci, à utiliser dans un procédé permettant de traiter une maladie médiée par un déficit de protéine pVHL, comprenant l'administration à un sujet d'au moins un dudit composé ou de son sel pharmaceutiquement acceptable ; le composé étant un composé de formule I 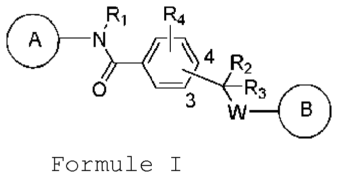 ou un sel pharmaceutiquement acceptable de celui-ci, dans lequel :
A est un groupe 3-pyridinyle qui est éventuellement substitué ; 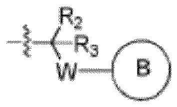 est attaché au cycle phényle en position 3 ou 4 ;
R₁, R₂ et R₃ sont chacun indépendamment choisis dans le groupe constitué par un atome d'hydrogène, un groupe alkyle éventuellement substitué, et un groupe alcényle éventuellement substitué ;
R₄ est un atome d'hydrogène, un groupe hydroxy, alkyle éventuellement substitué, alcényle éventuellement substitué, alcoxy éventuellement substitué, cycloalkyle éventuellement substitué, aryle éventuellement substitué, hétérocycloalkyle éventuellement substitué, hétéroaryle éventuellement substitué, halogéno, carboxy, nitro, sulfonyle, sulfinyle, ou amino éventuellement substitué ;
W est -NRSO₂-, - SO₂NR-, ou -NRCO-, où chaque R est indépendamment choisi dans le groupe constitué par un atome d'hydrogène, un groupe alkyle, alcényle, alcynyle, cycloalkyle, aryle, hétérocycloalkyle, et hétéroaryle, chacun d'entre eux, à l'exception de l'atome d'hydrogène, étant éventuellement substitué ; et
B est un cycle aryle éventuellement substitué,
à condition que si R₁, R₂ et R₃ sont chacun un atome d'hydrogène, et si W est -NHSO₂-, B ne soit pas un groupe 3-méthoxyphényle, 3,4-diméthylphényle, 2,3,4-trifluorophényle, 2,3,5,6-tétraméthylphényle, 2,5-diméthylphényle, 3-chlorophényle, 3-trifluorométhylphényle, 4-méthoxyphényle, 4-tertbutylphényle, 4-fluorophényle, ou 4-acétylphényle ;
où « cycloalkyle » fait référence à un groupe cyclique hydrocarboné saturé qui peut être un groupe cyclique hydrocarboné saturé ponté ou cagé ; et
« aryle » fait référence à un cycle aromatique carbocyclique à 5 ou 6 chaînons ; un système de cycle bicyclique dans lequel au moins un cycle est carbocyclique et aromatique ; ou un système de cycle tricyclique dans lequel au moins un cycle est carbocyclique et aromatique.

11. Composé ou sel pharmaceutiquement acceptable de celui-ci, destiné à une utilisation telle que définie dans la revendication 10, le composé ou sel étant tel que défini dans l'une quelconque des revendications 1 à 8.
